# EUROPEAN PATENT APPLICATION

(11) **EP 2 527 455 A2**
(43) Date of publication of application: **28.11.2012**
(21) Application number: 12181579.9
(22) Date of filing: 03.11.2009
(51) Int. Cl.: C12N 15/82, A01H 5/00, C12N 9/10

(54) **Vegetabile material, plants and a method of producing a plant having altered lignin properties**

(30) Priority: 03.11.2008 SE 0850065; 03.11.2008 US 110614 P
(62) Divisional of application: 09829400.2
(71) Applicant: Swetree Technologies AB, 904 03 Umeå (SE)
(72) Inventor: Hertzberg, Magnus, 903 39 Umeå (SE); Sundberg, Björn, 907 36 Umeå (SE); Sandberg, Göran, 905 82 Umeå (SE); Schrader, Jarmo, 31162 Bad Salzdetfurth (DE); Teeri, Tuula, 186 56 Täby (SE); Aspeborg, Henrik, 141 70 Segeltorp (SE); Wallbäcks, Lars, 905 91 Umeå (SE); Bhalerao, Rishikeshi, 907 41 Umeå (SE); Trygg, Johan, 904 35 Umeå (SE); Johansson, Karin, 904 40 Röbäck (SE); Karlsson, Ann, 905 88 Umeå (SE); Johnsson, Pär, 907 37 Umeå (SE)
(74) Representative: Höglund, Lars

(57) **Abstract**

The present invention is related to a set of genes, which when modified in plants gives altered lignin properties. The invention provides DNA construct such as a vector useful in the method of the invention. Further, the invention relates to a plant cell or plant progeny of the plants and wood produced by the plants according to the invention

Lower lignin levels will result in improved saccharification for bio-refining and ethanol production and improved pulp and paper. Increased lignin levels will utilise lignin properties for energy production.

The genes and DNA constructs may be used for the identification of plants having altered lignin characteristics as compared to the wild-type.

According to the invention genes and DNA constructs may also be used as candidate genes in marker assisted breeding.

## Description

### Technical field

The present invention is related to a set of genes, which when modified in plants gives altered lignin properties. More specifically, the invention relates to methods for phenotypically modifying plants and transgenic plants and plants obtained by a specific crossing method having altered expression of a gene resulting in a modified lignin expression. The invention also provides DNA construct such as a vector useful in the method of the invention. Further, the invention relates to a plant cell or plant progeny of the plants and wood produced by the plants according to the invention.

Lower lignin levels will result in improved saccharification for bio-refining and ethanol production and improved pulp and paper. Increased lignin levels will utilise lignin properties for energy production.

The genes and DNA constructs may be used for the identification of plants having altered lignin characteristics as compared to the wild-type.

According to the invention genes and DNA constructs may also be used as candidate genes in marker assisted breeding.

### Background

Wood from forest trees provides the biopolymers cellulose, hemicellulose and lignin for industrial uses. Major products produced from wood include pulp and paper, energy and building materials. However, we now see a shift where industry looks for novel production possibilities as well as for improved bio-refining of the wood raw material to more valuable products. This is driven by the aim to replace the use of fossil materials with renewable resources. Wood is such a resource of major importance, and it is therefore important to supply the industry with large amount of wood with designed properties for variable uses.

Lignin is a major wood biopolymer that is highly important for industrial processing of most wood based materials. Lignin biosynthesis has been well described by biochemical and genetic research tools (Baucher, Critical Reviews in Biochemistry and Molecular Biology, 38:305-350, 2003). All major enzymes in the biosynthetic pathway have been identified, and reverse genetics has been helpful in highlighting the major route of the pathway(s). Lignin is a highly complex biopolymer. It is composed of p-hydroxycinnamyl alcohol monomers and related compounds. This are polymerized oxidised and coupled in a chemical process, and the lignin is encrusting the cellulose and hemicellulose matrix in the wood cell walls. It links in complex, and hitherto, poorly described ways to the hemicellulose network with so-called "lignin carbon complexes". It has recently also been obvious that the lignin structure is very plastic and variable. Both lignin content and its composition are naturally variable between organs and cell types, and this variability can be mimicked and extended by genetic engineering (Vanholme et al. Current Opinion in Plant Biology 2008, 11:1-8). Not surprisingly in transgenic trees with modified lignin, pulping properties such as yield and kappa number has been modified (Bauhcer, Critical Reviews in Biochemistry and Molecular Biology, 38:305-350, 2003). More recent research has also demonstrated that saccharification needed for wood bio-refining and for example ethanol production is also modified when lignin is modified (Chen and Dixon. Nature Biotechnology Vol. 25 no 7: pp759-761, 2007). This paper shows that lower lignin levels results in improved saccharification. Saccharification is a process were the poly carbohydrates are hydrolysed into sugar monomers such as glucose. This process is expensive and advanced for lignocellulosic raw materials (Breaking the Biological Barriers to Cellulosic Ethanol: A Joint Research Agenda . A Research Roadmap Resulting from the Biomass to Biofuels Workshop, December 7-9, 2005, Rockville, Maryland).

To access the cellulose and hemicellulose for the saccharification, the cell wall structure has to be loosened, thereby enabling it for enzymatic degradation or hydrolysis. Furthermore, this pre-treatment may increase the surface area of the cellulose thereby enhancing its reactivity with the enzyme and the transformation to monosaccharides.

Despite the rather well researched lignin biosynthetic pathway recent observations point to the fact that lignin content can also be modified through perturbing genes related to other metabolic and cell wall biopolymer pathways in a rather unexplored, and not yet understood, fashion This is an unexplored potential to modify wood properties and lignin chemistry in trees.

The commercial value of engineering lignin content in wood is obvious, but the usefulness of more or less lignin depends upon the utilization of wood and the industrial process used. For energy production through burning or other combustion technology high lignin content is valuable because lignin has a high-energy value compared to other wood biopolymers.

Also, in chemical pulping, forage digestibility and processing of plant biomass to bio fuels lignin represent a major obstacle. This is because lignin is covalently bound to the carbon matrix, and energy is required to isolate or degrade the lignin. In pulp and paper processing, for example, it becomes a rest product ending up in the rest liquor, and separation of lignin from the cellulose is an energy consuming process. Therefore low lignin content has been viewed as a valuable property.

One example of genes involved in the biosynthetic pathway of lignin is the genes involved in the monolignol synthesis. Forster and colleagues describe in the US patent 7402428 that they can reduce or modulate the lignin content in plants by RNAi knockout of a gene in the monolignol biosynthetic pathway. This is one gene of many genes involved in the lignin synthesis pathway, due to different growth and environment condition very little is know how the this gene construct will function in field growth conditions and which genes will be best for different applications. Thus there is still a need for additional genes that can be used for modulation of lignin content in plants.

But ongoing research trying to establish wood bio refining, where all wood components are processed may change this view. The use of lignin as a dispergent, emulsion, binding agent or stabilizer has already been developed. If novel lignin based products are becoming more attractive, the lignin content of the wood may be looked at in different views. It is therefore of value to be able to modify lignin content in wood in a desirable way, e.g. up or down depending on the intended use.

Problems remaining are how to identify the potentially most important genes involved in regulation of lignin and expression of lignin related genes, in order to utilise lignin properties for energy production or remove unwanted lignin properties in paper production. In this present invention we examined a number of genes affecting the lignin levels in living trees.

### Brief description of the invention

The present invention pertains to genes, and DNA constructs SEQ ID No: 23 to 115 that can be used to modify lignin level in plants. This is done by modification of the expression level of these genes.

The invention relates to methods for phenotypically modifying plants and transgenic plants and plants obtained by a specific crossing method having altered expression of a gene resulting in a modified lignin expression. The invention also provides DNA constructs useful in the method of the invention. Further, the invention relates to a plant cell or plant progeny of the plants and wood produced by the plants according to the invention and uses thereof in applications where increased or decreased lignin content is an advantage.

The inventors have found that the down regulation and/or over expression of certain genes, gives changed wood chemistry in the produced transgenic trees. Especially it was realized that certain genes can be used to modify lignin levels. These genes have been identified from a program where gene have been transferred to trees and been down regulated by the use of RNAi and over expressed by high level expression promoters.

These genes were indentified to affect the lignin level after a two step analysis, firstly the wood was analysed with FT-IR analysis (general reference Griffiths, P. R. and De Haseth, J. A. Fourier Transform Infrared Spectrometry. New York: Wiley, 1986 and the text below) and secondly the wood was analysed with the Klason-lignin analysis (see below in text.).

The genes (SEQ ID No: 23 to 115, 157-195) have changed chemical wood properties, especially changed lignin levels.

The genes (SEQ ID 23 to 115, 157-195) can be expressed in plants for further use as energy crops, for example if the lignin content is increased a high-energy crop will be generated.

Furthermore, these genes (SEQ ID 23 to 115, 157-195) can when expressed in plants further be used to increase and improve saccharification potentials and thereby enhance the economics in producing fuels and chemicals from ligno-cellulose.

### Detailed description of the invention

### Definitions

Prior to discussing the present invention in further details, the following terms and conventions will first be defined:
Definition of "saccharification" is the process of converting a complex carbohydrate, such as starch, cellulose or hemicellulose, into sugars such as fermentable sugars. It is essentially a hydrolysis. The process may for example be accomplished by the use of enzymes or acids or other chemicals.

The term "transgenic plant" refers to a plant that contains genetic material, not found in a wild type plant of the same species, variety or cultivar.

"Over expression" refers to the increased expression of an mRNA or polypeptide compared to control plants, wherein said mRNA or polypeptide or protein is either not normally present in the host cell, or wherein said mRNA or polypeptide or protein is present in said host cell at a higher level than that normally expressed.

Over expression of the proteins of the instant invention may be accomplished by first constructing a chimeric gene in which the coding region is operably linked to a promoter capable of directing expression of a gene in the desired tissues at the desired stage of development. The chimeric gene may comprise promoter sequences and translation leader sequences derived from the same genes. 3' Non-coding sequences encoding transcription termination signals may also be provided. The instant chimeric gene may also comprise one or more introns in order to facilitate gene expression. A suitable promoter may be the CaMV 35 S promoter which may be used with *Agrobacterium* as a vector.

The term "RNA interference" or "RNAi" may refer to a process in which a double-stranded RNA molecule or a short hairpin RNA changes the expression of a nucleic acid sequence with which they share substantial or total homology.

The term "RNAi down-regulation" refers to the reduction in the expression of a nucleic acid sequence which may be mediated by one or more RNAi species. The term "RNAi species" refers to a distinct RNA Sequence that elicits RNAi.

The term "RNAi construction group" refers to different transgenic trees emanating from one RNAi construct.

The term "photoperiod" refers to the daily cycle of light and darkness.

The term "recombinant" when used with reference, e.g., to a cell, nucleotide, vector, protein, or polypeptide typically indicates that the cell, nucleotide, or vector has been modified by the introduction of a heterologous (or foreign) nucleic acid or the alteration of a native nucleic acid, or that the protein or polypeptide has been modified by the introduction of a heterologous amino acid, or that the cell is derived from a cell so modified. Recombinant cells express nucleic acid sequences (e.g., genes) that are not found in the native (non-recombinant) form of the cell or express native nucleic acid sequences (e.g. genes) that would be abnormally expressed under-expressed, or not expressed at all. The term "recombinant" when used with reference to a cell indicates that the cell replicates a heterologous nucleic acid, or expresses a peptide or protein encoded by a heterologous nucleic acid. Recombinant cells can contain genes that are not found within the native (non-recombinant) form of the cell. Recombinant cells can also contain genes found in the native form of the cell wherein the genes are modified and re-introduced into the cell by artificial means. The term also encompasses cells that contain a nucleic acid endogenous to the cell that has been modified without removing the nucleic acid from the cell; such modifications include those obtained by gene replacement, site-specific mutation, and related techniques.

In the context of the present invention "complementary" refers to the capacity for precise pairing between two nucleotides sequences with one another. For example, if a nucleotide at a certain position of an oligonucleotide is capable of hydrogen bonding with a nucleotide at the corresponding position of a DNA or RNA molecule, then the oligonucleotide and the DNA or RNA are considered to be complementary to each other at that position. The DNA or RNA strand are considered complementary to each other when a sufficient number of nucleotides in the oligonucleotide can form hydrogen bonds with corresponding nucleotides in the target DNA or RNA to enable the formation of a stable complex.

In the present context the expressions "complementary sequence" or "complement" therefore also refer to nucleotide sequences which will anneal to a nucleic acid molecule of the invention under stringent conditions.

The term "stringent conditions" refers to general conditions of high, weak or low stringency.

The term "stringency" is well known in the art and is used in reference to the conditions (temperature, ionic strength and the presence of other compounds such as organic solvents) under which nucleic acid hybridisations are conducted.

Hybridisation means matching one nucleic acid with another e.g. base pairing between single-stranded nucleic acid molecules with each other to form a duplex.

A "sub sequence" or a "fragment" is any portion of an entire sequence. Thus, a fragment or sub sequence refers to a sequence of amino acids or nucleic acids that comprises a part of a longer sequence of amino acids (e.g. polypeptide) or nucleic acids (e.g. polynucleotide), respectively.

In the present context, the term "homology" indicates similarities between two amino acid sequences or between two nucleotide sequences is described by the parameter "sequence identity".

The term "sequence identity" indicates a quantitative measure of the degree of homology between two amino acid sequences or between two nucleic acid sequences of equal length. If the two sequences to be compared are not of equal length, they must be aligned to give the best possible fit, allowing the insertion of gaps or, alternatively, truncation at the ends of the polypeptide Sequences or nucleotide Sequences. The sequence identity can be calculated as 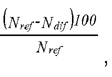 wherein N_{dif} is the total number of non-identical residues in the two Sequences when aligned and wherein N_{ref} is the number of residues in one of the sequences. Hence, the DNA sequence AGTCAGTC will have a sequence identity of 75% with the sequence AATCAATC (N_{dif}=2 and N_{ref}=8). A gap is counted as non-identity of the specific residue(s), i.e. the DNA sequence AGTGTC will have a sequence identity of 75% with the DNA sequence AGTCAGTC (N_{dif}=2 and N_{ref}=8).

With respect to all embodiments of the invention relating to nucleotide sequences, the percentage of sequence identity between one or more sequences may also be based on alignments using the clustalW software (http:/www.ebi.ac.uk/clustalW/index.html) with default settings. For nucleotide Sequence alignments these settings are: Alignment=3Dfull, Gap Open 10.00, Gap Ext. 0.20, Gap separation Dist. 4, DNA weight matrix: identity (IUB). Alternatively, the sequences may be analysed using the program DNASIS Max and the comparison of the sequences may be done at http://www.paralign.org/. This service is based on the two comparison algorithms called Smith-Waterman (SW) and ParAlign. The first algorithm was published by Smith and Waterman (1981) and is a well established method that finds the optimal local alignment of two sequences The other algorithm, ParAlign, is a heuristic method for sequence alignment; details on the method is published in Rognes (2001). Default settings for score matrix and Gap penalties as well as E-values were used.

The phrase "substantially identical" or "substantial identity" in the context of two nucleic acids or polypeptides, may refer to two or more sequences or sub-sequences that have at least about 60%, 65%, 70%, 75%, preferably 80% or 85%, more preferably 90%, 91%, 92%, 93%, 94%, 95%, 96%, 97%, 98%, 99%, 99.5%, or greater nucleotide or amino acid residue percent identity, respectively, comparison algorithm or by visual inspection. The substantially identity may refer to one gene, two or a group of genes separately and not to the other genes in the present invention, for example one gene might be 75% homologous and another might be 60 % homologous and further another might be 93 % homologous to the gene of interests. In certain aspects, the substantial identity exists over a region of amino acid sequences of at least about 50 residues in length, such as, at least about 100, 110, 120, 125, 130, 135, 140, 145, 150, 155, 160, or 165 amino acid residues. In certain aspects, substantial identity exists over a region of nucleic acid sequences of at least about 150 nucleic acid residues, such as at least about 200, 250, 300, 330, 360, 375, 400, 425, 450, 460, 480, 500, 600, 700, 800 such as at least about 900 nucleotides or such as at least about 1 kb, 2 kb, or such as at least about 3 kb. In some aspects, the amino acid or nucleic acid sequences are substantially identical over the entire length of the polypeptide sequence or the corresponding coding region.

Examples of "conservative substitutions" are within the group of basic amino acids (arginine, lysine and histidine), acidic amino acids (glutamic acid and aspartic acid), polar amino acids (glutamine and asparagine), hydrophobic amino acids (leucine, isoleucine, valine and methionine), aromatic amino acids (phenylalanine, tryptophan and tyrosine), and small amino acids (glycine, alanine, serine and threonine). Amino acid substitutions which do not generally alter the specific activity are known in the art and are described, for example, by Neurath and Hill, 1979. The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly as well as these in reverse.

The term "conservatively substituted variant" as used herein refers to a variant of a nucleotide sequence comprising one or more conservative substitutions.

Generally and in the present context, the term "silent substitution" refers to a base substitution which does not affect the sense of a codon and thus has no effect on polypeptide structure. As the skilled person will know silent substitutions are possible because of the degeneracy of the genetic code.

The term "conserved domain" may be a sequence of amino acids in a polypeptide or a sequence of nucleotides in DNA or RNA that is similar across multiple species. A known set of conserved sequences is represented by a consensus sequence. Amino acid motifs are often composed of conserved sequences. Additionally, the term "conserved sequence" refers to a base sequence in a nucleic acid sequence molecule or an amino acid sequence in a protein that has remained essentially unchanged throughout evolution. A "consensus sequence" may be defined in terms of an idealized sequence that represents the base most often present at each position in a nucleic acid sequence or the amino acid most often present at each position in a protein. A "consensus sequence" is identified by aligning all known examples of a nucleic acid Sequence or a protein so as to maximise their sequence identity. For a sequence to be accepted as a consensus sequence each particular base or amino acid must be reasonably predominant at its position and most of the sequences must be related to the consensus by only few substitutions, such as 1 or 2.

A homologue may also be in the form of an "insertional variant" of a protein, i.e. where one or more amino acid residues are introduced into a predetermined site in a protein. Insertions may comprise N-terminal and/or C-terminal fusions as well as intra-sequence insertions of single or multiple amino acids.

Homologues in the form of "deletion variants" of a protein are characterised by the removal of one or more amino acids from a protein.

Homologues in the form of "addition variants" of a protein are characterised by the addition of one or more amino acids from a protein, whereby the addition may be at the end of the sequence.

Substitution is another variant of a protein wherein one or more amino acids have been changed for another (other) amino acid(s).

The terms "Orthologs" and "Paralogs" -sequences are also a type of homologous sequences as described above. Several different methods are known by those of skill in the art for identifying and defining these functionally homologous sequences. Three general methods for defining orthologs and paralogs are described; an ortholog, paralog or homolog may be identified by one or more of the methods described below.

Orthologs and paralogs are evolutionarily related genes that have similar sequence and similar functions. Orthologs are structurally related genes in different species that are derived by a speciation event. Paralogs are structurally related genes within a single species that are derived by a duplication event.

Within a single plant species, gene duplication may cause two copies of a particular gene, giving rise to two or more genes with similar sequence and often similar function known as paralogs. A paralog is therefore a similar gene formed by duplication within the same species. Paralogs typically cluster together or in the same clade (a group of similar genes) when a gene family phylogeny is analyzed using programs such as CLUSTAL (Thompson et al.; Higgins et al.) Groups of similar genes can also be identified with pair-wise BLAST analysis (Feng and Doolittle). For example, a clade of very similar MADS domain transcription factors from *Arabidopsis* all share a common function in flowering time (Ratcliffe et al.), and a group of very similar AP2 domain transcription factors from *Arabidopsis* are involved in tolerance of plants to freezing (Gilmour et al.). Analysis of groups of similar genes with similar function that fall within one clade can yield sub-Sequences that are particular to the clade. These sub-sequences, known as consensus sequences, can not only be used to define the sequences within each clade, but define the functions of these genes; genes within a clade may contain paralogous sequences, or orthologous sequences that share the same function (see also, for example, Mount (2001), in Bioinformatics: sequence and Genome Analysis Cold Spring Harbor Laboratory Press, Cold Spring Harbor, N.Y., page 543.)

Speciation, the production of new species from a parental species, can also give rise to two or more genes with similar sequence and similar function. These genes, termed orthologs, often have an identical function within their host plants and are often interchangeable between species without losing function. Because plants have common ancestors, many genes in any plant species will have a corresponding orthologous gene in another plant species. Once a phylogenic tree for a gene family of one species has been constructed using a program such as CLUSTAL potential orthologous sequences can be placed into the phylogenetic tree and their relationship to genes from the species of interest can be determined. Orthologous sequences can also be identified by a reciprocal BLAST strategy. Once an orthologous sequence has been identified, the function of the ortholog can be deduced from the identified function of the reference sequence.

Orthologous genes from different organisms may have highly conserved functions, and very often essentially identical functions (Lee et al. and Remm et al.). Paralogous genes, which have diverged through gene duplication, may retain similar functions of the encoded proteins. In such cases, paralogs can be used interchangeably with respect to certain embodiments of the instant invention (for example, transgenic expression of a coding sequence). An example of such highly related paralogs is the CBF family, with three well-defined members in *Arabidopsis* and at least one ortholog in *Brassica napus*, all of which control pathways involved in both freezing and drought stress (Gilmour et al. and Jaglo et al.)

Hybrid aspen is a hybrid of the two species *Populus tremuloides* and *Populus tremula.*

The following references represent a small sampling of the many studies that demonstrate that orthologous transcription factor genes from diverse species are likely to function similarly (i.e., regulate similar target sequences and control the same traits), and that transcription factors may be transformed into diverse species to confer or improve traits.
(1) The *Arabidopsis* NPR1 gene regulates systemic acquired resistance (SAR); over-expression of NPR1 leads to enhanced resistance in *Arabidopsis.* When either *Arabidopsis* NPR1 or the rice NPR1 ortholog was overexpressed in rice (which, as a monocot, is diverse from *Arabidopsis*), challenge with the rice bacterial blight pathogen *Xanthomonas oryzae* pv. *Oryzae*, the transgenic plants displayed enhanced resistance (Chern et al.). NPR1 acts through activation of expression of transcription factor genes, such as TGA2 (Fan and Dong).
(2) E2F genes are involved in transcription of plant genes for proliferating cell nuclear antigen (PCNA). Plant E2Fs share a high degree of similarity in amino acid Sequence between monocots and dicots, and are even similar to the conserved domains of the animal E2Fs. Such conservation indicates a functional similarity between plant and animal E2Fs. E2F transcription factors that regulate meristem development act through common cis-elements, and regulate related (PCNA) genes (Kosugi and Ohashi).

The term "closely related" genes is used for genes that are orthologous or paralogous.

The term "promoter," as used herein, refers to a region of sequence determinants located upstream from the start of transcription of a gene and which are involved in recognition and binding of RNA polymerase and other proteins to initiate and modulate transcription. Promoters useful in plants need not be of plant origin. A "basal promoter" is the minimal sequence necessary for assembly of a transcription complex required for transcription initiation. Basal promoters frequently include a TATA box" element usually located between 15 and 35 nucleotides upstream from the site of initiation of transcription. Basal promoters also sometimes include a CCAAT box" element (typically a Sequence CCAAT) and/or a GGGCG Sequence, usually located between 40 and 200 nucleotides, preferably 60 to 120 nucleotides, upstream from the start site of transcription.

Promoters referred to herein as "constitutive promoters" may actively promote transcription under most, but not necessarily all, environmental conditions and states of development or cell differentiation. Examples of constitutive promoters include the cauliflower mosaic virus (CaMV) 35S transcript initiation region and the 1' or 2' promoter derived from T-DNA of *Agrobacterium tumefaciens,* and other transcription initiation regions from various plant genes, such as the maize ubiquitin-1 promoter, known to those of skill. Organ-specific promoters may be, for example, a promoter from storage sink tissues such as seeds, potato tubers, and fruits, or from metabolic sink tissues such as meristems. Furthermore, the promoter may be a leaf specific promoter such as the *rbcs* promoter from rice or tomato.

An "inducible promoter" in the context of the present invention refers to a promoter which is regulated under certain conditions, such as light, chemical concentration, protein concentration, conditions in an organism, cell, or organelle, etc. An example of an inducible promoter is the HSP promoter and the PARSK1, the promoter from the *Arabidopsis* gene encoding a serine-threonine kinase enzyme and which is induced by dehydration, abscissic acid and sodium chloride. In essence, expression under the control of an inducible promoter is "switched on" or increased in response to an applied stimulus. The nature of the stimulus varies between promoters and may include the above environmental factors. Whatever the level of expression is in the absence of the stimulus, expression from any inducible promoter is increased in the presence of the correct stimulus.

As used herein, the term "tissue specific" refers to a characteristic of a particular tissue that is not generally found in all tissues, or may be exclusive found in a tissue of interest. In the present application, "tissue specific" is used in reference to a gene regulatory element (promoter or promoter plus enhancer and/or silencer), the gene it encodes, or the polypeptide product of such a gene. In the context of a gene regulatory element or a "tissue specific promoter", the term means that the promoter (and also other regulatory elements such as enhancer and/or silencer elements) directs the transcription of a linked sequence in a cell of a particular lineage, tissue, or cell type, but is substantially inactive in cells or tissues not of that lineage, tissue, or cell type. A tissue specific promoter useful according to the invention is at least 5-fold, 10-fold, 25-fold, 50-fold, 100-fold, 500-fold or even 1,000 times more active in terms of transcript production in the particular tissue than it is in cells of other tissues or in transformed or malignant cells of the same lineage. In the context of a gene or the polypeptide product of a gene, the term tissue specific means that the polypeptide product of the gene is detectable in cells of that particular tissue or cell type, but not substantially detectable in certain other cell types. Particularly relevant tissue specific promoters include promoter sequences specifically expressed or active in the xylem forming tissue in a plant. Examples of such promoters are the Lmp1, Lmx2, Lmx3, Lmx4 and Lmx5 promoters, described in WO2004097024.

A "terminator sequence" refers to a section of genetic sequence that marks the end of gene or operon on genomic DNA for transcription. Terminator sequences are recognized by protein factors that co-transcriptionally cleave the nascent RNA at a polyadenylation signal, halting further elongation of the transcript by RNA polymerase. A nucleic acid is "operably linked" when it is placed into a functional relationship with another nucleic acid sequence. For instance, a promoter or enhancer is operably linked to a coding sequence if it increases the transcription of the coding sequence. Operably linked means that the DNA sequences being linked are typically contiguous and, where necessary to join two protein coding regions, contiguous and in reading frame. However, since enhancers generally function when separated from the promoter by several kilobases and intronic sequences may be of variable lengths, some polynucleotide elements may be operably linked but not contiguous.

In the context of the present invention the terms "transformation" and "transforming" are used interchangeably and as synonyms to "transfecting" and "transfection", respectively, to refer to the process of introducing DNA into a cell. The DNA constructs, including at least a portion of the gene or promoter of interest, can be introduced into host cells, which as stated previously, can be individual cells, cells in culture, cells as part of a host organism, a fertilized oocyte orgametophyte or an embryonic cell. By the term "introduction" when used in reference to a host cell is meant to refer to standard procedures known in the art for introducing recombinant vector DNA into the target host cell. Such procedures include, but are not limited to, transfection, infection, transformation, natural uptake, electroporation, biolistics and *Agrobacterium* mediated.

By "regenerable cell" is meant a plant cell from which a whole plant can be regenerated. It will be understood that the regenerable cell is a cell that has maintained its genetic potential, also known in the art as "totipotency". It will further be understood that the regenerable cells, when grown in culture, may need the appropriate stimuli to express the total genetic potential of the parent plant.

A number of genes analyzed using the analytical platform show interesting and most often unexpected commercial features.

One aspect of the present invention with intentionally changed (increased or decreased expression) levels of one gene's SEQ ID No: 23 to 115, 157-195 provide a method of producing a plant having modified lignin levels.

### Method of producing a plant having altered lignin properties

In specific embodiments of the invention advantageous plant phenotypes are generated by modifying, relative to the corresponding wild-type plant, the expression level of candidate genes that have been evaluated and selected according to the above criteria to modify lignin quantity, content and/or quality. According to these aspects a method is provided which comprises altering in the plant the level of a gene product of at least one gene comprising a nucleotide sequence selected from the group consisting of:
a) a nucleotide sequence from SEQ ID No: 23 to 115, 157-195 or;
b) a nucleotide sequence being at least 60% identical to a nucleotide sequence from SEQ ID NO 23 to 115, 157-195 or;
c) a sub sequence or fragment of a nucleotide Sequence of a) or b).

Higher lignin content may be obtained by over expression of at least one of the genes with SEQ ID NO: 113, 114, 115, 178 and 179 and/or down regulating at least one of the genes with SEQ ID NO: 24-26, 30, 32, 46, 47, 53, 54, 58,157, 159, 160 and 162 in the groups of a), b) or c).

Thus at least one, at least two, at least three, lat least four or all of SEQ ID NO: 1 13, 114, 115, 178 and 179 may be over expressed. Further, at least one, at least two, at least three, lat least four, at least five, at least six, at least seven, at least eight, at least nine, at least then, at least 11 , at least 12, at least 13, or all of SEQ ID NO: 24, 25, 26, 30, 32, 46, 47, 53, 54, 58, 157, 159, 160 and 162 may be down regulated.

Lower lignin content, may be obtained by over expression of at least one of the genes with SEQ ID NOM 76, 177 and 180, 181 , 182, 183, 184, 185, 186 and187 and/or down regulating at least one of the genes with SEQ ID NO: 23, 27, 28, 29, 31 , 33, 34, 35, 36, 37, 38, 39, 40, 41 , 42, 43, 44, 48, 49, 50, 51 , 52, 55, 56, 57, 158, 161 and 163 in the groups of a), b) or c).

Thus, at least one, at least two, at least three, lat least four, at least five, at least six, at least seven, at least eight, at least nine or all of the genes with SEQ ID NO: 176, 177 and 180, 181 , 182, 183, 184, 185, 186 and187 may be over expressed.

Further, at least one, at least two, at least three, lat least four, at least five, at least six, at least seven, at least eight, at least nine, at least then, at least 1 1 , 12, 13, 14, 15, 16, 17, 18, 19, 20, 21 , 22, 23, 24, 25, 26, 27 or all of SEQ ID NO: 23, 27, 28, 29, 31 , 33, 34, 35, 36, 37, 38, 39, 40, 41 , 42, 43, 44, 48, 49, 50, 51 , 52, 55, 56, 57, 158, 161 and 163 may be down regulated.

All these definitions regarding SEQ ID numbers mentioned above relating to the higher and lower lignin content are also combined with the above made definition of the homology language "substantially identical" or "substantial identity".

Plant phenotypes may be obtained by following technically modified crossing method comprising
i) selecting plant species expressing at least one of the nucleotide Sequences selected from the group consisting of
   a) a nucleotide sequence from SEQ ID NO 23 to 115, 157-195 or;
   b) a nucleotide sequence being at least 60% identical to a nucleotide sequence from SEQ ID NO 24-115, 157-195 or being at least 99.6% identical to the nucleotide sequence SEQ ID NO 23 or;
   c) a sub sequence or fragment of a nucleotide sequence of a) or b,
ii) crossing a plant species selected in i) with the same or another plant species selected in i),
iii) selecting plants with modulated expression of at least one of the nucleotide sequences selected from the group consisting of
   a) nucleotide sequence from SEQ ID NO 23 to 115, 157-195 or;
   b) a nucleotide sequence being at least 60% identical to a nucleotide sequence from SEQ ID NO 24-115, 157-195 or being at least 99.6% identical to the nucleotide sequence SEQ ID NO 23 or;
   c) a sub sequence or fragment of a nucleotide sequence of a) or b) compared to the plant species selected under i)
iv) optionally backcrossing one or more times the plants obtained in iii) and selecting plants with modulated expression of at least one of the nucleotide sequences selected from the group consisting of
   a) a nucleotide sequence from SEQ ID NO 23 to 115, 157-195 or;
   b) a nucleotide sequence being at least 60% identical to a nucleotide sequence from SEQ ID NO 24-115, 157-195 or being at least 99.6% identical to the nucleotide sequence SEQ ID NO 23 or;
   c) a sub sequence or fragment of a nucleotide sequence of a) or b) compared to any of the plant species used in i) and/or plants obtained in iii).

According to one aspect of the invention a method is provided comprising the following steps:
(i) providing an expression vector comprising a nucleotide sequence selected from the group consisting of
   a) a nucleotide sequence from SEQ ID NO 23 to 115, 157-195 or
   b) a nucleotide sequence being at least 60% identical to a nucleotide sequence from SEQ ID NO 24-115, 157-195 or being at least 99.6% identical to the nucleotide sequence SEQ ID NO 23 or
   c) a sub sequence or fragment of a nucleotide sequence of a) or b) and
   d) at least one regulatory element operable linked to the polynucleotide sequence, wherein said at least one regulatory element controls expression of the polynucleotide sequence in a target plant;
(ii) introducing the expression vector into at least one plant; and
(iii)selecting at least one transgenic plant that has a modulated lignin quantity compared to its wild type.

The sequences specified by Sequence ID numbers 59 to 93, 164-169, 170 and 188-195 represent sequences of the candidate genes as predicted from Populus t[eta]chocarpa, the sequences specified by sequence ID numbers 94 to 112, 157, 171 -175, represent sequences of the candidate genes from hybrid aspen and SEQ ID numbers 23 to 58 113 to 1 15 and 157-163, 176-187 as cloned from hybrid aspen., Additional sequence from these genes 5' as well as 3' to the sequence described in SEQ ID No: 23 to 1 15, and 157-195 is readily achievable using conventional cloning techniques, as the skilled person will understand, such as those described in Sambrook et al.

In further embodiments of the invention the nucleic acid sequences in a)-d) above are substantially identical to SEQ ID NO 23 to 115 and 157-195 as defined herein.

The modulated expression may be effected by introducing a genetic modification preferably in the locus of a gene comprising SEQ ID NO 23-1 15 and 157-195 encoding a polypeptide or a homologue of such polypeptide.

The modification may also for example be effected by one of: T-DNA activation, TILLING, homologous recombination, site-directed mutagenesis or directed breeding using one or more of SEQ ID NO 23 to 115 and 157-195 or a fragment thereof as markers in any step of the process of producing the desirable plants.

According to one aspect of the invention the modulation is decreased yield in lignin.

According to one other aspect of the invention the modulation is increased yield in lignin.

According to more particular embodiments of the invention, the method comprises the step of providing a nucleic acid construct, such as a recombinant DNA construct, comprising a nucleotide sequence selected from the group consisting of:
a) a nucleotide sequence comprising a sequence selected from SEQ ID No: 23 to 58 , 113 to 115 and 157-163, 176-187;
b) a complementary nucleotide sequence of a nucleotide sequence of a);
c) a sub-sequence or fragment of a nucleotide sequence of a) or b);
d) a nucleic acid sequence being at least 60% identical to any one of the sequences in a), b) and c); and but not SEQ ID No 23 where the identity is 99,6%
e) a nucleotide sequence which hybridizes under stringent conditions to a nucleotide sequence of a), b) or c).

In further embodiments of the invention the nucleic acid sequence in d) is substantially identical as herein defined to any one of the sequences in a), b), or c),

In preferred embodiments of this aspect of the invention the nucleotide sequence of a) is selected from the group consisting of SEQ ID NOs: 23 to 58.

A variety of methods exist in the art for producing the nucleic acid sequences and nucleic acid/DNA constructs of the invention. Procedures for identifying and isolating DNA clones are well known to those of skill in the art, and are described in, e. g. Sambrook et al., Molecular Cloning-A Laboratory Manual (3rd Ed.), Vol. 1-3, Cold Spring Harbor Laboratory, Cold Spring Harbor, New York, 2001.

Nucleic acid sequences of the present invention can be produced by a variety of *in vitro* amplification methods adapted to the present invention by appropriate selection of specific or degenerate primers. Examples of protocols sufficient to direct persons of skill through *in vitro* amplification methods, including the polymerase chain reaction (PCR) the ligase chain reaction (LCR), Qbeta-replicase amplification and other RNA polymerase mediated techniques (e. g., NASBA), e. g., for the production of the homologous nucleic acids of the invention are found in Sambrook, *supra.*

Alternatively, nucleic acid constructs of the present invention can be assembled from fragments made by solid-phase synthesis methods. Typically, fragments of up to approximately 100 bases are individually synthesized and then enzymatically or chemically ligated to produce a desired sequence, e. g., a polynucletotide encoding all or part of a transcription factor. For example, chemical synthesis using the phosphoramidite method is well known to the skilled person. According to such methods, oligonucleotides are synthesized, purified, annealed to their complementary strand, ligated and then optionally cloned into suitable vectors.

As mentioned, the above described sequences are from hybrid aspen and *Populus trichocarpa.* As the skilled person will understand, homologues of the described Sequences may be isolated from other species, non-limiting examples of which include acacia, eucalyptus, hornbeam, beech, mahogany, walnut, oak, ash, hickory, birch, chestnut, alder, maple, sycamore, ginkgo, palm tree, sweet gum, cypress, Douglas fir, fir, sequoia, hemlock, cedar, juniper, larch, pine, redwood, spruce and yew, apple, plum, pear, banana, orange, kiwi, lemon, cherry, grapevine, fig, cotton, bamboo, switch grass, red canary grass, *Miscantus species* and rubber plants. Useful homologues of the described sequences may also be isolated from hardwood plants from the *Salicaceae* family, e.g. from the *Salix* and *Populus* genus. Members of this genus are known by their common names: willow, poplar and aspen.

In particular, the method according to the present invention may comprise a step of providing a nucleic acid construct, such as a recombinant DNA construct, comprising a nucleotide sequence which relative to the particular sequences described, comprises conservative variations altering only one, or a few amino acids in the encoded polypeptide may also be provided and used according to the present invention. Accordingly, it is within the scope of the invention to provide and use a recombinant DNA construct comprising a nucleotide sequence which encodes a polypeptide comprising a conservatively substituted variant of a polypeptide encoded from a nucleotide sequence from SEQ ID NO 23 to 115 and 157-195.

Sequence alterations that do not change the amino acid sequence encoded by the polynucleotide are termed "silent" substitutions. With the exception of the codons ATG and TGG, encoding methionine and tryptophan, respectively, any of the possible codons for the same amino acid can be substituted by a variety of techniques, e. g., site-directed mutagenesis, available in the art. Accordingly, the present invention may also provide a recombinant nucleic acid construct, wherein the nucleotide sequence comprises a silent substitution in a nucleotide sequence.

In certain further embodiments of the invention, the sub-sequences or fragments of c) or d) have substantial sequence identity as herein defined to a conserved domain of a nucleotide sequence as described above under item a) or b), .

Thus, there are methods for identifying a sequence similar or paralogous or orthologous or homologous to one or more polynucleotides as noted herein, or one or more target polypeptides encoded by the polynucleotides, or otherwise noted herein and may include linking or associating a given plant phenotype or gene function with a Sequence.

### DNA construct

According to a second main aspect of the invention a DNA construct, such as a recombinant DNA construct, is provided comprising at least one sequence as described above. The DNA construct may be a vector. In particular, the recombinant DNA construct may comprise a nucleotide sequence selected from the group consisting of:
a) a nucleotide sequence comprising a sequence selected from SEQ ID NO: 23-115, 157-195 or;
b) a complementary nucleotide sequence of a nucleotide sequence of a) or;
c) a sub-sequence or fragment of a nucleotide sequence of a) or b) or;
d) a nucleic acid sequence being at least 60% identical to any one of the sequences in a), b) and c); but not SEQ ID NO 23, where the identity is 99,6% or;
e) a nucleotide sequence which hybridizes under stringent conditions to a nucleotide sequence of a), b) or c).

In selected embodiments of the invention the nucleic acid sequence in d) is substantially identical as herein defined to any one of the sequences in a), b) and c).

Variants of DNA constructs of a), b), c) an d) above that encode polypeptides having additions, deletions, substitutions or insertions of one or more amino acids are also within the scope of the invention as long as lignin properties are affected.

Also, in accordance with the discussion above, the nucleotide Sequence encodes a polypeptide comprising a conservatively substituted variant of a polypeptide of (a). Further, the nucleotide sequence comprises a silent substitution in a nucleotide sequence.

In additional embodiments of the pertaining to this aspect of the invention, the sub-sequences or fragments have substantial sequence identity as herein defined to a conserved domain of a nucleotide sequence as described above under item a).

In further embodiments and in accordance with the description above, the recombinant DNA construct further comprising a promoter, that may be constitutive, inducible, or tissue specific, operably linked to said nucleotide sequence. In particular, the recombinant DNA construct may further comprise a strong constitutive promoter in front of a transcribed cassette consisting of the full open reading frame of the gene followed by a terminator sequence.

In a particular embodiment of the present invention the nucleic acid construct, or recombinant DNA construct, further comprises a strong constitutive promoter in front of a transcribed cassette consisting of part of the target gene followed by a plant functional intron followed by the same part of the target gene in reverse orientation, the transcribed cassette is followed by an terminator sequence. The preferred vector is of such type with one of the nucleotide sequence of the invention is inserted in inverted repeat orientation.

Thus, according to one embodiment the invention relates to the method according to any one of claims 1 -13, wherein the recombinant DNA construct further comprises a strong constitutive promoter in front of a transcribed cassette consisting comprising a nucleotide sequence as defined in claim 1 followed by a plant functional intron followed by the nucleotide sequence as defined in claim 1 in reverse orientation The recombinant DNA construct may be used for transforming regenerable cells of a plant and regenerating a transgenic plant from said transformed cell. In the presently exemplified embodiments of the invention the recombinant DNA construct comprises the sequence of SEQ ID NO: 23-58 and 112-115..

### Construction of vectors

In general, those skilled in the art are well able to construct vectors of the present invention and design protocols for recombinant gene expression. For further details on general protocols for preparation of vectors reference is made to: Molecular Cloning: a Laboratory Manual: 3rd edition, Sambrook et al, 2001, Cold Spring Harbor Laboratory Press. Further information about plant vector and cloning in plants can be found in Gelvin SB and Schilperoort RA (eds) (2000) Plant Molecular Biology Manual. Dordrecht: Kluwer Academic Publishers. The promoter used for the gene may influence the level, timing, tissue, specificity, or inducibility of the over expression.

Generally, over expression of a gene can be achieved using a recombinant DNA construct having a promoter operably linked to a DNA element comprising a sense element of a segment of genomic DNA or cDNA of the gene, e.g., the segment should contain enough of the open reading frame to produce a functional protein and preferably the full open reading frame..

In pertinent embodiments of the invention the nucleic acid construct, or recombinant DNA construct, further comprising a constitutive, inducible, or tissue specific promoter operably linked to said nucleotide sequence.

In a presently preferred embodiment of the invention, the nucleic acid construct, or recombinant DNA construct, used for RNAi comprises the sequence of SEQ ID NO: 116 the vector named pK7GWIWG2(I), Karimi et al 2002.

In another preferred embodiment of the invention, the nucleic acid construct, or recombinant DNA construct, used for over expression comprises the sequence of SEQ ID NO: 117 the vector named pK2GW7, Karimi et al 2002.

### Approaches to obtaining altering the level of a gene product

Description of each gene and what is known about them and how it might be related to lignin is found in the experimental section below.

One aspect of the invention is to increase the expression of certain genes, non limiting examples how this can be done are presented here. Up regulation or over expression of a gene can for example be achieved by placing the full open reading frame of the gene behind a suitable promoter, which are described elsewhere, and usually placing terminator and poly-adenylation signal sequence 3' of the gene to be over expressed.

One other aspect of the invention is to decrease the expression of certain genes, non limiting examples how this can be done are presented here. Suppression of gene expression can for instance be achieved using a ribozyme or an antisense sequence, or down-regulated by an RNAi species. Several extensive descriptions on how genes can be down-regulated can be found in the literature and in patent literature as well, one example is the WO 2008/067841, which is hereby incorporated as reference.

One of the genes SEQ ID No: 23-115 and 157-195 could be used as targets for marker assisted breeding because changes in the gene regulatory sequences can give changes in the expression patterns and changes in the coding sequences can give changes in the gene function, and we have shown that manipulating these genes gives changes in the desired traits. This is usually referred to that the genes SEQ ID No: 23-115 and 157-195 can be used as candidate genes in marker assisted breeding Brady and Provart 2007, Varshney et al 2005, and Burke et al. 2007. This can be used to achieve both up and down regulation of gene activity.

One particular way to use this invention is to measure the expression of one or more of the genes SEQ ID No 23 to 115 and 157-195 using for example quantitative RT-PCR in natural populations and select for unusual high or low expression of the measured gene and use such plants as parents in a breeding program, this could be repeated for each breeding cycle. Many methods to quantify gene expression are known to the art, an efficient method is real time PCR, described in Bustin 2000.

In addition, the nucleic acid construct or recombinant DNA construct according to the invention may be used for the purpose of gene replacement in order to modify the plant lignin properties phenotype.

Suppression of endogenous gene expression can for instance be achieved using a ribozyme. Ribozymes are RNA molecules that possess highly specific endoribonuclease activity. The production and use of ribozymes are disclosed in US 4987071 and US 5543508. While antisense techniques are discussed below, it should be mentioned that synthetic ribozyme sequences including antisense RNAs can be used to confer RNA cleaving activity on the antisense RNA, such that endogenous mRNA molecules that hybridize to the antisense RNA are cleaved, which in turn leads to an enhanced antisense inhibition of endogenous gene expression.

Vectors in which RNA encoded by a relevant gene homologue is over-expressed can also be used to obtain co-suppression of a corresponding endogenous gene, e. g., in the manner described in US 5231020 to Jorgensen. Such co-suppression (also termed sense suppression) does not require that the entire gene sequence be introduced into the plant cells, nor does it require that the introduced sequence be exactly identical to the endogenous sequence of interest. However, the suppressive efficiency will be enhanced as specificity of hybridization is increased, e. g., as the introduced sequence is lengthened, and/or as the sequence similarity between the introduced sequence and the endogenous transcription factor gene is increased.

Vectors expressing an untranslatable form of gene, e. g., sequences comprising one or more stop codons, or nonsense mutation, can also be used to suppress expression of an endogenous gene, thereby reducing or eliminating its activity and modifying one or more traits. Methods for producing such constructs are described in US 5583021. In particular, such constructs can be made by introducing a premature stop codon into the gene.

One way of performing targeted DNA insertion is by use of the retrovirus DNA integration machinery as described in WO2006/078431. This technology is based on the possibility of altering the integration site specificity of retroviruses and retrotransposons integrase by operatively coupling the integrase to a DNA-binding protein (tethering protein). Engineering of the integrase is preferably carried out on the nucleic acid level, via modification of the wild type coding sequence of the integrase by PCR. The integrase complex may thus be directed to a desired portion or be directed away from an undesired portion of genomic DNA thereby producing a desired integration site characteristic.

Another technology that can be used to alter gene expression and gene activity is the TILLING, "Targeting Induced Local Lesions in Genomes", which is a non-transgenic way to alter gene function in a targeted way. This approach involves mutating a plant with for example ethyl methanesulfonate (EMS) and later locating the individuals in which a particular desired gene has been modified. The technology is described for instance in Slade and Knauf, 2005 and Henikoff, et al.

A method for abolishing the expression of a gene is by insertion mutagenesis using the T-DNA of *Agrobacterium tumefaciens*. After generating the insertion mutants, the mutants can be screened to identify those containing the insertion in an appropriate gene. Plants containing a single transgene insertion event at the desired gene can be crossed to generate homozygous plants for the mutation.

The polynucleotides and polypeptides of this invention can also be expressed in a plant in the absence of an expression cassette by manipulating the activity or expression level of the endogenous gene by other means, for example, by ectopically expressing a gene by T-DNA activation tagging, Ichikawa et al. (1997); Kakimoto et al. (1996). This method entails transforming a plant with a gene tag containing multiple transcriptional enhancers and once the tag has inserted into the genome, expression of a flanking gene coding sequence becomes deregulated, Ichikawa et al. (1997); Kakimoto et al. (1996). In another example, the transcriptional machinery in a plant can be modified so as to increase transcription levels of a polynucleotide of the invention (See, e. g., PCT Publications WO 96/06166 and WO 98/53057 which describe the modification of the DNA binding specificity of zinc finger proteins by changing particular amino acids in the DNA binding motif).

However, the recombinant DNA construct, comprising a nucleotide sequence as described above is particularly useful for sense and anti-sense suppression of expression, e. g., to down-regulate expression of a particular gene, in order to obtain a plant phenotype with altered lignin properties. That is, the nucleotide sequence of the invention, or sub-sequences or anti-sense sequences thereof, can be used to block expression of naturally occurring homologous nucleic acids. Varieties of traditional sense and antisense technologies are known in the art, e. g., as set forth in Lichtenstein and Nellen (1997). The objective of the antisense approach is to use a sequence complementary to the target gene to block its expression and create a mutant cell line or organism in which the level of a single chosen protein is selectively reduced or abolished.

For more elaborate descriptions of anti-sense regulation of gene expression as applied in plant cells reference is made to US 5107065, the content of which is incorporated herein in its entirety.

Gene silencing that is induced by double-stranded RNA is commonly called RNA interference or RNAi. RNA interference is a molecular mechanism in which fragments of double-stranded ribonucleic acid (dsRNA) interfere with the expression of a particular gene that shares a homologous sequence with the dsRNA. The process that is mediated by the same cellular machinery that processes microRNA, known as the RNA-induced silencing complex (RISC). The process is initiated by the ribonuclease protein Dicer, which binds and cleaves exogenous double-stranded RNA molecules to produce double-stranded fragments of 20-25 base pairs with a few unpaired overhang bases on each end. The short double-stranded fragments produced by Dicer, called small interfering RNAs (siRNAs), are separated and integrated into the active RISC complex. If one part of an RNA transcript is targeted by an RNAi molecule or construct, the whole transcript is down-regulated.

For more elaborate descriptions of RNAi gene suppression in plants by transcription of a dsRNA reference is made to US 6506559, US 2002/0168707, and WO 98/53083, WO 99/53050 and WO 99/61631, all of which are incorporated herein by reference in their entirety.

The presently preferred nucleic acid construct for RNAi is the pDONR vector (Invitrogen USA) and a destination vector pK2GW7 using Gateway technology (Invitrogen USA).

### Transformation of plant cells

In accordance with the present invention, the method comprise the further step of transforming regenerable cells of a plant with said nucleic acid construct or recombinant DNA construct and regenerating a transgenic plant from said transformed cell. When introducing the above DNA construct or vector into a plant cell, certain considerations must be taken into account, well known to those skilled in the art. The nucleic acid to be inserted should be assembled within a construct that contains effective regulatory elements that will drive transcription, as described above. There must be available a method of transporting the construct into the cell. Once the construct is within the cell, integration into the endogenous chromosomal material either will or will not occur.

Transformation techniques, well known to those skilled in the art, may be used to introduce the DNA constructs and vectors into plant cells to produce transgenic plants, in particular transgenic trees, with altered lignin properties.

A person of skills in the art will realise that a wide variety of host cells may be employed as recipients for the DNA constructs and vectors according to the invention. Non-limiting examples of host cells include cells in embryonic tissue, callus tissue type I, II, and III, hypocotyls, meristem, root tissue, tissues for expression in phloem, leaf discs, petioles and stem internodes.

As listed above, *Agrobacterium* transformation is one method widely used by those skilled in the art to transform tree species, in particular hardwood species such as poplar. Production of stable, fertile transgenic plants is now a routine in the art. Other methods, such as microprojectile or particle bombardment, electroporation, microinjection, direct DNA uptake, liposome mediated DNA uptake, or the vortexing method may be used where *Agrobacterium* transformation is inefficient or ineffective, for example in some gymnosperm species.

Alternatively, a combination of different techniques may be employed to enhance the efficiency of the transformation process, e.g. bombardment with *Agrobacterium* coated microparticles or microprojectile bombardment to induce wounding followed by co-cultivation with *Agrobacterium.*

It will be understood, that the particular choice of a transformation technology will be determined by its efficiency to transform certain plant species as well as the experience and preference of the person practising the invention with a particular methodology of choice. It will be apparent to the skilled person that the particular choice of a transformation system to introduce nucleic acid into plant cells is not essential to or a limitation of the invention, nor is the choice of technique for plant regeneration.

Following transformation, transgenic plants are preferably selected using a dominant selectable marker incorporated into the transformation vector. Typically, such a marker will confer antibiotic or herbicide resistance on the transformed plants and selection of transformants can be accomplished by exposing the plants to appropriate concentrations of the antibiotic or herbicide. A novel selection marker using the D-form of amino acids and based on the fact that plants can only tolerate the L-form offers a fast, efficient and environmentally friendly selection system. An interesting feature of this selection system is that it enables both selection and counter-selection.

Subsequently, a plant may be regenerated, e.g. from single cells, callus tissue or leaf discs, as is standard in the art. Almost any plant can be entirely regenerated from cells, tissues and organs of the plant, Gelvin SB and Schilperoort RA (eds) (2000) Plant Molecular Biology Manual. Dordrecht: Kluwer Academic Publishers. After transformed plants are selected and grown to maturity, those plants showing an altered lignin properties phenotype are identified. Additionally, to confirm that the phenotype is due to changes in expression levels or activity of the polypeptide or polynucleotide disclosed herein can be determined by analyzing mRNA expression using Northern blots, RT-PCR or microarrays, or protein expression using immunoblots or Western blots or gel shift assays.

### Plants

According to another aspect the invention provides a plant having altered lignin properties compared to its wild type. The plant may be transgenic

A transgenic plant may comprise a recombinant polynucleotide (DNA construct) comprising a nucleotide sequence capable of altering in the plant the level of a gene product of at least one of the genes SEQ ID 23-115 giving altered lignin content when comparing said group of transgenic plants grown for 8 -9 weeks in a greenhouse under a photoperiod of 18 hours, a temperature of 22°C/15°C (day/night) and a weekly were fertilized weekly Weibulls Rika S NPK 7-1-5 diluted 1 to 100 (final concentrations NO₃, 55 g/l; NH₄, 29 g/l; P, 12 g/l; K, 56 g/l; Mg 7,2 g/l; S, 7,2 g/l; B, 0,18 g/l; Cu, 0,02 g/l; Fe, 0,84 g/l; Mn, 0,42 g/l; Mo, 0,03 g/l; Zn, 0,13 g/l) with a group of wild-type plants grown under identical conditions. Growth conditions must be adapted to the particular species transform, since they have different growth optima. The alteration may vary between a decrease in lignin content from -6% to -20% and an increase in lignin content from +5% to +16%.

According to particular embodiments of the invention the level of a gene product of at least one gene comprising a nucleotide sequence selected from the group consisting of:
a) a nucleotide sequence from SEQ ID NO: 23-115, 157-195 or;
b) a nucleotide sequence being at least 60% identical to a nucleotide Sequence from SEQ ID NO 23-115, 157-195 or being at least 99.6% identical to the nucleotide sequence SEQ ID NO 23 or;
c) a sub sequence or fragment of a nucleotide sequence of a) or b) has been altered relative to the level found in the respective corresponding wild-type plant.

The invention especially relates to plants having higher lignin content, and to plants having lower lignin content, comprising the genes as mentioned under the heading "Method of producing a plant having altered lignin properties".

According to yet another embodiment of the invention, the transgenic plant comprises a recombinant polynucleotide (DNA construct) comprising a nucleotide sequence selected from the group consisting of:
a) a nucleotide sequence comprising a sequence selected from SEQ ID NO: 23-115, 157-195 or
b) a complementary nucleotide sequence of a nucleotide sequence of a) or
c) a sub-sequence or fragment of a nucleotide sequence of a) or b) or
d) a nucleic acid sequence being at least 60% identical to any one of the sequences in a), b) and c); but not SEQ ID NO 23, where the identity is 99.6% or
e) a nucleotide sequence which hybridizes under stringent conditions to a nucleotide sequence of a), b) or c).

In further embodiments of this aspect of the invention the nucleic acid sequence in d) is substantially identical as herein defined to any one of the sequences in a), b) or c).

As mentioned above the skilled person will realize that a variety of methods exist in the art for producing the nucleic acid sequences and polynucleotide constructs of the invention, e.g. by cloning techniques, assembly of fragments generated by solid phase synthesis.

In particular, the transgenic plant according to the present invention may comprise a recombinant DNA construct comprising a nucleotide sequence which relative to the particular sequences described, comprises conservative variations altering only one, or a few amino acids in the encoded polypeptide may also be provided and used according to the present invention. Accordingly, it is within the scope of the invention to provide a transgenic plant comprising a recombinant DNA construct comprising a nucleotide sequence which encodes a polypeptide comprising a conservatively substituted variant of a polypeptide of a) or d).

Accordingly, the present invention may also provide a recombinant DNA construct, wherein the nucleotide sequence comprises a silent substitution in a nucleotide sequence, that is, the recombinant DNA construct may comprise a sequence alteration that does not change the amino acid sequence encoded by the polynucleotide.

In certain further embodiments of the invention, the sub-sequences or fragments substantial sequence identity to a conserved domain of a nucleotide sequence as described above under item a) or d).

In further embodiments the transgenic plant provided according to the invention comprises a recombinant polynucleotide construct which further comprises a constitutive, inducible, or tissue specific promoter operably linked to said nucleotide sequence.

In still further embodiments the recombinant polynucleotide construct further comprises a strong constitutive promoter in front of a transcribed cassette.

In a presently preferred embodiment of the invention, the transgenic plant according to the invention comprises a recombinant DNA construct comprising the sequence of SEQ ID NO: 23 to 115, 157-195.

Another aspect of the invention provides a plant cell, plant progeny or any vegetable material of a transgenic plant or a plant with intentionally changed (increased or decreased expression) levels of one or more gene's SEQ ID No: 23 to 1 15, 157-195 according to the invention or the above mentioned variants thereof and comprising a recombinant polynucleotide.

A further aspect of the invention provides a plant cell, plant progeny or any vegetable material of a transgenic plant or a plant with intentionally changed (increased or decreased expression) levels of one or more gene's SEQ ID: 23 to 1 15, 157-195 according to the invention and comprising a recombinant polynucleotide, where the lignin content measured by the Klason method is modified.

The invention also relates to wood produced by a plant, which may be transgenic as defined above. It may comprise the nucleic acids of one or more of SEQ ID: 23 to 1 15, 157-195 or the variants thereof defined above.

Selections of plants may be done by measuring the lignin level according to the Klason method.

### Plant species

In accordance with the present invention, the transgenic plant may be a perennial plant which preferable is a woody plant or a woody species. In a useful embodiment, the woody plant is a hardwood plant which may be selected from the group consisting of acacia, eucalyptus, hornbeam, beech, mahogany, walnut, oak, ash, willow, hickory, birch, chestnut, poplar, alder, maple, sycamore, ginkgo, a palm tree and sweet gum. Hardwood plants from the Salicaceae family, such as willow, poplar and aspen including variants thereof, are of particular interest, as these two groups include fast-growing species of tree or woody shrub which are grown specifically to provide timber and biofuel.

In further embodiments, the woody plant is a conifer which may be selected from the group consisting of cypress, Douglas fir, fir, sequoia, hemlock, cedar, juniper, larch, pine, redwood, spruce and yew.

In useful embodiments, the woody plant is a fruit bearing plant which may be selected from the group consisting of apple, plum, pear, banana, orange, kiwi, lemon, cherry, grapevine and fig.

Other woody plants which may be useful in the present method may also be selected from the group consisting of cotton, bamboo and rubber plants.

Other plants which may be useful is grasses grown for biomass production, for example *Miscanthus* and Switchgrass.

The present invention extends to any plant cell of the above transgenic plants obtained by the methods described herein, and to all plant parts, including harvestable parts of a plant, seeds and propagules thereof, and plant explant or plant tissue. The present invention also encompasses a plant, a part thereof, a plant cell or a plant progeny comprising a DNA construct according to the invention. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced in the parent by the methods according to the invention.

### Improved lignin quality and amount

In addition, the nucleic acid construct or recombinant DNA construct according to the invention may be used for the purpose of gene replacement in order to modify the lignin level in the plant.

This invention relates to the problem of changing wood chemistry especially for changing lignin quality and amounts.

Such trees can be used in many applications. For example for these usages
1. Changing surface chemistry for e.g. composite material usage.
2. Saccharification potential with emphasis of changing lignin levels and/or composition and/or lignin binding to the carbohydrates of the cell wall.
3. Changing lignin levels for energy content.
4. Changing lignin levels for improved pulping properties.

The inventors have found that the down regulation and/or over expression of certain genes, gives changed wood chemistry in the produced transgenic trees. Especially we have found that certain genes can be used to modify lignin levels. These genes have been found within an RNAi gene mining program, with a gene selection a basic set up identical to the one described in the patent applications WO 2008/067840 and WO 2008/067841, but where the first level of analysis were done with FT-IR analysis of wood and the second level of analysis were done with Klason Lignin analysis.

Thus, according to one aspect the invention regards the use of a nucleotide sequence selected from the group consisting of
a) at least one over expressed gene chosen from genes with SEQ ID NO: 176, 177 and 180-187 and/or one down regulated gene chosen from of the genes with SEQ ID NO 23, 27 - 29, 31 , 33 - 44, 48 - 52, 55 -57, 158, 161 and 163 or,
b) a nucleotide sequence being at least 60% identical to a nucleotide sequence from SEQ ID NO 27 - 29, 31 , 33 - 44, 48 - 52, 55 -57, 158, 161 , 163 176, 177 and 180-187 or at least 99,6% identical to SEQ ID NO 23,
c) a sub sequence or fragment of a nucleotide sequence of a) or b), in a plant for lowering lignin levels improving saccharification for bio-refining and ethanol production and for lowering lignin levels in pulp and paper processing.

The preferred nucleotide sequences are selected from the group consisting of 23, 28, 29, 31 , 33 - 38, 40, 42, 55, 57, 158, 161 and 163 in a plant for lowering lignin levels improving saccharification for bio-refining and ethanol production and for lowering lignin levels in pulp and paper processing. See table 1 for lignin levels.

According to another aspect the invention relates to the use of a nucleotide sequence selected from the group consisting of
a) at least one over expressed gene chosen from genes with SEQ ID NO: 1 13, 114, 115, 178 and 179 and/or at least one down regulated gene chosen from genes with SEQ ID NO: 24-26, 30, 32, 46, 47, 53, 54, 58, 157, 159, 160 and 162 or
b) a nucleotide sequence being at least 60% identical to a nucleotide sequence from SEQ ID NO 24 - 26, 30, 32, 46, 47, 53, 54, 58, 113 - 1 15, 157, 159, 160, 162, 178 and 179 or
c) a sub sequence or fragment of a nucleotide sequence of a) or b) in a plant for increasing lignin levels for energy production.

The preferred nucleotide sequences are selected from the group consisting of 26, 30, 32, 46, or 113 - 1 15, 178-179 in a plant for increasing lignin levels for energy production.

Especially the invention regards the use of a nucleotide sequence selected from the group consisting of
a) at least one over expressed gene chosen from genes with SEQ ID NO: 113, 114 and 115 and/or at least one down regulated gene chosen from genes with SEQ ID NO: 24-26, 30, 32, 46, 47, 53, 54, 58, 157, 159, 160 and 162 or
b) a nucleotide sequence being at least 60% identical to a nucleotide sequence from SEQ ID NO 24 - 26, 30, 32, 46, 47, 53, 54, 58, 113 - 115, 157, 159, 160, 162, 178 and 179 or
c) a sub sequence or fragment of a nucleotide sequence of a) or b) in a plant for increasing lignin levels for energy production.

The invention also relates to the use of a nucleotide sequence selected from the group consisting of
a) at least one down regulated gene chosen from of the genes with SEQ ID NO a nucleotide sequence from genes with SEQ ID NO 23, 27 - 29, 31, 33 - 44, 48 - 52, 55 -57, 158, 161 and 163 or
b) a nucleotide sequence being at least 60% identical to a nucleotide sequence from SEQ ID NO , 27 - 29, 31 , 33 - 44, 48 - 52, 55 -57, 158, 161 , 163, 176, 177, 180 and 187 or being at least 99,6% identical to SEQ ID NO 23 or
c) a sub sequence or fragment of a nucleotide sequence of a) or b) in a plant for lowering lignin levels improving saccharification for bio-refining and ethanol production and for lowering lignin levels in pulp and paper processing.

The effect of the preferred gene are shown in tables 1a and 1b below.

**Table 1a Over expressed genes with higher lignin content vs wildtype**

| **Gene** | **Construct** | **T-test** | **Anova** | **<T int** | **>T Int** | **< Min** | **>Max** | **Ratio** | **Modulation** | **SEQ ID** | **+/- vs wt T89** |
|---|---|---|---|---|---|---|---|---|---|---|---|
| STT 167 | LMP1-002 | | | 0 | 1 | 0 | 1 | 1,02 | Over expression | Seq ID No: 178 | 9% |

**Table 1b Down regulated genes with higher lignin content vs wildtype**

| **Gene** | **Construct** | | **Anova** | **<T int** | **>T Int** | **< Min** | **>Max** | **Ratio** | **Modulation** | **SEQ ID** | **+/- vs wt T89** |
|---|---|---|---|---|---|---|---|---|---|---|---|
| STT 167 | KR472 | | | 0 | 0 | 0 | 1 | 1,02 | Down regulated RNAi | Seq ID No: 53 | 5% |

### Further uses of the invention

Moreover, the invention relates to the use of a nucleotide sequence selected from the group consisting of
a) a nucleotide sequence from SEQ ID NO 23-115, 157-195 or
b) a nucleotide sequence being at least 60% identical to a nucleotide sequence from SEQ ID NO 24-115, 157-195, or being at least 99,6% identical to SEQ ID NO 23 or
c) a sub sequence or fragment of a nucleotide sequence of a) or b) for the identification of plants having altered lignin characteristics as compared to the wild-type.

According to the invention these sequences may also be used as candidate genes in marker assisted breeding.

It should be noted that embodiments and features described in the context of one of the aspects of the present invention also apply mutatis mutandis to the all other aspects of the invention. For example the variant and homology language for defining genes and expressed polypeptides regards all aspects of the inventions such as DNA constructs, expressions products of the genes, vectors, plant cells, plant progeny, wood and the methods for producing and identification of plants according to the invention.

All patent and non-patent references cited in the present application, are hereby incorporated by reference in their entirety.

The invention will now be described in further details in the following non-limiting examples.

### Examples

In order to find and elucidate the function of genes involved in wood formation and wood growth, an extensive gene mining program was performed, resulting in the identification of genes useful in wood industrial applications.

### Gene Selection

The genes indentified for changing wood chemistry especially for changing lignin quality and amounts was done in a gene mining program, which is described in detail in the patent applications WO 2008/067840, WO 2008/067841 and WO 2009/084999, which are hereby incorporated as references.

### Cloning of the selected genes

Selected genes were subsequently cloned into a RNAi vector under the control of the CaMV 35S promoter (RNA interference vector, pK7GWIWG2(I)) using Gateway technology (Invitrogen USA), SEQ ID NO: 116. Two principal sets of cloning primers were used, one set was a universal primer pair binding to the vector and the poly-A tail, and the other set were gene-specific primers. The PCR product was first transferred into the pDONR vector and subsequently transferred into the destination vector pK7GWIWG2 according to manufacturers' recommendations (Invitrogen USA). The sequences of the genes and the corresponding construct, and cloning primer are listed in Table 2 and 3.

Full Sequence from *Populus trichocarpa* means sequences of the candidate genes as predicted from *Populus trichocarpa* genome sequence at JGI (G.A. Tuskan et al. Science 15 September 2006:Vol. 313. no. 5793, pp. 1596 - 1604). The predicted sequence corresponds to the sequences that best maps the actual transcript sequence from hybrid aspen or the corresponding gene model at JGI. Full Sequence from *Populus tremula x tremuloides* means the best sequence of the candidate genes resulting from resequencing of hybrid aspen EST's, sequencing of fragments cloned in the RNAi construct and EST sequences present in the Pop-db, sequence used for RNAi construct means the sequence of the actually cloned fragment in the RNAi constructs.

**Table 2 Summary of the cloning primers used.**

| **Gene** | **Construct** | **Cloning forward primer** | **Cloning reverse primer** |
|---|---|---|---|
| STT167 | KR472 | SEQ ID No:03 | SEQ ID No:11 |

**Table 3 Genes and the corresponding RNAi construct sequences**

| **Gene** | **Construct** | **Sequence used for RNAi construct** | **Full Sequence from *Populus trichocarpa*** | **Full Sequence from *Populus tremula x tremuloides*** |
|---|---|---|---|---|
| STT167 | KR472 | SEQ ID No:53 | SEQ ID No:88 | SEQ ID No:105 |

### Plant transformation

CaMV 35S: Inverted repeat DNA constructs were transformed into *Agrobacterium* and subSEQuent into Hybrid aspen, *Populus tremula* L. x P. *tremuloides* Minch. Clone T89, hereafter called "poplar", was transformed and regenerated essentially as described in Nilsson et al. (1992). Approximately 3-8 independent lines were generated for each construct. One such group of transgenic trees produced using one construct is hereafter called a "construction group", e.g. different transgenic trees emanating from one construct. Each transgenic line within each construction group, e.g. KR555-2B KR555-3A, KR555-2B and so on, are different transformation events and therefore most probably have the recombinant DNA inserted into different locations in the plant genome. This makes the different lines within one construction group partly different. For example it is known that different transformation events will produce plants with different levels of gene down-regulation when using RNAi constructs of the type used here.

### Plant growth

The transgenic poplar lines were grown together with their wild type control (wt) trees, in a greenhouse under a photoperiod of 18 h and a temperature of 22°C/15°C (day/night). The plants were fertilized weekly Weibulls Rika S NPK 7-1-5 diluted 1 to 100 (final concentrations NO₃, 55 g/l; NH₄, 29 g/l; P, 12 g/l; K, 56 g/l; Mg 7,2 g/l; S, 7,2 g/l; B, 0,18 g/l; Cu, 0,02 g/l; Fe, 0,84 g/l; Mn, 0,42 g/l; Mo, 0,03 g/l; Zn, 0,13 g/l). The plants were grown for 8-9 weeks before harvest. During this time their height and diameter was measured 1 to 2 times per week. A number of wild type trees (typically 15-25 trees) and a number of transgenic trees comprising several construction groups (typically 6-20 construction groups) were grown in parallel in the greenhouse under the same above conditions. All comparisons between the wild type trees and construction groups are made within each growth group.

### Sampling

Two principal types of harvest and sampling were performed. One general type was for example for chemical analysis, wood morphological analysis, gene expression analysis, wood density analysis and metabolomics analysis. And another type for dry weight measurements of bark, wood, leafs and roots.

### Selection of Construction Groups

In the first round of growth for each group of trees with a specific gene down regulated using RNAi, i.e. a construction group, a number of the following analyses was performed: FT-IR analysis. These data were analysed in order to single out the Construction Groups that showed a phenotypic variation compared to wild type control trees.

### Selection of construction groups using FT-IR and Multivariate Analysis

### FT-IR Analysis

All samples were freeze dried and milled to a fine powder using a ball mill (Retch NM 301) prior to FT-IR analysis. The measuring procedure has been shifted over time; samples have been measured as pure samples or as 2.5% sample in potassium bromide (KBr). Different instruments (Bruker IFS 66v and Bruker 55 Equinox, Bruker) have been used hence the wavelength range changed from 900-1800nm to 400-5200nm in the latter case only the fingerprint region 800-1860nm have been used for multivariate analysis.

### Baseline correction and normalization

Two different approaches have been used to normalize the samples prior to multivariate analysis in order to reduce effects such as baseline and concentration variations.

When the samples are measured using the instrument with the larger wavelength range a baseline is fitted for each sample using one point on each side of the fingerprint region (~800nm and ~1860nm) the baseline is subtracted and the area under the spectrum normalized to a constant sum.[ Orthogonal Projections to Latent Structures Discriminant Analysis Modeling on in Situ FT-IR Spectral Imaging of Liver Tissue for Identifying Sources of Variability Stenlund, Hans, Gorzsás, András, Persson, Per, Sundberg, Björn, and Trygg, Johan Anal. Chem., 2008, 10.1021/ac8005318 Web Release Date: August 20, 2008]

When samples are measured using the other instrument that only measures between 900-1800nm the first two principal components has been removed from data since they were considered to only contain variation related to baseline and concentration variations.

### Multivariate analysis

The goal with the multivariate analysis has been to detect constructions (genes) that in some means deviate from the wild type population. Principal component analysis (PCA) has been utilized to reduce the dimensionality from ~500 variables (recorded wavelengths) into ~5 principal components. All wild type samples in the same cultivation round are modelled using PCA and the different "construction samples" belonging to the same cultivation round has been predicted into that model to detect deviations in principal component space (score space) as well as in the residual. A construction that has at least 2 samples deviating in the same direction in the score space or in the residual has been scored as a changed phenotype and then been further analyzed by other methods.

A construction group that scored positive in the FT-IR analysis was in most cases analyzed for lignin content.

### Lignin measurements

Lignin content was measured at MoRe Research, Örnsköldsvik, using their internal method MoRe KA 10.219, which is based upon the Tappi method T 222 om-88 (http://www.tappi.org) with some modifications. The method is known as Klason-lignin and uses the fact that lignin is insoluble in acids.

The dried milled wood samples are pre hydrolyzed in 72% sulfuric acid which then is diluted to 2% and further hydrolyzed during refluxing for 9hr. The dispersed lignin is filtered of and then washed and dried. The weight of the dried lignin is measured and compared with the original weight to decide the lignin content.

### Statistical Evaluation

Each construction is subjected to 4 different tests;
1) T-test Blom ISBN 91-44-05592-7; the mean value for the samples belonging to each construction is compared with the mean value for the corresponding T89 samples, using a 2-tailed T-test assuming unequal variance. If the probability that the mean values are equal is less that 5% the construction is considered to have changed lignin content.
2) Anova; the between and within group (construction and wild type) variation is compared using an F-test Blom ISBN 91-44-05592-7. If the probability that the between and the within group variation is equal is lower than 5% the construction is considered to have changed lignin content. This is equivalent to a 2-tailed T-test assuming equal variance.
3) A 95 % confidence interval is calculated around the mean value for the wild type samples using T statistics. One or more lines of a construction have lignin content outside this confidence interval the construction is considered to affect the lignin content.
4) A non statistical measure, were the samples for each construction is compared with the lowest and highest value for the corresponding T89 samples. If one or more samples from one construction have higher lignin content than the highest or lower than the lowest lignin content for a T89 sample, it the construction is assumed to affect the lignin content.

Construction groups meeting one or more of these criteria were selected.

A number of the construction groups that showed differences in lignin levels were tested with RT-PCR in order to verify the down regulation of the targeted gene.

Real-time RT-PCR (Bustin 2000) was used to compare construct gene expression levels of the construction group with corresponding wild type group. The expression level of 26S ribosome regulatory subunit S2 was used as a reference to which construct gene expression was normalized. The comparative CT method was used for calculation of relative construct gene expression levels, where the ratio between construction and reference gene expression levels is described by (1 + E_{target})^{-CTtarget} / (1 + E_{reference})^{-CTreference} where E_{target} and E_{reference} are the efficiencies of construct and reference gene PCR amplification respectively and CT_{target} and CT_{reference} are the threshold cycles as calculated for construct and reference gene amplification respectively.

For total RNA extraction, stem samples (approx. 50mg) were harvested from greenhouse grown plants and flash frozen in liquid nitrogen. Frozen samples were ground in a bead mill. Total RNA was extracted using Aurum Total RNA Mini kit according to manufacturers' recommendations (Bio-Rad). cDNA synthesis was performed using iScript cDNA synthesis kit according to manufacturers recommendations (Bio-Rad).

For real-time PCR, cDNA template was mixed with SYBR Green Supermix according to manufacturers recommendations (Bio-Rad) and corresponding construct gene specific primers or internal reference gene specific primers, forward PCR primer SEQ ID NO: 123 and reverse PCR primer sequence SEQ ID NO: 124. Real-time PCR was run on a MyiQ PCR thermocycler (Bio-Rad) and analysed using included software iQ5. For each sample, reactions were set up in three to six replicates, with an equal number of replicates using construct gene specific primers and reference gene specific primers, and the average threshold cycle for the reaction replicates was subsequently used for calculation of relative construct gene expression levels.

The 96 well plate was covered with microfilm and set in the thermocycler to start the reaction cycle. By way of illustration, the reaction cycle may include the following steps: Initial denaturation at 95°C for 3 minutes 30 seconds followed by 40 rounds of amplification comprising the following steps 95°C for 10 seconds, 55°C for 30 seconds and 72°C for 40 seconds.

Selected genes were in a number of cases tested in over expression experiments were the full length open reading frame (ORF) for the gene corresponding to the RNAi construct were cloned and put under the CaMV 35S promoter and tested in transgenic trees. The RNAi constructs were from Hybrid aspen i.e. a hybrid of the two species *Populus tremuloides* and *Populus tremula.*

These plants were in many cases tested for lignin content and a number of these gene constructs also modified the cell wall chemistry.

### Cloning of full length genes for over expression analysis using the 35S promoter

For over expression analysis of a corresponding full length gene, previously down regulated using RNAi, the gene coding sequence (CDS) was predicted from the genome sequence of *Populus trichocarpa* (Tuskan et al., 2006). The gene models were compared to, and in some instances corrected based on, information published for homologous genes in Arabidopsis thaliana and other plant species. This was done using databases such as http://www.ncbi.nlm.nih.gov/ and http://www.arabidopsis.org/. Selected genes were subsequently cloned into an over-expression vector under the control of the CaMV 35S promoter. Cloning primers were designed to be gene specific and to include the full CDS and in some instances parts of the untranslated transcript region (UTR). The full length gene was amplified from hybrid aspen cDNA using Phusion high fidelity DNA polymerase (Finnzymes) and first transferred into the pDONR vector (Invitrogen USA) and subsequently transferred into the destination vector pK2GW7 (Karimi et al., 2002) using Gateway technology (Invitrogen USA). Constructs thus cloned have construct names starting with 35s. The sequences of the selected full length genes, PCR primers etc. are listed in Table 3.

### Cloning of full length genes for over expression analysis using the LMP1 promoter

For over expression analysis of a corresponding full length gene, previously down regulated using RNAi, the gene coding sequence (CDS) was predicted from the genome sequence of *Populus trichocarpa* (Tuskan et al., 2006). The gene models were compared to, and in some instances corrected based on, information published for homologous genes in Arabidopsis thaliana and other plant species. This was done using databases such as http://www.ncbi.nlm.nih.gov/ and http://www.arabidopsis.org/. Selected genes were subsequently cloned into an over-expression vector under the control of the LMP1 promoter, described in patent application WO 2004/097024. Cloning primers were designed to be gene specific and to include the full CDS and in some instances parts of the untranslated transcript region (UTR). The full length gene was amplified from hybrid aspen cDNA using Phusion high fidelity DNA polymerase (Finnzymes) and first transferred into the pDONR vector (Invitrogen USA) and subsequently transferred into the destination vector pPCV812-LMP1-Gateway using Gateway technology (Invitrogen USA). Constructs thus cloned have construct names starting with LMP1. The pPCV812 plant binary vector is described in Koncz et al., 1994. The pPCV812 vector has been further modified to create the pPCV812-LMP1-Gateway vector by insertion of the LMP1 promoter between the BgIII and SaII sites and by replacing the GUS reporter gene *uidA* with a Gateway recombination cassette (attR1-ccdB-attR2, Invitrogen Life Technologies) using BamHI and SacI sites. The sequences of the selected full length genes, PCR primers etc. are listed in Table 3.

Cloning of full length transcription factor genes for over expression analysis using the 35S promoter

The corresponding gene models for the selected transcription factor genes were extracted from data derived from the genome sequencing of *Populus trichocarpa* (Tuskan et al., 2006) using BLAST analysis. The gene models were compared to, and in some instances corrected based on, information published for homologous genes in Arabidopsis thaliana and other plant species. This was done using databases such as http://www.ncbi.nlm.nih.gov/ and http://www.arabidopsis.org/. Selected genes were subsequently cloned into an over-expression vector under the control of the CaMV 35S promoter. For isolation of cDNA, total RNA was isolated from stem, leaf and bark tissue sampled from hybrid aspen clone T89 plants and reverse transcribed to cDNA using Superscript III First Strand Synthesis System (Invitrogen). cDNA were then amplified by PCR with gene specific forward and reverse primers using Phusion high fidelity DNA polymerase (Finnzymes). PCR primers were selected as follows, the 5'-primer was placed at the start codon and the 3' reverse primer was placed 3' of the translational stop site. Forward primers were modified by the introduction of a Kozak sequence (5'-AGAACC-3') upstream and next to the start codon of each target gene. The amplified cDNAs were inserted into a Gateway entry vector pENTR/D-TOPO (Invitrogen), followed by transfer of the genes into the expression vector pK2GW7 (Karimi et al., 2002) using the Gateway LR recombination reaction (Invitrogen). The cloned genes were control sequenced and compared to the selected genes using standard techniques before sub cloning into the plant vector pK2GW7.

Table 4 PCR primer sequences (a) and sequences used for 35S over expression constructs (b)

Full Sequence from *Populus tremula x tremuloides* means the best Sequence of the candidate genes resulting from resequencing of hybrid aspen EST's, sequencing of cloned fragments etc. Sequence used for 35S over expression construct means the sequence of the actually cloned fragment in the over expression constructs.

**Table 4a**

| Gene | Construct | Cloning Forward primer | Cloning Reverse primer |
|---|---|---|---|
| STT167 | LMP1-002 | SEQ ID No:142 | SEQ ID No:150 |

**Table 4b**

| Gene | Construct | Sequence used for over expression construct | Full Sequence from *Populus trichocarpa* | Full Sequence from *Populus tremula x tremuloides* |
|---|---|---|---|---|
| STT167 | LMP1-002 | SEQ ID No:178 | SEQ ID No:189 | SEQ ID No:105 |

### Results

The isolated genes and the corresponding constructions that has been used is shown below, which passed the selection criteria according to the invention, table 5.

**Table 5: Summary of modulated lignin results**

| **Gene** | **Construct** | **T-test** | **Anova** | **<T int** | **>T Int** | **< Min** | **>Max** | **Ratio** | **Modulation** | **SEQ ID** | **+/- vs wt T89** |
|---|---|---|---|---|---|---|---|---|---|---|---|
| STT 167 | KR472 | | | 0 | 0 | 0 | 1 | 1,02 | Down reg. RNAi | No: 53 | 5% |
| STT 167 | LMP1-002 | | | 0 | 1 | 0 | 1 | 1,02 | Over expression | No: 178 | 9% |

The first column "Gene" denotes the gene number of the construction, which was used in transformation. The following two columns contain the result of a paired t-test and ANOVA respectively. "<T inv" and ">T inv" denotes the number of samples outside the T (95%) distribution for T89 on the lower and upper side respectively. ">Max" and "<Min" denotes the number of samples higher the highest T89 and lower than the lowest T89 sample respectively. Ratio is the ratio between construction average and T89 average.

### Gene STT 167

The RNAi construction group KR472 shows a maximum increase of lignin of 5% compared to the wild type average see Table 6 below.

The gene STT 167 is represented by Seq ID NO 53, 88, 105, 178 or 189 in different constructs, see table 1, 2, 3 and 5.

**Table 6**

| **Individual Name** | **Lignin Content (%)** |
|---|---|
| T89-12 | 18,5 |
| T89-17 | 18,4 |
| T89-18 | 19,2 |
| T89-20 | 19,1 |
| T89-21 | 18,3 |
| T89-5 | 17,1 |
| T89-9 | 19,1 |
| KR472-1A-1 | 19,4 |
| KR472-4A-2 | 18,5 |
| KR472-5B-2 | 18,6 |

| | |
|---|---|
| Number (KR472) | 3 |
| Average (KR472) | 18,83 |
| Standard deviation (KR472) | 0,49 |
| Number (T89) | 7 |
| Average (T89) | 18,53 |
| Standard deviation (T89) | 0,73 |
| Max (T89) | 19,20 |
| Min (T89) | 17,10 |
| | 18,53 +/- |
| Confidence Interval (T89) (95%) | 1,79 |
| Ratio (Number (KR472)/Average (T89)) | 1,02 |
| Ratio (Max(KR472)/Average(T89)) | 1,05 |
| Ratio (Min(KR472)/Average(T89)) | 1,00 |
| T-test | 0,4727 |
| Anova | 0,5343 |
| Num KR472 >Confidence Interval (T89) (95%) (20,32) | 0 |
| Num KR472 <Confidence Interval (T89) (95%) (16,74) | 0 |
| Num KR472 >Max (T89) (19,2) | 1 |
| Num KR472 <Min (T89) (17,1) | 0 |

### _References:

Aspeborg et al., Plant Physiology (2005), 137(3), 983-997
Baucher, Critical Reviews in Biochemistry and Molecular Biology, 38:305-350, 2003 Blom Statistikteori med tillämpningar ISBN 91-44-05592-7
Brady and Provart 2007. Journal of the Science of Food and Agriculture Vol 87, Pp 925 - 929,
Breaking the Biological Barriers to Cellulosic Ethanol: A Joint Research Agenda . A Research Roadmap Resulting from the Biomass to Biofuels Workshop, December 7-9, 2005, Rockville, Maryland
Burke et al 2007. Current opinion in genetics and development vol17, 525-532.
Bustin S A. Journal of Molecular Endocrinology ,2000. 25, 169-193.
Chen and Dixon. Nature Biotechnology Vol. 25 no 7: pp759-761, 2007
Chern et al. 2001) Plant J. 27: 101 113
Falco et al. US2005034176
Fan and Dong (2002) Plant Cell 14: 1377 1389
Feng and Doolittle (1987) J. Mol. Evol. 25: 351 360
Flinn et al in. US6,451,604
Gilmour et al. (1998) Plant J. 16: 433 442.
Griffiths, P. R. and De Haseth, J. A. Fourier Transform Infrared Spectrometry. New York: Wiley, 1986
Henikoff, Till, and Comai, Plant Physiol. 2004 Jun;135(2):630-6..
Heo et al., Plant and Cell Physiology (2005), 46(12), 2005-2018
Ichikawa et al. 1997 Nature 390 698-701;
Jaglo et al. (1998) Plant Physiol. 127: 910 917.
Kakimoto et al. 1996 Science 274: 982-985. Karimi M, Inze D and Depicker A. Treands in plant Sciences. (2002) vol7 no5 pp193-195
Kang et al., Cell. 2001 Jun 1;105(5):625-36
Klason-lignin analysis Tappi method T 222 om-88, http://.tappi.org
Koncz et al. (1994) Plant Molecular Biology Manual B2:1-22
Kosugi and Ohashi, (2002) Plant J. 29: 45 59
Lee et al. (2002) Genome Res. 12: 493 502
Lichtenstein and Nellen (1997), Antisense Technology: A Practical Approach IRL Press at Oxford University, Oxford, England Mount (2001), in Bioinformatics: Sequence and Genome Analysis Cold Spring Harbor
Laboratory Press, Cold Spring Harbor, N.Y., page 543.
Nilsson et al. (1992) Transgenic Research 1, pp209-220
Ratcliffe et al. (2001) Plant Physiol. 126: 122 132.
Remm et al. (2001) J. Mol. Biol. 314: 1041 1052
Rognes T, 2001, Nucleic Acids Research, 29, 1647-1652, 1989
Sambrook et al., Molecular Cloning-A Laboratory Manual (3rd Ed.), Vol. 1-3, Cold Spring Harbor Laboratory, Cold Spring Harbor, New York, 2001.
Slade and Knauf, Transgenic Res. 2005 Apr;14(2):109-15
Smith and Waterman (1981). Helene! DNA aligning
Thompson et al. (1994) Nucleic Acids Res. 22: 4673 4680.
Tuskan et al. Science 15 September 2006:Vol. 313. no. 5793, pp. 1596 - 1604
Vanholme et al. Current Opinion in Plant Biology 2008, 11:1-8
Varshney et al 2005. Trends in Plant Science, Vol 10, 621-630
Vasil et al. 1984, Cell Culture and Somatic Cell Genetics of Plants, Vol I, II and III, Laboratory Procedures.
Zhou et al. Plant and Cell Physiology (2006), 47(9), 1229-1240

US 2002/0168707
US 2005/034176
US 4987071
US 5107065
US 5231020
US 5543508
US 5583021
US 6451604
US 6506559
US 7402428
WO 91/14772
WO 96/06166
WO 98/53057
WO 98/53083
WO 99/53050
WO 99/61631
WO2004/097024
WO2006/078431
WO2008/067840
WO2008/067841
WO2008/006033

## Claims

1. A method of producing a plant having higher lignin content compared to its wild type, comprising altering in the plant the level of a gene product expressed from the gene comprising a nucleotide sequence selected from the group consisting of
i. the nucleotide sequence SEQ ID NO 53, 88, 105, 178 or 189;
ii. a nucleotide sequence being at least 60% identical to a nucleotide sequence from SEQ ID NO 53, 88, 105, 178 or 189; and
iii. a subsequence or fragment of a nucleotide sequence of a) or b).

2. The method according to claim 1 for producing a plant having higher lignin content, comprising over expression and/or down regulating the gene with SEQ ID NO: 178 and/or 53.

3. The method according to claim 1 or 2, the method steps comprising:
i. providing an expression vector comprising a nucleotide sequence selected from the group consisting of:
a) the nucleotide sequence SEQ ID NO 53, 88, 105, 178 or 189 or
b) a nucleotide sequence being at least 60% identical to a nucleotide sequence from SEQ ID NO 53, 88, 105, 178 or 189; and
c) a subsequence or fragment of a nucleotide sequence of a) or b); and
at least one regulatory element operable linked to the polynucleotide sequence, wherein said at least one regulatory element controls expression of the polynucleotide sequence in a target plant;
ii. introducing the expression vector into at least one plant; and
iii. selecting at least one transgenic plant that has a modulated lignin quantity compared to its wild type.

4. The method according to any one of claims 1-3, wherein said down-regulated expression is effected by introducing a genetic modification preferably in the locus of a gene comprising the nucleotide sequence SEQ ID NO 53, 88, 105, 178 or 189 encoding a polypeptide or a homologue of such polypeptide or wherein said modification is effected by one of: T-DNA activation, TILLING, homologous recombination, site-directed mutagenesis or directed breeding using a nucleotide sequence from SEQ ID NO 53, 88, 105, 178 or 189 or a fragment thereof as markers in any step of the process of producing the desirable plants.

5. The method according to any one of claims 1-4 comprising the step of providing a recombinant DNA construct comprising a nucleotide sequence selected from the group consisting of:
a) a nucleotide sequence from SEQ ID NO 53, 88, 105, 178 or 189;
b) a complementary nucleotide sequence of a nucleotide sequence of a);
c) a subsequence or fragment of a nucleotide sequence of a) or b);
d) a nucleic acid sequence being at least 60% identical to any one of the sequences in a), b) and c); and
e) a nucleotide sequence which hybridizes under stringent conditions to a nucleotide sequence of a), b) or c).

6. The method according to any one of claims 1-5, wherein the nucleotide sequence encodes a polypeptide comprising a conservatively substituted variant of a polypeptide of a) or wherein the nucleotide sequence comprises a silent substitution in a nucleotide sequence.

7. The method according to any one of claims 5 to 6, wherein the recombinant DNA construct further comprises a constitutive, inducible, or tissue specific promoter operably linked to said nucleotide sequence or wherein the recombinant DNA construct further comprises a strong constitutive promoter in front of a transcribed cassette consisting comprising a nucleotide sequence as defined in claim 4 followed by a plant functional intron followed by the nucleotide sequence as defined in claim 1 in reverse orientation.

8. The method according to any one of claims 5 to 7, wherein the method comprises the further step of transforming regenerable cells of a plant with said recombinant DNA construct and regenerating a transgenic plant from said transformed cell.

9. A plant having a having higher lignin content compared to its wild type, comprising a nucleotide capable of altering in the plant the level of a gene product of the gene comprises a nucleotide sequence selected from the group consisting of:
a) a nucleotide sequence from SEQ ID NOS: 53, 88, 105, 178 or 189;
b) a nucleotide sequence being at !east 60% identical to a nucleotide sequence from SEQ ID NOS: 53, 88, 105, 178 or 189;
c) a subsequence or fragment of a nucleotide sequence of a) or b).

10. A transgenic plant comprising a recombinant polynucleotide (DNA construct) comprising a nucleotide sequence selected from the group consisting of:
a) a nucleotide sequence from SEQ ID NOS: 53, 88, 105, 178 or 189;
b) a complementary nucleotide sequence of a nucleotide sequence of a);
c) a subsequence or fragment of a nucleotide sequence of a) or b);
d) a nucleic acid sequence being at !east 60% identical to any one of the sequences in a), b) and c); and
e) a nucleotide sequence which hybridizes under stringent conditions to a nucleotide sequence of a), b) or c).

11. A plant cell or plant progeny of a plant as described in claim 9 or 10.

12. Wood produced by a plant as described in claim 9 or 10.

13. A DNA construct, such as a vector, comprising at least one sequence described in claims 5 to 8.

14. A plant cell or plant progeny comprising the DNA construct according to claim 13.

15. Use of a nucleotide sequence selected from the group consisting of:
a) a nucleotide sequence from SEQ ID NO 53, 88, 105, 178 or 189;
b) a nucleotide sequence being at least 60% identical to a nucleotide sequence from SEQ ID NO 53, 88, 105, 178 or 189; and
c) a subsequence or fragment of a nucleotide sequence of a) or b) for the identification of plants having altered lignin characteristics as compared to the wild-type or as candidate genes in marker assisted breeding.


SEQUENCE LISTING

<110>  Swetree Technologies AB

<120>  A method for modulation of lignin content in plants

<130>  213225

<160>  195

<170>  PatentIn version 3.4

<210>  1
<211>  42
<212>  DNA
<213>  Artificial

<220>
<223>  Primer

<220>
<221>  misc_feature
<222>  (42)..(42)
<223>  n is a, c, g, or t

<400>  1
gggacaagtt tgtacaaaaa agcaggcttt tttttttttt vn                42

<210>  2
<211>  34
<212>  DNA
<213>  Artificial

<220>
<223>  Primer

<400>  2
agaaagctgg gtattgaata gccaaatatc tctc                34

<210>  3
<211>  31
<212>  DNA
<213>  Artificial

<220>
<223>  Primer

<400>  3
agaaagctgg gttccttaac ttgtccgaga g                31

<210>  4
<211>  34
<212>  DNA
<213>  Artificial

<220>
<223>  Primer

<400>  4
agaaagctgg gttagagttc gagaggtcat cttc                34

<210>  5
<211>  32
<212>  DNA
<213>  Artificial

<220>
<223>  Primer

<400>  5
agaaagctgg gtgcttagtg gtctgggttt ag                                    32


<210>  6
<211>  34
<212>  DNA
<213>  Artificial

<220>
<223>  Primer

<400>  6
agaaagctgg gtgaaggtgg tggttcaatt atac                                  34


<210>  7
<211>  49
<212>  DNA
<213>  Artificial

<220>
<223>  Primer

<400>  7
ggggaccact ttgtacaaga aagctgggtc gctgacacca ccactttcc                  49


<210>  8
<211>  50
<212>  DNA
<213>  Artificial

<220>
<223>  Primer

<400>  8
ggggaccact ttgtacaaga aagctgggta acgtgatttc aaagggtcgc                 50


<210>  9
<211>  54
<212>  DNA
<213>  Artificial

<220>
<223>  Primer

<400>  9
ggggaccact ttgtacaaga aagctgggtg taatacgact cactataggg cgaa            54


<210>  10
<211>  31
<212>  DNA
<213>  Artificial

<220>
<223>  Primer

<400>  10
aaaaagcagg ctttgttgtt gtttcgatct g                                    31


<210>  11
<211>  32
<212>  DNA
<213>  Artificial

<220>
<223>  Primer

<400>  11
aaaaagcagg ctcgagaaag aagaacctag tg                                   32


<210>  12
<211>  33
<212>  DNA
<213>  Artificial

<220>
<223>  Primer

<400>  12
aaaaagcagg ctatttgggt cattcttgct cac                                  33


<210>  13
<211>  33
<212>  DNA
<213>  Artificial

<220>
<223>  Primer

<400>  13
aaaaagcagg ctaatcagtt gcttctcatt gtc                                  33


<210>  14
<211>  33
<212>  DNA
<213>  Artificial

<220>
<223>  Primer

<400>  14
aaaaagcagg ctccatccct catcactaaa tcc                                  33


<210>  15
<211>  49
<212>  DNA
<213>  Artificial

<220>
<223>  Primer

<400>  15

ggggacaagt ttgtacaaaa aagcaggcta gaaagccagg ggagaatgc 49

<210> 16
<211> 53
<212> DNA
<213> Artificial

<220>
<223> Primer

<400> 16
ggggacaagt ttgtacaaaa aagcaggctg ctctgttgtt actctggaca tgc 53

<210> 17
<211> 54
<212> DNA
<213> Artificial

<220>
<223> Primer

<400> 17
ggggacaagt ttgtacaaaa aagcaggcta ttgattaacc atggagaaag caca 54

<210> 18
<211> 54
<212> DNA
<213> Artificial

<220>
<223> Primer

<400> 18
ggggacaagt ttgtacaaaa aagcaggcta agctgatgat caaatgagga catc 54

<210> 19
<211> 54
<212> DNA
<213> Artificial

<220>
<223> Primer

<400> 19
ggggacaagt ttgtacaaaa aagcaggcta ttaatcatgt caggtgatca aagg 54

<210> 20
<211> 52
<212> DNA
<213> Artificial

<220>
<223> Primer

<400> 20
ggggaccact ttgtacaaga aagctgggtc ctttttcccc tacaaatcat gg 52

<210> 21
<211> 54

<212>  DNA
<213>  Artificial

<220>
<223>  Primer

<400>  21
ggggaccact ttgtacaaga aagctgggtc aaggatgtat gcttggttct atga          54


<210>  22
<211>  54
<212>  DNA
<213>  Artificial

<220>
<223>  Primer

<400>  22
ggggaccact ttgtacaaga aagctgggta agaagccaaa ggacttaagc tgtc          54


<210>  23
<211>  1351
<212>  DNA
<213>  Artificial

<220>
<223>  Construct sequence

<400>  23
cgcacagaga ggatttcagg aagcgctggg gatgtgttgg atgatgatcc agtgggaagg      60

ttgaaggtct atgtttatga gcttccgagt aaatacaaca agaaactgct gcagaaggat     120

cccagatgtc tcacccatat gtttgctgca gaaatcttca tgcatagatt tctcttatcc     180

agcccagttc gaacccttaa cccagatgaa gcagattggt tttattcccc tatatacccc     240

acttgtgatc tcacacccat gggcttgccg ttgcccttca aatctcctag gatgatgaga     300

agtgcgatac agctaatctc ctccaattgg ccttactgga atcgtacgga aggggctgat     360

cacttttttg ttgtgcccca tgactttgga gcctgcttcc actatcagga agagaaagcc     420

gttgagcggg gcattcttcc gctactccag cgttctactt tggttcaaac ttttgggcaa     480

cgaaatcatg tgtgcttgaa tgaggggtca attacaattc ctccttttgc tcctcctcaa     540

aagatgcagg cccaccagat tccccctgac attccacggt ccatttttgt ctatttccgt     600

ggattgtttt atgatgtaaa taatgatcca gaaggtggtt attatgcaag aggagcaagg     660

gctgcagttt gggagaattt caagaacaat ccactcttcg acatctccac tgaccatcca     720

acaacatatt atgaagacat gcagcgagct atattttgct tgtgcccgct cggttgggct     780

ccatggagcc ctagattagt tgaagcagtg gtatttggat gcattcctgt catcatagca     840

gatgatattg ttttgccatt tgccgatgct atcccatggg aggaaattgg ggtgtttgta     900

gcagaggaag atgtccctaa cctggacaca atcctaacat ccataccacc agaggtgatt     960

ttaaggaaac aaaggcttct tgcaaatcct tctatgaaac gtgcaatgtt attcccacaa    1020

cctgcacaac caggtgatgc tttccaccaa atcctaaacg ggctggctcg taagttgccg 1080

cacgacagga gtgtttactt gaagtctggt cagaatattt tgaattggac agcaggacca 1140

gttggggacc tgaaaccttg gtaagagaag tgtcatttcc tcaacagaca tggctgtggt 1200

gtatctactg tttcatctaa tcgcaaggac tgtaaacttc tctttgaagt tttgattttt 1260

gtatagattc ttgaatttca cgtgtacatc tgggacatag ttcgatgctt taaataactt 1320

cgcatttttc ttggtatcgg caaaaaaaaa a 1351

<210> 24
<211> 813
<212> DNA
<213> Artificial

<220>
<223> Construct sequence

<400> 24
tgcagacttt gggaggtatt ctccaaatat agcaaatgtt ggcaaagatg tgattgcacc 60

atacaaacat gtaattaaaa gctatgccaa cgactcctct aactttgaca gccgcccgac 120

tttgctgtat tttcaaggag ccatatacag gaaagatggt ggctttgctc ggcaagaatt 180

attttatgct ctaaaagatg aaaaagacgt gcattttcag ttcggtagtg tacaaaagga 240

tggagtgagc aaagcttctc aaggaatgca ctcctccaaa ttctgcctta acatagcagg 300

tgacacaccc tcatcaaacc gcctctttga tgccattgcc agccactgcg ttccggtcat 360

catcagtgac gatattgagc tcccttatga agatgtcctc gactactctc agttctgcat 420

atttgtccgc acttcagatg ctgtcagaga aaagttcctc ataaatcttg tcaggagtat 480

taagaaggat gaatggacta gaatgtggca gaggttgaaa gaggtagaaa acttctttga 540

gttccaatat ccatcaaagg aaggagatgc agttcaaatg atatggcagg ctgtagcaag 600

aaaagtccct gcaattaggt tgaaggtaaa taagtttagg cgattttctc gttttgggac 660

ccctaaagat ggagtgttga gaaaaatacc atcacctagt aatttttggt gaaacattca 720

gtgtttcttt atagtgaact ttgaggttct gtttatatat aaaggtgaga aattcaattt 780

attctcactg cagattttgg caaaaaaaaa aaa 813

<210> 25
<211> 807
<212> DNA
<213> Artificial

<220>
<223> Construct sequence

<400> 25
   attagcaatg aaagaaccta tggcgaaaac agtccttgag aatggttctc agggacccaa 60
   cgtggcagaa tctgatatta tgaaatttcc acggaccgaa aggttgaata tagatgatga 120
   ccataacttc tgtaatattc cgtgtggcca aaatggtgat ggaaatctca tgtctgctgc 180
   tggaacatat gatgtatcac acttgaatgg atttgcagta ggcagccaga tgtctcttgc 240
   attgggattg caaagtaacg acagtgattc atttcccacg tttgatgggg cccatacgag 300
   aggtaacacc atatcagctt ccccggttgg gcataatgag gtggattatc actgtatgga 360
   tacaggaaag caacaagaca ggattgccag ttcccatcgg ttacatgatt ttgtggtttg 420
   agagaatcct tctagcttta cgagggagtt tcatcaatcc tgccaagaga aggattgatg 480
   gagttttgga taacataaag cagaagaaaa caagctcagt gatactggtt tgttattcat 540
   tcaagccccg tgctcttctc ttggtttgaa tagaagaaaa ccaacaccct tacagccaca 600
   gacatagatg taacataagg gcatggtgcc tgcttgtaat atacgtacat attacaggga 660
   ttgtagatat atcgatctgt gcatatacaa taagattttt gtgatctttt gtactcttaa 720
   gcaatttcta caagctctcc ctaattgtga cactgatgta tcatttcatt cctaattctg 780
   aaataaatgt gatttgctaa aaaaaaa 807

<210> 26
<211> 849
<212> DNA
<213> Artificial

<220>
<223> Construct sequence

<400> 26
agcagtcaga gcttataact agctaattga ttaaccatgg agaaagcaca cacaaaatct 60

gctcttaaga agcttgttaa ggctagctca cagtctgcat cgtggagcaa tgctgctaga 120

ggaatggcaa aagatgatct caaggatccg ctttatgaca aatccaaggt tgccccaaaa 180

cctcttgcaa aagaaaacac taagccccag gaattcaaac tccacactgg acaaagagct 240

ctcaaacgtg ccatgttcaa ctattctgtg gcaaccaaga tatatatgaa tgagcaacag 300

aagaggcaaa tagagaggat acaaaagatc atagaagaag aagaggttcg tatgatgagg 360

aaggagatgg ttccaagagc tcaattgatg ccttactttg acagaccttt ctttccccaa 420

agatcaagca ggccattgac agttcctaga gagccaagtt tccacatggt gaacagcaag 480

tgttggagct gcatccctga ggatgaactt tactactact ttgaacatgc tcatccccat 540

gatcatgcct ggaggcctgt taattaaata attgccatga tttgtagggg aaaaaggaga 600

gtggagtatt ctctctgtgc tgctaatttc ttcatgctgt ccaagttagt cttgtataat 660

ttggactttt ggagtgttga tagtgtttac tgtttagatc atgtggtagg tcttgtagtg 720

ggtgtaagta gtcgcttttt catagccgaa agtccttttc aatgggggat tttgcttacc 780

aataaggcgg atattgtact tcgttttgta ctattaaaaa gcaagtcacc ttcttccaaa 840

aaaaaaaaa 849

<210> 27

<211>  1139
<212>  DNA
<213>  Artificial

<220>
<223>  Construct sequence

<400>  27
ggaagaccta ctcaccgatg caggcatggt tctcactgca ccctctggct ccggcaataa      60

ttgaggtcaa ggcattgcat catttcgatt ggttcgcaaa ggggaaggtg ccagtcatgg     120

aagcaatgga aaaggatcag agggtgaggt cacagtttag agggggttcg tctgccattg     180

tagcaaataa cactgagaag cctcatgtta tcgcagcaaa actacaaaca cttagtccca     240

aatacaattc agtgatgaat cacatccgaa ttcatctacc tgagctgttc ccaagcctaa     300

acaaggttgt gttcctagac gacgacatag tggtgcagtc cgatctttcg cctctatggg     360

acgtcgacat ggatggaaag gttaacggtg cagtagaaac ctgtcgagga gaagacaagt     420

ccgtcatgtc aaagaagttg aagagctatt tgaacttctc tcatcctttg atatcagaaa     480

atttcaaacc caacgaatgt gcctgggctt atggaatgaa catatttgat ttagaggctt     540

ggaggaaaac cacataagca caacatacca tcactgggtt gaagagaact tgaaatcaga     600

cctgagcttg tggcagctag gaacactgcc tccaggtctg atagcattcc atggccacgt     660

ccacgttatc gatccctttt ggcacatgtt gggtcttggc taccaagaaa atacaagttt     720

ggcagatgct gagactgccg gagtcatcca tttcaacggt cgagcaaagc cttggctgga     780

tatagcattc cctcagctcc gccccctgtg ggcgaagtac atcaactttt ccgacaagtt     840

cataaagggc tgtcatatta ggccatctta acttgagttt tgagatgaaa atgttggctg     900

cgatcgtgat taatatatgt ctatatataa ggagaaaaaa agcagagaag agagagttct     960

tcacaaatcc aaggccttga atccagagag taaaatattc ataaccaagc atttgtttgc    1020

ttggtttttt tagaattcat cctctgtggg cgaacaaccc ttccctatta cttcaaatct    1080

tgattcaatt ttaatgagaa attaagcatg ttatggattt tcaacggcca aaaaaaaaa      1139

<210>  28
<211>  898
<212>  DNA
<213>  Artificial

<220>
<223>  Construct sequence

<400>  28
cgaaaaacga gatacacaag attaaccaaa ttgtagtaga tcccagcttg catcttcaaa      60

agaggagcca cctttattac gctgttgaga ctcgaacaac acctgatgct ttcaagatat     120

ttggaggttc gccctggctg attcttacaa gagctttcat ggagtactgt gtccaaggat     180

gggacaacct tccaagaaaa ctactaatgt acttcagcaa cacggcatcc ccacttgaat     240

cgtatttcca ctctgtcctc tgcaactctc ctgagtttca aaacacaaca gtaagcgatg     300

atttaaggta caatattctg gaaactacta cagatgggga atcaccttat gacaaaatgc 360

taaatggtgg agcagcattt gcaaggccat ttaaagaaga tgctgctgct ctaaacatga 420

tagatgagaa cgtcttgaac cgtgaaccca acggattggt gcctggaaaa tggtgcttag 480

accagggtat gaacaagagc tcagaggcat caaagcctcc aggggaggat ttgtgttcaa 540

cctggggtaa cattaatgat gtcaagccag gatcttatgg tatcaagctt gcatttttat 600

tgtctaagat tgccagtgaa gagaaattga caactagtca atgccttcaa gctacaaaaa 660

tggggtcatc atagaaccaa gcatacaacc tagtgtggat aatttttttc ttttaatcga 720

aaactatagg ttaaatcaag cttcttagcc atataaatcc tttgctaaat gcactacaaa 780

tgcatacttg cttgttaaaa caaaagctag aagcacccag agatatattg tacatacatt 840

cacattttat cgaggatttc tacaataaat ttattgtttt tcccccaaaa aaaaaaaa 898

<210> 29
<211> 1278
<212> DNA
<213> Artificial

<220>
<223> Construct sequence

<400> 29
   aatcactctt tcaacttcaa gctgagcgaa ttttgtgtgt ccaaagacta gcagcccaag 60
   ctttaagtga aggttctgga agctcagttt caatacattg gtacataatg accagcccgt 120
   ttactcatga ttccacacga tttttttttg aaaatcacaa gtactttggc cttgaagcag 180
   atcaggttac cttcttccag caaggcacca taccttgtgt ttccaaggat ggcagattta 240
   tcatggagac tccatttagg gtagctaagg ctccgggtgg gaatggagga gtatattcag 300
   cgctgaaata ttcaaaatta ttagaggata tggcctcaag agggatcaag tacgtggact 360
   gctatggggt tgacaacgca ctggtacgtg tagctgatcc agcttttttg ggatatttca 420
   ttgataaagg tgtagcagct gcagcaaaag ttgttcgtaa ggcatacccc caagaaaagg 480
   ttggtgtttt tgtaaggcaa ggtaaaggtg gacctcttac cgtggttgaa tacagtgagc 540
   tggatcagtc actggcttct gcagtcaatc aacaaactgg acgccttcgt tttgttggga 600
   gtaatgtgtg cttgcacatg ttctctttgg attttctaaa ccaagtggca aatggccttg 660
   agaaagacag catttaccat ctagctgaga aaagaattcc ttctattcat ggtgatacga 720
   tgggattaaa actagagcaa ttcatattcg atgcattccc atatgctcct tcaactgctc 780
   tttttgaggt accacgtgaa gaagaatttg cacctgtaaa aaatgccaat gggtcaaatt 840
   ttgacactcc tgagagtgct cggctgcttg ttcttcgact gcatactcgc tgggtggttg 900
   cagcaggtgg cttcttaaca cattcggtgc ctttatatgc aactggtgtg gaggtatcac 960
   cactttgctc ctatgctggc gaaaatctag aagccatatg ccggggaaga acatttcacg 1020
   caccttgtga gattacattc tagttaaatt tgtatccata tattcttgat ttgtacggct 1080
   cccattggaa aggggaaagg ggttatccat cccacgcatg tactgttgtg gtagattagc 1140
   gagagatatg aatctgtaag agttccaggt tttgttacat cctgggtgtg atgtcaagag 1200
   tgatactatt tgattaatgt atatcattca agttattgtt gagcaatgat aatcctctct 1260
   tcttcctaaa aaaaaaaa 1278

<210> 30
<211> 491
<212> DNA
<213> Artificial

<220>
<223> Construct sequence

<400> 30
gaaaagggtt gcaattatta caccaagaat ttccatggat cagttgaggc tttccatgta 60

gacaccgttg ataccacagg tgctggtgat gcatttgttg gtgctctcct ttgcaagatt 120

gttgatgacc aatctgtgct tgaggacgag ccaaggttga gagagatact aagatttgca 180

aatgcttgtg gagccattac aaccaccaaa aagggagcaa tccctgctct tcctactccg 240

gccgatgccc tcaaactggt gaacgaagga aaatgaactc ttttttttta atccgcactg 300

catttttccc tttccttttc agctcctggt tctcaattca atcgaagagc tgttgcaatc 360

tcaaattttc gcctttttct ctcgagtgta atgatttgaa agacttgcgc tttaaggtct 420

tgccaaataa atttttttct tttgtgttag ctttctccaa atattatatt tgtttaaagc 480

aaaaaaaaaa a 491

<210> 31
<211> 979
<212> DNA
<213> Artificial

<220>
<223> Construct sequence

<400> 31
   caaggaggta tattgagtgg agagggcggt gatgaaacca aagatattct tttattggac 60
   gtggctcccc tcaccctggg tattgaaact gttggtggag tgatgaccaa gttgattcca 120
   aggaacactg ctattccaac caagaaatct caagttttca ccacttacca ggatcagcag 180
   actactgtct ccattcaggt ctttgaaggt gaaaggagtc tcacaaagga ttgcaggctg 240
   cttggtaaat ttgatttgac tagcattcct ccagctccaa ggggaacccc tcaaattgag 300
   gtcacgtttg aggttgatgc caatggtatc ctgaatgtca aggctgaaga caagggcact 360
   ggtaaatcag aaaagatcac catcacgaat gacaagggtc gtttgagcca ggaggaaatc 420
   gagcgcatgg ttcgtgaggc agaggagttt gccgaggagg acaagaaggt gaaggagagg 480
   attgatgctc gtaacagttt ggagacctat gtatataaca tgaaaaacca gattaacgac 540
   aaagataagc ttgctgacaa gctcgagtct gatgagaagg agaaaataga aactgctacc 600
   aaggaggccc ttgaatggct ggatgacaac cagaatgccg agaaagagga ttatgaagag 660
   aagctcaagg aggtggaagc tgtttgcaac ccaatcatca ctgctgttta ccagagatct 720
   ggtggagctg caggtggtgg atcagctgaa gattccgagg acgactctca agatgaactc 780
   tgagaatctt tctgcttcct tctacccctc ctggaaagca agggcaagcg atgaggatgt 840
   aggctagatg aagtagagga tattgtaatt ttgttgatcc tagaagctaa agggttaaaa 900
   taacatatcc cctttttttg gttttttgag gttaaaaata catggaatag tttgtttcca 960
   aaaaaaaaaa aaaaaaaaa 979

<210> 32
<211> 701
<212> DNA
<213> Artificial

<220>
<223> Construct sequence

<400> 32
   ggaatggaga gctctaccgt tacatttgtg ataccaaggg agcttttgtg cagcctgctt 60
   tgtatgaggc ttttggattg actgttgtgg aggccatgac atgtggtttg ccaacctttg 120
   ctacttgcaa tggcggtcct gctgagatca ttgtgcatgg aaaatccgga ttccatattg 180
   atccttacca tggagtacag gctgctgaac tccttgttga cttctttgag aagtgcaagg 240
   ctgatcccac ttactgggac aaaatctccc agggaggcct gcagcgaatc caagagaagt 300
   atacctggaa aatttactct caaaggctcc tgactctcac aggagtttat ggcttctgga 360
   agcacgtttc caaccttgat caccgtgaga gccgtcgcta tctggaaatg ttctatgcac 420
   tgaaatatcg caaattggct gattctgttc ctttgactat cgagtaaatg gaactggaga 480
   aatcgaggaa acatgggttg gtttgagtcg ggttccgggt ccagaataat ggtcatttca 540
   cgatagtgat tggacaagaa aggctttgat cttctttttg tttttttgtt tttcctttct 600
   gtgtgcatct tttttgtttt cccttttctt ttcatcatgc agcctcttca atttcagtaa 660
   agattgtgtc tgttgagcta aaaaaaaaaa aaaaaaaaaa a 701

<210> 33
<211> 1439
<212> DNA
<213> Artificial

<220>
<223> Construct sequence

<400> 33
ctccaatcac gcgtccgctc tacacaggtc gatccaaagc aattttctcc attacaagag 60

aagccaagaa tttgtgacga acttggtata aattagtttt gttttgacaa gagaaagact 120

tgtttgttta atttccatgt gtctgaggag ggaacgaaga acaaccccag ggtgcttttc 180

attgttcgga cacctcttag attctcagac aacttgaaca caaaatattt cccgagttcc 240

aggatcattt tgaaggatac aaggaaagag gaataaaaag aaagaacaat ggcattcacg 300

ggaaccctgg ataagtgcaa ggcttgtgat aagactgttt attttgttga tatgatgtct 360

cttgaagggg tgccttatca taaatcctgc ttcaaatgca gccattgcaa aggaactctt 420

gtgatgagca actactcatc catggatgga gtcctctact gcaagactca ttttgagcaa 480

cctttcaagg agggtggaga tttcagcagg aattttcaga aagggaagcc cgagaggact 540

catgacctgt ctaggatccc cagcaaacta tctccagttg ttctgtggaa cccaggacaa 600

atgctccact tgcggcaaaa cagtgtaccc tttggagaag gtttgttttc agctctaaac 660

ttaaataaat ccattaggtc ctaaagacct gtgtgctatt cataccccta catttatcag 720

ataaatgacg aacatatttt aggtacatct tctctatggt agatggatct atctgagaaa 780

tgttttcaag tctctgttat ttaatagcca tgcctgtgat aatctggaat aagcacttaa 840

gtgctataac cattgatgac ataggttacc atggagggag aatgttacca caagacatgc 900

ttcaggtgtg ctcatggtgg atgtccactc acacactcat cttatgctgc tcttgacgga 960

gtcctctact gcaaggtcca cttcgcccag ctcttcatgg aaaagggaac ctacagccat 1020

gtccttgcat cagctgcaca taagagatca aattcgacac ctccggaact ggccgggtca 1080

aacccagagg aaggagctgc tgcggaggag gaaaagtcag aagagcaatc ttagacaaag 1140

cacatcatgt tttcttgcat tgctatcttt tcaagttgcc aggactgctt cgagttttca 1200

tgttgttgaa tattattgcc ggattgtgtt caaagtttgt ttttggattt gactatctat 1260

gttacaatgc cttgctacag gatattgaat tgcactagtt tttacaaatt cctcctcttg 1320

caatttaata ttcttggcaa caggatacaa atgaaaaacc caaatcctag gaaccagtat 1380

caacattcat tgatctcagc tctttgctca gatggattgc gttttccaaa aaaaaaaaa 1439

<210> 34
<211> 792
<212> DNA
<213> Artificial

<220>
<223> Construct sequence

<400> 34
cttgttcgat ccctgcacta atcctgatta atgcctgcat tcccttttag attttgtgtt 60

tgattttgtc ctgtaaggag ctggatatgc gggatcatat ggagagattt gtagttcttc 120

cattctccat cgcctgtgct tctcactcca gtgttgatgt ggcctccagt gaatcctcca 180

agaaaccaag acccgaaacc aaatcacatg catcaagagg acaagaaggg gaggaaagct 240

cttgtaaaga aaagacgaag aacagtacac ttggtttcct gctggctctt ccaaagcctt 300

gcatatccag tagcttgcac aaattgatta gaggcatcaa gactctctcc caagtatttg 360

tgtacaagga agaagacgag gagctaatgg aaagagagat ggaaatcgga tatccaactg 420

atgtgaagca tgtaacacac ataggattgg atggaactac gatgacaaat cctataaagg 480

gctgggattg tctgaaatct ccagaaataa ttccattccc ttcatttact ttaaggcagt 540

tcgagcttgc aatggctgca caagctcatg gacctcttgt tggggtcgat cattccaagc 600

ttgtttgatt cattgatttt tcttttcatt tcctgatctt gtttctttga cactagatga 660

ctgatgtgat gaagattgat caatgttttt gatggaggca ctggttgcag tgatgtgttt 720

ttggtgtttg tgtgggacct tgacaatgtt tttctgggtg gccattgaaa ttgttcttgc 780

aaaaaaaaaa aa 792

<210> 35
<211> 823
<212> DNA
<213> Artificial

<220>
<223> Construct sequence

<400> 35
gctagctagc tttgcagagt tctgtcgatt caatggcgac caaagtgtat attgtgtact 60

attccatgta tggacatgta gaggaactgg cagaagagat taagaaaggg gcttcatctg 120

ttgaaggtgt tgaggcccaa ttatggcagg ttcctgagac actgccagaa gaggtgcttg 180

gaaagatgag tgcaccacca aagagtgatg taccagtcat tacacctagt gaactcgctg 240

aagctgatgg ctttgtgctt ggattcccga caagatttgg aatgatggct gcccaattca 300

aagcttttct ggatgcaact ggaagtctat ggagaacaca gcaacttgct ggcaagcccg 360

ctggaatctt ctttagcact ggatcgcaag gtggtggcca agagaccaca gcgttgactg 420

ctattaccca acttgttcac catgggatga tatttgttcc cattggctac acatttggtg 480

ctggcatgtt tgagatggag aaggtgaaag gtggaagtcc ttacggcgca ggaactttcg 540

ctggggatgg gtcgagacag ccaaccgagc ttgaattgga gcaggctttc caccagggca 600

agtacattgc tgccatcaca aagaagctca aaggagctgc ttaagtttac tcaattacaa 660

gaatacggtc tggtcttcca ttttttcacc ctatagaata tgcatttgca aagaataatt 720

cgtattccac cgtttattat atgcttttca tgtgtgtaac atgatagtga ttattgtctc 780

aatgatatca aggttggttg tgttttcatt taaaaaaaaa aaa 823

<210> 36
<211> 1113
<212> DNA
<213> Artificial

<220>
<223> Construct sequence

<220>
<221>  misc_feature
<222>  (579)..(579)
<223>  n is a, c, g, or t

<220>
<221>  misc_feature
<222>  (639)..(639)
<223>  n is a, c, g, or t

<400>  36
gcggacgcgt gggctctttt ctttctttct ccatcaccca cgcgttttct tagtttcaat      60

tcttgtcaaa atagtcttga tcattttctc tgcaaaagaa attaatcatg tcaggtgatc     120

aaaggcctaa agattctgct gaaggctctt cgcgatctgg aggcgatcac caccagcttc     180

aatcacctcc tttgagccgg tatgagtcgc agaaacggcg agactggaac actttcgggc     240

agtacttgaa gaatcagaga cccccagttt cattatctca gtgtaattgc aatcatgtgc     300

ttgatttcct tcggtatctt gatcagtttg gcaagactaa ggttcatcac catgggtgcg     360

tcttctttgg acaacctgat cctcctgctc cttgtacatg ccctctaagg caagcttggg     420

ggagccttga cgccctaatc ggacgccttc gagcagcttt tgaggagcat ggaggatcag     480

cggagactaa cccttttgga aatggagcta ttcgggttta tttgcgtgaa gtgaaagagt     540

gtcaagctaa ggcaagaggg attccttaca agaagaagna gaagaagaag actcaaatag     600

gcccagagac gaagcaaagc cttcgatgca gacagcttng tcctttggct tcttccgata     660

aatggatcac aaaataggac aaatggaggt gaagttccag aaaggcaaat gttgggggcc     720

tagcctctgc ctcagtactt aatatatagc tagctagctt ctctctttgc agatagtgtt     780

acatcacagc ctacttctaa ataatcttgt cctcgcaggt tggtctaaga agctattcct     840

agcttatagg aattaaaact gtagtcgtaa tgttcatcga cttcttaaat agtaagacgt     900

tagcactata tgtccagctc tgttgtttct ttgctgcaga agaaaactag tgtcttcagc     960

taatggtgaa aatggctttt agcttctgcc caaatgtagc tttgggcctg cctctcatta    1020

catgagctgt gctttctagt aatccttcaa ttgtccaaag taccatgttt ttcttcatgt    1080

ttaatctcta gttaatttcc taaaaaaaaa aaa                                 1113


<210>  37
<211>  991
<212>  DNA
<213>  Artificial

<220>
<223>  Construct sequence

<400>  37
cgcaatccat cctatggtgc tttatctgac tatcagttta tgcgaccccg cgcacgtggc      60

aagaaacttc ttgaagagtg ggctgccccc ttgaaaagtg ttgaggatat atacgagaaa     120

ttcaaggtat actgccttgg gaagttgaaa agtagtcctt ggtcagaact agatggcctt     180

cagccagaga ctaaaatcat taatgaacag ctgggtaaga tcaacttgaa gggactcctg 240

actatcaata gtcaaccagc tgtcaatggg gaaaaatctg attctccatc tgttggttgg 300

ggtgggccag gagggtatgt atatcagaag gcttatctag agtttttctg ctccaaggac 360

aagctgaatg ctcttgtgga gaagtgcaag tctttccctt ttgtaactta cattgctgtg 420

aacaaaggag ggagttggat ttctaatgtt gccctgactg atgtgaatgc tgtaacctgg 480

ggagtcttcc cagcaaagga gattatccaa ccaactgttg tggatcccac cagcttcagt 540

gtgtggaagg atgaggcatt tgaaatctgg tcaagaggat gggcttcctt gtacccagag 600

ggtgacccat ccagaaccct acttgaagag gtgaagagca gctacttttt ggtcagcttg 660

gtagataatg attacatcca tggggatatt ttcgctgtct tcgccgattt atgatgcaag 720

gggctctttg caacccctgc atttgctaag agtatccaag tccccagaaa aatggtggtg 780

ctttacccta agttgaatcc cagctttaat catcttatcg gcatacattg tttttgaaat 840

tatttccgta ttgaaatgta ataattcatt gtgattttgc tactcccctc tccattatat 900

gctgcaaatt tgtatttgga gtggcaacta attctaagct ttaagaaaat atgattgcct 960

ctttttcttg gtacttccga aaaaaaaaaa a 991

<210> 38
<211> 1273
<212> DNA
<213> Artificial

<220>
<223> Construct sequence

<400> 38
ctctgctttc tctatctaga aaacatatct ctctttttcg tccctcacgt tcccttgcaa 60

tggcttccag tggagtgaac gacaagtcac tgatcgtcag cttcggggag atgctgatag 120

acttcgtacc aacggtctct ggcgtctccc tcgctgaagc tccggggttt gtgaaggcac 180

cgggtggtgc tccggcgaac gtggcgatag cggtggcgag gctgggagga aaggcggcat 240

ttgttggaaa acttggtgat gatgagtttg gcaacatgct cgccggaatt ttgaaggaaa 300

acggcgtgat cgctacaggg ataaattttg acacaggtgc taggactgct ctcgcgtttg 360

ttactctacg cgctgatgga gagcgtgagt ttatgtttta tagaaatcca agtgctgata 420

tgctactaag accagaagag ttaaatcttg agttaattag atctgctaag gttttccact 480

atggatcaat aagtttgatt gtggagccat gcagatcagc acatttacag gcaatgaggg 540

tggcgaagga tgcaggtgca ttgctttcgt atgacccaaa tctgaggctg ccattgtggc 600

catcagaaga ggaggcgcgt gagcagatat tgagcatctg ggacgaggca gatgtgatca 660

aagtcagtga taatgagctt gagttcctca ctggaagtga caaagttgat gatgaaactg 720

catgtcactt tggcgcccta actttaagtt gctcttggtc acccttggtg aaaagggttg 780

caattattac accaagaatt tccatggatc agttgaggct ttccatgtag acaccgttga 840

taccacaggt gctggtgatg catttgttgg tgctctcctt tgcaagattg ttgatgacca 900

atctgtgctt gaggacgagc caaggttgag agagatacta agatttgcaa atgcttgtgg 960

agccattaca accaccaaaa agggagcaat ccctgctctt cctactccgg ccgatgccct 1020

caaactggtg aacgaaggaa aatgaactct ttttttttaa tccgcactgc atttttccct 1080

ttccttttca gctcctggtt ctcaattcaa tcgaagagct gttgcaatct caaattttcg 1140

cctttttctc tcgagtgtaa tgatttgaaa gacttgcgct ttaaggtctt gccaaataaa 1200

tttttttctt ttgtgttagc tttctccaaa tattatattt gtttaaagca aaaatttctg 1260

caaaaaaaa aaa 1273

<210> 39
<211> 803
<212> DNA
<213> Artificial

<220>
<223> Construct sequence

<400> 39
cttcgtcatg ggggagaagg agcccaagaa agctgaatct gagggagcct ctctgcctac 60

acaagttcaa gaacatggac ctgttaagga agagaaagaa gtccctttaa atgattctgc 120

taatgagaag agctcggtcc tagttactga aaaggttgca gatccacctg ctactgcaaa 180

aaactccagg gggtcaaatg acagagatgc tgtgcttgcg agggttgaag cggagaaaag 240

atgtgctcta cttaaagcat gggaagaaaa tgagaaagct aaagcggaga acaaggctca 300

caagaaactc tctgccattg gatcatggga gacaatcaag agagaatctg tggaggcaaa 360

aataaagaag tatgaggaaa aagtggaaaa gaagaaggct gaatatgcag agaaaatgaa 420

gagcaaagta gccgaactcc acaaggcagc cgaggagaag aaagcaatga ttgaagcaaa 480

aaaaggggag gaccgtctca aggtagagga aactgcagcg aaattccgag caactggtta 540

tacaccaagg aagtgtctaa gatgttttgg caaattctaa tgaacaatag tggggtaaac 600

ggaagagaaa ggctgctaga attggatttg cttgattttt tggcttgtta ttttatgctg 660

tgctcaaata ttgttggttc agctgtaaat gtcttttgat atttgtaaaa accaataaat 720

gggagtgcat gtgttacatt acccaagttc tgacaagacc cgagtttgaa tttgatattg 780

tcttttgtgc gaaaaaaaa aaa 803

<210> 40
<211> 1116
<212> DNA
<213> Artificial

<220>
<223> Construct sequence

<400> 40

caaacaagca ctggcactgg caatggcaat gccacttctt cttcaactac caccaccact 60

tcttcttctt caacatcttc aagttccatg agcagtaata acagtaatgg caacaatggt 120

tgtcagcaga accaaagtaa ccagcttgga gagacaagat caagtcttta ttattcaaca 180

aatgcaatgt cttttgttac caagtctttg ttgcctacta gaagaagact caggcttgat 240

cctcctaaca agctcttctt cccttatgaa ccaggaaaac aggttaggag tgccattggc 300

ataaaaaaca ctagcaagtc tcatgtagct ttcaagttcc aaacaactgc accaaagagc 360

tgttacatgc gtcctcccgg ggctatactt gcacctggtg aaagtcttat tgcaactgta 420

ttcaagtttg tggagcctcc agagaacaac gagagactat ttgatcagaa gagcagggta 480

aagttcaaga tcatgagctt gaaagtgaaa ggagaaatgg aatatgttcc tgagatgttt 540

gatgagcaga aggatcaagt agcagttgag caaattttac gagttgtttt tcttgatcca 600

gaatgcccta gccctgcact ggaaaagctt aaaagacaac tggctgaggc tgaagctgag 660

ctcgaagcac gcaagaagcc tccagaagat gcaggccccc gagttgttgg cgaaggactt 720

gttatagatg agtggaaaga gcggagggaa agatacctgg cacgccagca ggttgaagtt 780

gattctgtgt gaagatcctg ttttccttgt aagcacttgt tttttcttga gctgaggttt 840

ccttcatgca atgttgaatg ctttgcctct cggtccgttg gtggaggcgg atgtgtgata 900

agtccggtcc agttaggggt gcagacataa gatttggaaa gcaggattta tcagcaaagc 960

ctgtaaaaaa aaggtaaaat caaggcattt gtcatgtcat attaagctgt aaggcactta 1020

attatgtact tgtgccctat ttattgtaga tatgggattt tgattaacga tgaattagtt 1080

aaatttgtgt tgtgaaaaaa aaaaaaaaaa aaaaaa 1116

<210> 41
<211> 868
<212> DNA
<213> Artificial

<220>
<223> Construct sequence


<220>
<221> misc_feature
<222> (586)..(586)
<223> n is a, c, g, or t

<400> 41
   gggccttata gacaaagatg tggtgaggac tgatagagca ctgtctttct atgatgggga 60
   tgataatcca aatgtgaata tcttacgtga tattctgttg acgtactcct tctataactt 120
   tgatcttggt tactgtcagg gtatgagtga tttgctatcc ccaattttgt ttgtgatgga 180
   ggatgaatca gaatcgtttt ggtgttttgt ggcactgatg gaacgtcttg gacccaattt 240
   taatcgtgat caaaatggca tgcactctca gctttttgca ttatccaagc tggtggagtt 300
   gcttgattgc ccattacata actatttcaa gcagaatgac tgcttgaatt acttcttttg 360
   tttccgctgg gttctaatac aatttaaaag ggaatttgaa tacaagaaaa caatgcgatt 420
   gtgggaagtg ttgtggaccc attatttgtc tgagcatctg caactgtatg tatgtgttgc 480
   aatcttgaag cgataccgca acaaaataat gggggagcgc atggactttg acacactctt 540
   gaaatttatc aacgagctga gtggccatat tgaccttgac gcaatnctta gagatgcaga 600
   ggctctgtgc atatgtgctg gtgagaatgg tgctgctcgc atcccaccag gaactccacc 660
   ttcattgcct actgagaatg agaatgcttt attatatgct caagatgatg aagtactgta 720
   atacagttcg tctcattggt acgatataat tggctttata ttttgttttc ttcatgaaat 780
   agctgtgtat aattgctgtt ttttcgaaat aaaatgttaa taatcaagca atcaactcca 840
   tttgctgacc tccttgaaaa aaaaaaaa 868

<210> 42
<211> 752
<212> DNA
<213> Artificial

<220>
<223> Construct sequence

<400> 42
gttcaataag ataagatcat caccacctcc aaaattcaag ttcttacagg atgcggatga 60

gaaactacat agaaggaaat tgatgcaaga agctggggag aaggtccaaa ggcatgatgt 120

ttttgctcag gatcatacca aaattcctgc tagttcaaat tcccacaaag acgaggatga 180

tgggcctttt atcaccatca ttattgacag gaacaaagaa agagagctta acgaacaaaa 240

tcatcaacta ccagactatc agtcaagcac ttcacaggta cttcctcttg ctactgcacc 300

ttcaacatca aaatcagcag ccaagaaaag ttccttcttt cattagatct ccgtgatcga 360

gtaaaacgag atagtttttt tttttttttt tcctatgtat ttctattaac caactttagt 420

cttcttttta ggagttcttt ttctttaatc tttctatggg atggtgttag aaggttaacc 480

ttgccaaacc cttttgcaat gtctgaaaaa tcttcgggtg ggtggggagt tggacacact 540

ctctgcagta taatgttaaa agttggacaa gtgcaggtgg gggtcttgag ggagtaatct 600

gttgtattca ctcttcatat atctctgagc tgtctgatat ctgtaatcat tctggtgagt 660

ggtggtacta ctgatgtttt tgtccaaaag gcactgtaag aattctttca gaccatgcac 720

ttctttattt atttttctaa aaaaaaaaaa aa 752

<210> 43
<211> 939
<212> DNA
<213> Artificial

<220>
<223> Construct sequence

<400> 43

tcgtacgccc acgcgtccgc acctaggcgc gcaccaaaga gggttaaatc aacactgaag 60

atcgataagt tggacgatat tcgcaatgaa tggattgatt ctgatattct aatatttaat 120

tcagggcatt ggtggacacc gagtaaactc tttgaaatgg gctgttattt tctggtaggc 180

agatcgctga agctaggaat gcctatcact gctgcctttg aaagggcact gcatacatgg 240

gcatcatggc ttaacactac cataaatgca aatagaacaa gcgtgttctt ccgcactttt 300

gaatcttccc attggagtgg ccggaaccgc ctttcttgca aagtgactcc gcagccttca 360

tcaagaactg gagggaggga tcgtagccca atatctgaca ccataatcaa ggttgtgaag 420

gcaatggccg ttcccgtaac agttttgcat gtgacaccca tgggtgcatt ccggagtgat 480

gctcatgttg gcacttggag tgacaatcca tctgtccctg attgcagcca ctggtgctta 540

cctggtgtac ctgatatgtg gaacgagatt ctcttgtcaa atatgttgtc caggaattaa 600

gaggccattc tttccataag ctagaaaatg caactgtgta gtgtgttcca tattcttccc 660

tcttcaattg accccattca atttccaaac cttctactca actactttca gtacatacga 720

agatcttctc tgctgagaag ttgtgtgcat ttgtcacaaa tattgtttgt tgtgtagttc 780

agggatccaa aaattacttg atttgtctgt tctagttgca cttgagttca catgtaatgg 840

taatgatttc tctccagcgt tagctgaaat ctggaagctc ttctcttcag atgccaccga 900

gctgttgtac aataaaagcg ttattgcaaa aaaaaaaaa 939

<210> 44
<211> 556
<212> DNA
<213> Artificial

<220>
<223> Construct sequence

<400> 44
ggggaagttt tagaagctgt cataatgaag gatcggccca cgggtcgtgc tcgaggattt 60

ggttttgttg ttttcgcaga ccctgctgtt gctgagagag ttgtgatgga aaaacacctt 120

atagatggta gaaatgttga ggcaaagaaa gcagttccta gagaggatca gaacaccctg 180

aacaaaaaca gtagcagcgt taatggttca cctggccctg cccgaacaaa gaagatattt 240

gtaggaggtt tagcatctac agttacagag agtgacttta ggaagtactt tgatcagttt 300

ggagtgatta cagatgtggt agttatgtat gatcacaaca ctcaaaggcc aagaggtttt 360

ggattcatca cctatgattc agaggaagca gtggatagag tattgcacaa aacctttcat 420

gaactcaacg gtaaaatggt tgaggtcaag cgggctgtcc ccaaagagtt atctccaggg 480

ccaactagga accagttagg ggggtttaat tatagtccta gtagagtcag tagcttcctc 540

aatggttaca ctcagg 556

<210> 45
<211> 580

<212>  DNA
<213>  Artificial

<220>
<223>  Construct sequence

<400>  45
tggtgctgga gattttgata ttggtctagg aggggtaggg tatgggagga acaataggac      60

agctgttgca ccagcatcat ctcatgctgc atctaatggt ggttatgatg gggcttatgc     120

agacttttat gagaagggct cattatacgg ggataatacc tggcaatctt caccttcgga     180

actggatgtg tctggctcat ttggttttgg gcttggaaat gcaacttctg atgttatgac     240

taaaaattct gctggttatg ttggtggtta tagtgttgct aatagacaat caaatagagg     300

aattgctgca taggatgatt atgatatagg tgacggtgaa gtgaatctcg tacatgtttc     360

ttgttcagat gatcaagttt caactttgct catcatatag agcagtcaca aggaaatctg     420

tgtatggttt gagcttttga gatttttcta atataggttt tctttcttgg aattttatta     480

tgtaaaatca cattgtgggg tatactcaac atgattgtat gatgcatctt tggtgacagc     540

tatattaaaa aaggaaacaa tttttagcaa aaaaaaaaaa                           580


<210>  46
<211>  1441
<212>  DNA
<213>  Artificial

<220>
<223>  Construct sequence


<220>
<221>  misc_feature
<222>  (967)..(967)
<223>  n is a, c, g, or t

<400>  46
ctctttactt gtgcacgctt aaagaagaga tcacacaatg gcaaaccctt ctctctgtct      60

cctcttcctc ctctctctcc tgaccccagc cctcgtctcc tcttccccgg ttcaggaccc     120

cgaacttgta gttcaagaag ttcatagggc catcaatgcc tctaggagga agttgggata     180

tctctcttgt ggaaccggca accccattga tgactgctgg agatgcgatc ccaattggga     240

gaagaaccgc cagaggctag ctgattgtgc gattgggttt ggtaagaatg ccattggtgg     300

aagaaatggt aagatttatg tggttacaga atctggtagt gatgatcccg tgaaccctaa     360

gccagggact ctcaggcatg ctgtcattca agaagagcca ttgtggatca tttttgctcg     420

tgacatgaca attcaattga aggaagaact gatcatgaac tcgttcaaga ctatcgatgg     480

tagaggtgcg agtgtccata ttgctggtgg tccatgcatt actatacagt atgttaccaa     540

cattattata catggactaa acatacacga ttgcaagaga ggagggaatg ctatggtgag     600

ggactcccca aaccactttg gatggaggac tgtatcagat ggtgatggtg tgccatcttt     660

ggtggtctca catctgggtg gaccataatt cattgtcaaa ttgtaacgat ggactcgttg 720

atgccatcca tgggcctcag ccatcaccat ttcgaacaat tacatgaccc accatgataa 780

agtcatgctt ctggggcata gtgattccta tactcaagac aagaacatgc aagtcaccat 840

agccttcaat cactttggag agggtcttgt ccagagaatg ccaagatgca gacatggata 900

tttccatgtg gtcaacaatg actacaccca ttgggaaatg tacgccattg gagggagtgc 960

tagcccnacc atcaacagcc aaggcaacag atttgtagca cctgacatca ggttcagtaa 1020

agaggtaaca aaacatgaag atgcaccaga gagtgaatgg aagaattgga attggaggtc 1080

tgaaggagat ctactgttga atggagcgtt tttcgtagca tctggcgcag gcgcttcatc 1140

aagctatgct agggcatcaa gcttgggtgc aagaccatct tcacttgtcg gtccaatcac 1200

gatgggggca ggtgcactta actgcaagaa ggggggtcgt tgctagctga ttgtgaaaga 1260

ttacacaata ttttgtgact agtaatttgg actataatta attagctggc tagaaaagta 1320

attaagcaga agtggaatta aaaaatgagg agagtgaaga aaagtccgag cactagtttc 1380

tcctggcttt ttcttccctc caaattttta ttctttttac ccgaaaaaaa aaaaaaaaaa 1440

a 1441

<210> 47
<211> 650
<212> DNA
<213> Artificial

<220>
<223> Construct sequence

<400> 47
cgagaccagt ggccttttct atccaagaat caagcatgga taaaacagaa caagatcttg 60

attcttgttc tgttgtcaac accaatgtat catcaccaga gttcttcaac gccttcgagt 120

ttatttcctc gatgtcatcc ggctttgatc tgtctggtct ttttgagact aagaggaaat 180

caagctcaat gttcacttca aaattttcag cgagtgcgat catggaaaaa attgaagggg 240

ttgcaagggg gctgagttat aaggtggcaa aagtcaaaga tttcaaagtg acgttacaag 300

gtccatgtga ggggagaaag gggaagctgg cggtgacggc ggaggtgttc gaggtggcac 360

cggaggttgc ggtggtggag ttctccaagt cttccggaga taccttggag tatactaggt 420

tctgcgagga agatgttagg cctgcactaa aagacatcgt ttggacatgg caaggagaca 480

atgcttgtaa tcaagataac aacaatagtc atgtagaaga tcctgagatt caaatgttgt 540

agaaaggctt tgatcttaat ttggtgagtt ttaggatttt atataatctg taaataagtt 600

tttgttattt aattaatgca tcaagttgag tgttcttggg aaaaaaaaaa 650

<210> 48
<211> 957
<212> DNA
<213> Artificial

<220>
<223>  Construct sequence


<220>
<221>  misc_feature
<222>  (214)..(214)
<223>  n is a, c, g, or t

<400>  48
cccacgcgtc cggtattttc tgtggaggag aaacaaaaag caatctcttg ggtttttgtt      60

cttttaaaga tttggtttgg gttctctctt tgaaagttat caagaacagt aaaggggctc     120

ttgattgact gatgcaagtt taagattttc tattcttttt agagaaagaa agaagttttc     180

ttttgaaatg aatttgatgt gtgaggtttt cttngctgaa gaagaagaga aggttgaagg     240

agagtgggaa aaggagagat agtatttacc aaagccaagg tagctagcag aagtagagag     300

gcagatagca ataaactaca aaataaaatt tcatctaagg ggttttagtt tgttctgctt     360

ctgctgggta attggcaatg tctgctacta taatcagcga ccctatggtg gtctctgcac     420

cggagacaca agcaacagca gcagccgcag ctgcggcaac gacagcaaca caactcattg     480

cacagataga ggttgagtcc gtcagatgtg attgctgtgg cctaatcgaa gagtgcacgc     540

cagaatacat tgaaagagta cgcgaaagat atcatggaaa atggatttgt gggttatgca     600

cggaggctat aaaatatgaa attgtaagga ctgagaggct tattagcact gaagaagcaa     660

tgacaaagca catgaacgtc tgcaagaagt ttgtctcctc agggccacct cctgatccaa     720

caactcattt gatagccgcc atgaggcaaa tcctgagaag aagcttggat tctccgagag     780

gttcaaggtc aaccccaagt agtcctaaca aaacgaacgg agcaatccgt gtcgctgcac     840

ttgctaggtc cgaaagttgc ttccctgctt tgtctggttg atagataatt ctgagcaccg     900

tggaagacgg ggtgattcat aaaatggtga tgaagctatt ggaagcaaaa aaaaaaa        957


<210>  49
<211>  1020
<212>  DNA
<213>  Artificial

<220>
<223>  Construct sequence

<400>  49
gtcaagataa agtcccattc tttttcctag ccccaagttt gtttgagggg tgataaaaaa      60

tgaaccaaga gatgaatggt gttgacactg agattgatca gaaccaccaa gagaatgtgc     120

aagagaaaat cgattatgtg tttaaggtgg tggtgatcgg tgactctgca gtgggcaaga     180

cgcaaattct ttccaggttt accaagaatg aattctgctt tgattcaaag tctaccatcg     240

gtgtcgagtt cccagactag gactgtcatc attaaagaca aggtcatcaa ggctcagatc     300

tgggatactg ctggccaaga aaggtaccgg gcagtgacaa gcgcatacta tagaggggca     360

ctaggggcca tgttagtcta cgacattacc aagagaccaa cgtttgatca tgtggctagg 420

tgggtggagg agctccgagc ccatgctgac agctcaattg tgatcatgct gatcggaaac 480

aaggctgatc ttgtggacct cagggcagtt ccaacagaag acgcggtgga atttgcagag 540

gatcaaggcc tctttttttc tgagacatca gcccttagtg gtgacaatgt ggatggtgca 600

tttttcaggc tgctagaaga aatttacggt gtgatttgta agaagtcatt ggaatgtggc 660

aatggaaaac cccatgctgc tgatgccata acgcttagag gttctaagat tgatggcata 720

tcagggacgg atctggggat tagtgagatg aagaaattat ctgcttgctc gtgttgattt 780

gatcatttct cttgtgaatt gtgtactata agacttcacc actcccatgt tcttaattga 840

ttctgtggct ttctttggaa agtggtgatc ggtcgtgtgg tgagggtggc aagttttttc 900

ttttctgtga cctgtcaaga ttttagcagt attgtacttg tcttacagaa cccatgaatt 960

tggttttttt atatgtattg atttggatgg atggttttcc ttttcctctg aaaaaaaaaa 1020

<210> 50
<211> 1160
<212> DNA
<213> Artificial

<220>
<223> Construct sequence


<220>
<221> misc_feature
<222> (449)..(449)
<223> n is a, c, g, or t

<220>
<221> misc_feature
<222> (557)..(557)
<223> n is a, c, g, or t

<220>
<221> misc_feature
<222> (585)..(585)
<223> n is a, c, g, or t

<400> 50
atgaactaga aattcaaagc ttccaacaca tattttaatg gcttacaatc cttcttcaag 60

gtcttgttct tatgattctt tttgtttcct attcattaca ttcgttcttc catatttctt 120

cctcacaaaa gctcaagctg caagccatgg tagaacctca tgtggtacca ttccaataaa 180

ctaccctttt ggcatcgatg atggctgtgg cagtccatat tacaggcaca tgcttctatg 240

ctccgattcg ggcattctcg agcttcgaac gccttccggg agataccatg ttcgtagcat 300

aagctactca gaccctcaca tgatagtcac cgatccattc atgtggaagt gtcaagatgg 360

tcatcacttt cgtgcaacta gggcatttag ccttgatgca agcacacatt taacactctc 420

ctctcaaaat gactacctct tcttcaatng tagtgaagag aaagtgattg ttgagccaaa 480

acctatcttc tgcgagaggt ttcctgatcg gtgcgactcg acatgtgata gtgctagtta 540

cctttgcagg cacttgncca ggatgtggtg ctgcattagg aggangttct tgctgctctt 600

acttcccaaa agcgaccgaa tctttgaggc tgatgctgaa gtattgtgct agttacacta 660

gtatttattg gagaattaat ggtgcaaatg ctcctgatga tcatgtacct gagtatggta 720

ttagagttga ttttgacatt ccagtgacta cagattgcct tcaatgtcaa gacatgaaga 780

aaggaggtgg aagatgtgga tttgacacaa aatcacagaa tttcttatgt ctgtgcaatc 840

agagatcaaa tgtcacaaca tattgcaatg gtatatcaca gcagcagtag ccgtagcaag 900

gcaggaataa ttgcagggac tgtaactgga gtttcagctg ctggggcctt aggaattggt 960

gctggtctat ggtattggaa gaaagtgaga gcctcagcac cagtaacgtg tggggttcaa 1020

agcaatgaga ataggctctt ttaagaacaa atcaacaaaa gtgaatacta atcactttct 1080

cttttctaat ttccctattt tttgagttgc ctttcgcact ttaataagca atttaatgga 1140

tctgttttct ttgttttaaa 1160

<210> 51
<211> 896
<212> DNA
<213> Artificial

<220>
<223> Construct sequence

<400> 51
cattatccca aaaaacgagc aaggcagata cagcagccaa aaccatgtca cccggtcccg 60

tcgttgaggc tgaacccgcc gccgccgtgg aagatacaca gaagaagaag cctcctcagc 120

aggacgtgga tgaacctgtg gtggaggacg tgaaagaaga tgagaaggaa gaggacgacg 180

acgatgatga tgaagacgac gatgatgaag atgatgacaa ggatgatgat accccaggtg 240

ctaatgggag ttccaagcag agcagaagtg aaaagaagag tcgcaaggca atgttgaagc 300

ttggcatgaa acctgttact ggtgttagca gagtcaccat caagagaacc aaaaatatac 360

tgttttttat ctcaaagcct gatgtcttca agagccaaaa ttctgagacc tatatcatat 420

ttggagaggc aaagatagag gatttgagct ctcagctgca gacacaggct gctcagcagt 480

ttagggtgcc agacatgtca tctatgctac caaaatcaga tgcttctact gcagctgctg 540

ctgcaccagc agatgaagaa gaggaagaag tcgatgagac tggggttgag cctagggaca 600

ttgatcttgt tatgacacag gctggagttt ctaggagcaa ggctgtcaag gctctccaga 660

cgaacaatgg ggacattgtc agtgctatca tggagcttac tacataggtt ggctccctgg 720

ttactctcct attttctgct cacaagttct tggaacaatt taatcatggt agtcacattg 780

gcttgccatc tatgaggtcg ctaattatcc attgtttgtg tcaaatttga gattattacc 840

tattgcggtt tttctttagt agcaagctct tattgtgctc tttgcaaaaa aaaaaa 896

<210> 52

<211>  415
<212>  DNA
<213>  Artificial

<220>
<223>  Construct sequence

<400>  52
attgaatagc caaatatctc tctctctgga ctagaatgtg aaggagcagt tagcgaagga      60

aagattggag aatgccggag ctggtgtacc aagagcagag ctcgtcgaga tcaggattta     120

gagctcgaga tgcaagtcct gactctgtaa ttttcactct ggagtccaac ttcagtctct     180

tctcttctgc ttctgctagc gtcgatcgct gctcttttgc ttccgatgct catgatcatg     240

actctctcgc ctctgaaatc tctctggtaa aaccatttct ctaacttccc tgcatgcatg     300

cgttttctaa cccatggttt ttgagtgagt tttttgagag tgcaatgcag catttggcag     360

ctggtcatga tcaacaagag aactcttcga gtggtccaga tcgaaacaac aacaa          415


<210>  53
<211>  566
<212>  DNA
<213>  Artificial

<220>
<223>  Construct sequence

<400>  53
tccttaactt gtccgagaga ggagcaattt ttaacaccgg gcttgcctca catttttcat      60

tggaaaatgc atgcctgaca attccattgg ggcattttat cagaatgttt aaaatattta     120

tattttgcgt cggctctata gcatatgctt ataagcattg aaatatgatt caactgaagt     180

attcgcacat gttttgctgt ttacgtttta tatgattgtt gtgtttagtt ctccacaagc     240

agcctggcag ttattcgatc cggtatcttg acgttatctt atcacatgct gtaaatccta     300

tggctcaaac tggcaattat tcaaggcagt tcttaaagag ttcaaggttt tctttcctgt     360

tgatcaacct ttgtaaagct tatgaagcaa ttgtgcaaat aattggtgct cgacagaagg     420

caatctctta gttcgctcac gacatttcta gccgattttc gggaaaacat tagtggactg     480

gagtcattga ttcaatcaag gtgaaattgg aaatcattta gaaaagcaat tcctgttctt     540

tgacaacact aggttcttct ttctcg                                          566


<210>  54
<211>  581
<212>  DNA
<213>  Artificial

<220>
<223>  Construct sequence

<400>  54
tagagttcga gaggtcatct tcttgttata aaatcctcaa aagcttgagg tgaaaaaata      60

agcaagggag aggagagagc tagctagcta gtcaagttaa gagtcagaaa acaagaatgt     120

cttccaggaa gaaaaagaag gcagctcttt atgagaagtt acgtgctgct accaattcta 180

atgctatgaa caaaacttca atcatagtag acgcgtcaaa atatattgga gagttgaaga 240

acaaggtgga taggctaaaa aaagaaatcg gaacatcttc aaccccccaa aattcattac 300

ctgcgcaggt cacagtggaa aacctagaaa agggtttcct tattaatgta ttttcaggaa 360

agaattgccc tggattactt gtctccatac ttgaagcctt cgaggaacta ggccttgatg 420

tgctcgatgc tagggtttct tgtgaagaca atttccaact tgaagcgatt ggtggagacc 480

aaaaccaagg ccatgatgct caagtagtga aacaagcagt gctgcaagct atccataact 540

ggaatgaagg cagctagcta gtgagcaaga atgacccaaa t 581

<210> 55
<211> 598
<212> DNA
<213> Artificial

<220>
<223> Construct sequence

<400> 55
gcttagtggt ctgggtttag tgagaaatgt gattggacac tgtttgagtg ggcgcggtgg 60

aggattcctc ttcttcgggg atgatcttta tgattcatct cgtgtagctt ggacaccaat 120

gtcacccaat tcgaaacact attcacctgg attcgccgaa ttgacttttg atgggaaaac 180

tactgggttc aaaatcctga tcgtagcttt tgacagcggg gcctcttata cttaccttaa 240

ttctcaggcg tatcaaggtc taatttctct gatgaagaga gaaatacccg cgaagcattt 300

gacagaagcg ctggatgatc agacgcttcc gatttgctgg aaaggacgga aacctttcaa 360

aagcgtacgt gatgtcaaga aatacttcaa gacctttgca ttgagcttta agaatggcgg 420

aaaatctaaa actcaacttg aattcccacc agaagcttat cttatagtat catccaacgg 480

aaacgcttgc ttgggaattc taaatggtac agaagtaggt ctgaatgatc tgaatgtcat 540

tggagacata tcaatgcaag acagagtggt gatttatgac aatgagaagc aactgatt 598

<210> 56
<211> 273
<212> DNA
<213> Artificial

<220>
<223> Construct sequence

<400> 56
gaaggtggtg gttcaattat acacatagtt gaccacatgg atttggagcc ttggagtgtg 60

cctgaagtac tacggccgct gtatgaatcc tcaactgtac ttgctcaaaa gacaacaatg 120

gtggctctac gccagctgcg gcagatagct caggaagctt ctcagtctaa tgtgaccaac 180

tggggcagac gacctgcagc tctacgagca ctgagccaga ggttgagcag gggttttaat 240

gaggctctca atggatttag tgatgaggga tgg                                273

<210>  57
<211>  274
<212>  DNA
<213>  Artificial

<220>
<223>  Construct sequence

<400>  57
cgctgacacc accactttcc tccttgacag cccctccttc ctcaacttcc ttcaacatct      60

caacaccaat agccattgcg attgtgaaga tgataataca aacgccacta ttgactctat     120

catccccacc ataaaaatca cttcttgcat gctagaaatg gatccaatgc ttgtgtgtgc     180

agtgtgtaaa gatcagttct tgattgatgt tgaggccaag cagctgccct gcagtcacct     240

gtaccatcca ggttgcattc tcccctggct ttct                                274

<210>  58
<211>  259
<212>  DNA
<213>  Artificial

<220>
<223>  Construct sequence

<400>  58
aacgtgattt caaagggtcg caaaggaact caaatagagc cccaagatcc cacagaacta      60

gagttattga cagaggttcg aacgagtttc caaaagaagg ggaggaggct taaagacgtt     120

ttgaggagtg gaaactgcat actgaggaag ttccaaaaac atcgagagga cgattctaat     180

caagttcttt acttcttctc tcaagtggat atgaagttag tagcaagggt tctgagcatg     240

tccagagtaa caacagagc                                                 259

<210>  59
<211>  1464
<212>  DNA
<213>  Populus trichocarpa

<400>  59
caccctgaag ccccatttcg gtttttagtc gaaatcttgg gttgggggct ggaaatatga      60

ggacatgctt gtgggttttt gccgtggttc ttgttttcgg ttttgttgat ggaaaagaaa     120

ttgaaaggtt gcgcacggag aggatttcag gaagcgctgg ggatgtgttg gatgatgatc     180

cagtgggaag gttgaaggtc tatgtttatg agcttccgag taaatacaac aagaaactgc     240

tgcagaagga ccccagatgt ctcacccata tgtttgctgc tgaaatcttc atgcatagat     300

ttctcttatc cagcccagtt cgaaccctta atccagatga agcagattgg ttttattccc     360

ctatataccc cacttgtgat ctcacaccca tgggcttgcc gttgcccttc aaatctccta     420

ggatgatgag aagtgcgata cagctaatct cctccaattg gccttactgg aatcgtacgg     480

aaggggctga tcactttttt gttgtgcccc atgactttgg agcctgcttc cactatcagg 540

aagagaaagc cattgagcgg ggcattctcc cgttactcca gcgttctact ttggttcaaa 600

cttttgggca acgaaatcat gtgtgcttga atgaggggtc aattacaatt cctccttttg 660

ctcctcctca aaagatgcag gcccaccaga ttccccctga cattccacgg tccatttttg 720

tctatttccg tggattgttt tatgatgtaa ataatgatcc agaaggtggt tattatgcaa 780

gaggagcaag ggctgcagtt tgggagaatt tcaagaacaa tccactcttc gacatctcca 840

ctgaccatcc aacaacatat tatgaagaca tgcagcgagc tatattttgc ttgtgcccgc 900

tcggttgggc cccgtggagt cctagattag ttgaagcagt ggtatttgga tgcattcctg 960

tcatcatagc agatgatatt gttttgccat ttgccgatgc tatcccatgg gaggaaattg 1020

gggtgtttgt agcagaggaa gatgtcccta acctggacac aatcctaaca tctataccac 1080

cagaagtgat tttaaggaaa caaaggcttc ttgcaaatcc ttctatgaaa cgtgcaatgt 1140

tattcccaca acctgcacaa ccaggtgatg ctttccacca aatactaaac gggctggctc 1200

gtaagttgcc gcacgacagg agtgtttact tgaagtctgg tcaaaatatt ttgaattgga 1260

cagcaggacc agttggggac ctgaaacctt ggtaagagaa gtgtcatttc ctcaacagac 1320

atggctgtgg tgtatccact gtttcatcta atcgcaagga ctgtaaactt ctctttgaag 1380

ttttgatttt tgtatagatt cttgaatttc acatgtacat ctgggacata gttcgatgct 1440

ttaaataact tcgcattttt cttg 1464

<210> 60
<211> 1495
<212> DNA
<213> Populus trichocarpa

<400> 60
attgcaatgg ctgagaagca ctcctcatca tcccttgggg ttgtttctag gaaatctatg 60

ttttgcttgt tttctttcgt gtctgccttg tttatactgt catggttttt tatgttgcgt 120

tccacggtca gtgccggatt tattgacctt agtttgttac ctcatcctaa acttaattct 180

aaagatgatt taggagatgt gaaatgtaac agttttgata ataattgcaa tcaagttctt 240

aaggttttca tgtatgattt gccttctgag tttcattttg gactcttgga ttggaaacct 300

caagggggta gtgtttggcc tgatcttaga gctaaagtgc ctgcatatcc tggtgggctg 360

aacttgcaac atagtattga atattggctt acaatggatc ttcttgcttc agagattcct 420

ggcataccta gagctggtag tgcagtaaga gttcaaaatt ctagtgaagc tgatgttata 480

tttgtgccat tcttttcttc tataagctat aaccgatatt ccaaggtgaa tccacaccaa 540

aagaagagca agaacaaatc attggaagaa aagttggtca agtttgtgac ttcgcaaaag 600

gaatggaaga ggtctggggg gcgagaccat atcattttgg ctcaccatcc aaatagcatg 660

ttatatgcaa ggatgaagct atggacggca atgttcatac ttgcagactt tgggaggtat 720

tctccaaata tagcaaatgt tggcaaagat gtgattgcac catacaaaca tgtaattaaa 780

agctatgcca acgactcctc taactttgac agccgcccga ctttgctgta ttttcaagga 840

gccatataca ggaaagatgg tggctttgct cggcaagaat tattttatgc tctaaaagat 900

gaaaaagacg tgcattttca gttcggtagt gtacaaaagg atggagtgag caaagcttct 960

caaggaatgc actcctccaa attctgcctt aacatagcag gtgacacgcc ctcatcaaac 1020

cgcctctttg atgccattgc cagccactgc gttccggtca tcatcagtga tgatatcgag 1080

ctcccttatg aggatgtcct tgactactct caattctgca tttttgtccg cacttcagat 1140

gctgtcagag aaaagttcct cataaacctt gtcaggagta ttaagaagga tgaatggact 1200

agaatgtggc agaggttgaa agaggtagaa aacttctttg agttccaata tccatcaaag 1260

gaaggcgatg cagttcaaat gatatggcag gctgtagcaa gaaaagtccc tgcaattagg 1320

ttgaaggtaa ataagtttag gcgattttct cgttttggga cccatgaaga tggagtgtta 1380

agaaaaatac catcacctag taatttttgg tgaaacattc agtgtttctt tatactttga 1440

ggttctgttt atatataaag gtgagaaatt caatttattc tcactacaga ttttg 1495

<210> 61
<211> 2487
<212> DNA
<213> Populus trichocarpa

<400> 61
atggctacct attacccaac ttcaagaagt cagaataaca atttgcacgc tttgcttaca 60

ggggaccaga aacttgcttc ttattctgat ctgccttccg atcttagtag catgaaaagt 120

tatacgaacc acactccagc tgctgggtca tactcggaca tattgtatgg gggttccttg 180

tcttctcaaa atggtgctga attctcatct agtggagcta gaaatgagat agtattcatt 240

ccacctacaa gtgacacgat gaatttacaa tctgttggtg ggcagttgaa tacagcagca 300

ggtaacctgg ttggtgactc tgttagtgga gattcccagg ctgttccgcc aaggatgcac 360

cttggcattc cagactgtga gcagaatttc cagtctcaag gattaccact gaggcttggc 420

atgcaggtgc aatctgcagt ttctatgcct tcacttcagt atcagtatct aaaccaaaat 480

ttcccttcat ctctgagttc acatctgctt gtgccagaga agtggacttt accctgtgaa 540

ggtgatgaaa gcaatcaaag caaggagttg agagaatttg aaggattgcc tggatttgcc 600

ggaagcagcc ataaccctat caaaacagag tcttcacaca atcctcagta catagttggc 660

ctcagagata tgcatgctga aatgaatatg tatggattgt ctggttatgc taacacactc 720

ttgaactcca gatacctcaa gtcagtgcaa cacttgctcg atgaggtggt taatgtaaaa 780

aaggctttga agcagcctca atccaacaaa tgctccgatg acttcaagga gagtgatagg 840

aggccgagta gctgttctat gcttccctca tcaaatgtga aaccaccgga tcctgctgag 900

tctactgccg actccactcc tgagctatcg ccagtagaac ggcaggactt acttgacaaa 960

aagactaagc ttctgtccat gctggaagag gtagacagaa aatacaaaca gtattaccat 1020

caaatgcaaa ttgtggtgtt atattttgac acggtggctg gtcatggggc agctaaatca 1080

tatacagcac tcgccctcca aacaatttct cgtcactttc gctgtttgcg cgatgcaatc 1140

agtggccaaa tagaagttat catgaaaagg cttggggagc aaggtacttc accaaatggt 1200

caaggaggta taccacgtct ccgttatgta gatcatcaga ccagacaaca gagggctctc 1260

cagcagcttg gtgtgatgcg acatgcttgg aggcctcaaa gggggctccc tgagagctct 1320

gtttcagtcc ttcgagcttg gctctttgag catttccttc atccctaccc gagtgattca 1380

gagaaaatca tgttagcaag gcaggcaggc ttgaccagaa gccaggtcgc gaactggttt 1440

ataaatgcgc gggtgcgtct ttggaagccc atggtcgagg acatgtacaa agaagaattt 1500

ggtgattcag agacaaactc caaatcttct ttggacgaaa caaccaaagc ccatggagat 1560

aaatcaggca atcacttgac atctgagaat aggctacgtg agttgtatga gagtgtgaca 1620

tctacagctg ctgatatttc ccagccaggg caagcccatg acataaaatc tagtcatatt 1680

cttgaattag aaatgaaaga acctatggcg aaaacagtcc ttgagaatgg ttctcaggga 1740

cccaatgtgg cagaatctga tattatgaaa tttccacggg accgaaggtt gaatatagat 1800

gatgaccata acttctgtcc acatgggaat attccgtgtg gccaaaatgg tgatggaaat 1860

cttatgtctg ctgctgctac atatgatgta tcacacttga atggatttgc agtaggcagc 1920

cagatgtctc ttgcattggg attgcaaagt aacgacagtg attcatttcc cacgtttgat 1980

ggggcccata tgagaggtaa cactatatca gcttcctcgg tggggcataa tgaggtggat 2040

tatcactgta tggatacagg aaagcaacaa gacaggattg ccaattccca tcggttacat 2100

gattttgtgg tttaagagaa tccttctagc tttacgaggg agtttcatca atcctgccaa 2160

gagaaggatt gatggagttt tggataacat aaagcagaag aaaacaagct cagtgatatt 2220

ggtttgctat tcattcaagc cccgtgctct tctcttggtt tgaatagaag aaacccaaca 2280

cccttacagt cacagacata gatgtaacat aagggcatgg tgcctgcttg taatatacgt 2340

acagattaca gggattgtag atatatctgt gcatatacaa taagattttt gtgatctttt 2400

gtactcttaa ggaatttcta caagctctcc ctaattgtga cactgatgta tcatttcatt 2460

cctaattctg aaataaatgt gatttgc 2487

<210> 62
<211> 957
<212> DNA
<213> Populus trichocarpa

<400> 62
attcaattca attgtaccag cagcagcagc agtagtcaga gcttataact agctaattga 60

ttaaccatgg agaaagcaca tacaaaatct gctcttaaga agcttgttaa ggctagctca 120

cagtctgcac catggagcaa tgctgctaga ggaatggcaa aagatgatct caaggatccg 180

ctttatgaca aatccaaggt tgccccaaaa ccttttgcaa aagaaaacac taagccccaa 240

gaattcaaac tccacactgg acaaagagct ctcaaacgtg ccatgttcaa ctattctgtg 300

gcaaccaaga tatatatgaa tgagcaacag aagaggcaaa tagagaggat acaaaagatc 360

atagaagaag aagaggttcg tacgatgagg aaggagatgg ttccaagagc tcaattgatg 420

ccttactttg acagaccttt ctttccccaa agatcaagca ggccattgac agttcctaga 480

gagccaagtt tccacatggt gaacagcaag tgttggagct gcatccctga ggatgaactt 540

tactactact ttgaacatgc tcatccccat gatcatgcct ggaagcctgt taagtaaata 600

aattgccatg atttgtaggg gaaaaaggag agtggagtat tctctctgtg atgttaattt 660

cttcatgctg tccaagttag tcttgtataa tttggagtgt tgatagtgtt tactgtttag 720

atcatgtggt aggtcttgta gtgggtgtaa gtagtcgctt tttcatagcc gaaagtcctt 780

ttcaatgggg ggttttgctt accaataagg cggatattgt acttcgtttt gtactattaa 840

aaagcaagtc accctctttt ctgtctttta gcagtatggt cgtgcatgcc caattaagca 900

cttcatttta ttttgctgga aatgaattat caaagcagac tataattctt ttagcca 957

<210> 63
<211> 2017
<212> DNA
<213> Populus trichocarpa

<400> 63
acttgggttt ctcgcatttc tactcatcgc cagttcttct ttgggtgtga ttgccaaata 60

atgacatagc caaccaagag aaagaaaacg aggcacagct ctatttcggg cttcttgttt 120

cttggtttcg ctgtgttgtg tgctcggttt aaaacaaaat gcagcttcat atatcgccga 180

gtttgaggca tgttacagtt tttcctggaa aaggtgtcag agagtttatc aaagtgaggg 240

ttggagctag aagggtttct tatcgaatgc tcttctattc tctcttgttc ttcacgtttc 300

ttctccggtt tgtgttcgtt ttgtccacag ttgactccat tgatggagaa accaagtgct 360

ctacactagg ctgcttgggg aaaagactag ggccaaggat attggggaga aggcttgact 420

cggctgtacc agaggttatg tttcaagtgc tagaacaacc acttggcaat gatgaattaa 480

agggaaggag tgatattcct caaacattag aggaatttat ggacgaagtc aagaacacaa 540

gattagacgc gaaacttttt gctctcaagc tcagagaaat ggtcactcta ctcgaacaaa 600

ggactagaaa tgccaaaatc caagaatatc tgtacaggca tgtagcatca agtagcatac 660

cgaagcagct tcattgcctt gccttgaggc tagccagtga gcactccact aatgcagctg 720

ctcgacttca gctccccttg ccggaactcg tccctgcgct tgttgacaat acttattttc 780

actttgtcct ggcttcagac aatgtgcttg ctgctgctgt tgttgccaat tcacttgtcc 840

aaaatgcgtt gcgcccccag aagtttgtgc tgcacataat aacagatagg aaaacttatt 900

cacctatgca agcatggttc tcactgcacc ctttggctcc ggcaataatt gaggtcaagg 960

cattgcatca tttcgattgg ttcgcaaagg ggaaagtgcc agtcatggaa gcaatggaaa 1020

aggatcagag ggtgaggtca cagtttagag ggggttcatc tgcaattgta gcaaataaca 1080

ccgagaagcc tcatattatc gcagcaaaac tacaaacact tagtcccaaa tacaattcag 1140

tgatgaatca catccgaatt catctacctg agttgttccc aagcctaaac aaggttgtgt 1200

tcctagacga tgacatagtg gtgcagtccg atctttcgcc tctctgggac attgacatga 1260

atggaaaggt taacggggca gtagaaacct gtcgaggaga agacaagttt gtcatgtcaa 1320

agaagttgaa gagctatttg aacttctctc atcccttgat atcagaaaat ttcaagccga 1380

acgaatgtgc ctgggcttat ggcatgaaca tatttgattt agaggcttgg aggaaaacca 1440

acataagcac aacataccat cactgggttg aagagaactt gaaatcagac ctgagcttgt 1500

ggcagctagg aacactacct ccaggtctga tagcattcca tggccacgtc cacgttatcg 1560

atcccttttg gcacatgttg ggtctaggct accaagaaaa tacaagtttg gcagatgctg 1620

agactgccgg cgtcatccat ttcaatggtc gagcaaagcc ttggctagat atagcattcc 1680

ctcagctccg ccccctgtgg gctaagtaca tcaacttttc tgacaagttc ataaagggct 1740

gtcatattag gccatcttaa cttgaatttt gagatgaaaa tgttgactgc gatcgtgatt 1800

aatatatgtc tatatataag gagaaaaaag cagagaggag agagttcttc acaaatccaa 1860

ggccttgaat ccagaatgta aaatattcat aaccaggcat ttgtctgctt ggttttttta 1920

gaattcatcc tctgtgggtg aacaaccctt ccttattact tcaaatcttg attcaatttt 1980

aatgagaaat taagcatgtt atggattttc aaccgtt 2017

<210> 64
<211> 1496
<212> DNA
<213> Populus trichocarpa

<400> 64
aagctgatga tcaaatgagg acatcaaaaa tttcttcagg gaattcaggc tatcatgcat 60

ggattcttgc ttttgcaatg agtctactga tactgattgc cttgtcgaag tcatggtttt 120

atgatcacgc ttctgctact gcaagtgagg atttgcaata cttttcagtg attgtccctt 180

caaaagggcg ggcctatcct cctgttcttg cttattggat atgtggtact agtggagatg 240

ggaagagaat gttaaggcta ttaaaagcaa tttatcatcc aaggaaccaa tatctattgc 300

agcttgatgc tgaatcctca gattatgaaa gggcagagtt agttgtttca gttcaatctg 360

aaagtttgtt tcaagcatac ggtaatgtta atgttgttgg aaaaggttat gctatcaacg 420

aaatgggatc gtctgctctt gctgccatac tcaacgctgc tgcattgctt cttaagctga 480

gtgcagactg ggattggttc atcaatttaa gtgtctcaga ctatcctcta gtgagtcaag 540

atgatctcct ccatgccttc acttccttgc caagagatct caacttcatc aactatacta 600

atgacactgc gaaaaacgag atacacaaga ttaaccaaat tgttgtagac cccagcttac 660

atcttcagaa gagtagccac ctttattacg ctgttgagac tcgaacaaca cctgatgctt 720

tcaagatatt tggaggttca ccctggctga ttcttacaag agctttcatg gagtactgtg 780

tccaaggatg ggacaacctt ccaagaaaac tactaatgta cttcagcaac acggcatccc 840

cacttgaatc gtatttccac tctgtcctct gcaactctcc tgagtttcaa aacacaacag 900

taagcaatga tttaaggtac aatattctgg aaactactac agatggggaa tcaccttatg 960

acaaaatgct aaatggtgga gcagcatttg caaggccatt taaagaagat gctgctgcac 1020

taaacatgat agatgagaat gtcttgaacc gtgaacccaa tggattggtg cctggaaaat 1080

ggtgcttaga ccaaggtctg aacaagagct cagaggcatc aaagcctcca ggggaggatt 1140

tgtgttcaac ttggggtaac attaatgatg tcaagccagg atcttatggt atcaagcttg 1200

catttttatt gtctaagatt gccggtgaag agaaattgac aactagtcaa tgccttcaag 1260

ctacacaaat ggggtcatca tagaaccaag catacatcct tgtgtggata atttgtttct 1320

tttaatcgaa aactataggt taaatcaagc ttcttagcca tatgaatcct ttgctaaatg 1380

cactataaag gcgtacttgc ttgttagaac aaaagctaga agcacccagt gatatattgt 1440

acatacattc acattttatc gaggatttct acaataaact cattgttttt ccctta 1496

<210> 65
<211> 1703
<212> DNA
<213> Populus trichocarpa

<400> 65
gagagagaga atcttcgtat tgaaatgagg gaacctattg aaaccaacaa cggatcacct 60

ccaccactac ctccacaagc gttgctcgag aggcttaaag attatggcca ggaagacgct 120

tttgccctct gggacgagct ctctactgaa gagcgagaac tccttgtcaa ggacgtcgag 180

agcttagatc ttccaaggtt ggatcggata atccgatgtt cgcttcgatc tcaaggccta 240

ccggcggcgg caattgagcc ggttccggag aataccgtgt cgacggtaga agatagaacg 300

atagaagaaa gagagagatg gtggaagatg ggattgaaag caatctctga tggcaaattg 360

gctgttgtgc tattatctgg tggccagggg acaaggctag gaagttcaga tccaaaagga 420

tgtttcaata tagcgcttcc atctggcaaa tcactctttc aacttcaagc tgagcgaatt 480

ttgtgtgtcc aaagattagc agcccaagct tcaagtgaag gttctggaag ctcagtttcg 540

atacattggt acataatgac cagcccgttt actcatgatt ccacacgatt tttttttgaa 600

aatcacaagt acttcggcct tgaagcagat caggttacct tcttccagca aggcaccata 660

ccttgtgttt ccaaggatgg cagatttatc atggagactc catttagggt agctaaggct 720

ccggatggga atggaggagt atattcagcg ctgaaatatt caaaattatt agaggatatg 780

gcctcaagag ggatcaagca cgtggactgc tatggggttg acaacgcact ggtacgtgta 840

gctgatccag cttttttggg atatttcatt gataaaggtg tagcagctgc agcaaaagtt 900

gttcgtaagg cataccccca agaaaaggtt ggtgtttttg taaggcaagg taaaggtgga 960

cctcttaccg tggttgaata cagtgagctg gatcagtcac tggcttctgc agttaatcaa 1020

caaactggac gccttcgttt ttgttggagt aatgtgtgct tgcacatgtt ctctttggat 1080

tttctaaacc aagtggcaaa tggccttgag aaagacagca tttaccatct agctgagaaa 1140

agaattcctt ctattcatgg tgatacgatg ggattaaaac tagagcaatt catattcgat 1200

gcattcccat atgctccttc aacagctctt tttgaggtac cacgtgaaga agaatttgca 1260

cctgtaaaaa atgccaatgg gtcaaatttt gacactcctg agagtgctcg gctgcttgtt 1320

cttcgactgc attctcgctg ggtggttgca gcaggtggct tcttaacaca ttcggtgcct 1380

ttatatgcaa ctggtgtgga ggtatcacca ctttgctctt atgctggcga aaatctagag 1440

gccatatgcc ggggaagaac atttcacgca ccttgtgaga ttacattcta gttaaatctg 1500

tatccaaata ttcttgattt gtacggatcc cattggaaag gggttatcca tcccacgcat 1560

gtgttgtggt agattggcga gagataagag ttccaggttt tgttacatcc tgggtgtgat 1620

gtcaagaatg atacaatttg attaacgtat atcattcaag ttattgttga gcaatgataa 1680

tcctctcttc ttcttttttc caa 1703

<210> 66
<211> 1312
<212> DNA
<213> Populus trichocarpa

<400> 66
ctctctctct ctccctctcc ctctccctgc cattccctcc tttgctttct ctatccagaa 60

aacacacaac tctctttctc gtccctcacg ttctcttgca atggcttcca atggagtgaa 120

cgacaagtca ctgatcgtca gcttcgggga gatgctgata gacttcgtcc cgacggtctc 180

tggcgtctcc ctcgctgaag ctccggggtt tgtgaaggca ccgggtggtg ctccggcgaa 240

cgtggcgata gcggtggcga ggctgggagg aaaggcggca tttgttggaa aacttggtga 300

tgatgagttc ggcaacatgc tcgccggaat tttgaaggaa aacggcgtga tcgctactgg 360

gataaatttt gacacaggtg ctaggactgc tctcgcgttt gttactctac gcgctgatgg 420

agagcgtgag tttatgtttt atagaaatcc aagtgctgat atgttactaa ggccagaaga 480

gttaaatctt gagttaatta gatctgctaa ggttttccac tatggatcaa taagtttgat 540

tgtggagcca tgcagatcag cacatttaca ggcaatgagg gtggcgaagg atgcaggtgc 600

attgctttcg tatgacccaa atctgaggct gccattgtgg ccatcagcag aggaggcgcg 660

tgagcagata ttgagcatct gggacgaggc agatgtggtc aaagtcagtg ataatgagct 720

tgagttcctc actggaagtg acaaaattga tgatgaaact gctatgtcac tctggcgtcc 780

taactttaag ttgctcttgg tcactcttgg tgaaaagggt tgcaattatt acaccaagaa 840

tttccatgga tcagttgagg ctttccatgt ggacactgtt gataccacag gtgctggtga 900

ttcatttgtt ggtgctctcc tttgcaagat tgttgacgac cactctgtgc ttgaggatga 960

gccaaggctg agagagatac ttagatttgc aaatgcttgt ggagccatta caaccaccaa 1020

aaagggagca atccctgctc tgcctactcc ggccgatgcc ctcaaactgg tgaaagaagg 1080

aaaataaact atttttttgt taatccgcac tgcattttat ccctttcctt ttcagctcct 1140

ggttctcaat tcaatcgaag agctgttgca atctcaaatt ttcgcctttt tctcttgagt 1200

gtaatgatgg aaaagacttg cgctttaagg tcttgccaaa taaaattttt cttttaaaca 1260

aattgatctt gggcatcaag cagcaatcat caaagtgtta gctttctcca aa 1312

<210> 67
<211> 2353
<212> DNA
<213> Populus trichocarpa

<400> 67
gaaacactga aacaagcaga actcaacatc acacgctctc gggaatatta ttcactctcg 60

aaacaaccag aaactcctcc attgtagatt cagagagaga gggaggcaga cagacagaga 120

gtaggcgatg gcgggcacgt ggagagcacg tggagctgtg gtatccgcga ttctcttatt 180

cgggtgtttg tttgcgattt cgattgcgaa agaggaggca acgaagctag gaacagtaat 240

tggaatagat cttggaacga catattcatg tgttggtgtt tacaagaacg gtcatgtgga 300

aatcatagca aatgaccaag gaaaccgtat taccccatcg tgggtggctt ttactgacag 360

tgagaggttg atcggtgaag cagccaagaa tctggcggct gtcaatcccg agaggaccat 420

ctttgatgtc aagagactta ttggaagaaa gtttgaggat aaagaggtgc agaaggacat 480

gaaacttgtg ccttacaaga ttgtgaataa ggatggaaag ccttatattc aagttaagat 540

taaggatggg gagactaagg tcttcagtcc tgaagagata agtgctatga ttttgaccaa 600

gatgaaggaa acagctgagg cattccttgg aaagaaaatt aaggatgctg ttgtcactgt 660

tcctgcatac ttcaatgatg cccaaaggca agctaccaag gatgctggtg tcatagctgg 720

actcaatgtg gctagaatta ttaatgagcc aactgccgct gctattgcct atggtttgga 780

taagaagggt ggtgaaaaga acattcttgt ctttgatctt ggtggtggta catttgatgt 840

cagtgtcttg acaattgaca acggtgtctt tgaggttctt tctaccaatg gtgacacaca 900

tttgggaggt gaggattttg accagaggat tatggagtac ttcattaagt tgatcaagaa 960

aaagcatgga aaggatatca gcaaggacaa cagggctctt ggaaaactca ggagggaatg 1020

tgagcgtgcc aagagagctc ttagcagcca gcaccaggtc cgtgtagaga ttgaatcact 1080

ccatgatggt atggatttct ctgagccact gaccagagcc cgttttgaag agttgaacaa 1140

cgatttgttc agaaagacca tgggacctgt gaagaaggct atggacgatg ctggtttgga 1200

gaagaatcag attgatgaga ttgttcttgt tggtggaagt accagaattc caaaggttca 1260

acaacttctt aaggattact ttgatggaaa ggagcctaac aagggtgtga accctgacga 1320

ggctgttgct tttggtgctg ctgttcaagg aggtatattg agtggagagg gcggtgatga 1380

aaccaaagat attcttcttt tggacgtggc tcccctcacc ctgggtattg aaactgttgg 1440

tggagtgatg accaagttga ttccaaggaa cactgttatt ccaaccaaga aatctcaagt 1500

tttcaccact taccaggatc agcagactac tgtctccatt caggtctttg aaggtgaaag 1560

gagtctcaca aaggattgca ggctgcttgg taaatttgat ttgactagca ttcctccagc 1620

tccaagggga acccctcaaa ttgaggtcac atttgaggtt gatgccaatg gtatcctgaa 1680

tgtcaaggcc gaagacaagg gcactggtaa atcggagaag atcaccatca ctaatgacag 1740

gggtcgtttg agccaggagg aaatcgagcg catggttcgt gaggcagagg aatttgccga 1800

ggaggacaag aaggtgaagg agaggattga tgctcgtaac agtttggaga cctatgtata 1860

taacatgaaa aaccagatta acgacaaaga taagcttgct gacaagctcg agtctgatga 1920

gaaggagaaa atagaaactg ctaccaagga cgcccttgaa tggctggacg acaaccagaa 1980

tgccgagaaa gaggactatg aagagaagct caaggaggtg gaagctgttt gcaacccaat 2040

catcactgcc gtgtaccaga gatctgctgg agccccaggt ggtggatcag cagaagattc 2100

cgaggacgac tctcaagatg aactctgaga atctttctgc ttccttctac ccctgctgga 2160

aagcaagggc aagggatgag gatgtaggct agatgaagtt ttgctgatcc tagaagctaa 2220

agggttaaaa taacatatcc cccttttttt ggtttttgga ggttaaaaat acatggaata 2280

gtttgttttc tctacatgtt taaaacacct tctacgagtt atagaaggta ggtccgtgag 2340

attgagtagt gtt 2353

<210> 68
<211> 2774
<212> DNA
<213> Populus trichocarpa

<400> 68
ggagctatgt ctgtacttac tcgtgtccaa agcattcgtg aacgtttgga tgagaccctc 60

aagactcatc gcaacgagat tgttgcctta ctgaccagga ttgaaggcaa gggaaaagga 120

attcttcaac accaccagat tattgctgag tttgaagcaa tccctgaaga gatcaggaag 180

atattggcgg gtggtgcttt ttctgaagtt ctcagatcca cacaggaagc aattgttttg 240

cctccatggg ttgcccttgc tgtgcgccca aggcctggtg tctgggagta cgtcagagtg 300

aatgtccaag cacttgttgt tgaggagttg cgtgttgctg agtatcttca tttcaaggag 360

gaacttgttg atggaggctc caatggcaac ttcgtgcttg aattggactt tgaaccattc 420

agtgcatctt tccctcgccc aactctttcg aagtacattg gtaacggtgt tgagttcctc 480

aaccgccacc tttcagctaa gttgttccat gacaaggaaa gcctgcaccc cctgcttgca 540

tttctcaagg tgcactgtca caaggggaag aatatgatgc tgaatgacag aattcataac 600

ctagactctc tgcaatatgt tctgaggaag gccgaggaat atctgtcttc tctgaaacct 660

gagactccat actctcaatt cgaacacaag ttccaggaga tcggcttaga gagaggatgg 720
ggcaatactg ctgagcgtgt tcttcagatg attcaacttc ttttggatct tcttgaggca 780
ccagatccct gcactcttga aactttcctt ggcagaattc ctatggtctt caatgttgtg 840
atcatgtccc ctcatggata cttcgcccaa gacaatgttt tggggtatcc tgataccgga 900
ggccaggttg tttacatttt ggatcaagtt cgtgccttgg agagtgaaat gcttctgcgt 960
atcaagcagc aaggacttga tatcaccccc cgaattctca ttattactcg actgctccct 1020
gatgcagtag gaaccacttg tggacaacgt ctggagaaag tctatggatc ggagcattgt 1080
gatattcttc gagttccctt cagagatgaa aagggaatgg tccggaaatg gatctctcgc 1140
tttgaagttt ggccatacct agaaacttac actgaggatg ttgctgctga aattgctaag 1200
gagttgcagg gaaagcctga tctgatcatt ggaaattaca gtgatggaaa cgttgttgcc 1260
tccttgctag cacacaaatt aggtgttaca gagtgcacta ttgcacatgc tctagaaaaa 1320
acaaagtacc cagattcaga tatttactgg aagaagtttg atgaaaagta ccacttttca 1380
tgccagttta cagctgatct ttttgccatg aaccatacag atttcatcat caccagcaca 1440
ttccaagaga ttgctggaag caaggatact gttggacagt acgagagcca cactgctttc 1500
actctccctg gcctctacag agttgttcat ggtatcgatg tatttgatcc caaattcaac 1560
attgtatccc ctggtgctga cgagagcata tacttccctt acactgagaa gaaacttagg 1620
ttgacttctt tccatgaaga aattgaagaa cttctttaca gctccgttga gaacgacgag 1680
cacttatgtg tgctaaaaga ccgcaacaag ccaattctat tcaccatggc gaggctggac 1740
agagttaaga atttaactgg tcttgtagaa tggtatggaa agaataccaa gctgcgcgaa 1800
ttagctaatc ttgttgtagt tggtggtgat agaagaaagg agtctaaaga tatagaagag 1860
caagctgaga tgaagaaaat gtacagtcat atagagaaat acaaattgaa tggccagttc 1920
agatggattt cttctcagat gaaccgcgtg aggaatggag agctctaccg ttacatttgt 1980
gataccaagg gagctttcgt gcagcctgct ttgtatgagg cttttggatt gactgttgtt 2040
gaggccatga catgtggttt gccaaccttt gctacttgca atggtggtcc tgctgagatc 2100
attgtgcatg gaaaatccgg attccatatt gatccttacc atggagtaca ggctgctgaa 2160
ctccttgttg acttctttga gaagtgcaag gctgatccca gttactggga caaaatctcc 2220
cagggaggcc tgcagcgaat ccaagagaag tatacctgga aaatttactc tcaaaggctc 2280
ctgactctca caggagttta tggcttctgg aagcatgttt ccaaccttga tcatcgtgag 2340
agccgtcgct atctggaaat gttctatgca ctcaaatatc gcaaattggc tgattctgtt 2400
cctttgacta tcgagtaaat ggagctggag aaatcaagga aacatgggtt ggtttgagtc 2460
gggttccggg tccagaataa tggtgtcatt tcacgatagt gattggacaa gaaaggcttt 2520
gatcttcttt ttgttttttt gtttttcctt tctgtgtgta tcttttttgt ctttcctttt 2580
cttttcatca tgctgcctct tgaatttcag taaagattgt gtcttttgag ttcatcatcc 2640

cctctatcca tttcttgcaa attctctgtc tcttgagatt ggaaatcatg ggaaatgatc 2700

cagcatcaat tcatgtattt ctcctcaata ctcgagattg gaagttgatg gctattcttc 2760

atcattatat aaag 2774

<210> 69
<211> 1428
<212> DNA
<213> Populus trichocarpa

<400> 69
ctccaatcac gtgtccgctt tacacaggtc gatccaaagc aattttctcc attgcaagag 60

aagccaagaa tttgtgacga acttggtata aattagtttt gttttgacaa gagagagact 120

tctttgttta atttccatgt gtctgaggag ggaacgaaga acaaccccag ggtgcttttc 180

attgttcgga cacctcttag attctcagac aacttgaaca caaaaatatt tcccgagttc 240

caggatcatt ttgaaggata caaggaaaga ggaataaaaa gaaagaacaa tggcattcac 300

gggaaccctg gataagtgca aggcttgtga taagactgtt tattttgttg atatgatgtc 360

tcttgaaggg gtgccttatc ataaatcctg cttcaaatgc agccactgca aaggaactct 420

tgtgatgagc aactactcat ccatggatgg agtcctctac tgcaagactc attttgagca 480

acttttcaag gagggtggag atttcagcaa gaattttcag aaagggaagc ccgagaggac 540

tcatgacctg tctaggatcc ccagcaagct atcttcagtg ttctgtggaa cccaagacaa 600

atgctccgct tgcggcaaaa cagtgtaccc attggagaag gtttgttttc agctctgaac 660

ttaaataaat ccattaggtc ctaaagacct gtgtgctatt tataccccta catttatcag 720

ataaatgacc aacatattct taggtacatc ttatctatta tagatggatc tatctgagaa 780

atgttttcaa gtctctgtta tttaataggc atgcctatga taatctggaa taagtacaga 840

tgtgctataa ccattgatga cataggttac catggaggga gaatgttacc acaagacatg 900

cttcaggtgt gctcatggtg gatgtccact cacacattca tcttatgctg ctcttgatgg 960

agtcctctac tgcaaggtcc acttcgctca gctcttcatg gaaaagggaa cctacagcca 1020

tgtccttgca tcagctgcac acaagagatc aaattccaca cctcctgaac tggccgggtc 1080

aaacccagag gaaggagctg ctgtggagga ggaaaagcca gaagagcaat cttaaacaaa 1140

gcacatcatg ttttcttgca ttgctatctt ttcaagttgc caggactgct ttgagttttc 1200

atgttgttga atattattgc cggattgtgt tcaaagtttg tttttggatt tgactatctg 1260

tgttacagat gccttgctac aggatattga attgcaatag tttttacaaa ttcctcctct 1320

tgcaatttaa tactcttggc aacaggatac aaatgaaaaa cccaaatcct aggaaccagt 1380

atcaacattt attgatctca gctctttgct cagatggctt gcgttttc 1428

<210> 70
<211> 776

<212>  DNA
<213>  Populus trichocarpa

<400>  70
cttgttcaat cccggcacta ctcctgaatg cctgcattcc cttttagatt ttgtgtttga      60

ttttgtcctg taaggaggat atgcgggatc atatggaaag atttgtagtt cttccattct     120

ccatcgcctg tgcctctcac tccagtgttg atgtggcctc cagtgaatcc tgcaagaaac     180

caaaacccga aaccaaatca catgcaacaa gaggacaaga aggggaggaa agctcttgta     240

aagaaaagac gaagaacaat acatttggtt tcctgctggc tcttccaaag ccttgcatat     300

ccagtagcat tcacaaattg attaggggca ttaagactct ctcccaagta tttgtgtaca     360

aagaagaaga cgaggagcta atggaaagag agatggaaat cggatatcca actgatgtga     420

agcatgtaac acacatagga ttggatggaa ctactatgac aaatcctatt aagggctggg     480

aatgcctgaa atctccagaa ataattccat tcccttcatt tactttaagg cagttcgagc     540

ttgcaatggc tgcacaagct catggacctc ttgttggggt cgatcattcc aagcttgttt     600

gattcattga tttttctttt catttcctga tcttgtttct ttgacactag atgactgatg     660

tgatgaagat tgatcaatgt tttgatggga ggcactggtt gcagtgatgt gtttatggtg     720

tgtgtgtggg accttgacaa tgtttttctg ggtggccatt gaaattgttc ttgcaa         776

<210>  71
<211>  819
<212>  DNA
<213>  Populus trichocarpa

<400>  71
gagtgtaagc tagctagctt tgcagagttc tgccgattca atggcgacca aagtgtatat      60

tgtgtactat tccatgtatg gacatgtaga gaaactggca gaagagatta agaaaggggc     120

ttcatctgtt gaaggtgttg aggcccaatt atggcaggtt cctgagacac tgccagaaga     180

ggtgcttgga aagatgagtg caccaccaaa gagtgatgta ccaatcatta cacctggtga     240

actcgctgaa gctgatggat ttgtgtttgg attcccgaca agatttggaa tgatggctgc     300

ccaattcaaa gcttttctgg atgcaactgg aggtctatgg aaaacacagc aacttgctgg     360

caagcccgct ggaatcttct ttagcactgg atcgcaaggt ggtggccaag agaccacagc     420

gttgactgct attacccaac ttgttcacca tgggatgata tttgttccca ttggctacac     480

atttggtgct ggcatgtttg agatggagaa ggtgaaaggt ggaagtcctt acggcgcagg     540

aactttcgct ggggatgggt caagacagcc aaccgagctt gaattggagc aggctttcca     600

ccagggcaag tacattgctg ccatcacaaa gaagctcaaa ggagctgctt aagtttactc     660

aattacaaga atacggtctg gtcttccatt ttttcaccct atagaatatg catttgcaaa     720

gaataattct tattctactg tttattatat gcttttcatg tgtgtaacac gatagtgatt     780

attgtctcaa tgatatcaag gttggttgtg ttttcattt                            819

<210> 72
<211> 1246
<212> DNA
<213> Populus trichocarpa

<400> 72
aaggaaaaga aatcaaaatt gttgctaatt ggacgagagg aagaaaagga gaacaaaagc 60

accttcatag ggcccatttc atgtctcctc atgagaccta agcttccccc cccactgcac 120

aatcacacta ttcttctttc atctcttttc tttccttctt tctccatcac ccacccgttt 180

tcttaatttc aattcttgtc aaaatagtct tgatcatttt ctctgcaaaa gaaattaatc 240

atgtcaggtg atcaaaggcc taaagattct gctgaaggct cttcgcgatc tggaggcgat 300

caccaccagc ttcaatcagc tcctttgagc cggtatgagt cgcagaaacg gcgagactgg 360

aacactttcg ggcagtactt gaagaatcag agacccccag tttcattatc tcagtgtaat 420

tgcaatcatg tgcttgattt ccttcggtat cttgatcagt ttggcaagac taaggttcat 480

ctacatgggt gcgtcttctt tggacaacct gatcctcctg ctccttgtac atgccctcta 540

aggcaagctt gggggagcct tgatgcccta atcggacgcc ttcgagcagc ttttgaggag 600

catggaggat cagcggagac taaccctttt ggaaatggag ctattcgggt ttatttgcgt 660

gaagtgaaag agtgtcaagc taaggcaaga gggattcctt acaagaagaa gaagaagaag 720

aagactcaaa taaggccaag agacgaagca aagccttcga tgcagacagc ttaagtcctt 780

tggcttcttc cgataaatgg atcacaaaat aggacaaatg gagatgaagt tccagaaagg 840

caaatgttgg gggtctagcc tctgcttcag tacttattat atagctagct agcttctctc 900

tttgcagata gtgttacatc acagcctact tctaaataat cttgtcctgt caggttggtc 960

taagaagcta ttcctagctt ataggaatta aaactgtagt cgtaatgttt ctactacatc 1020

ctcgacttct taattagtaa gacgttaaca ctatatgtcc agctctgttg tttctttgct 1080

gcagaagaaa actagtgtct tcagctaatg gtgaaaatgg cttttagctt ctgcctaaat 1140

gtagctttgg gcttgcctct cattagatga gctgtactta ctagtaatcc ttcaattgtc 1200

caaagtacca agtttttctt catgtttaat cttcagttaa tttcct 1246

<210> 73
<211> 2176
<212> DNA
<213> Populus trichocarpa

<400> 73
acaactacaa ctgctacttg taggtgtggg tttgctctta actctttcat tctatctgcg 60

atcctctctc cctccccctt ggcacttgtc aagcagcaga gaatgaaggt tattgataag 120

ataagggcgg cagcagcagc aggagattca aatggaggag atagcagcag caataataag 180

gttgttttct ccttcgaatt ctttcctcca aagaccgatg atggagtgga caacctgttc 240

gagagaatgg acaggatggt ctctcacaat ccttccttct gtgacatcac ttggggtgct 300

ggtggctcca ccgcagatct gaccttagac atcgccaaca agatgcagaa catcatctgt 360

gtcgagacta tgatgcatct cacctgcacc aacatgcccg ttgagaagat cgatcacgcc 420

ctcgaaacca tcaaatccaa tggaatccag aatgttcttg ctcttcgtgg tgatcctcct 480

cacggtcagg ataaattcgt ccagatccaa ggcggcctcg cttgcgctct cgacctcgtc 540

aaacatatca gatccaaata tggtgattac ttcggcatta ctgtcgctgg ctatccagag 600

gcacatcctg atgtgattgg aagtgatgga tttgctacgc ttgaggatta ccataaagat 660

cttgcttatt tgaagcagaa ggtggatgct ggggctgatc taattgttac tcaactcttt 720

tatgatactg atattttctt gaaatttgtg aatgactgcc gccaaatcgg aatcaattgt 780

cccattgtcc ctggtattat gcctattaat aactacaagg gcttcatacg tatggctggt 840

ttttgcaaaa ccaaggtacc agccgaggtt actgcggcat tggagcctat caaggataat 900

gaagaagccg tcagagccta tgggattcac cttggaactg aaatgtgcaa gaagatttta 960

gctcatggaa tcaagacgtt gcatctttat actctgaaca tggagaaatc tgctttagct 1020

atattaatga atcttggttt gattgaagag tccaagatta caaggccgtt accatggcgc 1080

cccccgacaa atgttttccg tgtaaaagaa gatgtccgtc cgatattttg ggctaatcgt 1140

ccaaagagct acctaacaag gacaataggt tgggaccagt accctcatgg ccgatggggt 1200

gattctcgca atccatccta tggtgcttta tctgactatc agtttatgcg accccgggca 1260

cgtggcaaga aacttcttga agagtgggct gcccccttga aaagtgttga ggatatatac 1320

gagaaattca aggtatactg ccttgggaag ttgaaaagta gtccttggtc agaactagat 1380

ggccttcagc cagagactaa aatcattaat gaacagctgg gtaagatcaa cttgaaggga 1440

ttcctgacta tcaatagtca accagctgtc aatggggaaa aatctgattc tccatctgtt 1500

ggttggggtg ggccaggagg gtatgtatat cagaaggctt atctagaatt tttctgctcc 1560

aaggacaagc tgaatgctct tgttgacaag tgcaagtctt tcccttttgt aacttacatt 1620

gctgtgaaca aaggagggag ttggatctct aacgttgccc tgactgatgt gaatgctgta 1680

acctggggag tcttcccagg aaaggagatt atccaaccaa ctgttgtgga tcccactagc 1740

ttcagtgtgt ggaaggatga ggcatttgaa atctggtcaa gaggatgggc ttccttgtac 1800

ccagagggtg acccatccag aacgctactt gaagaggtgc agagcagcta ctttttggtc 1860

agcttggtag ataatgatta catccatggg gacatttttg ctgtcttcgc ggatttatga 1920

tgctaggggc cctttgcagc cctgcacttt tctaagagtc tatccaagtc cccaaaaaat 1980

ggtgctttat cctaagttga atcccagctt taatcttctt atggcataca ttgtttttga 2040

aattatttcc gtattgaaat gtaataataa tccattgtga ttttgctact cccctctcca 2100

ttatatgctg caaatttgta tttggagtgg caactaattg taagcttaaa gaaaatatga 2160

ttgcctcttt ttcttg 2176

<210>  74
<211>  1304
<212>  DNA
<213>  Populus trichocarpa

<400>  74
ctctctctct ctccctctcc ctctccctgc cattccctcc tttgctttct ctatccagaa      60

aacacacaac tctctttctc gtccctcacg ttctcttgca atggcttcca atggagtgaa     120

cgacaagtca ctgatcgtca gcttcgggga gatgctgata gacttcgtcc cgacggtctc     180

tggcgtctcc ctcgctgaag ctccggggtt tgtgaaggca ccgggtggtg ctccggcgaa     240

cgtggcgata gcggtggcga ggctgggagg aaaggcggca tttgttggaa aacttggtga     300

tgatgagttc ggcaacatgc tcgccggaat tttgaaggaa aacggcgtga tcgctactgg     360

gataaatttt gacacaggtg ctaggactgc tctcgcgttt gttactctac gcgctgatgg     420

agagcgtgag tttatgtttt atagaaatcc aagtgctgat atgttactaa ggccagaaga     480

gttaaatctt gagttaatta gatctgctaa ggttttccac tatggatcaa taagtttgat     540

tgtggagcca tgcagatcag cacatttaca ggcaatgagg gtggcgaagg atgcaggtgc     600

attgctttcg tatgacccaa atctgaggct gccattgtgg ccatcagcag aggaggcgcg     660

tgagcagata ttgagcatct gggacgaggc agatgtggtc aaagtcagtg ataatgagct     720

tgagttcctc actggaagtg acaaaattga tgatgaaact gctatgtcac tctggcgtcc     780

taactttaag ttgctcttgg tcactcttgg tgaaaagggt tgcaattatt acaccaagaa     840

tttccatgga tcagttgagg ctttccatgt ggacactgtt gataccacag gtgctggtga     900

ttcatttgtt ggtgctctcc tttgcaagat tgttgacgac cactctgtgc ttgaggatga     960

gccaaggctg agagagatac ttagatttgc aaatgcttgt ggagccatta caaccaccaa    1020

aaagggagca atccctgctc tgcctactcc ggccgatgcc ctcaaactgg tgaaagaagg    1080

aaaataaact atttttttgt taatccgcac tgcattttat ccctttcctt ttcagctcct    1140

ggttctcaat tcaatcgaag agctgttgca atctcaaatt ttcgcctttt tctcttgagt    1200

gtaatgatgg aaaagacttg cgctttaagg tcttgccaaa taaaattttt cttttgtgtt    1260

agctttctcc aaatattata tttgtttaaa gctaaaattt ctgc                      1304

<210>  75
<211>  853
<212>  DNA
<213>  Populus trichocarpa

<400>  75
gaagtatttg gtgacaattg tcttcgtaca tgggtttccg ggtcttggtg gcgaaagtaa      60

aagacttcgt tatgggggag aaggagccca ggaaagctga atctgaggga gcttctctgc     120

ctacacaagc tgaagaacat ggacctgtta aagaagagaa agaagcccct ttaaatgatt     180

atgctaatga gaagagctcg gtcctggtta ctgaaaaggt tgcagatcca cctgctactg     240

caaaaaactc aagggggcca aatgacagag atgctgtgct tgcgagggtt gaagcggaga 300

aaagatgtgc tctaattaaa gcatgggaag aaaatgagaa agctaaagcg gagaacaggc 360

tcacaagaaa ctctctgcca ttggatcatg ggagacaatc aagagagaat ctgtggaggc 420

aaaaataaag aagtatgagg aaaaagtgga aaagaagaag gctgaatatg cagagaaaat 480

gaagaacaaa gtagccgaac tccacaaggc agccgaggag aagaaagcaa tgattgaagc 540

aaaaaaaggt gaggaccgtc tcgaggtaga ggaaactgca gcgaaattcc gagcaactgg 600

gtatacacca aggaagtgtc taagatgttt tggcaaattc taatgaacaa cagtggggta 660

aacgggagag aaaaggctgc tagaattgga tttgcttgat tttttggctt gttagtttat 720

gctgtgctca aatattgttg gttcagctgt aaatgtcttt tgatatttgt aaaaaacagt 780

aaatgggagt gcatgcgtta cattacccaa gttctgacaa gacccgagtt tgaatttgat 840

attttctttt gtg 853

<210> 76
<211> 849
<212> DNA
<213> Populus trichocarpa

<400> 76
caaacaagca ctggcactgg cactggcaat ggcaatgcca cttcttcttc aactaacacc 60

accacttctt cttcttcaac atcttcaagt tccatgagca gtaataacag taatggcaac 120

aatggttgtc agcagaacca aagtaaccag cttggagagg caagatcaag tctttattat 180

tcaacaaatg caatgtcttt tgttaccaag tctttgttgc ctactagaag aagactcagg 240

ctcgatcctc ctaacaagct cttcttccct tatgaaccag gaaaacaggt taggagtgcc 300

attggcataa aaaacactag caagtctcat gtagctttca agttccaaac aactgcacca 360

aagagctgtt acatgcgtcc tcccggggct atacttgcac ctggtgaaag tcttattgca 420

actgtattca agtttgtgga gcctccagag aacaatgaga gactactgga tcagaagagc 480

agggtaaagt tcaagatcat gagcttgaaa gtgaaaggag aaatggaata tgttcctgag 540

atgtttgatg agcagaagga tcaagtagca gttgagcaaa ttttacgagt tgtttttctt 600

gatccagaat gccctagccc tgcactggaa aagcttaaaa gacaactggc tgaggctgaa 660

gctgagctcg aagcacgcaa gaagcctcca gaagatgcag gcccccgagt tgttggcgaa 720

ggacttgtta tagatgagtg gaaagagcgg agggaaagat acctggcacg ccagcaggtt 780

gaagttgatt ctgtgtgaag atcctgtttc cttgcaagca cttgtttttt cttgagctga 840

ggtttcctt 849

<210> 77
<211> 1598
<212> DNA
<213> Populus trichocarpa

<400> 77
atgggtttta accgggtttt gcagaaaacc ttgtcctctt tagagttgcc tgtttatatt 60

gcaagtggac catctgcatc cgtttcagat ggtggtgaat cttcatcttg tgaactccaa 120

gagaggattg gtgactctat ccgtgatgaa attcctaggc cttctcaaaa tcctgggagg 180

cagaagcata agagtcatga tcctgctcga gatcttacga tccatgtatt ggaaaaattc 240

tctcttgtga ccaaatttgc tcgggacaca agttcacaac tatttcgaga aagtaatagc 300

aatggctatg gtgctgtaga aaggaaaagt tctagctact ctctccctga tgtcccccat 360

aagccatcca tggatgcaga gatagctctt gaggaaggtc ctgttccctc agatcctctg 420

gagttcgata aaatgacact cgtatgggga aaaccacgac agccaccctt ggggtcagaa 480

gagtgggcta ctttcttgga ttctgaaggg cgagtcatgg attcaaaagc cttaaaaaag 540

agaatattct atggtggagt tgagcacaca acacgcagag aggtctggcc atttttgctg 600

ggatatcatg cttatgattc aacgtatgcg gagagggaat acctcaagtc ttccaaaaag 660

tcagagtatg agacagtaag acaacaatgg cagagtatct ctactgaaca agcaaagaga 720

tttacaaaat ttagggaaag gaagggcctt atagacaaag atgtggtgag gactgataga 780

gcgctgtctt tctatgatgg ggatgataat ccaaatgtga atatcttacg tgatattctg 840

ttgacgtact ccttctataa ctttgatctt ggttactgtc agggtatgag tgacttacta 900

tccccaattt tgtttgtgat ggaggatgaa tcagaatcgt tttggtgttt tgtggcactg 960

atggaacgtc ttggacccaa ttttaatcgc gatcaaaatg gcatgcactc tcagcttttt 1020

gcattatcca agctggtgga gttgcttgat tgcccattac ataactattt caagcagaat 1080

gactgcttga attacttctt ttgtttccgc tgggttctaa tacaatttaa aagggaattt 1140

gaatacaaga aaacaatgcg attgtgggag gtgttgtgga cccattactt gtctgagcat 1200

ctgcacctgt atgtatgtgt tgcaatcttg aagcgatacc gcaagaagat aatgggggag 1260

cacatggact ttgacacgct cttgaaattt atcaatgagc tgagtggcca tattgacctt 1320

gacgcaatac ttagagatgc agaggctctg tgcatatgtg ctggtgagaa tggtgctgct 1380

cacatcccac caggaactcc accttcattg cctactgaga atgagaatgc tttattatat 1440

gctcaagatg atgaagtact gtaatacagt tcttctcatt ggtacgatat aagcggcttt 1500

attttttgtt ttcttcatca aatagctgtg tataattgct gttttttcga aataaaatgt 1560

taataatcaa gcaatcaact ccatttgctg tcctcctt 1598

<210> 78
<211> 1222
<212> DNA
<213> Populus trichocarpa

<400> 78
agagattgga gatgattaca gcagcccagc gagagagctc ttctttatgt tctgtttaag 60

aaagccatct tctttaaggc acaatcaaga actatgttca tcagtgagaa taacagatgc 120

acttgtccaa cacgagcaag aatcccagct caatattaat acaagcaaag atatattgct 180

ccggccctgg agtgatgata atgtggagac agagctcatg aggctgcata gtctctcagg 240

tccaccaaga tttctcttta caattgttga agaaacaaag gaggacttag agtctgaaga 300

tggcaggtct agaggtgatc ctaagagtgc gaaaggatca agaagtagaa gcttaagtga 360

tttgcttcgt actgtggaga ctccatatct aacccctctt tcttctccgc catttttcac 420

acctcctctt actcctagct ataatcagat tggattcaat catctctttg aatcgtcaaa 480

agatgcagag ttcaataaga taagatcatc accacctcca aaattcaagt tcttacagga 540

tgcggatgag aaactacata gaaggaaatt gatgcaagaa gcaggggaga aggtccaaag 600

gcatgatgtt tttgctcagg atcataccaa aatacctgct agttcaaatt cacacaaaga 660

tgaggatgat gggcctttta tcaccatcat tattgacagg aacaaagaaa gagagcttaa 720

tgaacaaaat catcaactac cagactacca gtcaagcact tcccaggtac ttcctcttgc 780

tacttcacct tcaacatcaa aatcagcagc caagaaaagt tccttctttc attagatctc 840

cgtgatcaag taaaacgaga tagttttttt ttttccccat gtatttctat taaccaactt 900

tagtcttctt tttaggagtt ctttttcttt aatattttta tgggatcgtg ttagaaggtt 960

aaccttgcca aacccttttg caatgtctga aaaatcttcg ggtgggtggg ggagttggac 1020

acactctctg cagtataatg ttaaaagttg gacaagtgca agtgggggtc ttgagggagt 1080

gatctgttgt cttcactctt catgaatctc tgagctgtct gatatctgta atcattctgg 1140

tgagtggtgg tactactgat gtttttgtcc aaaaggcact gtaagaattc tttcaagacc 1200

attcacttct ttatttattt tt 1222

<210> 79
<211> 1418
<212> DNA
<213> Populus trichocarpa

<400> 79
atggcatatg tatatgtgat tctgtttcca agatttcagc cagttattaa taagacgtat 60

gagactcccc atttcaatag ttctgtaagt gagtgcaatt attttgaggg aaactggata 120

caagatgaga gttacccttt atatgatgca tctcagtgtc ctttcgctga gaatggattt 180

aattgcttgg ctaatgggag aagagacgga ggttatacaa aatggaggtg gaagcctaaa 240

aattgtgaaa ttccgaggtt taatgcgcgt gaaattcttg aaaagatgcg tgggaagcga 300

attgtttttg ttggtgattc attgagtaga acacagtggg agtctttgat atgtatgctc 360

atgacaggtg tggaggacaa gagaagtgtt tatgaaatca atgggaataa aatcaccaag 420

cagattaggt ttttaggtgt acggtttagt tcttttgact tgagaattga tttctataga 480

tcagtttttc tggtgcagcc tggtccggca cctaggcgcg cgccaaagag ggttaaatca 540

acactgaaga tcgataagtt ggacgatatt cgcaatgaat ggattgattc tgatattcta 600

atatttaatt cagggcattg gtggacaccg agtaaacttt ttgaaatggg ctgttatttt 660

ctggtaggcg gatcgctgaa gctaggaatg cctatcactg ctgccttcga aagggcactg 720

catacatggg catcatggct taatactacc ataaatgcaa atagaacaag cgtgttcttc 780

cgcacttttg aatcttccca ttggagtggc cggaaccgcc tttcttgcaa agtgactcgg 840

cggccttcat caagaactgg agggagggat cgtagcctga tatctgacat cataatcaag 900

gttgtgaagg caatggcagt tcccgtaaca gttttgcatg tgacacccat gggtgcattc 960

cggagtgatg ctcatgttgg cacttggagt gataatccat ctgtgcctga ttgcagccac 1020

tggtgcttac ctggtgtacc tgatatgtgg aacgagattc tcttgtcaaa tatgttgtcc 1080

aggaattaat aggccattct ttcccataag ctagaaaatg caactgtgta gtgtgttcca 1140

tattcttccc tcttcaattg accccattca atttccaaac cttctactca actactttca 1200

atacatacga agattttctc tgctgagaag ttgtgtgcat ttgtcacaaa tattgtttgt 1260

tgtgtagttc agggatccaa aaattacttg atttgtcttt tctagttgca cttgagttca 1320

catgtaatgg taatgatttc tttccagcgt tagctgaaat ctggaagctc ttctcttcag 1380

atgccaccga gctgttgtac aataaaagcg ttattgct 1418

<210> 80
<211> 2038
<212> DNA
<213> Populus trichocarpa

<400> 80
aggtacccat ttttttgttg ttggaaacaa acccattttc tctctctgtc tttgtgtctt 60

tctttgataa attttaatca tcaacaacaa cgacaacagt aacttgcaaa gagagaaaga 120

gggaggagag agagcaaaaa agaaagggtc tctctcaccc tctcttattg ttatgtctcc 180

tcgcagatct tagattcttt cagtttccta tctgggttct tctttttgtt tgtcaggatt 240

cctggattgc ttttgattga tatttgattg ctcaaaggtt gtttctactt ttggattaaa 300

tccaagagtg accttttgtt gtaaatatcc aagctttgct ttgtattttt gacaagaatg 360

gaaatggagc ttggaaagtt gttcattggt gggatttcat gggacacgaa cgaagaccgt 420

cttaaggagt atttccgggc ttttggggaa gttttagaag ctgtcataat gaaggatcgg 480

gccacgggtc gtgctcgagg atttggtttt gttgttttcg cagaccctgc tgttgctgag 540

agagttgtga tggaaaagca ccttatagat ggtagaaatg ttgaggcaaa gaaggcagtt 600

cctagagagg atcagaacac cctgaacaaa aacagtagca gcgttaatgg ttcacctggc 660

cctgcccgaa caaagaagat atttgtagga ggtttagcat ctacagttac agagagtgac 720

tttaggaagt actttgatca gtttggggtg attacagatg tggtggtcat gtatgatcac 780

aacactcaaa ggccaagagg ttttggattc atcacctacg attcagagga agcagtggat 840

agagtattgc acaaaacctt tcatgaactc aacggtaaaa tggttgaggt caagcgggct 900

gtccccaaag agttatctcc aggaccagct aggaaccagt taggggggtt taactatagt 960

cctagtagag tcagtagctt cctcaatggt tacactcagg gatacaatcc aagttttgtt 1020

ggagggtatg gcgttagaat ggatggcagg tttagtcctg ttactgctgg gcggagtaac 1080

ttttctccat ttggtagtgg ttatgggatg ggattgaatt ttgagcaggt gttgaaccca 1140

gtttatgggg gaaattcaaa catcagttct aatgctggct atggacgagt aagtccttca 1200

tatagtggaa atgcaagcag gtacagcaac cccattggat ttagtggagg caatggagga 1260

agtagttctg tcttaaatcc aattgcccac tctttatggg gaaatggaag tattaatcat 1320

gcttctaaca ccacaaactc cagttctttt atgagttctg gaactgggag ctcaggaatg 1380

ggttctttta gcagtatggg agcactttgg ggttcctctg ctaattctga acaaggtgga 1440

ggagttcgcg cagtcaatag cagtaatctt agctttggtg gtggagattt tgatattggt 1500

ctagaagggg tagggtatgg aaggaacagt aggacaggtg ttgcaccagc atcatctcat 1560

gctgcatcta atggtggtta tgatggggct tatgcagact tttatgagaa gggctcatta 1620

tacggggata atacctggca atcttcacct tcggaactgg atgtgtctgg ctcatttggt 1680

tttgggcttg gaaatgcaac ttctgatgtt atgactaaaa attctgctgg ttatgttggt 1740

ggctatagtg ttgctaatag acaatcaaat agaggaattg ctgcatagga tgattatgat 1800

ataggtgacg gtgaagtgaa tttcgtacat gtttcttgtc cagatgatca tgtttcaact 1860

ttgctcatca caaggaaatc tgtgtatggt ttgagctttt gagatttttc taatataggt 1920

tttctttctt ggaattttat taagatgtaa aatcacattg tggggtatac tcaacatgat 1980

tgtatgatgc atctttggtg agagctatat taaaaaagga aacaattttt tagcatta 2038

<210> 81
<211> 1611
<212> DNA
<213> Populus trichocarpa

<400> 81
acactcttcc ctctctttac ttgtgcacgc ttaaagaaga gatcacacaa tggcaaaccc 60

ttctctctgt ctcctcctcc tcctctctct cctgacccca gccctcgtct cctcttcccc 120

ggttcaggac ccagaatttg tagtacaaga agtacatagg gccatcaatg cctctaggag 180

gaagttggga tatctctctt gtggaaccgg caaccccatt gatgactgct ggagatgcga 240

tcccaattgg gagaagaacc gccagaggct agctgattgt gcaattgggt ttggtaagaa 300

tgccattggt ggaagaaatg gtaagattta tgtggttaca gaatctggta atgatgatcc 360

cgtgaaccct aagccaggga ctctcaggca tgctgtcatt caagaagagc ctttgtggat 420

catttttgct cgtgacatga caattcaatt gaaggaagaa ctgatcatga actcgttcaa 480

gactatcgat ggtagaggtg caagtgtcca tattgccggt ggcccatgca ttactataca 540

gtatgtgacc aacattatta tacatggact aaacatacac gattgcaaga gaggagggaa 600

tgctatggtg agggactccc caaaccactt tggatggagg actgtatcag atggtgatgg 660

tgtgtccatc tttggtggtg ctcacatctg ggtggaccat aattcattgt caaattgtaa 720

cgatggactc gttgatgcca tccatgggtc ctcagccatc accatttcga acaattacat 780

gacccaccat gataaagtca tgcttctggg gcatagtgat tcctacactc aagacaagaa 840

catgcaagtc accatagcct tcaatcactt tggagaaggt cttgtccaga gaatgccaag 900

atgtagacat ggatatttcc atgtggtcaa caatgactac acccattggg aaatgtacgc 960

cattggaggg agtgctagcc caaccatcaa cagccaaggc aacagatttg tagcacctga 1020

catcaggttc agtaaagagg taacaaaaca tgaagatgca ccagagagtg aatggaagaa 1080

ttggaattgg aggtctgaag gagatctact attgaatgga gcatttttcg tagcatctgg 1140

cgcaggcgct tcatcaagct atgctagggc atcaagcttg ggtgcaagac catcttcact 1200

tgtcggtcca atcacgatgg gggcaggtgc acttaactgc aggaaggggg gtcgttgcta 1260

gctgattgtg aaagattaaa caatattttg tgacgagtaa tttggactat aattaattag 1320

ctggctagaa aagtaattaa gcagaaggga attaaaaaat gaggagggtg aagaaaagtc 1380

caagcactag tttcccctgg ctttttcttc cctccaaatt tttattcttt ttacccattg 1440

ttatctgctc ttatctttcc tcccccttct ccttttgtag tcaaaccttg aagattacaa 1500

cctcagcctg ttactctttg aacctaaggc attctccttg agaattcttg gtgggcatag 1560

agaaaacaag tgctcaagtg ttgatatagt atatatcaat tatctattca t 1611

<210> 82
<211> 1798
<212> DNA
<213> Populus trichocarpa

<400> 82
atataaaaat aatactaggt gctctccaat ggctgcaaaa ccatcaacag cgttcctttt 60

ctcttctata ttttaaagaa aaaacacctt ctttcaacaa ccttttgatg gattccatca 120

gaattagcat ctcttcaatc ctttcttgga ttcccttaga acatgttctt tgatagccaa 180

atcaccaaag ttgaacaaaa agagaaaatc ttgttgagtt cttgagctaa acgagaggag 240

gaggagaatt gtttttcgtt tttcaaatat ggaggagagg catgttattt ttgggaagta 300

tgaaatgggt aggcttttag ggaagggaac ttttgctaaa gtttactatg ggaaacactt 360

ggtgacagga gagagtgtgg caatcaaagt cataagcaaa gatcaagtca agaaagaagg 420

gatgatggag caaatccaga gagaaatctc agtcatgcgt cttgttcgtc atcccaacat 480

tgtagagctc aaggaagtca tggctaccaa gacaaaaatc ttcttcatca tggagtatgt 540

tcgaggagga gagttgtttg ccaaagtagc caaaggaagg ctgaaagaag aagctgctcg 600

aaaatatttc cagcaactaa tcagcgcaat tgattattgt catagcagag gtgtttatca 660

tagagatttg aagccagaga atatgttgct tgatgaagat gaaaacttga aaatctctga 720

ttttggcttg tcagccttgc ctgaacaatt tcggcaagat gggcttttgc atactcagtg 780

tggaactcct gcttatgttg ccccagaagt cttgagaaag aaaggctatg atggatcaaa 840

agctgatata tggtcatgtg gggtgattct ttatgtgctt cttgcaggat tcttgccatt 900

tcaagatgag aatgttatga agatgtacag gaaagtcttc aaggctgaat atcagtttcc 960

gccttggttt tcaacagatt ctaagaggtt gatttcaaga cttcttgttg ctgatcctga 1020

aaagagaatc acaattcctg ccataatgag aaatcattgg tttcttaaag ggttttcaag 1080

accagtggcc ttttctatcc aagaatcaag catggatcaa acaggacaag aacaagatct 1140

tgattcttgt tctgttgtca acaccaaggt atcatcacca gaattcttca atgcattcga 1200

gtttatttcc tcaatgtcat ccggctttga tctgtctagt ctttttgaga ctaagaggaa 1260

atcaggctct atgttcactt caaaattttc agcgagtgct atcatggaaa aaattgaagg 1320

ggttgcaaaa gggctgagtt ataaggtggc aaaagtcaaa gatttcaaag tgacgttaca 1380

aggtccatgc gagggtagaa aggggaagct ggcagtgacg gcggaggtgt tcgaggtggc 1440

accggaggtt gcggtggtgg agttctccaa gtcttccgga gataccttgg agtatactaa 1500

gttctgtgag gaagatgtta ggcctgcact aaaagacatc gtttggacat ggcaaggaga 1560

caatgtttgt aataaagata acaacaatag tcatgtagaa gatcgtgaaa ttcaaatgtt 1620

gtagaaatta aggctttgat cttaatttgg tgagttaatt ttaggatttt atataatctg 1680

taaataagtt ttcgttattt aattaatgca tcaagttgag tgtttttgtt aagctagcac 1740

agtgtgtgtc tatatatata tatatatata tatattatta agcttgaatt caagatta 1798

<210> 83
<211> 1178
<212> DNA
<213> Populus trichocarpa

<400> 83
gtattttctg tggaggagaa acaaaaagca atctcttggg ttttaaagat ttggtttggg 60

ttctctcttt gaaagttatc aagaacagta aaggggctct tgattgactg atgcaagttt 120

aagattttct attcttttta gagaaagaag gaagttttct tttgaaatga atttgatgtg 180

tgaggttttc tttgctgaag aagaagagaa ggttgaagaa ggagagtggg aaaaggagag 240

atagtattta ccaaagccaa ggtagctagc agaagtagag aggcagatag ctataaacaa 300

catacaaaat aaaatttcat ctaaggggtt ttagtttgtt ctgcttctgc tgggtaattg 360

gcaatgtcta ctactataat cagtgaccct atggtggtct cagcaccaga gacacaagca 420

acagcagcag ctgcagctgc agcaacgaca gcaacacaac tcattgcaca gatagaggtt 480

gagtcagtca aatgtgattg ctgtggccta atcgaagagt gcacaccagc atacattgaa 540

agagtacgcg aaagatatca tggaaaatgg atttgtgggt tatgtgcgga ggctataaaa 600

tatgagattg taaggactga gaggcttatt agcactgaag aagcaatgac aaagcacatg 660

aacttctgca agaagtttgt ttcctcaggg ccacctcctg atccaacaac tcatttgata 720

gccgccatga gacaaatcct gagaagaagc ttggattctc cgagaggttt aaggtcaacc 780

ccaagtagtc caaacaaaac gaacggagca attcgtgccg ctgcacttgc taggtccgaa 840

agttgcttcc ctgctctgtc tggttgatat ataatactga gcatcgtgga agacggggtg 900

attcataaaa tggtgatgaa gctattggaa aaaaaaaaga cagaaaaaaa cacattccat 960

tttcatgcaa ataagaaaga tcaaaggatt gaagttaggt gcctaatcta gctacttata 1020

tagttgttaa atacagaact tcctgtcaca agaaaaatca cggaactccc ttttttcctg 1080

aagctttata tttcagctgt acgtacttgt gtaatctgtc tttttaaaac catggcttcc 1140

ttcctctttt taccaaatta tatttctcaa ttttgata 1178

<210> 84
<211> 1010
<212> DNA
<213> Populus trichocarpa

<400> 84
gtcaagataa agtcccattc tttttccttg ccccaagttt gtttgagggg ttataaaaaa 60

tgaaccaaga gatgaatggt gttgatacag agattgatca gaaccaccaa gagaatgtgc 120

aagagaaaat cgattatgtg tttaaggtgg tggtgatcgg tgactctgca gtaggcaaga 180

cgcaaattct ttccaggttt accaagaatg aattctgctt tgattcaaag tctaccatcg 240

gtgtcgagtt ccagactagg actgtcatca ttaaagacaa ggtcatcaag gctcagatct 300

gggatactgc tggccaagaa aggtaccggg cagtgacaag cgcatactat agaggggcat 360

taggggctat gttagtctac gacattacca agagaccaac gtttgatcat gtggctaggt 420

gggtggagga gctccgagcc catgctgaca actcaattgt gatcatgctg attggaaaca 480

aggctgatct tgtggacctc agggcagttc caacagaaga cgcggtggaa tttgcagagg 540

agcaaggcct atttttttct gagacatcag cccttagtgg tgacaatgtg gacggtgcat 600

ttttcaggct gctagaagaa atttacggtg tgatttgtaa gaagtcattg gaatgtggca 660

atggaaatcc ccatgctgct gatgccataa cgcttagagg ttctaagatt gatggcatat 720

cagggactga tctggagatt agtgagatga agaaattatc tgcttgctcg tgttgatttg 780

atcatttttc tagtgaattg tgtactataa gactttacca ctcgatgttc ttaattgatt 840

ctgtggcttt ctttggaaag tggtgatcgt ttcggtagtg tggtaagggt ggcaagtttt 900

ttcttctctg tgacctgtca agattttagc agtattgtac ttgtcttaca gaaaccatga 960

atttggtttt tttatatgta ttgatttgga tggatggttt tccttttcct 1010

<210> 85
<211> 1159
<212> DNA

<213>  Populus trichocarpa

<400>  85
atgaactaga aattcaaagc ttccatcaca tattttaatg gcttacaatc cttcttcaag      60

gtcctgtttt tatgattctt tttgtttcct attcattaca ttcgttcttc catttttctt     120

cctcacaaaa gctcaagctg caagccattg tagaacctca tgtggtacca ttccaataaa     180

ctaccctttt ggcatcgatg atggctgtgg cagtccatat tacaggcaca tgcttttatg     240

ctccgattcg ggcattctcg agcttcgaac gccttccggg agataccaag ttcgtagcat     300

aagctactca gaccctcaca tgatagtcac cgatccattc atgtggaagt gtaaagatgg     360

tcatcacttt cgtgcaacta gggcatttag ccttgataca agcacacatt taacactctc     420

ctctcaaaat gactacctct tcttcaattg cagtgaagag aaagtgattg ttgagccaaa     480

acctatcttc tgcgagaggt ttcctgatcg gtgcgactcg acatgtgata gtgctagtta     540

cctttgcagg cacttgccag gatgtggtgc tgcattagga ggacgttctt gctgctctta     600

cttcccaaaa gcgaccgaat ctttgaggct gatgctgaag tattgtgcta gttacactag     660

tatttattgg agaattaatg gtgcaaatgc tcctgatgat catgtacctg agtatggtat     720

tagagttgat tttgacattc cagtgactac agattgcctt caatgtcaag acatgaagaa     780

aggaggtgga agatgtggat ttgacacaca atcacagaat ttcctatgtc tgtgcaatca     840

gagatcaaat gtcacaacat attgcaatgg tatatcacag cagcagtagc catagcaagg     900

caggaataat tgcagggact gtaactggag tttcagctgc tggggcatta ggaattggtg     960

ctggtctatg gtattggaag aaagtgagag cctcagcacc agtaacatgt ggggttcaaa    1020

gcaatgagaa taggctcttt taagaacaaa tcaacaaaag tgaataccaa tcactttctc    1080

ttttctaatt tccctatttt ttgagttgcc tttcgcactt taataagcaa cttaatggat    1140

ctgttttctt tgttttaaa                                                 1159


<210>  86
<211>  884
<212>  DNA
<213>  Populus trichocarpa

<400>  86
cattatccca aaaaacgagt aaggcatata cagcagccaa aaccatgtca cccggtcccg      60

tcgttgaggc tgaacccgcc gccgccggtg gaagatacac agcagaagaa gcctcctcac     120

aggacgtgga tgaacctgtg gtggaggacg tgaaagaaga tgagaaggaa gaggacgatg     180

atgatgatga tgaggacgat gatgatgaag atgatgacaa ggatgatgat accccaggtg     240

ctaatgggag ttccaagcag agcagaagtg aaaagaagag tcgcaaggca atgttgaagc     300

ttggcatgaa acctgttact ggtgttagca gggtcaccat caagagaacc aaaaatatac     360

tgttttttat ctcaaagcct gatgtcttca agagcccaaa ttctgagacc tatatcatat     420

ttggagaggc aaagatagag gatttgagct ctcagctgca gacacaggct gctcagcagt     480

ttagggtgcc agacatgtca tctatgttac caaaaccaga tgcttctact gcagctgctg 540

ctgcaccagc agatgaagaa gaggaagaag tcgatgagac aggggttgag cctagggaca 600

ttgatcttgt tatgacacag gctggagttt ctaggagcaa ggctgtcaag gctctccaga 660

cgaataatgg ggacattgtc agtgctatca tggagcttac tacttaggat ggctccctgg 720

ttactctctt attttatgct gacaagttct cggaacaatt taatcatggt agtcaaattg 780

gcttgccatc tatgaggtcg ctaattatcc attgtttgtg tcaaatctga gatttttacc 840

tattgcggtt tttctttagt agcaggctct tattgtgctc tttg 884

<210> 87
<211> 2371
<212> DNA
<213> Populus trichocarpa

<400> 87
atgccattga atagccaaat ctctctctct cttagaatgt gaaggagcag ttagcgaagg 60

aaaaattgga gaatgccgga gctggtgtac caagagcaga gctcgtcgag atcaggattt 120

agagctcgag atgcaagtcc tgactctgta attttcactc tagagtccaa cttcagtctc 180

ttctcttctg cttctgctag cgtcgatcgc tgctcttttg cttccgatgc tcatgatcat 240

gactctctcg cctctgaaat ctctctggta aaaccatttc tctaacttcc ctgcatgcat 300

gcgttttcta acccatggtt tttgagtgag ttttttgaga gtgcaatgca gcatttggca 360

gctggtcatg atcaacaaga gaactcttcg agtggtccag atcgaaacaa caacaagcag 420

aagcagcaca ctcacacccg tctctccaga aaagcagaaa aagttaaagc tgttaaaaaa 480

gaagacaaca gaattagttc tgtagaagac gatattcatc tcctagattc tgcaagaagc 540

tccttctctc tcgctctcaa agagtgtcag gagaggagac cgagatctga agcgatcaca 600

aagaaaccag acaggcggag gcctgcgtca ctagatctta acaatgtggt cacatcttct 660

tcgccgagat tgggtaacat gaagaagagt atagcttgtt catctcgaaa atctggtaca 720

tttccgagtc ctgggacccc aaattacctt aatcagtatc attctagtgt tggaatgcag 780

aagggttgga gttcagagag agtgccgttg ccaaataata gtaataggcg gcaagtgatg 840

aatactactg gagctgctgt tttgccttat aataataatg gaaggacatt gccgtccaaa 900

tgggaggatg ctgagaggtg gatatttagt cctgtttcgg gagatggggt tgttaggagt 960

tcgattcagc ctgcacagag gaggcctaag tcaaagagtg gaccacttgg accaccggga 1020

gttgcgtatt attccttgta ttcgcctggg atgcaggtgt tcgatggagg gaatgcgggg 1080

aattttgttg ctggttctcc gttttcagct ggtgttattg ctgctgatgg attgggtatt 1140

aggtcacatg ggagccatgg tgtgtcattt cccatgcgga cagagccctg tatggctcgt 1200

tcggttagtg tgcatggttg ttctgagatg gtagctcagt cttcgttgcc atcacaagat 1260

gaaaagcttg atggtgtcaa ggatgctgca actgatatct cccgtgttgt ttcaagaagg 1320

gacatggcca cccaaatgag ccctgtaggt agtaatcatt catctcccac caggaagcca 1380

tcattctcca cctccacccc ctctgtccta cctattgtgg aactgcagag cgtaccttcc 1440

tctagatctg agaccaggga tgtacaggtg gatgagaggg ttactgtcac aaggtggtct 1500

aagaaacaca gagcccgcaa ccatggaaaa agttcacaag ttgttgatga ctggagaaag 1560

aaagctgctg atactctttc atcaggttgg gatgtttcag aggctggaaa gagcatttca 1620

aaggtcaaaa gagaggaagc taaaatcaca gcatgggaga acctgcagaa ggcaaaagct 1680

gaggcagaaa taaggaaact tgaggttctt tttccccacg cgttttcgtc gtacaatttt 1740

gactaacttg aaggattaat tcacttaatg atcagccttg gtgcagcaaa gaagatattt 1800

tctctaatat ttcctttctg ctcacttgaa agctttgcta gtacttaatt ataagatact 1860

ggtcttaact gactagattc attgcagatg aagcttgaga aaaagagatc atcatctatg 1920

gatagaatta tgaataagct gagatcagct caaaagagag cccaggaaat gaggagctcg 1980

gtgctagcaa accaggcaca tcaagtttcc acgaactctc acaaagttat atcattccgc 2040

agaacccgtc agaagggttc cctgagtggt tgtttcacat gccatgcttt ctgatatctc 2100

atccagccac atgttggtac acttcgatag atataaaacc tggtcaaatt gcaggaaaaa 2160

ctgcagcaga atatggaagc gtataggagg gaaaccacca ttgacactat tttggtgtca 2220

atggagttga gttggggcgt gaagaacaag tactactgtt aagatttcag acgggacaca 2280

agaccctgga tggtatcaaa atctccagga aatttgtcta ctaaaccttg tatgcatgta 2340

tgtatgtatg tatatcaaca attattatta t 2371

<210> 88
<211> 2514
<212> DNA
<213> Populus trichocarpa

<400> 88
tttttttta aacctcctcc tcggatatta tatacttcgt ttctctcttt ctctgccatt 60

aatcatacct cacaccgatc cagattttgt ttgctcataa acagagggag tgggagagac 120

ccccgtttaa cttttctttt tttccttttg aagtaatacg acatctccct ctcctcctcc 180

tcctcctcct cctcctctat ttgttcttga tttcgattat ctgatcggac ggtcacggtc 240

ttatttgtat tcacctgcaa tatcgtacgg ccagggatct ctcttctctt cttactgagc 300

aagttgcatc tcataagcac tggcgatcat ggagctgaat ggtcaaacaa gagtgagaag 360

aaaggaccat tttgctcata caaacggtga tttagcatta ccaagtgttg gtgatgtaga 420

tccctggact gcatgggcat ataagcctcg aactatttcg ttgttactta ttggtgcttg 480

ctttctaata tgggcaagtg gagccctaga tccagagagc tgcacatctg gtgatgttgt 540

tacatctgtg aaaaggggta tatgggcaat gactgcagtt tttcttggtt attgcttgct 600

acaggcccct tcaacggttc taataaggcc acatccagca atttggcgct tagttcatgg 660

attggccatt gtttatcttg ttgccctcac atttctgctt tttcagaagc gtgatgatgc 720

gcggcaattt atgaagtttc tccatcctga ccttggaatt gaactacctg aaagatcata 780

tggtgctgat tgtcgcattt atgtgcctga aaatcctaca agcaagttta agaacgtttt 840

ggacactctt tttgatgaat ttgttctagc acatatcttt ggatggtggg gcaaggctat 900

attaatccgt aatcagccac ttctatgggt attgtcaatt ggttttgagc tgatggagtt 960

taccttccgc cacatgctac caaacttcaa tgaatgctgg tgggacagta tcattcttga 1020

tattttgata tgcaattggt ttggcatttg ggctggaatg catacagtcg ggtattttga 1080

tggaaaaaca tacgagtggg ttggtataag tcgccaacct aatattatga gcaaggtcaa 1140

acgaacattg gaacaattta cacctgcaca gtgggacaaa gatgaatggc atcccttgct 1200

tggtccatgg cgatttatcc aagttcttag tctctgtatt gtcttcctga ctgtagagct 1260

caacacattc tttttgaagt ttgtctatgg ggttcctcct cggaaccctg taatagtgta 1320

caggttgatc ttgtggtggc taattgccat acctacaaca cgtgaataca attcatatct 1380

ccaagaccga aagcctgtga aaaaggtagg cgctttttgt tggctttccc ttgctatttg 1440

catcgtggaa cttctcattt gcatcaagtt tggacatggt ctttatccca aaccaatgcc 1500

tgcatggttg gtcatcttct ggacatctgt tggagttagc cttgtaatat tcctgattat 1560

gtggtcatgg aaaagtttgg gaagaaagag acgatgaatt tgctagcatg gtacactatt 1620

cttttatttc ttcatgttct tatgggtata catcttgtaa atgccctata gcttcatgga 1680

attgtaaatg cttattctct taggttagtg gatcacagtt ttagagtatt caattctttt 1740

atgataggta gttggaaaat gcctctaatg gtaggttcat gtgttgctaa tttgcagcaa 1800

tcatcaaatt ggtaattttc ccttttaact tgtctgagag aggagcaatt tttaacaccg 1860

ggcttgcctc acatttttca ttggaaaatg catgcctgac aattccattg ggcattttta 1920

tcagaatgtt taaaatattt atattttgcg tcggctctat agcatatgct tataagcatt 1980

gaaatatgat tcaactgaag tattcgcaca tgttttgctg tttacgttta tatgattgtt 2040

gtgtttagtt cgccacaagc agcctggcag ttattcgatc cggtatcttg acgttatctt 2100

atcacctgct gtaaatccta tggctcaaac tggcaattat tcaaggcagt tcttgaaaga 2160

gttcaaggtt ttctttcctg ttgatcgacc tttgtaaagc ctatgaagca attgtgcaaa 2220

taattggtgc tcgacagaag gcaatctcta gtttgctcac gacaattcta gccgattttc 2280

gggaaaacat ttgtggactg gagtcattga ttcaatcaag gtgaaattgg aaatcattta 2340

gaaaagcaat tcctgttctt tgacaacact aggttcttct ttctcgatcc gtgcccatgt 2400

cactgtgtgt atatatagaa atagaaaaag tgtatctctc ttaatcatgc aggattttgt 2460

tttttgcagt caatatgccc atgacttata atctcagtct tcttcgaaaa cact 2514

<210> 89

<211>  852
<212>  DNA
<213>  Populus trichocarpa

<400>  89
acgaacaagc aatgatagag ttcgagaggt catcttcttg ttataaaatc ctcaaaagct      60

cgaggtgaaa aaataagcaa gggagaggag agagctagct agctagtcaa gttaagaggc     120

agaaaacaag aatgtcttcc aggaagaaaa agaaggcagc tctttatgag aagttacgtg     180

ctgctaccaa ttctaatgct atgaacaaaa cctcaatcat agtagacgcg tcaaaatata     240

ttggagagtt gaagaacaag gtggataggc taaaaaaaga aatcggaaca tcttcaaccc     300

cccaaaattc attacctgcg caggtcacag tggaaaacct agaaaagggt ttccttatta     360

atgtattttc aggaaagaat tgccctggat tacttgtctc catacttgaa gccttcgagg     420

aactaggcct tgatgtgctt gatgctaggg tttcttgtga agacaatttc caacttgaag     480

cgattggtgg agaccaaaac caaggccatg atgctcaagt agtgaaacag gcagtgctgc     540

aagctatcca taactggaat gaaggcagct agctagtgag caagaatgac ccaaatattt     600

tcttccccta tcttcctgta cgctactgag aaattgggaa tttgtatatt gaatttcaag     660

cacactaggc agtcctaatt ccaagttagt agtcagtcaa aatgtgcagc aatgggattc     720

tggtaattaa ttttcagttt ttaattaagc aacttttacc ttcgatgatc tatacatata     780

tttgagcata aaatgcttaa ttaaaatccg tatgattggt ttcagctaat taaggagctc     840

tttggcaaga tc                                                         852


<210>  90
<211>  1320
<212>  DNA
<213>  Populus trichocarpa

<400>  90
agagttccat catccattgt cttaccactt catgggaatg tctatcctac tgggttctat      60

aatgtgaccc tcaacattgg ccaaccatca aaaccttact ttctcgatgt agatactgga     120

agtgatctta catggctcca atgtgatgtt ccccgtgccc aatgcacaga ggcaccgcat     180

ccatattaca aacccagtaa caatctagtg gcttgtaagg accccatttg tcaatccttg     240

cacaccggtg gtgaccaaag atgtgaaaac ccaggacagt gtgactatga ggttgagtat     300

gctgatggtg gatcatccct tggcgtcctt gtcaaggatg cctttaatct caactttaca     360

agtgaaaagc gccagagtcc tcttctggcc cttgggttgt gtggatatga tcagcttcct     420

ggtgggactt atcatcccat agacggagtg cttggtcttg gccggggaaa acctagcatt     480

gtatcacagc ttagtggtct gggtttagtg agaaatgtga ttggacactg tttgagtggg     540

cgcggtggag gattcctctt cttcggggat gatctttatg attcatctcg tgtagcttgg     600

acaccaatgt cacccaatgc gaaacactat tcacctggat ttgctgaatt gacttttgat     660

gggaaaacta ctgggttcaa aaacctgatc gtagcttttg atagtggggc ctcttatact     720

taccttaatt ctcaggtgta tcaaggtcta atttctctga taaagagaga attatccacg 780

aagcctttga gagaagcact ggatgatcag acgcttccga tttgctggaa aggacggaaa 840

cctttcaaaa gcgtacgtga tgtcaagaaa tacttcaaga cctttgcatt gagctttgcg 900

aatgacggaa aatctaaaac tcagcttgaa ttcccaccag aagcttatct tatagtatca 960

tctaagggaa acgcctgctt gggagttcta aatggtacag aagtaggtct gaatgatctg 1020

aatgtcattg gagacatatc aatgcaagac agagtggtga tttatgacaa tgagaagcaa 1080

ctgattggat gggcacctcg aaattgcgac aggattccta aatccagaag cattatcatt 1140

tgatgacagg tgttctaaac gttaaaaaaa acttgtcctg tacagaaagc tgctaagctg 1200

taacattgta ttcttccacc attacagcag attccttgta aagtaatact atgtatatgt 1260

ggtctggtga atcagacccc acaattatac ttgatgggtt atgaaaattt aataaaataa 1320

<210> 91
<211> 2811
<212> DNA
<213> Populus trichocarpa

<400> 91
atggaaatgt cctgcaagga tggtaagcag cctatcatgg acaatggcaa gtatgtccgg 60

tacacgcctg agcaggtcga agccctggaa aggctctatc atgattgtcc caaacccagc 120

tccattcgcc gccagcagct cattagggag tgtccaattc tctccaatat tgagcccaaa 180

caaatcaaag tttggttcca aaatagaaga tgtcgagaga aacagaggaa agaagcgtct 240

cgcctccagg cggtgaatag gaagctgagt gcaatgaaca agcttctgat ggaagagaat 300

gataggttgc agaagcaggt gtcgcagctg gtgtatgaga atggctactt tcgccaacat 360

acccataaca caccgcttgc aaccaaagat acaagctgtg aatcagtggt gacaagcggt 420

caacaccacc tgacacctca gcatccgcca agggatgcta gtcctgcagg gcttttgtcc 480

attgcagaag agactttaac agagtttctt tcgaaggcta ctggaactgc tgtagagtgg 540

gtccaaatgc ctggaatgaa gcctggtccg gattccagtg gaatcgttgc tatttctcat 600

ggttgtgctg gtgtgggagc acgagcttgt ggcctagtgg gtcttgaacc tacaagggtt 660

gctgaaatcc tcaaggatcg gccatcatgg tttcgtgatt gccgagctgt ggatgtgctt 720

aatgtgctgc ccaccgcaaa tggtggaacc attgagctgc tttatatgca gctctatgcg 780

ccaactacgt tggcacctgg tcgtgacttc tggttgttgc gttatacttc tgttttagaa 840

gatggaagcc ttgtggtttg tgagagatct ctgaaaaata ctcaaaatgg cccgagcatg 900

ccaccagtgc agcattttgt gagagcagaa atgctcccaa gtgggtatct ggtacggcct 960

tgtgaaggtg gtggttcaat catacacata gttgaccaca tggatttgga gccttggagt 1020

gtgcctgaag tactacggcc gctgtatgaa tcctcaactg tacttgctca aaagacaaca 1080

atggtggctc tacgccagct gcggcagata gctcaggaag cttctcagtc caatgtgacc 1140

aactggggca gacgacctgc agctctacga gcactgagcc agaggttgag caggggtttt 1200

aatgaggctc tcaatggatt tagtgatgag ggatggtcaa tgatcggaaa tgatggcatg 1260

gatgatgtta ctatcctcgt gaactcatct cctgacaagt tgatgggttc aaatctttcc 1320

ttcactaatg ggtttccagc tgtcagcagt gctgtcctgt gtgctaaagc atcgatgctt 1380

ttacagaatg tccctcctgc aatccttctc agattcttgc gagagcacag gtcagaatgg 1440

gcagataaca atattgatgc ctatgcagct gcagcagtta aagttggtcc ttttagccta 1500

caaggttctc gagttggaag tttcgggggt caagttatac ttccgctggc tcacactatt 1560

gaacatgaag agttcctgga ggtcataaaa ctggaaggtg ttggccattc tcctgaagat 1620

ccaataatgc ccagagatgt gtttctttta caactctgct gtggaatgga cgagaatgct 1680

gttggaacat gtgctgaact tatatttgct cccattgatg ctacttttgc tgatgatgca 1740

ccgcttttac cttctggttt tcgcatcatt ccccttgatt ctgggaagga agcctccagt 1800

ccaaatcgta ccttagatct tgcggctgct cttgaagtcg gaccagctgg aaacagagca 1860

tccagtgatc attctgctaa ttctggttgc acaagatctg taatgacaat cgcatttgaa 1920

tttgcgtttg agagccacat gcaagaacat gtagcatcaa tgactcggca atatatccgc 1980

agcattatat cttcagttca gagggtggca ttagcactat ctcctcatct tggttcacag 2040

gctggtcttc ggtcaccact gggtactcct gaagcacaga cacttgctcg ttggatctgc 2100

cagagctaca ggagctattt gggtgtggag ctactcaaat ccaatggcga aggaagtgaa 2160

tctattctga aaaccttgtg gcatcattca gatgctatta tgtgctgctc actgaaggcg 2220

ttgcccatct ttacttttgc aaaccaagct ggacttgaca tgcttgagac aaccttagtt 2280

gcactgcaag acataacttt ggaaaagata tttgatgatc atggaagaaa aactctctgc 2340

tcagaattct cacagattat gcaacagggt tttacttgtc ttcaaggtgg tatctgtttg 2400

tcaagcatgg gcagaccagt ttcatatgaa agagctgtgg cctggaaagt gttgaatgaa 2460

gaagaaaatg cgcattgcat ctgctttatg tttataaact ggtcttttgt ctgagacttg 2520

aaaccagaat ctagtggagt ggctgattta agagtgagaa ctctatatct gtcttatttt 2580

gctggcttgt atttctttta tgtataacat gttgcaaccg tggagcacct agttcatttg 2640

ctatgttata aatttgtgtg gggttcatta tagtagagat tatggacgag acgatatagt 2700

tgccggggat cattttgttt ctgactggtt tgttcttaag tcctcacctg ggtctatgtt 2760

gattattgca accgaaatct gtcaatcctg gtttttcctg tgttgcttac a 2811

<210> 92
<211> 886
<212> DNA
<213> Populus trichocarpa

<400> 92
gttcagggaa tcagcctagc caagaagaga ttacaacgcc agaagctaag tcacactgat 60

cagagcaaca aaaaaagtac accaacaatg tcttccaccc caacaaatga atggcaaacc 120

tactggtgtc atgaatgtga cctcagcatc caccttctca ccaccaccac tcctctttgt 180

cctcactgcc accatgactt tcttgaactc atggacccca ttcccacctc caccgccgct 240

gacaccacca ctttcctcct tgacagcccc tccttcctca acttccttca acatctcaac 300

accaatagcc attgcgattg tgaagatgat aatataaacg ccactattga ctctatcatc 360

cccaccataa aaatcacttc ttgcatgcta gaaatggatc caatgcttgt gtgtgcagtg 420

tgtaaagatc agttcttgat tgatgttgag gccaagcagc tgccctgcag tcacttgtac 480

catccaggct gcattctccc ctggctttct aaccacaact cttgtcctct ctgtcgcttc 540

cagttacaaa cgccagtagt cagagaggag aatttggaaa attggtctcc tgatcatcct 600

catcatgatg ctaatcatgc tcatgtcggg gttttgtcca cctctcttcc tccacacttt 660

tgaatttgaa tgcaggtaaa taaacgcttc tggggatttt gggtttttgg tttggaattg 720

atcgagtgaa gtgtaaatca acagtgaacg attgtgttac acaaagaact tgtgatggtt 780

gtgcatctcc ttctcccctt cagactttca ctatggatat tgtaaagtaa taccttgttt 840

gggattggat tttggccaga aatacagcgt ggttgaattg attttt 886

<210> 93
<211> 887
<212> DNA
<213> Populus trichocarpa

<400> 93
gacccccttc aatcgattgc aaggcatgaa gcgtcagctc cagcattgac atcccttctt 60

ccacagaaat tcctgctaag caagcgcaga aagtccagtt ctgacccgat gatgaacttt 120

gttagggaat atcataatga tttggaagtt gtgtatgttg ggcaactctg cctttcatgg 180

gaaatccttc attggcagta tgaaaaggca ctcgagctat gggattccga cccttatggg 240

atgcgacagt acaatgaagt tgctggtgaa tttcaacagt ttcaagtgat attgcaaaga 300

tttatagaga atgaaccttt tgaaggtcca cgggtgaaaa attacattaa gaatcgatac 360

gtgttgcgta atctccttca agttccggta ataaaagagg acagtatgaa ggataaaaag 420

gcaagacgaa aaggaaggga tgatggttct attacaagtg atatgctagt agagataatg 480

gaagaatcaa taaggatttt ttggcgattt gttcgatctg ataaagatgc acaaaacgtg 540

atttcaaagg gtcgcaaagg aactcaaata gagccccaag atcccactga actagagtta 600

ttgacagagg tgcgaacgaa tttccaaaag aaggagagga ggcttaaaga cgtcttgaga 660

agtggaaact gcatactgaa gaagttccaa aaacatcgag aggacaattc taatcaagtt 720

ctttacttct tctctcaagt ggatatgaag ttagtagcga gggttctgag catgtccaga 780

gtaacaacag accagctact atggtgtcac aataaattaa gcaagataaa ttttgttagc 840

cggaagatac acgtagagcc atcatttttg ctttttccat catgatg 887

<210> 94
<211> 1471
<212> DNA
<213> Populus tremula x tremuloides

<400> 94
ccccatttcg gtttttagtc gaaatcttgg gttgggggct ggaaatatga ggacatgctt 60

gtgggttttt gctttggttc ttgttttcgg ttttgttgat ggaaaaaaaa ttgaaaggtt 120

gcgcacagag aggatttcag gaagcgctgg ggatgtgttg gatgatgatc cagtgggaag 180

gttgaaggtc tatgtttatg agcttccgag taaatacaac aagaaactgc tgcagaagga 240

ccccagatgt ctcacccata tgtttgctgc agaaatcttc atgcatagat ttctcttatc 300

cagcccagtt cgaaccctta atccagatga agcagattgg ttttattccc ctatataccc 360

cacttgtgat ctcacaccca tgggcttgcc gttgcccttc aaatctccta ggatgatgag 420

aagtgcgata cagctaatct cctccaattg gccttactgg aatcgtacgg aaggggctga 480

tcactttttt gttgtgcccc atgactttgg agcctgcttc cactatcagg aagagaaagc 540

cgttgagcgg ggcattcttc cgctactcca gcgttctact ttggttcaaa cttttgggca 600

acgaaatcat gtgtgcttga atgaggggtc aattacaatt cctccttttg ctcctcctca 660

aaagatgcag gcccaccaga tcccccctga cattccacgg tccatttttg tctatttccg 720

tggattgttt tatgatgtaa ataatgatcc agaaggtggt tattatgcaa gaggagcaag 780

ggctgcagtt tgggagaatt tcaagaacaa tccactcttc gacatctcca ctgaccatcc 840

aacaacatat tatgaagaca tgcagcgagc tatattttgc ttgtgcccac tcggttgggc 900

tccatggagc cctagattag ttgaagcagt ggtatttgga tgcattcctg tcatcatagc 960

agatgatatt gttttgccat ttgccgatgc tatcccatgg gaggaaattg gggtgtttgt 1020

agcagaggaa gatgtcccta acctggacac aatcctaaca tccataccac cagaagtgat 1080

tttaaggaaa caaaggcttc ttgcaaatcc ttctatgaaa cgtgcaatgt tattcccaca 1140

acctgcacaa ccaggtgatg ctttccacca aatcctaaac gggctggctc gtaagttgcc 1200

gcacgacagg agtgtttact tgaagtctgg tcagaatatt ttgaattgga cagcaggacc 1260

agttggggac ctgaaacctt ggtaagagaa gtgtcatttc ctcaacagac atggctgtgg 1320

tgtatctact gtttcatcta atcgcaagga ctgtaaactt ctctttgaag ttttgatttt 1380

tgtatagatt cttgaatttc acgtgtacat ctgggacata gttcgatgct ttaaataact 1440

tcgcattttt cttggtaaaa aaaaaaaaaa a 1471

<210> 95
<211> 1351
<212> DNA
<213> Populus tremula x tremuloides

<400> 95

cagcttcatt gccttgccct gaggctagcc agtgagcact ccaccaatgc agctgctcga 60

ctgcagctcc ccttgccgga actcgtccct gcgctcgttg acaatactta ttttcacttt 120

gtcctggctt ccgacaatgt gctcgccgct gctgttgttg ccaattcact tgtccaaaat 180

gcgctgcgcc cccagaagtt tgtgttacac ataataacag ataggaagac ctactcaccg 240

atgcaggcat ggttctcact gcaccctctg gctccggcaa taattgaggt caaggcattg 300

catcatttcg attggttcgc aaaggggaag gtgccagtca tggaagcaat ggaaaaggat 360

cagagggtga ggtcacagtt tagagggggt tcgtctgcca ttgtagcaaa taacactgag 420

aagcctcatg ttatcgcagc aaaactacaa acacttagtc ccaaatacaa ttcagtgatg 480

aaccacatcc gaattcatct acctgagctg ttcccaagcc taaacaaggt tgtgttccta 540

gacgatgaca tagtggtgca gtccgatctt tcgccgctct gggacatcga catggatggg 600

aaggttaacg gggcagtaga aacctgtcga ggagaagaca agttcgtcat gtcaaagaag 660

ttgaagagct atttgaactt ctctcatcct ttgatatcag aaaatttcaa acccaacgaa 720

tgtgcctggg cttatggaat gaacatattt gatttagagg cttggaggaa aaccaacata 780

agcacaacat accatcactg ggttgaagag aacttgaaat cagacctgag cttgtggcag 840

ctaggaacac tgcctccagg tctgatcgca ttccatggcc acgtccacgt tatcgatccc 900

ttttggcaca tgttgggtct tggctaccaa gaaaatacaa gtttggcaga tgctgagact 960

gccggagtca tccatttcaa cggtcgagca aagccttggc tagatatagc attccctcag 1020

ctccgccccc tgtgggcgaa gtacatcaac ttttccgaca agttcataaa gggctgtcat 1080

attaggccat cttaacttga gttttgagat gaaaatgttg gctgcgatcg tgattaatat 1140

atgtctatat ataaggagaa aaaaagcaga gaagagagag ttcttcacaa atccaaggcc 1200

ttgaatccag agagtaaaat attcataacc aagcatttgt ttgcttggtt tttttagaat 1260

tcatcctctg tgggcgaaca acccttccct attacttcaa atcttgattc aattttaatg 1320

agaaattaag catgttatgg attttcaacg g 1351

<210> 96
<211> 1507
<212> DNA
<213> Populus tremula x tremuloides


<220>
<221> misc_feature
<222> (597)..(597)
<223> n is a, c, g, or t

<400> 96
aagctgatga tcaaatgagg acatcaaaaa tttcttcagg gaattcaggc taccatgtat 60

ggattcttgc ttttgcaatg agtctactga tactgattgc cttgtcgaag tcatggtttt 120

atgatcacgc ttctgctgct gcaagtgagg atttgcagta cttttcagtg atcgtccctt 180

caaaagggcg ggactatcct cctgttcttg cttattggat atgtggtacc agtggagatg 240

ggaagagaat gttaaggcta ttaaaagcaa tttatcatcc aaggaaccaa tatctattgc 300

aacttgatgc ggaatcctca gattatgaaa gggcggagtt ggttgtttca gttcaatctg 360

aaagtttgtt tcaagcattc ggtaatgtta atgttgttgg aaaaggtttt gctatcaacg 420

aaatgggatc ctctgctctt gctgccatac tcaacgctgc tgcgttgctt cttaagctga 480

gtacagatgg ggactggttc atcaatttaa gtgtctcaga ctatcctcta gtgagtcaag 540

atgatctcct ccatgccttc acttccttgc caagagatct caacttcatc aactatncta 600

atgacactgc gaaaaacgag atacacaaga ttaaccaaat tgtagtagat cccagcttgc 660

atcttcaaaa gaggagccac ctttattacg ctgttgagac tcgaacaaca cctgatgctt 720

tcaagatatt tggaggttcg ccctggctga ttcttacaag agctttcatg gagtactgtg 780

tccaaggatg ggacaacctt ccaagaaaac tactaatgta cttcagcaac acggcatccc 840

cacttgaatc gtatttccac tctgtcctct gcaactctcc tgagtttcaa aacacaacag 900

taagcgatga tttaaggtac aatattctgg aaactactac agatggggaa tcaccttatg 960

acaaaatgct aaatggtgga gcagcatttg caaggccatt taaagaagat gctgctgctc 1020

taaacatgat agatgagaac gtcttgaacc gtgaacccaa cggattggtg cctggaaaat 1080

ggtgcttaga ccagggtatg aacaagagct cagaggcatc aaagcctcca ggggaggatt 1140

tgtgttcaac ctggggtaac attaatgatg tcaagccagg atcttatggt atcaagcttg 1200

catttttatt gtctaagatt gccagtgaag agaaattgac aactagtcaa tgccttcaag 1260

ctacaaaaat ggggtcatca tagaaccaag catacatcct tgtgtggata atttttttct 1320

tttaatcgaa aactataggt taaatcaagc ttcttagcca tataaatcct ttgctaaatg 1380

cactacaaat gcatacttgc ttgttaaaac aaaagctaga agcacccaga gatatattgt 1440

acatacattc acattttatc gaggatttct acaataaatt tattgttttt cccccaaaaa 1500

aaaaaaa 1507

<210> 97
<211> 1903
<212> DNA
<213> Populus tremula x tremuloides

<400> 97
ttttggggta tcctgatacc ggaggccagg ttgtttacat tttagatcaa gttcgtgcct 60

tggagaatga aatgcttctg cgtatcaagc agcaaggact tgatatcatc ccccgaattc 120

tcattattac tcgactgctc cctgatgcag taggaaccac ttgtggacaa cgtctggaga 180

aagtctatgg atcggagcat tgtgatattc ttcgagttcc cttcagagat gaaaagggaa 240

tggtccggaa atggatctct cgctttgaag tttggccata cctagaaact tacactgagg 300

atgttgctgc tgaaattgct aaggagttgc agggaaagcc tgatctgatc attggaaatt 360

acagtgatgg aaacgttgtt gcctccttgc tagcacacaa attaggtgtt acagagtgca 420

ctattgcaca tgctctagaa aaaacaaagt acccagattc agatatttac tggaagaagt 480

ttgatgaaaa gtaccacttt tcatgccagt ttacagctga tctttttgcc atgaaccata 540

cagatttcat catcaccagc acattccaag agattgctgg aagcaaggat actgttggac 600

agtacgagag ccacacagct ttcactctcc ctggcctcta cagagttgtt catggtatcg 660

atgtatttga tcccaaattc aacattgtat cccctggtgc tgacgagagc atatacttcc 720

cttacactga ggagaaactt aggctgactt ctttccatga agaaattgaa gaacttcttt 780

acagccccgt tgagaacgac gagcacttat gtgtgctaaa agaccgcaac aagccaattc 840

tattcaccat ggcgaggctg gacagagtta agaatttaac tggtcttgta gaatggtatg 900

gaaagaatac caagctgcgc gagttagcta atcttgttgt agttggtggt gatagaagaa 960

aggagtctaa agatatagaa gagcaagctg agatgaagaa gatgtacagt catatagaga 1020

aatacaactt gaatggccag ttcagatgga tttcttctca gatgaaccgc gtgaggaatg 1080

gagagctcta ccgttacatt tgtgatacca agggagcttt tgtgcagcct gctttgtatg 1140

aggcttttgg attgactgtt gtggaggcca tgacatgtgg tttgccaacc tttgctactt 1200

gcaatggcgg tcctgctgag atcattgtgc atggaaaatc cggattccat attgatcctt 1260

accatggagt acaggctgct gaactccttg ttgacttctt tgagaagtgc aaggctgatc 1320

ccacttactg ggacaaaatc tcccagggag gcctgcagcg aatccaagag aagtatacct 1380

ggaaaattta ctctcaaagg ctcctgactc tcacaggagt ttatggcttc tggaagcacg 1440

tttccaacct tgatcaccgt gagagccgtc gctatctgga aatgttctat gcactgaaat 1500

atcgcaaatt ggctgattct gttcctttga ctatcgagta aatggaactg gagaaatcga 1560

ggaaacatgg gttggtttga gtcgggttcc gggtccagaa taatggtcat ttcacgatag 1620

tgattggaca agaaaggctt tgatcttctt tttgtttttt tgtttttcct ttctgtgtgc 1680

atcttttttg ttttcccttt tcttttcatc atgcagcctc ttcaatttca gtaaagattg 1740

tgtctgttga gttcatcatc ccttatctga tctctatcca tttcttgcaa attctctgtc 1800

tcttgagttg gaaatcatgg gaattgatcc aacatcaatt cctgtatttc tcctcagtac 1860

tcgagattgg aagttgatgg ctattcttca tcattatata aag 1903

<210> 98
<211> 2198
<212> DNA
<213> Populus tremula x tremuloides

<400> 98
   gacaactaca actgctactt gtcggtgtgg gtttgctctt aactctttca ttctatctgc 60
   gatcctctct ccctccccct tggcacttgt caagcagcag agaatgaagg ttattgataa 120
   gataagggcg gcagcagcag caggagattc aaatggagga gatagcagca gcaataataa 180
   ggttgttttc tccttcgaat tctttcctcc aaagaccgat gatggagtgg acaacctgtt 240
   cgagagaatg gacaggatgg tctctcacaa tccttccttc tgtgacatca cttggggtgc 300
   tggtggctcc accgcagatc tgaccttaga catcgccaac aagatgcaga acatcatctg 360
   tgtcgagact atgatgcatc tcacctgcac caacatgccc gttgagaaga tcgatcacgc 420
   cctcgaaacc atcaaatcca atggaatcca gaatgttctt gctcttcgtg gtgatcctcc 480
   tcacggtcag gataaattcg tccagatcca aggcggcctc gcttgcgctc tcgacctcgt 540
   caaacatatc agatccaaat atggtgatta cttcggcatt actgtcgctg gctatccaga 600
   ggcacatcct gatgtgattg gaagtgatgg atttgctacg cctgaggatt accataaaga 660
   tcttgcttat ttgaagcaga aggtggatgc tggggctgat ctaattgtta ctcaactgtt 720
   ttatgatact gatattttct tgaaatttgt gaatgactgc cgccaaatcg gaatcacttg 780
   tcccattgtc cctggtatta tgcccattaa taactacaag ggcttcatac gtatgactgg 840
   tttttgcaaa accaaggtac ccgccgaggt tactgcagca ttggagccta tcaaggataa 900
   tgaagaagcc gtcagagcct atgggattca ccttggaact gaaatgtgca agaagatttt 960
   agctcatgga atcaagacat tgcatctcta tactctgaac atggagaaat ccgctttagc 1020
   tatattaatg aatcttggtt tgattgaaga gtccaagatt acaaggccgt taccatggcg 1080
   cccccgacaa aatgttttcc gtgtaaaaga agatgtccgt ccgatatttt gggctaatcg 1140
   tccaaagagc tacctaacaa ggacaatagg ttgggaccag taccctcatg gccgatgggg 1200
   tgattctcgc aatccatcct atggtgcttt atctgactat cagtttatgc gaccccgcgc 1260
   acgtggcaag aaacttcttg aagagtgggc tgcccccttg aaaagtgttg aggatatata 1320
   cgagaaattc aaggtatact gccttgggaa gttgaaaagt agtccttggt cagaactaga 1380
   tggccttcag ccagagacta aaatcattaa tgaacagctg ggtaagatca acttgaaggg 1440
   actcctgact atcaatagtc aaccagctgt caatggggaa aaatctgatt ctccatctgt 1500
   tggttggggt gggccaggag ggtatgtata tcagaaggct tatctagagt ttttctgctc 1560
   caaggacaag ctgaatgctc ttgtggagaa gtgcaagtct ttcccttttg taacttacat 1620
   tgctgtgaac aaaggaggga gttggatttc taatgttgcc ctgactgatg tgaatgctgt 1680
   aacctgggga gtcttcccag caaaggagat tatccaacca actgttgtgg atcccaccag 1740
   cttcagtgtg tggaaggatg aggcatttga aatctggtca agaggatggg cttccttgta 1800
   cccagagggt gacccatcca gaaccctact tgaagaggtg aagagcagct actttttggt 1860
   cagcttggta gataatgatt acatccatgg ggatattttc gctgtcttcg ccgatttatg 1920
   atgcaagggg ctctttgcaa cccctgcatt tgctaagagt atccaagtcc ccagaaaaat 1980
   ggtggtgctt taccctaagt tgaatcccag ctttaatcat cttatcggca tacattgttt 2040
   ttgaaattat ttccgtattg aaatgtaata attcattgtg attttgctac tcccctctcc 2100
   attatatgct gcaaatttgt atttggagtg gcaactaatt ctaagcttta agaaaatatg 2160
   attgcctctt tttcttggta cttccgaaaa aaaaaaaa                                2198

<210>  99
<211>  1548
<212>  DNA
<213>  Populus tremula x tremuloides

<220>
<221>  misc_feature
<222>  (1266)..(1266)
<223>  n is a, c, g, or t

<400>  99
   gtggaccatc tgcatccgtt tcagatggtg gtgaatcttc atcttttgaa ctccaagaga      60
   ggattggtga ctctatccgt gatgaaattc ctaggccttc tcaaaatcct ggtaggcaga     120
   agcataagag tcatgatcct gctcgagatc ttacgatcca tgtattggaa aaattctctc     180
   ttgtgaccaa atttgctcgg gacacaagtt cacaactatt tcgagaaagt aatagcaatg     240
   gctatggtgc tgtagaaagg aaaagttcta gctactctct ccctgatgtc ccccataagc     300
   catccatgga tgcagagatg gctcccgagg aaggtcctgt tccctcagat cctctggagt     360
   tcgataaaat gacactcgta tggggaaaac cacgacagcc acccttgggg tcatgaagag     420
   tgggctactt tcttggattc tgaagggcga gtcatggatt caaaagcctt aaaaaagaga     480
   atattctatg gtggagttga gcacacaaca cgcagagagg tctggccatt tttgctggga     540
   tatcatgctt atgattcaac gtatgcggag agggaatacc tcaagtcttc caaaaagtca     600
   gagtatgaga cagtaagaca acaatggcag agtatctcta ctgaacaagc aaagagattt     660
   acaaaattta gggaaaggaa gggccttata gacaaagatg tggtgaggac tgatagagca     720
   ctgtctttct atgatgggga tgataatcca aatgtgaata tcttacgtga tattctgttg     780
   acgtactcct tctataactt tgatcttggt tactgtcagg gtatgagtga tttgctatcc     840
   ccaattttgt ttgtgatgga ggatgaatca gaatcgtttt ggtgttttgt ggcactgatg     900
   gaacgtcttg gacccaattt taatcgtgat caaaatggca tgcactctca gctttttgca     960
   ttatccaagc tggtggagtt gcttgattgc ccattacata actatttcaa gcagaatgac    1020
   tgcttgaatt acttcttttg tttccgctgg gttctaatac aatttaaaag ggaatttgaa    1080
   tacaagaaaa caatgcgatt gtgggaagtg ttgtggaccc attatttgtc tgagcatctg    1140
   caactgtatg tatgtgttgc aatcttgaag cgataccgca acaaaataat gggggagcgc    1200
   atggactttg acacactctt gaaatttatc aacgagctga gtggccatat tgaccttgac    1260
   gcaatnctta gagatgcaga ggctctgtgc atatgtgctg gtgagaatgg tgctgctcgc    1320
   atcccaccag gaactccacc ttcattgcct actgagaatg agaatgcttt attatatgct    1380
   caagatgatg aagtactgta atacagttcg tctcattggt acgatataat tggctttata    1440
   ttttgttttc ttcatgaaat agctgtgtat aattgctgtt ttttcgaaat aaaatgttaa    1500
   taatcaagca atcaactcca tttgctgacc tccttgaaaa aaaaaaaa 1548

<210> 100
<211> 648
<212> DNA
<213> Populus tremula x tremuloides

<220>
<221> misc_feature
<222> (579)..(579)
<223> n is a, c, g, or t

<400> 100
gacatcctgt gtgctccaag aatataaaaa taatactagg tgctctctaa tggctgcaaa 60

accaacaaca gcgttccttt tctcttctat attttaaaga aaaaacacct tctttcaaca 120

accctttgat ggattccatc agaattagca tctcttcaat cctttcttgg attcccttaa 180

aacatgttct ttgatagcca aatcaccaaa gttgaacaga aaaggaaaat cttgttgagt 240

tcttgagcta aacgagagga ggaggagaat tgtttttcgt ttttcagata tggaggagag 300

gcatgttatt tttgggaagt atgaaatggg gaggctttta gggaagggaa cttttgctaa 360

agtttactat gggaaacact tggtgacagg agagagtgtg gcaatcaaag tcataagcaa 420

agatcaagtc aagaaagaag ggatgatgga gcaaatccag agagaaatct cagtcatgcg 480

tcttgttcgt catcccaaca ttgtagagct caaggaagtc atggctacca agactaaaat 540

cttcttcatc atggagtatg tccgaggagg agagttgtnt gccaaagtag ccaaaggaag 600

gctgaaagaa gaagctgctc gaaaatattt ccagcaacta atcagcgc 648

<210> 101
<211> 1030
<212> DNA
<213> Populus tremula x tremuloides

<400> 101
tgatgaagat gagcaacttg aaaatctgct gattttggct tgtcaccctt gccggaacag 60

tctccggcaa cgacgggctt ttgtactact cagtgcggca actcctgctt atgttgcccc 120

ggaagtcttg agaaagaaag gctatgatgg catcaaaagc tgatatatgg tcatgtggag 180

tgattcttta tgttcttctt gcagggttct tgccatttca agacgagaat gttatgaaga 240

tgtacaggaa agttttcaag gctgaatatc agttcccccc ttggtttcaa cagattccaa 300

gaggttgatt tcaagacttc ttgttgctga tcctgaaaag agaatcacaa ttcctgccat 360

aatgagaaat cattggtttc ttaaagggtt ttcgagacca gtggcctttt ctatccaaga 420

atcaagcatg gataaaacag atcaagatct tgattcttgt tctgttgtca acaccaatgt 480

atcatcacca gagttcttca acgccttcga gtttatttcc tcgatgtcat ccggctttga 540

tctgtctggt ctttttgaga ctaagaggaa atcaagctca atgttcactt caaaattttc 600

agcgagtgcg atcatggaaa aaattgaagg ggttgcaagg gggctgagtt ataaggtggc 660

aaaagtcaaa gatttcaaag tgacgttaca aggtccatgt gaggggagaa aggggaagct 720

ggcggtgacg gcggaggtgt tcgaggtggc accggaggtt gcggtggtgg agttctccaa 780

gtcttccgga gataccttgg agtatactag gttctgcgag gaagatgtta ggcctgcact 840

aaaagacatc gtttggacat ggcaaggaga caatgtttgt aataaagata acaacaatag 900

tcatgtagaa gatcctgaga ttcaaatgtt gtagaaaggc tttgatctta atttggtgag 960

ttttaggatt ttatataatc tgtaaataag tttttgttat ttaattaatg tatcaagttg 1020

agtgttcttg 1030

<210> 102
<211> 1215
<212> DNA
<213> Populus tremula x tremuloides

<400> 102
cccacgcgtc cggtattttc tgtggaggag aaacaaaaag caatctcttg ggtttttgtt 60

cttttaaaga tttggtttgg gttctctctt tgaaagttat caagaacagt aaaggggctc 120

ttgattgact gatgcaagtt taagattttc tattcttttt agagaaagaa agaagttttc 180

ttttgaaatg aatttgatgt gtgaggtttt cttcgctgaa gaagaagaga aggttgaagg 240

agagtgggaa aaggagagat agtatttacc aaagccaagg tagctagcag aagtagagag 300

gcagatagca ataaactaca aaataaaatt tcatctaagg ggttttagtt tgttctgctt 360

ctgctgggta attggcaatg tctgctacta taatcagcga ccctatggtg gtctctgcac 420

cggagacaca agcaacagca gcagccgcag ctgcggcaac gacagcaaca caactcattg 480

cacagataga ggttgagtcc gtcagatgtg attgctgtgg cctaatcgaa gagtgcacgc 540

cagaatacat tgaaagagta cgcgaaagat atcatggaaa atggatttgt gggttatgca 600

cggaggctat aaaatatgaa attgtaagga ctgagaggct tattagcact gaagaagcaa 660

tgacaaagca catgaacgtc tgcaagaagt ttgtctcctc agggccacct cctgatccaa 720

caactcattt gatagccgcc atgaggcaaa tcctgagaag aagcttggat tctccgagag 780

gttcaaggtc aaccccaagt agtcctaaca aaacgaacgg agcaatccgt gtcgctgcac 840

ttgctaggtc cgaaagttgc ttccctgctt tgtctggttg atagataatt ctgagcaccg 900

tggaagacgg ggtgattcat aaaatggtga tgaagctatt ggaagaaaat aaaaaaaaga 960

gagaaaaaaa cacattccat tttcatgcaa ataagaaaga tcaaaggatt gaagttaggt 1020

gcctaatcta gccacttata tagttgttta atacagaact tcctgtcaga agaaaaatca 1080

tggaactccc ttttttcctg aagctttata tttcagctgt acgtacttgt gtaatctgtc 1140

tttttgaaac catggtttcc ttcctctttt taccaagctg tatctctcaa ttttgataaa 1200

aaaaaaaaaa aaaaa 1215

<210>  103
<211>  484
<212>  DNA
<213>  Populus tremula x tremuloides

<400>  103
ggatgccatt gaatagccaa atatctctct ctctggacta gaatgtgaag gagcagttag      60

cgaaggaaag attggagaat gccggagctg gtgtaccaag agcagagctc gtcgagatca     120

ggatttagag ctcgagatgc aagtcctgac tctgtaattt tcactctgga gtccaacttc     180

agtctcttct cttctgcttc tgctagcgtc gatcgctgct cttttgcttc cgatgctcat     240

gatcatgact ctctcgcctc tgaaatctct ctggtaaaac catttctcta acttccctgc     300

atgcatgcgt tttctaaccc atggtttttg agtgagtttt ttgagagtgc aatgcagcat     360

ttggcagctg gtcatgatca acaagagaac tcttcgagtg gtccagatcg aaacaacaac     420

aagcagcagc agcagcagca gcacactcac acccgtctct ccagaaaagc agaaaaagtt     480

aaag                                                                  484

<210>  104
<211>  812
<212>  DNA
<213>  Populus tremula x tremuloides

<400>  104
tgatactctt ccatcgggtt gggatgtttc agaggctgga aagagcattt caaaggtcaa      60

aagagaggaa gccaaaatca cagcatggga gaacctgcag aaggcaaaag ctgaggcaga     120

aataaggaaa cttgaggttc tttttcccca cgcgtttcgt cgtacaattt tgacacttaa     180

acgattaatt cacttaatga tcagccttgg tgcagcaaag aagatattct atctaatatt     240

tcctttctgc ccacttgaaa gcttagctaa tacttaacta taagatactg gtcttaactg     300

actcgattca tttcagatga agcttgagaa aaagagatcg tcatctatgg atagaattat     360

gaataagctg agatcagctc aaaagagagc ccaggaaatg aggagctcgg tgctagcaaa     420

ccaggcacat caagtttcca cgaactctca caaagtttta tcattccgca gaacccgtca     480

gaagggttcc ctgagtggtt gtttcacatg ccatgctttc tgatatctca tccagccaca     540

tgttggtaca ctttgataga tataatacct ggtcaaattg caggaaaaac tgcagcagaa     600

tgtggaagcg tataggaggg aaaccaccat tgacactatt ttggtgtcaa tggagttgag     660

ttggggcgtg aagaacaagt actactgtta agatttcaga cgggacacaa accctggatg     720

gtatcaaaat ctccaggaaa tttgactgct aaaccttgta tgtatgcatg tatgtatgta     780

tgtatatcaa cgattattat tattattatt at                                   812

<210>  105
<211>  2550
<212>  DNA
<213>  Populus tremula x tremuloides

<220>
<221>  misc_feature
<222>  (127)..(127)
<223>  n is a, c, g, or t

<400>  105
gccttccgtt ttttttttaa acctcctcct cagatattat atacttcgtt ttctctcttc      60

tctgccatta atcatacttc acaccgatcc aggttttgtt tgctcctaaa cagagagtgg     120

gagaganccg tttaactttt ctttttttcc ttttgaagta atactacatc tccctctcct     180

cctcctcctc cacctcctcc tctatttgtt cttgatttcg attatctgat cggacggtca     240

cggtcttatt tgtattcgcc tgcaatatcg tacggcctgg gatctctctt ctcttactga     300

gcaagttgca tctcataagc actggcgatc atggagctga atggtcaaac aagagtgaga     360

agaaaggacc attttgctca tacaaacggt gatttagcat tagcaagtgt tggtgatgta     420

gatccctgga ctgcatgggc atataagcct cgaactattt cgctgttact tattggtgct     480

tgctttctaa tatgggcaag tggagcccta gatccagaga gctgtacatc tggtgatgtt     540

gttacatctg tgaaaagggg tatatgggca atgactgcag tttttcttgg ttattgcttg     600

ctacaagccc cttcaacggt tctaataagg ccacatccag caatttggcg cttagttcat     660

ggattggcca ttgtttatct tgttgccctc acatttctgc tttttcagaa gcgtgatgat     720

gcgcggcaat ttatgaagtt tctccatcct gaccttggaa ttgaactacc tgaaagatca     780

tatggtgctg attgtcgcat ttatgtgcct gaaaatccta caagcaagtt taagaacgtt     840

ttggacactc tttttgatga atttgttcta gcacatatct ttggatggtg gggcaaggct     900

atattaatcc gtaatcagcc acttctatgg gtattgtcaa ttggttttga gctgatggag     960

tttaccttcc gccacatgct accaaacttc aatgaatgct ggtgggacag tatcattctt    1020

gatattttga tatgcaattg gtttggcatt tgggctggaa tgcatacagt cgggtatttt    1080

gatggaaaaa catacgagtg ggttggtata agtcgccaac ctaatattat gagcaaggtc    1140

aaacgaacat tggaacaatt tacacctgca cagtgggaca aagatgaatg gcatcccttg    1200

cttggtccat ggcgatttat ccaagttctt agtctctgta ttgtcttcct gactgtagag    1260

ctcaacacat tctttttgaa gttttgtcta tgggttcctc ctcggaaccc tgtaatagtg    1320

tacaggctga tcttgtggtg gctaattgcc atacctacaa cacgtgaata caattcatat    1380

ctccaagacc gaaagcctgt gaaaaaggtt ggtgcttttt gttggctttc ccttgctatt    1440

tgcattgtgg aacttctcat ttgcatcaag tttggacatg gtctttatcc caaaccaatg    1500

cctgcatggt tggtcatctt ctggacatct gttggagtta gccttgtaat attcctgatt    1560

atgtggtcat ggaaaagttt gggaagaaag agacgatgat tttgctagca tggtacacta    1620

ttcttttatt ccttcatgtt cttatgggta tacatcttgt aaatgcccta tagcttcatg    1680

gaattgtaaa tgcttattct cttaggttag tggatcacag ttttagagta ttcaattctt    1740

ttatgatagg tagttggaaa atgcttctaa tggtaggttc atgtgttgct aatttgcagc 1800

agtcatcaaa atggtaattt tccttaactt gtccgagaga ggagcaattt ttaacaccgg 1860

gcttgcctca catttttcat tggaaaatgc atgcctgaca attccattgg ggcattttat 1920

cagaatgttt aaaatattta tattttgcgt cggctctata gcatatgctt ataagcattg 1980

aaatatgatt caactgaagt attcgcacat gttttgctgt ttacgtttta tatgattgtt 2040

gtgtttagtt ctccacaagc agcctggcag ttattcgatc cggtatcttg acgttatctt 2100

atcacatgct gtaaatccta tggctcaaac tggcaattat tcaaggcagt tcttaaagag 2160

ttcaaggttt tctttcctgt tgatcaacct ttgtaaagct tatgaagcaa ttgtgcaaat 2220

aattggtgct cgacagaagg caatctctta gttcgctcac gacatttcta gccgattttc 2280

gggaaaacat tagtggactg gagtcattga ttcaatcaag gtgaaattgg aaatcattta 2340

gaaaagcaat tcctgttctt tgacaacact aggttcttct ttctcgatcc gtgcccatgt 2400

cactgtgtgt gtatatatag aaacataaaa agtgtatctc tctcaatcat gcaggatttt 2460

gtattttgca atcaatacgc ccatgacgta tactctcagt cttcttcgaa aacactctta 2520

ccaaaaaaaa aaaaaaaaaa aaaaaaaaaa 2550

<210> 106
<211> 787
<212> DNA
<213> Populus tremula x tremuloides

<400> 106
ggacgaacaa gcaatgatag agttcgagag gtcatcttct tgttataaaa tcctcaaaag 60

cttgaggtga aaaaataagc aagggagagg agagagctag ctagctagtc aagttaagag 120

tcagaaaaca agaatgtctt ccaggaagaa aaagaaggca gctctttatg agaagttacg 180

tgctgctacc aattctaatg ctatgaacaa aacttcaatc atagtagacg cgtcaaaata 240

tattggagag ttgaagaaca aggtggatag gctaaaaaaa gaaatcggaa catcttcaac 300

cccccaaaat tcattacctg cgcaggtcac agtggaaaac ctagaaaagg gtttccttat 360

taatgtattt tcaggaaaga attgccctgg attacttgtc tccatacttg aagccttcga 420

ggaactaggc cttgatgtgc tcgatgctag ggtttcttgt gaagacaatt tccaacttga 480

agcgattggt ggagaccaaa accaaggcca tgatgctcaa gtagtgaaac aagcagtgct 540

gcaagctatc cataactgga atgaaggcag ctagctagtg agcaagaatg acccaaatat 600

tttcttcccc tatcttcctg tacgctactg agaaattggg aatttgtata ttgaatttca 660

agcacactag gcagtcctaa ttccaagtta gtagtcagtc aaaaagtgca gcaatgggat 720

tctggtaatt aattttcagt ttttgattaa ttaagcaact tttaccttcg atgatgtaaa 780

aaaaaaa 787

<210>  107
<211>  880
<212>  DNA
<213>  Populus tremula x tremuloides

<400>  107
gatgggaact ggcatatggc ggggaaaact agcattgtat cacagcttag tggtctgggt      60

ttagtgagaa atgtgattgg acactgtttg agtgggcgcg gtggaggatt cctcttcttc     120

ggggatgatc tttatgattc atctcgtgta gcttggacac caatgtcacc caattcgaaa     180

cactattcac ctggattcgc cgaattgact tttgatggga aaactactgg gttcaaaatc     240

ctgatcgtag cttttgacag cggggcctct tatacttacc ttaattctca ggcgtatcaa     300

ggtctaattt ctctgatgaa gagagaaata cccgcgaagc atttgacaga agcgctggat     360

gatcagacgc ttccgatttg ctggaaagga cggaaacctt tcaaaagcgt acgtgatgtc     420

aagaaatact tcaagacctt tgcattgagc tttaagaatg gcggaaaatc taaaactcaa     480

cttgaattcc caccagaagc ttatcttata gtatcatcca acggaaacgc ttgcttggga     540

attctaaatg gtacagaagt aggtctgaat gatctgaatg tcattggaga catatcaatg     600

caagacagag tggtgattta tgacaatgag aagcaactga ttggatgggc acctggaaat     660

tgcgacaggc ttcctaaatc cagaagcatt atcatttgat gacaggtgtt ctaaacgtta     720

aaaaaaacaa ttgtcctgta cagaaagctg ctaagctgta acattgtatt cttccgccat     780

tacagcagat tccttgtaga gtaaaactat gtatatgtgg tctggtgaat cagaccccac     840

aattatactt gattggttat gaaaatttaa taaaataacg                          880


<210>  108
<211>  486
<212>  DNA
<213>  Populus tremula x tremuloides

<400>  108
agatcactga aaaatactca gaatggcccg agcatgccac cagtgcagca ttttgtgaga      60

gcagaaatgc tcccaagtgg gtatctggta cggccttgtg aaggtggtgg ttcaattata     120

cacatagttg accacatgga tttggagcct tggagtgtgc ctgaagtact acggccgctg     180

tatgaatcct caactgtact tgctcaaaag acaacaatgg tggctctacg ccagctgcgg     240

cagatagctc aggaagcttc tcagtctaat gtgaccaact ggggcagacg accagcagct     300

ctacgagcac tgagccagag gttgagcacg gggttttaat gaggctctca atggatttag     360

tgatgaggga tggtcaatga tcggaaatga tggcatggat gatgttacta tcctcgtgaa     420

ctcatctcct gacaagttga tgggttcaaa tctttccttc actaatgggt atccagctgt     480

cagcag                                                                486


<210>  109
<211>  854
<212>  DNA

<213>  Populus tremula x tremuloides

<400>  109
ggccacatgc aagagcatgt agcatcaatg gctcggcaat atatccgcag cattatatct      60

tcagttcaga gggtggcatt agcactatct ccttctcatc ttggttcaca ggctggtctt     120

cggtcaccac tgggtactcc tgaagcacag acacttgctc gttggatctg ccagagctac     180

aggagctatt tgggtgtgga gctactcaaa tccaatggcg aaggaagtga atctattctg     240

aaaaccttgt ggcatcattc agatgccatt atgtgctgct cactgaaggt gttgcccgtc     300

tttacttttg caaaccaagc tggacttgac atgcttgaga caacattagt tgcactgcaa     360

gacataactt tggaaaagat atttgatgat catggaagaa aaactctctg ctcagaattc     420

ccacagatca tgcaacaggg ttttacttgt cttcaaggtg gtatctgttt gtcaagcatg     480

ggcagaccag tttcatatga aagagctgtg gcctggaaag tgttgaatga agaagaaaat     540

gcgcattgct tctgctttat gtttataaac tggtcttttg tctgagactt gaaaccagaa     600

tctagtggag tggctgattt aagagtgaga actctatacc tgtcttattt tgctggcttg     660

tatttcttct atgtataaca tgttacaacc gtggagcacc cagttcattt gctatgttat     720

aaatttgtgt ggggttcatt atcgtagaga ttgtggacga gacgatctag ttgccgggga     780

tcattttgtt tctgactggt ttgttctgaa gtcctcacct gggtctatgt tgattattgc     840

aacccaaatc tgtc                                                       854


<210>  110
<211>  607
<212>  DNA
<213>  Populus tremula x tremuloides

<400>  110
ggaatcagcc tagccaagta gagattacaa cgccaaaagc taggtcacac tgatcagagc      60

aacaacaaaa gtacaccaac aatgtcttcc accccaacaa atgaatggca aacctactgg     120

tgtcatgaat gtgacctcag catccacctt ctcaccacca ccactcctct ttgccctcac     180

tgccaccatg actttcttga actcatggac cccattccca cctccaccac cgctgacacc     240

accactttcc tccttgacag cccctccttc ctcaacttcc ttcaacatct caacaccaat     300

agccattgcg attgtgaagc atgataatac aaacgccact attgactcta tcatccccac     360

cataaaaatc acttcttgca tgctagaaat ggatccaatg cttgtgtgtg cagtgtgtaa     420

agatcagttc ttgattgata gttgaggcca agcagcatgc cctgcagtca cctgtaccat     480

ccaggttgca ttctcccctg gctttctaac cacaactctt gtccctctct gtcgcttcca     540

gttacaaacg ccagtatgtc agagaggaga attacgaaac ttgggtctcc tgatcatcct     600

catcatg                                                               607


<210>  111
<211>  259

<212>  DNA
<213>  Populus tremula x tremuloides

<400>  111
gggttttgac cacctttttt cctccacact tttgaatttg aatgcaggta aataaatgct      60

tttggggatt ttgggttttt ggtttggaat tgatcgagtg aagtgtaaat caacagtgaa     120

cgattgtgtt acacaaagaa cttgtgaggg ttgggcatcc ccttcccccc ttcagatttc     180

cactatggat attgtaaagt aaccccttgt ttgggattgg attttggcca aaaaaacagg     240

gtggttgaat tgattttttt                                                 259


<210>  112
<211>  475
<212>  DNA
<213>  Populus tremula x tremuloides

<400>  112
gcacgggtga aaagttacat taagaatcga tatgtgttgc gcaatctcct tcaagttccg      60

gtaataaaag aggacagtat gaaggataaa aaggcaagac aaaaaggaag ggatgatgat     120

tcaattacaa gtgatatgct agtagagata atggaagaat caataaggat tttttggcga     180

tttgttcgat ctgataaaga tgcacaaaac gtgatttcaa agggtcgcaa aggaactcaa     240

atagagcccc aagatcccac agaactagag ttattgacag aggttcgaac gagtttccaa     300

aagaaggaga ggaggcttaa agacgttttg aggagtggaa actgcatact gaggaagttc     360

caaaaacatc gagaggacga ttctaatcaa gttctttact tcttctctca agtggatatg     420

aagttagtag caagggttct gagcatgtcc agagtaacaa cagagcagct actat          475


<210>  113
<211>  572
<212>  DNA
<213>  Artificial

<220>
<223>  Construct sequence


<220>
<221>  misc_feature
<222>  (77)..(77)
<223>  n is a, c, g, or t

<400>  113
attgattaac catggagaaa gcacacacaa aatctgctct taagaagctt gttaaggcta      60

gctcacagtc tgcatcntgg agcaatgctg ctagaggaat ggcgaaagat gatctcaagg     120

atccgcttta tgacaaatcc aaggttgccc caaaacccct tgcaaaagaa aacacgaagc     180

cccaagaatt caaactccac actggacaaa gagctctcaa acgtgccatg ttcaactatt     240

ctgtggcaac caagatatat atgaatgagc aacagaagag gcaaatagag aggatacaaa     300

agatcataga agaagaagag gttcgtatga tgaggaagga gatggttcca agagctcaat     360

tgatgcctta ctttgacaga cctttctttc cccaaagatc aagcaggcca ttgacagttc 420

ctagagagcc aagtttccac atggtgaaca gcaagtgttg gagctgcatc cctgaggatg 480

aactttacta ctactttgaa catgctcatc cccatgacca tgcctggaag cctgttaagt 540

aaataattgc catgatttgt aggggaaaaa gg 572

<210> 114
<211> 1302
<212> DNA
<213> Artificial

<220>
<223> Construct sequence

<220>
<221> misc_feature
<222> (597)..(597)
<223> n is a, c, g, or t

<400> 114
aagctgatga tcaaatgagg acatcaaaaa tttcttcagg gaattcaggc taccatgtat 60

ggattcttgc ttttgcaatg agtctactga tactgattgc cttgtcgaag tcatggtttt 120

atgatcacgc ttctgctgct gcaagtgagg atttgcagta cttttcagtg atcgtccctt 180

caaaagggcg ggactatcct cctgttcttg cttattggat atgtggtacc agtggagatg 240

ggaagagaat gttaaggcta ttaaaagcaa tttatcatcc aaggaaccaa tatctattgc 300

aacttgatgc ggaatcctca gattatgaaa gggcggagtt ggttgtttca gttcaatctg 360

aaagtttgtt tcaagcattc ggtaatgtta atgttgttgg aaaaggtttt gctatcaacg 420

aaatgggatc ctctgctctt gctgccatac tcaacgctgc tgcgttgctt cttaagctga 480

gtacagatgg ggactggttc atcaatttaa gtgtctcaga ctatcctcta gtgagtcaag 540

atgatctcct ccatgccttc acttccttgc caagagatct caacttcatc aactatncta 600

atgacactgc gaaaaacgag atacacaaga ttaaccaaat tgtagtagat cccagcttgc 660

atcttcaaaa gaggagccac ctttattacg ctgttgagac tcgaacaaca cctgatgctt 720

tcaagatatt tggaggttcg ccctggctga ttcttacaag agctttcatg gagtactgtg 780

tccaaggatg ggacaacctt ccaagaaaac tactaatgta cttcagcaac acggcatccc 840

cacttgaatc gtatttccac tctgtcctct gcaactctcc tgagtttcaa aacacaacag 900

taagcgatga tttaaggtac aatattctgg aaactactac agatggggaa tcaccttatg 960

acaaaatgct aaatggtgga gcagcatttg caaggccatt taaagaagat gctgctgctc 1020

taaacatgat agatgagaac gtcttgaacc gtgaacccaa cggattggtg cctggaaaat 1080

ggtgcttaga ccagggtatg aacaagagct cagaggcatc aaagcctcca ggggaggatt 1140

tgtgttcaac ctggggtaac attaatgatg tcaagccagg atcttatggt atcaagcttg 1200

catttttatt gtctaagatt gccagtgaag agaaattgac aactagtcaa tgccttcaag 1260

ctacaaaaat ggggtcatca tagaaccaag catacatcct tg 1302

<210> 115
<211> 556
<212> DNA
<213> Artificial

<220>
<223> Construct sequence

<400> 115
attaatcatg tcaggtgatc aaaggcctaa agattctgct gagggctctt cgcgatctgg 60

cggcgaccac taccagcctc aaccggctcc tttgagcagg tatgagtcgc agaaacggcg 120

agactggaac acttttgggc agtacttgaa gaatcagaga cccccagttt cactatctca 180

gagtaattgc aaccacgtgc ttgatttcct tcggtatctc gatcagtttg gcaagactaa 240

ggttcatcta catggctgtg tcttttttgg acaacctaat cctcctgctc cttgtacctg 300

ccctctaagg caagcttggg ggagcctcga tgccctaatc ggacggcttc gagcagctta 360

tgaggagcac ggaggatctg cagagaccaa cccctttgga aatggagcta ttcgggttta 420

tttgcgtgaa gtgaaagagt gtcaagctaa ggcaaggggg attccataca agaagaaaac 480

gaagaagaag actcaaataa gggcaagaaa tgaagcgaag cctcctatgc agtcagctta 540

agtcctttgg cttctt 556

<210> 116
<211> 4470
<212> DNA
<213> Artificial

<220>
<223> Cloning vector pDONR201

<400> 116
ctttcctgcg ttatcccctg attctgtgga taaccgtatt accgctagcc aggaagagtt 60

tgtagaaacg caaaaaggcc atccgtcagg atggccttct gcttagtttg atgcctggca 120

gtttatggcg ggcgtcctgc ccgccaccct ccgggccgtt gcttcacaac gttcaaatcc 180

gctcccggcg gatttgtcct actcaggaga gcgttcaccg acaaacaaca gataaaacga 240

aaggcccagt cttccgactg agcctttcgt tttatttgat gcctggcagt tccctactct 300

cgcgttaacg ctagcatgga tctcgggccc caaataatga ttttattttg actgatagtg 360

acctgttcgt tgcaacaaat tgatgagcaa tgctttttta taatgccaag tttgtacaaa 420

aaagcagaac gagaaacgta aaatgatata aatatcaata tattaaatta gattttgcat 480

aaaaaacaga ctacataata ctgtaaaaca caacatatcc agtcactatg aatcaactac 540

ttagatggta ttagtgacct gtagtcgacc gacagccttc caaatgttct tcgggtgatg 600

ctgccaactt agtcgaccga cagccttcca aatgttcttc tcaaacggaa tcgtcgtatc 660

cagcctactc gctattgtcc tcaatgccgt attaaatcat aaaaagaaat aagaaaaaga 720
ggtgcgagcc tcttttttgt gtgacaaaat aaaaacatct acctattcat atacgctagt 780
gtcatagtcc tgaaaatcat ctgcatcaag aacaatttca caactcttat acttttctct 840
tacaagtcgt tcggcttcat ctggattttc agcctctata cttactaaac gtgataaagt 900
ttctgtaatt tctactgtat cgacctgcag actggctgtg tataagggag cctgacattt 960
atattcccca gaacatcagg ttaatggcgt ttttgatgtc attttcgcgg tggctgagat 1020
cagccacttc ttccccgata acggagaccg gcacactggc catatcggtg gtcatcatgc 1080
gccagctttc atccccgata tgcaccaccg ggtaaagttc acgggagact ttatctgaca 1140
gcagacgtgc actggccagg gggatcacca tccgtcgccc gggcgtgtca ataatatcac 1200
tctgtacatc cacaaacaga cgataacggc tctctctttt ataggtgtaa accttaaact 1260
gcatttcacc agtccctgtt ctcgtcagca aaagagccgt tcatttcaat aaaccgggcg 1320
acctcagcca tcccttcctg attttccgct ttccagcgtt cggcacgcag acgacgggct 1380
tcattctgca tggttgtgct taccagaccg gagatattga catcatatat gccttgagca 1440
actgatagct gtcgctgtca actgtcactg taatacgctg cttcatagca cacctctttt 1500
tgacatactt cgggtataca tatcagtata tattcttata ccgcaaaaat cagcgcgcaa 1560
atacgcatac tgttatctgg cttttagtaa gccggatcca cgcgattacg ccccgccctg 1620
ccactcatcg cagtactgtt gtaattcatt aagcattctg ccgacatgga agccatcaca 1680
gacggcatga tgaacctgaa tcgccagcgg catcagcacc ttgtcgcctt gcgtataata 1740
tttgcccatg gtgaaaacgg gggcgaagaa gttgtccata ttggccacgt ttaaatcaaa 1800
actggtgaaa ctcacccagg gattggctga gacgaaaaac atattctcaa taaacccttt 1860
agggaaatag gccaggtttt caccgtaaca cgccacatct tgcgaatata tgtgtagaaa 1920
ctgccggaaa tcgtcgtggt attcactcca gagcgatgaa aacgtttcag tttgctcatg 1980
gaaaacggtg taacaagggt gaacactatc ccatatcacc agctcaccgt ctttcattgc 2040
catacggaat tccggatgag cattcatcag gcgggcaaga atgtgaataa aggccggata 2100
aaacttgtgc ttatttttct ttacggtctt taaaaaggcc gtaatatcca gctgaacggt 2160
ctggttatag gtacattgag caactgactg aaatgcctca aaatgttctt tacgatgcca 2220
ttgggatata tcaacggtgg tatatccagt gatttttttc tccattttag cttccttagc 2280
tcctgaaaat ctcgataact caaaaaatac gcccggtagt gatcttattt cattatggtg 2340
aaagttggaa cctcttacgt gccgatcaac gtctcatttt cgccaaaagt tggcccaggg 2400
cttcccggta tcaacaggga caccaggatt tatttattct gcgaagtgat cttccgtcac 2460
aggtatttat tcggcgcaaa gtgcgtcggg tgatgctgcc aacttagtcg actacaggtc 2520
actaatacca tctaagtagt tgattcatag tgactggata tgttgtgttt tacagtatta 2580
tgtagtctgt tttttatgca aaatctaatt taatatattg atatttatat cattttacgt 2640

ttctcgttca gctttcttgt acaaagtggg cattataaga aagcattgct tatcaatttg 2700

ttgcaacgaa caggtcacta tcagtcaaaa taaaatcatt atttgccatc cagctgcagc 2760

tctggcccgt gtctcaaaat ctctgatgtt acattgcaca agataaaaat atatcatcat 2820

gaacaataaa actgtctgct tacataaaca gtaatacaag gggtgttatg agccatattc 2880

aacgggaaac gtcgaggccg cgattaaatt ccaacatgga tgctgattta tatgggtata 2940

aatgggctcg cgataatgtc gggcaatcag gtgcgacaat ctatcgcttg tatgggaagc 3000

ccgatgcgcc agagttgttt ctgaaacatg gcaaaggtag cgttgccaat gatgttacag 3060

atgagatggt cagactaaac tggctgacgg aatttatgcc tcttccgacc atcaagcatt 3120

ttatccgtac tcctgatgat gcatggttac tcaccactgc gatccccgga aaaacagcat 3180

tccaggtatt agaagaatat cctgattcag gtgaaaatat tgttgatgcg ctggcagtgt 3240

tcctgcgccg gttgcattcg attcctgttt gtaattgtcc ttttaacagc gatcgcgtat 3300

ttcgtctcgc tcaggcgcaa tcacgaatga ataacggttt ggttgatgcg agtgattttg 3360

atgacgagcg taatggctgg cctgttgaac aagtctggaa agaaatgcat aaacttttgc 3420

cattctcacc ggattcagtc gtcactcatg gtgatttctc acttgataac cttatttttg 3480

acgaggggaa attaataggt tgtattgatg ttggacgagt cggaatcgca gaccgatacc 3540

aggatcttgc catcctatgg aactgcctcg gtgagttttc tccttcatta cagaaacggc 3600

tttttcaaaa atatggtatt gataatcctg atatgaataa attgcagttt catttgatgc 3660

tcgatgagtt tttctaatca gaattggtta attggttgta acactggcag agcattacgc 3720

tgacttgacg ggacggcgca agctcatgac caaaatccct taacgtgagt tttcgttcca 3780

ctgagcgtca gaccccgtag aaaagatcaa aggatcttct tgagatcctt tttttctgcg 3840

cgtaatctgc tgcttgcaaa caaaaaaacc accgctacca gcggtggttt gtttgccgga 3900

tcaagagcta ccaactcttt ttccgaaggt aactggcttc agcagagcgc agataccaaa 3960

tactgtcctt ctagtgtagc cgtagttagg ccaccacttc aagaactctg tagcaccgcc 4020

tacatacctc gctctgctaa tcctgttacc agtggctgct gccagtggcg ataagtcgtg 4080

tcttaccggg ttggactcaa gacgatagtt accggataag gcgcagcggt cgggctgaac 4140

ggggggttcg tgcacacagc ccagcttgga gcgaacgacc tacaccgaac tgagatacct 4200

acagcgtgag ctatgagaaa gcgccacgct tcccgaaggg agaaaggcgg acaggtatcc 4260

ggtaagcggc agggtcggaa caggagagcg cacgagggag cttccagggg gaaacgcctg 4320

gtatctttat agtcctgtcg ggtttcgcca cctctgactt gagcgtcgat ttttgtgatg 4380

ctcgtcaggg gggcggagcc tatggaaaaa cgccagcaac gcggcctttt tacggttcct 4440

ggccttttgc tggccttttg ctcacatgtt 4470

<210> 117

<211>  13599
<212>  DNA
<213>  Artificial

<220>
<223>  Cloning vector pK7GWIWG2(I)

<400>  117
ctagagggcc cgacgtcgca tgcctgcagg tcactggatt ttggttttag gaattagaaa      60

ttttattgat agaagtattt tacaaataca aatacatact aagggtttct tatatgctca     120

acacatgagc gaaaccctat aagaacccta attcccttat ctgggaacta ctcacacatt     180

attctggaga aaaatagaga gagatagatt tgtagagaga gactggtgat ttttgcggac     240

tctagcatgg ccgcgggata tcacaagttt gtacaaaaaa gctgaacgag aaacgtaaaa     300

tgatataaat atcaatatat taaattagat tttgcataaa aaacagacta cataatactg     360

taaaacacaa catatccagt cactatggcg gccgcattag gcaccccagg ctttacactt     420

tatgcttccg gctcgtataa tgtgtggatt ttgagttagg atccggcgag attttcagga     480

gctaaggaag ctaaaatgga gaaaaaaatc actggatata ccaccgttga tatatcccaa     540

tggcatcgta aagaacattt tgaggcattt cagtcagttg ctcaatgtac ctataaccag     600

accgttcagc tggatattac ggccttttta aagaccgtaa agaaaaataa gcacaagttt     660

tatccggcct ttattcacat tcttgcccgc ctgatgaatg ctcatccgga attccgtatg     720

gcaatgaaag acggtgagct ggtgatatgg gatagtgttc acccttgtta caccgttttc     780

catgagcaaa ctgaaacgtt ttcatcgctc tggagtgaat accacgacga tttccggcag     840

tttctacaca tatattcgca agatgtggcg tgttacggtg aaaacctggc ctatttccct     900

aaagggttta ttgagaatat gtttttcgtc tcagccaatc cctgggtgag tttcaccagt     960

tttgatttaa acgtggccaa tatggacaac ttcttcgccc ccgttttcac catgggcaaa    1020

tattatacgc aaggcgacaa ggtgctgatg ccgctggcga ttcaggttca tcatgccgtc    1080

tgtgatggct tccatgtcgg cagaatgctt aatgaattac aacagtactg cgatgagtgg    1140

cagggcgggg cgtaaacgcg tggatccggc ttactaaaag ccagataaca gtatgcgtat    1200

ttgcgcgctg atttttgcgg tataagaata tatactgata tgtatacccg aagtatgtca    1260

aaaagaggtg tgctatgaag cagcgtatta cagtgacagt tgacagcgac agctatcagt    1320

tgctcaaggc atatatgatg tcaatatctc cggtctggta agcacaacca tgcagaatga    1380

agcccgtcgt ctgcgtgccg aacgctggaa agcggaaaat caggaaggga tggctgaggt    1440

cgcccggttt attgaaatga acggctcttt tgctgacgag aacagggact ggtgaaatgc    1500

agtttaaggt ttacacctat aaaagagaga gccgttatcg tctgtttgtg gatgtacaga    1560

gtgatattat tgacacgccc gggcgacgga tggtgatccc cctggccagt gcacgtctgc    1620

tgtcagataa agtctcccgt gaactttacc cggtggtgca tatcggggat gaaagctggc    1680

gcatgatgac caccgatatg gccagtgtgc cggtctccgt tatcggggaa gaagtggctg    1740

atctcagcca ccgcgaaaat gacatcaaaa acgccattaa cctgatgttc tggggaatat 1800
aaatgtcagg ctcccttata cacagccagt ctgcaggtcg accatagtga ctggatatgt 1860
tgtgttttac agtattatgt agtctgtttt ttatgcaaaa tctaatttaa tatattgata 1920
tttatatcat tttacgtttc tcgttcagct ttcttgtaca aagtggtgat atcactagtg 1980
cggccgcctg caggtcgacc atatggtcga cctgcaggcg gccgcactag tgatgctgtt 2040
atgttcagtg tcaagctgac ctgcaaacac gttaaatgct aagaagttag aatatatgag 2100
acacgttaac tggtatatga ataagctgta aataaccgag tataaactca ttaactaata 2160
tcacctctag agtataatat aatcaaattc gacaatttga ctttcaagag taggctaatg 2220
taaaatcttt atatatttct acaatgttca aagaaacagt tgcatctaaa cccctatggc 2280
catcaaattc aatgaacgct aagcttaata tgactctcaa taaagtctca taccaacaag 2340
tgccacctta ttcaaccatc aagaaaaaag ccaaaattta tgctactcta aggaaaactt 2400
cactaaagaa gacgatttag agtgttttac caagaatttc tgtcatctta ctaaacaact 2460
aaagatcggt gtgatacaaa acctaatctc attaaagttt atgctaaaat aagcataatt 2520
ttacccacta agcgtgacca gataaacata actcagcaca ccagagcata tatattggtg 2580
gctcaaatca tagaaactta cagtgaagac acagaaagcc gtaagaagag gcaagagtat 2640
gaaaccttac ctcatcattt ccatgaggtt gcttctgatc ccgcgggata tcaccacttt 2700
gtacaagaaa gctgaacgag aaacgtaaaa tgatataaat atcaatatat taaattagat 2760
tttgcataaa aaacagacta cataatactg taaaacacaa catatccagt cactatggtc 2820
gacctgcaga ctggctgtgt ataagggagc ctgacattta tattccccag aacatcaggt 2880
taatggcgtt tttgatgtca ttttcgcggt ggctgagatc agccacttct tccccgataa 2940
cggagaccgg cacactggcc atatcggtgg tcatcatgcg ccagctttca tccccgatat 3000
gcaccaccgg gtaaagttca cgggagactt tatctgacag cagacgtgca ctggccaggg 3060
ggatcaccat ccgtcgcccg ggcgtgtcaa taatatcact ctgtacatcc acaaacagac 3120
gataacggct ctctctttta taggtgtaaa ccttaaactg catttcacca gtccctgttc 3180
tcgtcagcaa aagagccgtt catttcaata aaccgggcga cctcagccat cccttcctga 3240
ttttccgctt tccagcgttc ggcacgcaga cgacgggctt cattctgcat ggttgtgctt 3300
accagaccgg agatattgac atcatatatg ccttgagcaa ctgatagctg tcgctgtcaa 3360
ctgtcactgt aatacgctgc ttcatagcac acctcttttt gacatacttc gggtatacat 3420
atcagtatat attcttatac cgcaaaaatc agcgcgcaaa tacgcatact gttatctggc 3480
ttttagtaag ccggatccac gcgtttacgc cccgccctgc cactcatcgc agtactgttg 3540
taattcatta agcattctgc cgacatggaa gccatcacag acggcatgat gaacctgaat 3600
cgccagcggc atcagcacct tgtcgccttg cgtataatat ttgcccatgg tgaaaacggg 3660

ggcgaagaag ttgtccatat tggccacgtt taaatcaaaa ctggtgaaac tcacccaggg 3720
attggctgag acgaaaaaca tattctcaat aaacccttta gggaaatagg ccaggttttc 3780
accgtaacac gccacatctt gcgaatatat gtgtagaaac tgccggaaat cgtcgtggta 3840
ttcactccag agcgatgaaa acgtttcagt ttgctcatgg aaaacggtgt aacaagggtg 3900
aacactatcc catatcacca gctcaccgtc tttcattgcc atacggaatt ccggatgagc 3960
attcatcagg cgggcaagaa tgtgaataaa ggccggataa aacttgtgct tatttttctt 4020
tacggtcttt aaaaaggccg taatatccag ctgaacggtc tggttatagg tacattgagc 4080
aactgactga aatgcctcaa aatgttcttt acgatgccat tgggatatat caacggtggt 4140
atatccagtg atttttttct ccattttagc ttccttagct cctgaaaatc tcgccggatc 4200
ctaactcaaa atccacacat tatacgagcc ggaagcataa agtgtaaagc ctggggtgcc 4260
taatgcggcc gccatagtga ctggatatgt tgtgttttac agtattatgt agtctgtttt 4320
ttatgcaaaa tctaatttaa tatattgata tttatatcat tttacgtttc tcgttcagct 4380
tttttgtaca aacttgtgat atcactagtg cggccgcctg caggtcgact agaatagtaa 4440
attgtaatgt tgtttgttgt ttgttttgtt gtggtaattg ttgtaaaaat acggatcgtc 4500
ctgcagtcct ctccaaatga aatgaacttc cttatataga ggaagggtct tgcgaaggat 4560
agtgggattg tgcgtcatcc cttacgtcag tggagatatc acatcaatcc acttgctttg 4620
aagacgtggt tggaacgtct tctttttcca cgatgctcct cgtgggtggg ggtccatctt 4680
tgggaccact gtcggcagag gcatcttgaa cgatagcctt tcctttatcg caatgatggc 4740
atttgtaggt gccaccttcc ttttctactg tccttttgat gaagtgacag atagctgggc 4800
aatggaatcc gaggaggttt cccgatatta ccctttgttg aaaagtctca atagcccttt 4860
ggtcttctga gactgtatct ttgatattct tggagtagac gagagtgtcg tgctccacca 4920
tgttgacgaa gattttcttc ttgtcattga gtcgtaaaag actctgtatg aactgttcgc 4980
cagtcttcac ggcgagttct gttagatcct cgatctgaat ttttgactcc atggcctttg 5040
attcagtagg aactactttc ttagagactc caatctctat tacttgcctt ggtttatgaa 5100
gcaagccttg aatcgtccat actggaatag tacttctgat cttgagaaat atatctttct 5160
ctgtgttctt gatgcagtta gtcctgaatc ttttgactgc atctttaacc ttcttgggaa 5220
ggtatttgat ctcctggaga ttattactcg ggtagatcgt cttgatgaga cctgccgcgt 5280
aggcctctct aaccatctgt gggtcagcat tctttctgaa attgaagagg ctaatcttct 5340
cattatcggt ggtgaacatg gtatcgtcac cttctccgtc gaactttctt cctagatcgt 5400
agagatagag aaagtcgtcc atggtgatct ccggggcaaa ggagatcagc ttggctctag 5460
tcgaccatat gggagagctc aagcttagct tgagcttgga tcagattgtc gtttcccgcc 5520
ttcagtttaa actatcagtg tttgacagga tatattggcg ggtaaaccta agagaaaaga 5580
gcgtttatta gaataacgga tatttaaaag ggcgtgaaaa ggtttatccg ttcgtccatt 5640

tgtatgtgca tgccaaccac agggttcccc tcgggatcaa agtactttga tccaacccct 5700

ccgctgctat agtgcagtcg gcttctgacg ttcagtgcag ccgtcttctg aaaacgacat 5760

gtcgcacaag tcctaagtta cgcgacaggc tgccgccctg cccttttcct ggcgttttct 5820

tgtcgcgtgt tttagtcgca taaagtagaa tacttgcgac tagaaccgga gacattacgc 5880

catgaacaag agcgccgccg ctggcctgct gggctatgcc cgcgtcagca ccgacgacca 5940

ggacttgacc aaccaacggg ccgaactgca cgcggccggc tgcaccaagc tgttttccga 6000

gaagatcacc ggcaccaggc gcgaccgccc ggagctggcc aggatgcttg accacctacg 6060

ccctggcgac gttgtgacag tgaccaggct agaccgcctg gcccgcagca cccgcgacct 6120

actggacatt gccgagcgca tccaggaggc cggcgcgggc ctgcgtagcc tggcagagcc 6180

gtgggccgac accaccacgc cggccggccg catggtgttg accgtgttcg ccggcattgc 6240

cgagttcgag cgttccctaa tcatcgaccg cacccggagc gggcgcgagg ccgccaaggc 6300

ccgaggcgtg aagtttggcc cccgccctac cctcaccccg gcacagatcg cgcacgcccg 6360

cgagctgatc gaccaggaag gccgcaccgt gaaagaggcg gctgcactgc ttggcgtgca 6420

tcgctcgacc ctgtaccgcg cacttgagcg cagcgaggaa gtgacgccca ccgaggccag 6480

gcggcgcggt gccttccgtg aggacgcatt gaccgaggcc gacgccctgg cggccgccga 6540

gaatgaacgc caagaggaac aagcatgaaa ccgcaccagg acggccagga cgaaccgttt 6600

ttcattaccg aagagatcga ggcggagatg atcgcggccg ggtacgtgtt cgagccgccc 6660

gcgcacgtct caaccgtgcg gctgcatgaa atcctggccg gtttgtctga tgccaagctg 6720

gcggcctggc cggccagctt ggccgctgaa gaaaccgagc gccgccgtct aaaaaggtga 6780

tgtgtatttg agtaaaacag cttgcgtcat gcggtcgctg cgtatatgat gcgatgagta 6840

aataaacaaa tacgcaaggg gaacgcatga aggttatcgc tgtacttaac cagaaaggcg 6900

ggtcaggcaa gacgaccatc gcaacccatc tagcccgcgc cctgcaactc gccggggccg 6960

atgttctgtt agtcgattcc gatccccagg gcagtgcccg cgattgggcg gccgtgcggg 7020

aagatcaacc gctaaccgtt gtcggcatcg accgcccgac gattgaccgc gacgtgaagg 7080

ccatcggccg gcgcgacttc gtagtgatcg acggagcgcc ccaggcggcg gacttggctg 7140

tgtccgcgat caaggcagcc gacttcgtgc tgattccggt gcagccaagc ccttacgaca 7200

tatgggccac cgccgacctg gtggagctgg ttaagcagcg cattgaggtc acggatggaa 7260

ggctacaagc ggcctttgtc gtgtcgcggg cgatcaaagg cacgcgcatc ggcggtgagg 7320

ttgccgaggc gctggccggg tacgagctgc ccattcttga gtcccgtatc acgcagcgcg 7380

tgagctaccc aggcactgcc gccgccggca caaccgttct tgaatcagaa cccgagggcg 7440

acgctgcccg cgaggtccag gcgctggccg ctgaaattaa atcaaaactc atttgagtta 7500

atgaggtaaa gagaaaatga gcaaaagcac aaacacgcta agtgccggcc gtccgagcgc 7560

acgcagcagc aaggctgcaa cgttggccag cctggcagac acgccagcca tgaagcgggt 7620

caactttcag ttgccggcgg aggatcacac caagctgaag atgtacgcgg tacgccaagg 7680

caagaccatt accgagctgc tatctgaata catcgcgcag ctaccagagt aaatgagcaa 7740

atgaataaat gagtagatga attttagcgg ctaaaggagg cggcatggaa aatcaagaac 7800

aaccaggcac cgacgccgtg gaatgcccca tgtgtggagg aacgggcggt tggccaggcg 7860

taagcggctg ggttgtctgc cggccctgca atggcactgg aacccccaag cccgaggaat 7920

cggcgtgacg gtcgcaaacc atccggcccg gtacaaatcg gcgcggcgct gggtgatgac 7980

ctggtggaga agttgaaggc cgcgcaggcc gcccagcggc aacgcatcga ggcagaagca 8040

cgccccggtg aatcgtggca agcggccgct gatcgaatcc gcaaagaatc ccggcaaccg 8100

ccggcagccg gtgcgccgtc gattaggaag ccgcccaagg gcgacgagca accagatttt 8160

ttcgttccga tgctctatga cgtgggcacc cgcgatagtc gcagcatcat ggacgtggcc 8220

gttttccgtc tgtcgaagcg tgaccgacga gctggcgagg tgatccgcta cgagcttcca 8280

gacgggcacg tagaggtttc cgcagggccg gccggcatgg ccagtgtgtg ggattacgac 8340

ctggtactga tggcggtttc ccatctaacc gaatccatga accgataccg ggaagggaag 8400

ggagacaagc ccggccgcgt gttccgtcca cacgttgcgg acgtactcaa gttctgccgg 8460

cgagccgatg gcggaaagca gaaagacgac ctggtagaaa cctgcattcg gttaaacacc 8520

acgcacgttg ccatgcagcg tacgaagaag gccaagaacg gccgcctggt gacggtatcc 8580

gagggtgaag ccttgattag ccgctacaag atcgtaaaga gcgaaaccgg gcggccggag 8640

tacatcgaga tcgagctagc tgattggatg taccgcgaga tcacagaagg caagaacccg 8700

gacgtgctga cggttcaccc cgattacttt ttgatcgatc ccggcatcgg ccgttttctc 8760

taccgcctgg cacgccgcgc cgcaggcaag gcagaagcca gatggttgtt caagacgatc 8820

tacgaacgca gtggcagcgc cggagagttc aagaagttct gtttcaccgt gcgcaagctg 8880

atcgggtcaa atgacctgcc ggagtacgat ttgaaggagg aggcggggca ggctggcccg 8940

atcctagtca tgcgctaccg caacctgatc gagggcgaag catccgccgg ttcctaatgt 9000

acggagcaga tgctagggca aattgcccta gcaggggaaa aaggtcgaaa aggtctcttt 9060

cctgtggata gcacgtacat tgggaaccca aagccgtaca ttgggaaccg gaacccgtac 9120

attgggaacc caaagccgta cattgggaac cggtcacaca tgtaagtgac tgatataaaa 9180

gagaaaaaag gcgatttttc cgcctaaaac tctttaaaac ttattaaaac tcttaaaacc 9240

cgcctggcct gtgcataact gtctggccag cgcacagccg aagagctgca aaaagcgcct 9300

acccttcggt cgctgcgctc cctacgcccc gccgcttcgc gtcggcctat cgcggccgct 9360

ggccgctcaa aaatggctgg cctacggcca ggcaatctac cagggcgcgg acaagccgcg 9420

ccgtcgccac tcgaccgccg gcgcccacat caaggcaccc tgcctcgcgc gtttcggtga 9480

tgacggtgaa aacctctgac acatgcagct cccggagacg gtcacagctt gtctgtaagc 9540

ggatgccggg agcagacaag cccgtcaggg cgcgtcagcg ggtgttggcg ggtgtcgggg 9600
cgcagccatg acccagtcac gtagcgatag cggagtgtat actggcttaa ctatgcggca 9660
tcagagcaga ttgtactgag agtgcaccat atgcggtgtg aaataccgca cagatgcgta 9720
aggagaaaat accgcatcag gcgctcttcc gcttcctcgc tcactgactc gctgcgctcg 9780
gtcgttcggc tgcggcgagc ggtatcagct cactcaaagg cggtaatacg gttatccaca 9840
gaatcagggg ataacgcagg aaagaacatg tgagcaaaag gccagcaaaa ggccaggaac 9900
cgtaaaaagg ccgcgttgct ggcgtttttc cataggctcc gcccccctga cgagcatcac 9960
aaaaatcgac gctcaagtca gaggtggcga aacccgacag gactataaag ataccaggcg 10020
tttccccctg gaagctccct cgtgcgctct cctgttccga ccctgccgct taccggatac 10080
ctgtccgcct ttctcccttc gggaagcgtg gcgctttctc atagctcacg ctgtaggtat 10140
ctcagttcgg tgtaggtcgt tcgctccaag ctgggctgtg tgcacgaacc ccccgttcag 10200
cccgaccgct gcgccttatc cggtaactat cgtcttgagt ccaacccggt aagacacgac 10260
ttatcgccac tggcagcagc cactggtaac aggattagca gagcgaggta tgtaggcggt 10320
gctacagagt tcttgaagtg gtggcctaac tacggctaca ctagaaggac agtatttggt 10380
atctgcgctc tgctgaagcc agttaccttc ggaaaaagag ttggtagctc ttgatccggc 10440
aaacaaacca ccgctggtag cggtggtttt tttgtttgca agcagcagat tacgcgcaga 10500
aaaaaaggat ctcaagaaga tcctttgatc ttttctacgg ggtctgacgc tcagtggaac 10560
gaaaactcac gttaagggat tttggtcatg catgatatat ctcccaattt gtgtagggct 10620
tattatgcac gcttaaaaat aataaaagca gacttgacct gatagtttgg ctgtgagcaa 10680
ttatgtgctt agtgcatcta atcgcttgag ttaacgccgg cgaagcggcg tcggcttgaa 10740
cgaatttcta gctagacatt atttgccgac taccttggtg atctcgcctt tcacgtagtg 10800
gacaaattct tccaactgat ctgcgcgcga ggccaagcga tcttcttctt gtccaagata 10860
agcctgtcta gcttcaagta tgacgggctg atactgggcc ggcaggcgct ccattgccca 10920
gtcggcagcg acatccttcg gcgcgatttt gccggttact gcgctgtacc aaatgcggga 10980
caacgtaagc actacatttc gctcatcgcc agcccagtcg ggcggcgagt tccatagcgt 11040
taaggtttca tttagcgcct caaatagatc ctgttcagga accggatcaa agagttcctc 11100
cgccgctgga cctaccaagg caacgctatg ttctcttgct tttgtcagca agatagccag 11160
atcaatgtcg atcgtggctg gctcgaagat acctgcaaga atgtcattgc gctgccattc 11220
tccaaattgc agttcgcgct tagctggata acgccacgga atgatgtcgt cgtgcacaac 11280
aatggtgact tctacagcgc ggagaatctc gctctctcca ggggaagccg aagtttccaa 11340
aaggtcgttg atcaaagctc gccgcgttgt ttcatcaagc cttacggtca ccgtaaccag 11400
caaatcaata tcactgtgtg gcttcaggcc gccatccact gcggagccgt acaaatgtac 11460

ggccagcaac gtcggttcga gatggcgctc gatgacgcca actacctctg atagttgagt 11520
cgatacttcg gcgatcaccg cttcccccat gatgtttaac tttgttttag ggcgactgcc 11580
ctgctgcgta acatcgttgc tgctccataa catcaaacat cgacccacgg cgtaacgcgc 11640
ttgctgcttg gatgcccgag gcatagactg taccccaaaa aaacatgtca taacaagaag 11700
ccatgaaaac cgccactgcg ccgttaccac cgctgcgttc ggtcaaggtt ctggaccagt 11760
tgcgtgacgg cagttacgct acttgcatta cagcttacga accgaacgag gcttatgtcc 11820
actgggttcg tgcccgaatt gatcacaggc agcaacgctc tgtcatcgtt acaatcaaca 11880
tgctaccctc cgcgagatca tccgtgtttc aaacccggca gcttagttgc cgttcttccg 11940
aatagcatcg gtaacatgag caaagtctgc cgccttacaa cggctctccc gctgacgccg 12000
tcccggactg atgggctgcc tgtatcgagt ggtgattttg tgccgagctg ccggtcgggg 12060
agctgttggc tggctggtgg caggatatat tgtggtgtaa acaaattgac gcttagacaa 12120
cttaataaca cattgcggac gtttttaatg tactgaatta acgccgaatt gaattatcag 12180
cttgcatgcc ggtcgatcta gtaacataga tgacaccgcg cgcgataatt tatcctagtt 12240
tgcgcgctat attttgtttt ctatcgcgta ttaaatgtat aattgcggga ctctaatcat 12300
aaaaacccat ctcataaata acgtcatgca ttacatgtta attattacat gcttaacgta 12360
attcaacaga aattatatga taatcatcgc aagaccggca acaggattca atcttaagaa 12420
actttattgc caaatgtttg aacgatctgc ttgactctag ctagagtccg aaccccagag 12480
tcccgctcag aagaactcgt caagaaggcg atagaaggcg atgcgctgcg aatcgggagc 12540
ggcgataccg taaagcacga ggaagcggtc agcccattcg ccgccaagct cttcagcaat 12600
atcacgggta gccaacgcta tgtcctgata gcggtccgcc acacccagcc ggccacagtc 12660
gatgaatcca gaaaagcggc cattttccac catgatattc ggcaagcagg catcgccgtg 12720
ggtcacgacg agatcctcgc cgtcgggcat ccgcgccttg agcctggcga acagttcggc 12780
tggcgcgagc ccctgatgct cttcgtccag atcatcctga tcgacaagac cggcttccat 12840
ccgagtacgt gctcgctcga tgcgatgttt cgcttggtgg tcgaatgggc aggtagccgg 12900
atcaagcgta tgcagccgcc gcattgcatc agccatgatg gatactttct cggcaggagc 12960
aaggtgagat gacaggagat cctgccccgg cacttcgccc aatagcagcc agtcccttcc 13020
cgcttcagtg acaacgtcga gcacagctgc gcaaggaacg cccgtcgtgg ccagccacga 13080
tagccgcgct gcctcgtctt ggagttcatt cagggcaccg gacaggtcgg tcttgacaaa 13140
aagaaccggg cgcccctgcg ctgacagccg gaacacggcg gcatcagagc agccgattgt 13200
ctgttgtgcc cagtcatagc cgaatagcct ctccacccaa gcggccggag aacctgcgtg 13260
caatccatct tgttcaatca tgcctcgatc gagttgagag tgaatatgag actctaattg 13320
gataccgagg ggaatttatg gaacgtcagt ggagcatttt tgacaagaaa tatttgctag 13380
ctgatagtga ccttaggcga cttttgaacg cgcaataatg gtttctgacg tatgtgctta 13440

gctcattaaa ctccagaaac ccgcggctga gtggctcctt caacgttgcg gttctgtcag 13500

ttccaaacgt aaaacggctt gtcccgcgtc atcggcgggg gtcataacgt gactccctta 13560

attctcatgt atgataattc gagggtaccc ggggatcct 13599

<210> 118
<211> 11135
<212> DNA
<213> Artificial

<220>
<223> Cloning vector pKSGW7

<400> 118
ctcccatatg gtcgactaga gccaagctga tctcctttgc cccggagatc accatggacg 60

actttctcta tctctacgat ctaggaagaa agttcgacgg agaaggtgac gataccatgt 120

tcaccaccga taatgagaag attagcctct tcaatttcag aaagaatgct gacccacaga 180

tggttagaga ggcctacgcg gcaggtctca tcaagacgat ctacccgagt aataatctcc 240

aggagatcaa ataccttccc aagaaggtta aagatgcagt caaaagattc aggactaact 300

gcatcaagaa cacagagaaa gatatatttc tcaagatcag aagtactatt ccagtatgga 360

cgattcaagg cttgcttcat aaaccaaggc aagtaataga gattggagtc tctaagaaag 420

tagttcctac tgaatcaaag gccatggagt caaaaattca gatcgaggat ctaacagaac 480

tcgccgtgaa gactggcgaa cagttcatac agagtctttt acgactcaat gacaagaaga 540

aaatcttcgt caacatggtg gagcacgaca ctctcgtcta ctccaagaat atcaaagata 600

cagtctcaga agaccaaagg gctattgaga cttttcaaca aagggtaata tcgggaaacc 660

tcctcggatt ccattgccca gctatctgtc acttcatcaa aaggacagta gaaaaggaag 720

gtggcaccta caaatgccat cattgcgata aaggaaaggc tatcgttcaa gatgcctctg 780

ccgacagtgg tcccaaagat ggacccccac ccacgaggag catcgtggaa aaagaagacg 840

ttccaaccac gtcttcaaag caagtggatt gatgtgatat ctccactgac gtaagggatg 900

acgcacaatc ccactatcct tcgcaagacc cttcctctat ataaggaagt tcatttcatt 960

tggagaggac tccggtattt ttacaacaat accacaacaa aacaaacaac aaacaacatt 1020

acaatttact attctagtcg acctgcaggc ggccgcacta gtgatatcac aagtttgtac 1080

aaaaaagctg aacgagaaac gtaaaatgat ataaatatca atatattaaa ttagattttg 1140

cataaaaaac agactacata atactgtaaa acacaacata tccagtcact atggcggccg 1200

cattaggcac cccaggcttt acactttatg cttccggctc gtataatgtg tggattttga 1260

gttaggatcc ggcgagattt tcaggagcta aggaagctaa aatggagaaa aaaatcactg 1320

gatataccac cgttgatata tcccaatggc atcgtaaaga acattttgag gcatttcagt 1380

cagttgctca atgtacctat aaccagaccg ttcagctgga tattacggcc tttttaaaga 1440

ccgtaaagaa aaataagcac aagttttatc cggcctttat tcacattctt gcccgcctga 1500

tgaatgctca tccggaattc cgtatggcaa tgaaagacgg tgagctggtg atatgggata 1560

gtgttcaccc ttgttacacc gttttccatg agcaaactga aacgttttca tcgctctgga 1620

gtgaatacca cgacgatttc cggcagtttc tacacatata ttcgcaagat gtggcgtgtt 1680

acggtgaaaa cctggcctat ttccctaaag ggtttattga gaatatgttt ttcgtctcag 1740

ccaatccctg ggtgagtttc accagttttg atttaaacgt ggccaatatg gacaacttct 1800

tcgcccccgt tttcaccatg ggcaaatatt atacgcaagg cgacaaggtg ctgatgccgc 1860

tggcgattca ggttcatcat gccgtctgtg atggcttcca tgtcggcaga atgcttaatg 1920

aattacaaca gtactgcgat gagtggcagg gcggggcgta aacgcgtgga tccggcttac 1980

taaaagccag ataacagtat gcgtatttgc gcgctgattt ttgcggtata agaatatata 2040

ctgatatgta tacccgaagt atgtcaaaaa gaggtgtgct atgaagcagc gtattacagt 2100

gacagttgac agcgacagct atcagttgct caaggcatat atgatgtcaa tatctccggt 2160

ctggtaagca caaccatgca gaatgaagcc cgtcgtctgc gtgccgaacg ctggaaagcg 2220

gaaaatcagg aagggatggc tgaggtcgcc cggtttattg aaatgaacgg ctcttttgct 2280

gacgagaaca gggactggtg aaatgcagtt taaggtttac acctataaaa gagagagccg 2340

ttatcgtctg tttgtggatg tacagagtga tattattgac acgcccgggc gacggatggt 2400

gatccccctg gccagtgcac gtctgctgtc agataaagtc tcccgtgaac tttacccggt 2460

ggtgcatatc ggggatgaaa gctggcgcat gatgaccacc gatatggcca gtgtgccggt 2520

ctccgttatc ggggaagaag tggctgatct cagccaccgc gaaaatgaca tcaaaaacgc 2580

cattaacctg atgttctggg gaatataaat gtcaggctcc cttatacaca gccagtctgc 2640

aggtcgacca tagtgactgg atatgttgtg ttttacagta ttatgtagtc tgttttttat 2700

gcaaaatcta atttaatata ttgatattta tatcatttta cgtttctcgt tcagctttct 2760

tgtacaaagt ggtgatatcc cgcggccatg ctagagtccg caaaaatcac cagtctctct 2820

ctacaaatct atctctctct atttttctcc agaataatgt gtgagtagtt cccagataag 2880

ggaattaggg ttcttatagg gtttcgctca tgtgttgagc atataagaaa cccttagtat 2940

gtatttgtat ttgtaaaata cttctatcaa taaaatttct aattcctaaa accaaaatcc 3000

agtgacctgc aggcatgcga cgtcgggccc aagcttagct tgagcttgga tcagattgtc 3060

gtttcccgcc ttcagtttaa actatcagtg tttgacagga tatattggcg ggtaaaccta 3120

agagaaaaga gcgtttatta gaataacgga tatttaaaag ggcgtgaaaa ggtttatccg 3180

ttcgtccatt tgtatgtgca tgccaaccac agggttcccc tcgggatcaa agtactttga 3240

tccaacccct ccgctgctat agtgcagtcg gcttctgacg ttcagtgcag ccgtcttctg 3300

aaaacgacat gtcgcacaag tcctaagtta cgcgacaggc tgccgccctg cccttttcct 3360

ggcgttttct tgtcgcgtgt tttagtcgca taaagtagaa tacttgcgac tagaaccgga 3420

gacattacgc catgaacaag agcgccgccg ctggcctgct gggctatgcc cgcgtcagca 3480

ccgacgacca ggacttgacc aaccaacggg ccgaactgca cgcggccggc tgcaccaagc 3540

tgttttccga gaagatcacc ggcaccaggc gcgaccgccc ggagctggcc aggatgcttg 3600

accacctacg ccctggcgac gttgtgacag tgaccaggct agaccgcctg gcccgcagca 3660

cccgcgacct actggacatt gccgagcgca tccaggaggc cggcgcgggc ctgcgtagcc 3720

tggcagagcc gtgggccgac accaccacgc cggccggccg catggtgttg accgtgttcg 3780

ccggcattgc cgagttcgag cgttccctaa tcatcgaccg cacccggagc gggcgcgagg 3840

ccgccaaggc ccgaggcgtg aagtttggcc cccgccctac cctcaccccg gcacagatcg 3900

cgcacgcccg cgagctgatc gaccaggaag gccgcaccgt gaaagaggcg gctgcactgc 3960

ttggcgtgca tcgctcgacc ctgtaccgcg cacttgagcg cagcgaggaa gtgacgccca 4020

ccgaggccag gcggcgcggt gccttccgtg aggacgcatt gaccgaggcc gacgccctgg 4080

cggccgccga gaatgaacgc caagaggaac aagcatgaaa ccgcaccagg acggccagga 4140

cgaaccgttt ttcattaccg aagagatcga ggcggagatg atcgcggccg ggtacgtgtt 4200

cgagccgccc gcgcacgtct caaccgtgcg gctgcatgaa atcctggccg gtttgtctga 4260

tgccaagctg gcggcctggc cggccagctt ggccgctgaa gaaaccgagc gccgccgtct 4320

aaaaaggtga tgtgtatttg agtaaaacag cttgcgtcat gcggtcgctg cgtatatgat 4380

gcgatgagta aataaacaaa tacgcaaggg gaacgcatga aggttatcgc tgtacttaac 4440

cagaaaggcg ggtcaggcaa gacgaccatc gcaacccatc tagcccgcgc cctgcaactc 4500

gccggggccg atgttctgtt agtcgattcc gatccccagg gcagtgcccg cgattgggcg 4560

gccgtgcggg aagatcaacc gctaaccgtt gtcggcatcg accgcccgac gattgaccgc 4620

gacgtgaagg ccatcggccg gcgcgacttc gtagtgatcg acggagcgcc ccaggcggcg 4680

gacttggctg tgtccgcgat caaggcagcc gacttcgtgc tgattccggt gcagccaagc 4740

ccttacgaca tatgggccac cgccgacctg gtggagctgg ttaagcagcg cattgaggtc 4800

acggatggaa ggctacaagc ggcctttgtc gtgtcgcggg cgatcaaagg cacgcgcatc 4860

ggcggtgagg ttgccgaggc gctggccggg tacgagctgc ccattcttga gtcccgtatc 4920

acgcagcgcg tgagctaccc aggcactgcc gccgccggca caaccgttct tgaatcagaa 4980

cccgagggcg acgctgcccg cgaggtccag gcgctggccg ctgaaattaa atcaaaactc 5040

atttgagtta atgaggtaaa gagaaaatga gcaaaagcac aaacacgcta agtgccggcc 5100

gtccgagcgc acgcagcagc aaggctgcaa cgttggccag cctggcagac acgccagcca 5160

tgaagcgggt caactttcag ttgccggcgg aggatcacac caagctgaag atgtacgcgg 5220

tacgccaagg caagaccatt accgagctgc tatctgaata catcgcgcag ctaccagagt 5280

aaatgagcaa atgaataaat gagtagatga attttagcgg ctaaaggagg cggcatggaa 5340

aatcaagaac aaccaggcac cgacgccgtg gaatgcccca tgtgtggagg aacgggcggt 5400
tggccaggcg taagcggctg ggttgtctgc cggccctgca atggcactgg aacccccaag 5460
cccgaggaat cggcgtgacg gtcgcaaacc atccggcccg gtacaaatcg gcgcggcgct 5520
gggtgatgac ctggtggaga agttgaaggc cgcgcaggcc gcccagcggc aacgcatcga 5580
ggcagaagca cgccccggtg aatcgtggca agcggccgct gatcgaatcc gcaaagaatc 5640
ccggcaaccg ccggcagccg gtgcgccgtc gattaggaag ccgcccaagg gcgacgagca 5700
accagatttt ttcgttccga tgctctatga cgtgggcacc cgcgatagtc gcagcatcat 5760
ggacgtggcc gttttccgtc tgtcgaagcg tgaccgacga gctggcgagg tgatccgcta 5820
cgagcttcca gacgggcacg tagaggtttc cgcagggccg gccggcatgg ccagtgtgtg 5880
ggattacgac ctggtactga tggcggtttc ccatctaacc gaatccatga accgataccg 5940
ggaagggaag ggagacaagc ccggccgcgt gttccgtcca cacgttgcgg acgtactcaa 6000
gttctgccgg cgagccgatg gcggaaagca gaaagacgac ctggtagaaa cctgcattcg 6060
gttaaacacc acgcacgttg ccatgcagcg tacgaagaag gccaagaacg gccgcctggt 6120
gacggtatcc gagggtgaag ccttgattag ccgctacaag atcgtaaaga gcgaaaccgg 6180
gcggccggag tacatcgaga tcgagctagc tgattggatg taccgcgaga tcacagaagg 6240
caagaacccg gacgtgctga cggttcaccc cgattacttt ttgatcgatc ccggcatcgg 6300
ccgttttctc taccgcctgg cacgccgcgc cgcaggcaag gcagaagcca gatggttgtt 6360
caagacgatc tacgaacgca gtggcagcgc cggagagttc aagaagttct gtttcaccgt 6420
gcgcaagctg atcgggtcaa atgacctgcc ggagtacgat ttgaaggagg aggcggggca 6480
ggctggcccg atcctagtca tgcgctaccg caacctgatc gagggcgaag catccgccgg 6540
ttcctaatgt acggagcaga tgctagggca aattgcccta gcaggggaaa aaggtcgaaa 6600
aggtctcttt cctgtggata gcacgtacat tgggaaccca aagccgtaca ttgggaaccg 6660
gaacccgtac attgggaacc caaagccgta cattgggaac cggtcacaca tgtaagtgac 6720
tgatataaaa gagaaaaaag gcgatttttc cgcctaaaac tctttaaaac ttattaaaac 6780
tcttaaaacc cgcctggcct gtgcataact gtctggccag cgcacagccg aagagctgca 6840
aaaagcgcct acccttcggt cgctgcgctc cctacgcccc gccgcttcgc gtcggcctat 6900
cgcggccgct ggccgctcaa aaatggctgg cctacggcca ggcaatctac cagggcgcgg 6960
acaagccgcg ccgtcgccac tcgaccgccg gcgcccacat caaggcaccc tgcctcgcgc 7020
gtttcggtga tgacggtgaa aacctctgac acatgcagct cccggagacg gtcacagctt 7080
gtctgtaagc ggatgccggg agcagacaag cccgtcaggg cgcgtcagcg ggtgttggcg 7140
ggtgtcgggg cgcagccatg acccagtcac gtagcgatag cggagtgtat actggcttaa 7200
ctatgcggca tcagagcaga ttgtactgag agtgcaccat atgcggtgtg aaataccgca 7260
cagatgcgta aggagaaaat accgcatcag gcgctcttcc gcttcctcgc tcactgactc 7320

gctgcgctcg gtcgttcggc tgcggcgagc ggtatcagct cactcaaagg cggtaatacg 7380

gttatccaca gaatcagggg ataacgcagg aaagaacatg tgagcaaaag gccagcaaaa 7440

ggccaggaac cgtaaaaagg ccgcgttgct ggcgtttttc cataggctcc gcccccctga 7500

cgagcatcac aaaaatcgac gctcaagtca gaggtggcga aacccgacag gactataaag 7560

ataccaggcg tttccccctg gaagctccct cgtgcgctct cctgttccga ccctgccgct 7620

taccggatac ctgtccgcct ttctcccttc gggaagcgtg gcgctttctc atagctcacg 7680

ctgtaggtat ctcagttcgg tgtaggtcgt tcgctccaag ctgggctgtg tgcacgaacc 7740

ccccgttcag cccgaccgct gcgccttatc cggtaactat cgtcttgagt ccaacccggt 7800

aagacacgac ttatcgccac tggcagcagc cactggtaac aggattagca gagcgaggta 7860

tgtaggcggt gctacagagt tcttgaagtg gtggcctaac tacggctaca ctagaaggac 7920

agtatttggt atctgcgctc tgctgaagcc agttaccttc ggaaaaagag ttggtagctc 7980

ttgatccggc aaacaaacca ccgctggtag cggtggtttt tttgtttgca agcagcagat 8040

tacgcgcaga aaaaaaggat ctcaagaaga tcctttgatc ttttctacgg ggtctgacgc 8100

tcagtggaac gaaaactcac gttaagggat tttggtcatg catgatatat ctcccaattt 8160

gtgtagggct tattatgcac gcttaaaaat aataaaagca gacttgacct gatagtttgg 8220

ctgtgagcaa ttatgtgctt agtgcatcta atcgcttgag ttaacgccgg cgaagcggcg 8280

tcggcttgaa cgaatttcta gctagacatt atttgccgac taccttggtg atctcgcctt 8340

tcacgtagtg gacaaattct tccaactgat ctgcgcgcga ggccaagcga tcttcttctt 8400

gtccaagata agcctgtcta gcttcaagta tgacgggctg atactgggcc ggcaggcgct 8460

ccattgccca gtcggcagcg acatccttcg gcgcgatttt gccggttact gcgctgtacc 8520

aaatgcggga caacgtaagc actacatttc gctcatcgcc agcccagtcg ggcggcgagt 8580

tccatagcgt taaggtttca tttagcgcct caaatagatc ctgttcagga accggatcaa 8640

agagttcctc cgccgctgga cctaccaagg caacgctatg ttctcttgct tttgtcagca 8700

agatagccag atcaatgtcg atcgtggctg gctcgaagat acctgcaaga atgtcattgc 8760

gctgccattc tccaaattgc agttcgcgct tagctggata acgccacgga atgatgtcgt 8820

cgtgcacaac aatggtgact tctacagcgc ggagaatctc gctctctcca ggggaagccg 8880

aagtttccaa aaggtcgttg atcaaagctc gccgcgttgt ttcatcaagc cttacggtca 8940

ccgtaaccag caaatcaata tcactgtgtg gcttcaggcc gccatccact gcggagccgt 9000

acaaatgtac ggccagcaac gtcggttcga gatggcgctc gatgacgcca actacctctg 9060

atagttgagt cgatacttcg gcgatcaccg cttcccccat gatgtttaac tttgttttag 9120

ggcgactgcc ctgctgcgta acatcgttgc tgctccataa catcaaacat cgacccacgg 9180

cgtaacgcgc ttgctgcttg gatgcccgag gcatagactg taccccaaaa aaacatgtca 9240

taacaagaag ccatgaaaac cgccactgcg ccgttaccac cgctgcgttc ggtcaaggtt 9300

ctggaccagt tgcgtgacgg cagttacgct acttgcatta cagcttacga accgaacgag 9360

gcttatgtcc actgggttcg tgcccgaatt gatcacaggc agcaacgctc tgtcatcgtt 9420

acaatcaaca tgctaccctc cgcgagatca tccgtgtttc aaacccggca gcttagttgc 9480

cgttcttccg aatagcatcg gtaacatgag caaagtctgc cgccttacaa cggctctccc 9540

gctgacgccg tcccggactg atgggctgcc tgtatcgagt ggtgattttg tgccgagctg 9600

ccggtcgggg agctgttggc tggctggtgg caggatatat tgtggtgtaa acaaattgac 9660

gcttagacaa cttaataaca cattgcggac gtttttaatg tactgaatta acgccgaatt 9720

gaattatcag cttgcatgcc ggtcgatcta gtaacataga tgacaccgcg cgcgataatt 9780

tatcctagtt tgcgcgctat attttgtttt ctatcgcgta ttaaatgtat aattgcggga 9840

ctctaatcat aaaaacccat ctcataaata acgtcatgca ttacatgtta attattacat 9900

gcttaacgta attcaacaga aattatatga taatcatcgc aagaccggca acaggattca 9960

atcttaagaa actttattgc caaatgtttg aacgatctgc ttgactctag ctagagtccg 10020

aaccccagag tcccgctcag aagaactcgt caagaaggcg atagaaggcg atgcgctgcg 10080

aatcgggagc ggcgataccg taaagcacga ggaagcggtc agcccattcg ccgccaagct 10140

cttcagcaat atcacgggta gccaacgcta tgtcctgata gcggtccgcc acacccagcc 10200

ggccacagtc gatgaatcca gaaaagcggc cattttccac catgatattc ggcaagcagg 10260

catcgccgtg ggtcacgacg agatcctcgc cgtcgggcat ccgcgccttg agcctggcga 10320

acagttcggc tggcgcgagc ccctgatgct cttcgtccag atcatcctga tcgacaagac 10380

cggcttccat ccgagtacgt gctcgctcga tgcgatgttt cgcttggtgg tcgaatgggc 10440

aggtagccgg atcaagcgta tgcagccgcc gcattgcatc agccatgatg gatactttct 10500

cggcaggagc aaggtgagat gacaggagat cctgccccgg cacttcgccc aatagcagcc 10560

agtcccttcc cgcttcagtg acaacgtcga gcacagctgc gcaaggaacg cccgtcgtgg 10620

ccagccacga tagccgcgct gcctcgtctt ggagttcatt cagggcaccg gacaggtcgg 10680

tcttgacaaa aagaaccggg cgcccctgcg ctgacagccg gaacacggcg gcatcagagc 10740

agccgattgt ctgttgtgcc cagtcatagc cgaatagcct ctccacccaa gcggccggag 10800

aacctgcgtg caatccatct tgttcaatca tgcctcgatc gagttgagag tgaatatgag 10860

actctaattg gataccgagg ggaatttatg gaacgtcagt ggagcatttt tgacaagaaa 10920

tatttgctag ctgatagtga ccttaggcga cttttgaacg cgcaataatg gtttctgacg 10980

tatgtgctta gctcattaaa ctccagaaac ccgcggctga gtggctcctt caacgttgcg 11040

gttctgtcag ttccaaacgt aaaacggctt gtcccgcgtc atcggcgggg gtcataacgt 11100

gactccctta attctcatgt atgataattc gagct 11135

<210>  119
<211>  20
<212>  DNA
<213>  Artificial

<220>
<223>  Construct specific primer

<400>  119
gggtaagatc aacttgaagg                                                    20


<210>  120
<211>  20
<212>  DNA
<213>  Artificial

<220>
<223>  Construct gene specific primer

<400>  120
acttctccac aagagcattc                                                    20


<210>  121
<211>  22
<212>  DNA
<213>  Artificial

<220>
<223>  Construct-specific forward primer

<400>  121
gtctatggat cggagcattg tg                                                 22


<210>  122
<211>  20
<212>  DNA
<213>  Artificial

<220>
<223>  Construct-specific reverse primer

<400>  122
cagcagcaac atcctcagtg                                                    20


<210>  123
<211>  22
<212>  DNA
<213>  Artificial

<220>
<223>  PCR primer

<400>  123
cactatgtcc tctacttcct tg                                                 22


<210>  124
<211>  19
<212>  DNA
<213>  Artificial

<220>
<223>  PCR primer

<400>  124
atggtcttgg gtcttcctg                                                    19


<210>  125
<211>  20
<212>  DNA
<213>  Artificial

<220>
<223>  PCR primer

<400>  125
cactggtacg tgtagctgat                                                   20


<210>  126
<211>  20
<212>  DNA
<213>  Artificial

<220>
<223>  PCR primer

<400>  126
gtaagaggtc cacctttacc                                                   20


<210>  127
<211>  52
<212>  DNA
<213>  Artificial

<220>
<223>  Forward primer

<400>  127
ggggaccact ttgtacaaga aagctgggtt ccgagaaaga aaatggtgac cg               52


<210>  128
<211>  53
<212>  DNA
<213>  Artificial

<220>
<223>  Forward primer

<400>  128
ggggaccact ttgtacaaga aagctgggta accaaccctg tacaaatacc acg              53


<210>  129
<211>  52
<212>  DNA
<213>  Artificial

<220>
<223>  Forward Primer

<400>  129
ggggaccact ttgtacaaga aagctgggtg ctgcagaaca gatgcttact cc               52

<210>  130
<211>  52
<212>  DNA
<213>  Artificial

<220>
<223>  Forward primer

<400>  130
ggggaccact ttgtacaaga aagctgggtg aagaattagc aatgggcaaa cc          52


<210>  131
<211>  52
<212>  DNA
<213>  Artificial

<220>
<223>  Forward primer

<400>  131
ggggaccact ttgtacaaga aagctgggta ttcacatggg aaaggtagtg gg          52


<210>  132
<211>  53
<212>  DNA
<213>  Artificial

<220>
<223>  Forward primer

<400>  132
ggggaccact ttgtacaaga aagctgggtt ttctctaggt cccagacagt tgg         53


<210>  133
<211>  52
<212>  DNA
<213>  Artificial

<220>
<223>  Forward primer

<400>  133
ggggaccact ttgtacaaga aagctgggtt aagtggtcca cgaaacagtt gg          52


<210>  134
<211>  52
<212>  DNA
<213>  Artificial

<220>
<223>  Reverse primer

<400>  134
ggggacaagt ttgtacaaaa aagcaggctt ctttgcctaa ctttgggacc tc          52


<210>  135
<211>  50
<212>  DNA

<213>  Artificial

<220>
<223>  Reverse primer

<400>  135
ggggacaagt ttgtacaaaa aagcaggcta atcggaaccg tctgtcttgg                50


<210>  136
<211>  50
<212>  DNA
<213>  Artificial

<220>
<223>  Reverse primer

<400>  136
ggggacaagt ttgtacaaaa aagcaggctt tacaagatgc tccgaatggg                50


<210>  137
<211>  53
<212>  DNA
<213>  Artificial

<220>
<223>  Reverse primer

<400>  137
ggggacaagt ttgtacaaaa aagcaggctc agaaaacaga taatcaacga gcg            53


<210>  138
<211>  51
<212>  DNA
<213>  Artificial

<220>
<223>  Reverse primer

<400>  138
ggggacaagt ttgtacaaaa aagcaggctg aagcacaacg gaagactgag g              51


<210>  139
<211>  54
<212>  DNA
<213>  Artificial

<220>
<223>  Reverse primer

<400>  139
ggggacaagt ttgtacaaaa aagcaggctt tgataaggtt tgtctaatct cccg           54


<210>  140
<211>  54
<212>  DNA
<213>  Artificial

<220>
<223>  Reverse primer

<400>  140
ggggacaagt ttgtacaaaa aagcaggctc gcttttgttc ttattatagc tggc          54


<210>  141
<211>  54
<212>  DNA
<213>  Artificial

<220>
<223>  Forward primer

<400>  141
ggggacaagt ttgtacaaaa aagcaggctg agttcttgag ctaaacgaga ggag          54


<210>  142
<211>  23
<212>  DNA
<213>  Artificial

<220>
<223>  Forward primer

<400>  142
cggtcttatt tgtattcgcc tgc                                            23


<210>  143
<211>  19
<212>  DNA
<213>  Artificial

<220>
<223>  Forward primer

<400>  143
atgcaaccag gcttggatg                                                 19


<210>  144
<211>  27
<212>  DNA
<213>  Artificial

<220>
<223>  Forward primer

<400>  144
atggtggaga agatcatcaa tatgaac                                        27


<210>  145
<211>  20
<212>  DNA
<213>  Artificial

<220>
<223>  Forward primer

<400>  145
atggagtttg atgggctggc                                                20


<210>  146

<211>  23
<212>  DNA
<213>  Artificial

<220>
<223>  Forward primer

<400>  146
atgttgatgg ctttaaaatc ccg                                               23


<210>  147
<211>  23
<212>  DNA
<213>  Artificial

<220>
<223>  Forward primer

<400>  147
atggacaaca atacctctcg ctc                                               23


<210>  148
<211>  25
<212>  DNA
<213>  Artificial

<220>
<223>  Forward primer

<400>  148
atggaaacaa agtgtccagt agatg                                             25


<210>  149
<211>  55
<212>  DNA
<213>  Artificial

<220>
<223>  Reverse primer

<400>  149
ggggaccact ttgtacaaga aagctgggtt aactcaccaa attaagatca aagcc           55


<210>  150
<211>  26
<212>  DNA
<213>  Artificial

<220>
<223>  Reverse primer

<400>  150
gcaaattagc aacacatgaa cctacc                                            26


<210>  151
<211>  22
<212>  DNA
<213>  Artificial

<220>

<223>  Reverse primer

<400>  151
taaatgcagt aaatgatgga gc                                                22


<210>  152
<211>  28
<212>  DNA
<213>  Artificial

<220>
<223>  Reverse primer

<400>  152
attacacaaa tattcacact agaaaggg                                          28


<210>  153
<211>  22
<212>  DNA
<213>  Artificial

<220>
<223>  Reverse primer

<400>  153
caggggatat ctagcttagg gc                                                22


<210>  154
<211>  22
<212>  DNA
<213>  Artificial

<220>
<223>  Reverse primer

<400>  154
gaagttaacg ccaagcagat gc                                                22


<210>  155
<211>  27
<212>  DNA
<213>  Artificial

<220>
<223>  Reverse primer

<400>  155
gacaaactta tggacccgtt aataatc                                           27


<210>  156
<211>  24
<212>  DNA
<213>  Artificial

<220>
<223>  Reverse primer

<400>  156
ctactttttg ttggggtctt gctc                                              24

<210>  157
<211>  336
<212>  DNA
<213>  Artificial

<220>
<223>  Construct sequence

<400>  157
tccgagaaag aaaatggtga ccgttgatga aattcggaag gcgcagaggg cagagggccc      60

tgccaccatc ttggccatcg gaacatcaac ccctccgaac tgtgttgatc aaagcaccta     120

tcctgactat tattttcgta tcacaaacag cgagcataag gtggagctta aagaaaaatt     180

caagcgaatg tgcgagaaat ccatgatcaa gaagagatac atgcacttga cggaggagat     240

cttgaaagaa aatcctagtg tatgcgaata catggcacct tcattggatg ctaggcaaga     300

catggtggtg gtggaggtcc caaagttagg caaaga                               336

<210>  158
<211>  322
<212>  DNA
<213>  Artificial

<220>
<223>  Construct seqeunce

<400>  158
aaccaaccct gtacaaatac cacgggttcc aacacagtac caggcactga gccgcagttc      60

cccaattacc cgagccttct aaccaaatgc catttgaatg tctccaacac aagggatgga     120

gctgtgatgc ctgcttgtgt tcccaacctg gatccaagtc ttgtgctcac taacttcaca     180

cggactacat actccgataa ccttaatgat actaagttta caggagctgg gattggttct     240

gatggtaact ggatagttgt tgttctgacc acaagcacac ccgagggaag ttttgtgact     300

tccaagacag acggttccga tt                                              322

<210>  159
<211>  349
<212>  DNA
<213>  Artificial

<220>
<223>  Construct sequence

<400>  159
gctgcagaac agatgcttac tccagcttct ccagctctcc cattctctct atgattgccc      60

tcttgcttgt ttcttgaagc tccccagcga gaatgaattc atcaagtatc aaataaacct     120

tgtgaaagtt aaacaccaaa tctagctcac acacattgct gaagaaatga tccaatatct     180

ccacaaataa atgaatgcac tccaaataag ccaattcgtt atccgttatg tcgacacaca     240

gcgaaaaaaa caatccagca taccgcctgt gtttttgaaa ggcaatcttg gaattttttt     300

ttcctttgtt ttttttccct gtggtctccc cattcggagc atcttgtaa                 349

<210> 160
<211> 326
<212> DNA
<213> Artificial

<220>
<223> Construct sequence

<400> 160
gaagaattag caatgggcaa accagatggt aaacgagctt ctcctggaag tcaggtcagt 60

gtgcactata ttggtaagct gaagaacgga aaagtatttg actctaatgt cggaagggct 120

ccctttaaat tccgcctagg tgttggacaa gtcataaaag gatgggatgt tggagttaat 180

ggtatgcgag ttggggacaa aagaagactc acagttccac catcgatggg ttacggagat 240

cggggtgctg gcgggaagat acctccaaac tcatggcttg tgtttgatgt tgagttggtg 300

aacgctcgtt gattatctgt tttctg 326


<210> 161
<211> 296
<212> DNA
<213> Artificial

<220>
<223> Construct sequence

<400> 161
attcacatgg gaaaggtagt gggtccctta tcagtggggc gatgggaggg ttaagtttgg 60

aagaagggag taactacgag aaagagaaag ttgctactga taaggagagt cacagctacc 120

atgattatcg tcgcgaatat tgaaggaata gagagactgt gtgcgtgtgt gtgtgaaggt 180

atatttgagc tttttatact cgcattgtgg ctttgactga tggatatact tttgtatgta 240

catgcaaaaa attatcataa ctgctagatg ctgccctcag tcttccgttg tgcttc 296


<210> 162
<211> 248
<212> DNA
<213> Artificial

<220>
<223> Construct sequence

<400> 162
tttctctagg tcccagacag ttgggaacca acagcatcaa ttctgcaaat caaggaatat 60

cttacataaa ggaaggaaga aaaattcagg ggcaaaagat gtttcttcga cctgacctct 120

cttctcatct tcgagatgaa gcagcttcag ttgtctcatc attttctggg ccattatctc 180

cctgaattcc aattcggaca tttgaatcga aaatcacatg tcacgggaga ttagacaaac 240

cttatcaa 248


<210> 163

<211>  234
<212>  DNA
<213>  Artificial

<220>
<223>  Constsruct sequence

<400>  163
taagtggtcc acgaaacagt tggtgatttg cactccaact aggttatgct ctttaattgg      60

ttggatttct tttgacaggc aaacttgcct gttttagtgt gctctataat aacagtcttg     120

aaaaataagt gtacttttaa gacaacaatg catatggtat ttcatcaatt aagtgaagtt     180

tctgtatttg aatggaaagc tgatgagagg ccagctataa taagaacaaa agcg           234


<210>  164
<211>  1176
<212>  DNA
<213>  Populus trichocarpa

<400>  164
atggtgaccg ttgatgaaat tcggaaggcg cagagggcag agggccctgc caccatcttg      60

gccattggaa catcaacccc tccgaattgt gttgatcaaa gcacctatcc tgactattat     120

tttcgtatca ccaacagcga gcataaggtg gagcttaaag aaaagttcaa gcgaatgtgc     180

gagaaatcca tgatcaagaa gaggtacatg cacttgactg aggagatctt gaaagaaaat     240

cctagtgtgt gcgaatacat ggcaccttca ttggatgcta ggcaggacat ggtggtggtc     300

gagataccaa agttgggcaa agaagcagct gccaaggcca tcaaggaatg gggccagccc     360

aagtccaaaa taacccattt ggtcttttgc acaaccagtg gtgttgacat gcccggggct     420

gactatcaac tcactaagct cttgggtctt cgctcatcag taaaacgttt catgatgtac     480

cagcaaggtt gttttgctgg tgggacggtg ctccgcctag ctaaagacct tgctgaaaac     540

aacaaaggtg ctcgcgtgct agtcgtgtgc tcagaaataa cagcagtgac atttcgtggc     600

cctagtgaca cccacctcga tagtcttgtg ggtcaagcgt tgtttggtga cggcgcagct     660

gctatcataa taggctcgga tcctgtgctg ggggtcgaga agcctttgtt tgagctggtg      720

tctgccgccc agactattct acccgatagt gagggggcta tagatggaca tcttcgcgaa     780

gttgggctta catttcacct tctcaaggat gtgcccgggc ttatttcaaa aaacgtcgaa     840

aagagtctaa ccgaggcttt caagcccttg ggcatctcgg attggaactc ccttttctgg     900

attgcgcacc ctggtgggcc cgcaattctg gaccaggtgg aggctaagtt ggagcttaaa     960

cctgaaaaac tgcgagccac tcgacaagta ctggctgact atggtaacat gtccagtgca    1020

tgtgtgctat ttattttgga cgagatgagg aagaaatctg ccaaggatgg gcttaaatct    1080

actggagaag ggttggaatg gggcgtcctt ttcgggttcg ggcctgggct tactgttgag    1140

acagtggtgc tccacagtct gcctgccaca atttag                              1176


<210>  165

<211>  618
<212>  DNA
<213>  Populus trichocarpa

<400>  165
atggcttctt caagattttc tcttctcttc ccattctttg ttttcttcat tattctctgc      60

ttgatcagtc accctgtcat ttgtgacggt gatcaggagg atgcccttct tcaagggatt     120

aacaactaca ggacatcctt caatttgact accctcacaa aaaatgataa cgcagagtgc     180

cttgctgagg agatagctga ccaatttaag aaccaacctt gtacaaatac cacgggttcc     240

aacacagtac caggcactga gccgcagttc cccaattacc cgagccttct agccaaatgc     300

catttgaatg tctccaacac aagggatgga gctgtgatgc ctgcttgtgt tccccacctg     360

gatccaagtc ttgtgctcac taacttcaca cggactccat actccgataa ccttaatgat     420

actaagttta caggagctgg gattggttct gatggtaact ggatcgttgt tgttctgacc     480

acaagcacac ctgagggaag ttatgtgact tccaagacag acggttcgga ttacaatgca     540

gccaatttga ccgccaagaa tactggtcta atctatcact tgctgttttt gctgataggt     600

tctctcttct tgttgtga                                                   618

<210>  166
<211>  429
<212>  DNA
<213>  Populus trichocarpa

<400>  166
atgatccgat ttatactcct gcaaaacaga caaggcaaga ctcgtctcgc taaatactat      60

gttcctcttg aggattccga gaagcacaaa gtcgaatacg aggttcaccg attggttgtt     120

aatagagatg ctaaattcac gaattttgtt gagtttagga cacacaaggt gatatacagg     180

cggtatgctg gattgttttt ttcgctgtgt gtcgacataa cggataacga attggcttat     240

ttggagtgca ttcatttatt tgtggagata ttggatcatt tcttcagcaa tgtgtgtgag     300

ctagatttgg tgtttaactt tcacaaggtt tatttgatac ttgatgaatt cattctcgct     360

ggggagcttc aagaaacaag caagagggca atcatagaga gaatgggaga gctggagaag     420

ctggagtaa                                                             429

<210>  167
<211>  1503
<212>  DNA
<213>  Populus tricocharpa

<400>  167
atgagcttct ggggtattga agtgaaacca ggcaagcctc atccttatca ctctgatgat      60

gtgcaaggaa agcttcgggt aactcaggca actttgggcc ttggttcgtc aaaagaaagg     120

agcatactcc agtgttcagt tggacacagg agtccaattt ttgtatgttc actgttgcca     180

gggaaagctg aaagttgctc tctgaatctt gaatttacgg atgaattagt agcgttctca     240

gttattggac cacaaagcat ccatctttgt ggttactttg attctgtaga aggagatcac 300

ctccgagatg aatatgaata tgattcggga gaggatattg ttgatacgga gtcggatgag 360

tccagtgaat atgattatga tgatgaatat gatgacgacg atgatgatct tcaaatgtat 420

tccccttcgc ctgttcccaa aagtggagtt gtaattgagg agataactga agatgacaaa 480

cctacaaaag aaaatggcaa gtctaaacgg ctaaagaaga agaataatca atcaagtgac 540

aaggaagacc aaaacaactc tcaacaacaa attgttctta agagggatag aggtatttct 600

gttctggaaa gtgaagatga agatggtttt ccaatttctt cctctgcaaa aagcaaagat 660

acagttcagg agcagcaagc agaactagat ggacaaaaag ataaggaaac aacccaagag 720

actaagaaaa agatggcaag tgaggataat gatgacacta gaaaaaagag aaaagttaaa 780

agcgttgatc aagatggtca accagaaagg aaaacaaaga aaaagaagaa gcagagagaa 840

cgaggtacag aggcaaaggt tgatgaaatg gatgacaagg aagagatcaa caatgcctct 900

agagatgaaa tcgagtccaa gcaagtgaaa aagcaggatt ctaccaaccg aaacaaacat 960

gaacagaggg accttgacac tgatgcagat agtatgcccg gtgaagattc atcggataag 1020

aaaaagaaga agaataagaa gaagaaaaag acccaggata gtggagcaac aactaatgat 1080

caagctgttt cagctgcggg aggtgaagcc aagtcttcat tggagtctga ggataagcaa 1140

tctacagcca agtcctcaca agttagaacc ttttcaaatg gattggttat tgaagaatta 1200

gcaatgggca aaccagatgg taaacgagct tctcctggaa gtcaggtcag tgtgcactat 1260

attggtaagc tgaagaacgg aaaaatattt gactctaatg tcgggagggc tccctttaaa 1320

ttccgcctag gtgttggaca agtcataaaa ggatgggatg ttggagttaa tggtatgcga 1380

gttggggaca aaagaagact cacaattcca ccatcgatgg gttacggaga tcggggtgct 1440

ggcgggaaga tacctccaaa ctcatggctt gtgtttgatg ttgagttggt gaacgctcgt 1500

tga 1503

<210> 168
<211> 927
<212> DNA
<213> Populus trichocarpa

<400> 168
atggcttcct cgaagtctca cgatctcgag atcacaataa tctcagctaa acacctcaaa 60

aacgttaatt ggcgaaacgg cgatctcaaa ccgtacgcaa ccttctatct cgacaactcg 120

gaccgccgac tcgcaactca cgctgacgac tcactctcaa ctcgcccagt ctggaacgaa 180

cgatttactc tccccataac tcgccagatc tacgattcag tcctcaccct cgagatcttc 240

cactccaagc catcggaaac cccccaacct ctcgtaggca ctgtgaaatt cccactgagt 300

aatctcatgg tctcggatga gtcgctgtcc tgcgaagtcc tcacgctcga gctcctacgt 360

ccctctggtc gtccacaggg taaagtccgg gtgaaactag aggtgaaaga acggcctttg 420

cctccgccag tgcaggatta ccatactgcc cctaattata gtcattatta caaccctgcc 480

cctgcccccc ctcctcctcc tccaccacct cctgcacgtg actacaggga atactctcca 540

tctccatacg gctacgccga tccgtacggt tactaccctg cctattatcc tccacaacca 600

ccccgacctc tctacaatcg agcatccaat tacagcttgc caggtgggcc ttctgctcca 660

gttgatctat ctgcccaatc atcaccatcg ccctacgatc acaagccgcc gccgccgccg 720

ccgggattgt tccaaaagac gtccaattat ggtgtgccaa gtggaccgtc ggctcccgtc 780

gattattcac atgggaaagg tagtgggtcc cttatcagtg gggcaatggg agggttaagt 840

ttggaagaag ggagtaacta cgagaaagag aaagttgcta ctgataagga gagtcacagc 900

taccatgatt atcgtcgcga atattga 927

<210> 169
<211> 1716
<212> DNA
<213> Populus trichocarpa

<400> 169
atgtggatat ctacttcagg aattgcaggc aagactgcct cttggaaatc aatgatatta 60

atttatcaat caataacagc attgcttttt tcttcacgat ctctttccct tcctcttaat 120

atgaaacaga tgatatggga agaagttggg gaagatcagt ttgaacgaga gaaggtcctg 180

ctggatttag aacaagagtg cttagaggtt tataggagaa aagttgacaa tgcaaacata 240

tcaagagctc gcctgcatca agagctggcg gaatctgaag ctgaattcac ccatcttctt 300

ctgtcccttg gtgaacgatc actcccagga cggccagaaa gaaagtcagg aacactgaag 360

gagcagctag atgcgatcac cccagctcta cgggagatgc gtttgagaaa agaagagagg 420

gtgaatcaat ttagatctgt gcaaggtcaa attcaaaaaa tttctgcaga aattgcaggt 480

caatcagtgt acgatgaccc gataacaaat gtcaaggtaa atgagaatga tctttcatta 540

aagaaacttg aggaatacca aattgagcta caaaggctgc acaatgagaa gaatgacagg 600

ctccagctag tggacacata tattgacaca atccatgatt tgtcctcaac attaggaatg 660

gaatcctcca tgatcataac aaaggtgcat ccaactttga atgaattgtg tggaatatca 720

aaaaatataa gtgacagtat tctggataaa ctcaacagca ctgtggaatc tctcaaagaa 780

gaaaaacaaa agcggcttga gaagcttcat cagcttggaa aggcactgaa aaacttgtgg 840

aatctaatgg acacgcccta taaagattgt cactcatttt ccaatgtcac ggacttatta 900

tttctctcat cagatgaagt atcaggtcct ggaagccttg ctctaaatat aatccagcag 960

gctaaaaaag gggagattga ccatgccgat ctcctcatga gcttggatga gcagatatca 1020

agagcaaaag aggaagcttc tagcaggaag gctataatgg aaaaggttga aaagtggata 1080

ttagcacgtg atgaagagcg ctggttagaa gaatacagca tggacgagaa tcgatattca 1140

gttcgcagag gtgctcataa gaatctgcaa cgtgcagaac gtgcccgggt aatagtcaac 1200

aagatcccag ttctagtggc atcgctcgta gcaaagactg agagttggga agaggaaaga 1260

aacaaagtat tcctgtatga tggagtaccc ctgctggcaa tgctggaaga gtataacata 1320

tcgaggcagg agatggaaga ggaaaagcaa agacaaaggg ctagttcatc caacactcta 1380

caggcaaagg aaaagaaggt cccaagccat gtagaagttg agcaagaaaa tttgatcggg 1440

tcaagctcaa ggccaagcac cagcagtcga cgtctttcaa acaagagctt gaatggagga 1500

tttagcaatg caacgccttt aaacagaagg ctttctcttg gtcccagaca gctgggaacc 1560

aacagcatca attctgcaaa tcaaggaata tcttacataa aggaaggaag aaaaattcag 1620

gggcaaaaga tgtttcttcg acctgacctc tcttctcatc ttcgagatga agcagcttca 1680

gttgtctcat catttctggg gccattatct ccctga 1716

<210> 170
<211> 2763
<212> DNA
<213> Populus trichocarpa

<400> 170
atgcaagcag cctccggaac taagcgtggt tctagtggtt ccccggtgat tgataagcaa 60

gggagggcag tggccttgaa cgctgggggc agtgtatcat cttcatcagc cttctattta 120

catttagaga gagttgttag ggcattggca tttctccaga agtccaaaga tgcctgtaaa 180

aacaaatggg aagcagtttc tatacctcgt ggcacccttc aggtaacgtt tctccataaa 240

ggatttgatg agacacgccg gcttggtctt ccgaatgaaa cagagcagat ggtgcggcaa 300

gcctctgcac caggtgaaac tggaatgttg gttgttgact ctgtggtgcc atgtgggcca 360

gctgataggc aattggagtc aggggatgtg cttgtgcgtg tgaatgggga agtgactact 420

cagtttttaa aattggaggc attgcttgat gacagtgttg acaaaaagat cgaattacag 480

attgaaaggg gtggcacatc attaaccgtt gatatagtgg ttcaggattt gcacttgata 540

actcctgact gcttcttaga agtaagtggt gcagtgatac accgtttgtc ttatcaacag 600

gccagaaact tctgcttcca atgtggtctt gtatatgttt cggaccccgg atacatgcta 660

tctagggctg gcattcctcg tcatgccatc attaagaagt ttgccgatga tgagatatca 720

caacttgaag atgtaatctc tgttctgtct aagctatgta agggtgatag agtgccattg 780

gagtatataa gctacaggga tcgccatcga agaaagtcta ccctggtcac aattgatcgc 840

catgaatggt acgatgctcc gaagatatac acacgggatg atagttctgg tttatggatg 900

gcaaggcctg ctattcaacc gaccaccctg cagttgtcac cttgtagtag caatgtcact 960

caaggtctaa acagccaagc atcttcattg aatagtgaat caactcctgc tgaaggtacg 1020

gatcaagcta acaaccagga gttgacacat gatattttaa gaacagaagc cggttatgaa 1080

catatttctg aggaggtcca ttccagggaa gagtgtgatg ttaaaactaa taagcaacag 1140

gtacagggga acttgtcctc tgatgaaatt gcagttgctg accattcctc gcttgaaatt 1200

ggagaaatga aattggagac tccaggtacc actgaaatta cagtttcgaa tggctatgaa 1260

ggtgcaatag cagcagcaac taatgcttca tttgcggaat gtgtggtgga gcccactctt 1320

gtaacattgg aggtcaatgt gccaccatca tgtttgcttg atggcatcca ttcagtgcat 1380

gcttctggga ctggtgttgt tgtacatcat tctcaagaca tgggattggt tgctattgac 1440

aagaacaccg ttgaaacatc tgcatgtgat gtgatgctgt catttgctgc ttttcctatc 1500

gagattccag gagaggttgt ctttcttcat cctgtttaca attatgctct tgttggttat 1560

gatccctctg ctctgggagc tgttggtgct tctatggttc gtgctgctga gttacttcct 1620

gagcctgcat tatgtcgtgg agatccaata tatctgattg ggttaagtaa aaatcaacga 1680

gcaaagtcta ggaaatcaat tgtgacaaac ccttatgtta cattaaattt tggctatgct 1740

gatcgtccac ggtatagagc aataaatatg gaagtgattg agcttgacac tgattttggt 1800

aatgcattta ctggtgtgct atgtaatgag catggaaagg ttcaggctat atgggggagc 1860

ttttcaaata agccaaaatc tagtcacact acatcaaagg atcatcaatt tgttcgaggt 1920

gtcccaatct acatgattag ccaagtactt gacaaaatca tatctggtgc aaatggggct 1980

tctattctca taaacggtat caaaaggtca atgccacttg ttaggacttt agaggtggaa 2040

ctttgctcta gattgctttc aaaggcccgg agttttggtc ttggtgatga atggatccaa 2100

agacttgtaa agaaagaccc aatgagacga caagttttgc gtgttaaagg ttgtttggct 2160

ggatctaaca ctgagaatct attaaaacaa ggcgatatgt tgttggcaat caacaaagag 2220

ccagttactt gcttccaaga tgtagaaaat gcatgtcaag cattagagaa ctgtgttgac 2280

agtgatggga agcttaaaat taccatttgt cggcagggag gtgaagttga tctacttgtt 2340

ggaacagaca ttagggatgg taatggtaca acacgagcag taaattggtg tggctgtctt 2400

gttcaggatc ctcatccagc agttcgtgct cttggatttc ttcctggtga aggtcatggt 2460

gtctatgcag caatgtgctg ccgtggaagt cctgcagata gatatgctct aggtgctctt 2520

agatggattg ttcgagtcaa tggaaaaccc actcctgact tggatgcttt tgtaaatgtg 2580

acaaagggat tacgatatga tgagttcgtt cgtgtaaaga ccatcaatct ggatgggaag 2640

ccacgagtgc taacgttgaa gcaggacttg cactactggc ctacatggga gttgaggttt 2700

gatccaaaca ccgctaggtg gcggagagag acaataaagg ctttagattg caatattgaa 2760

taa 2763

<210> 171
<211> 651
<212> DNA
<213> Populus tremula x Populus tremuloides

<400> 171
agaaaacaac agtagcttca attagcagca gcagcaatgg cttcttcaag atttctcttt 60

ctcttcccat tctttgtttt cttcattatt ctctgcttga tcagtcaccc tgtcatttgt 120

gacggtgatg aggaggatgc tcttcttcta gggattaaca agtataggac atccttcaat 180

ttgagtaccc tcacaaaaaa tgataacgca gagtgccttg cagaggagat agctgaccaa 240

tttaagaacc aaccctgtac aaataccacg ggttccaaca cagtaccagg cactgagccg 300

cagttcccca attacccgag ccttctaacc aaatgccatt tgaatgtctc caacacaagg 360

gatggagctg tgatgcctgc ttgtgttccc aacctggatc caagtcttgt gctcactaac 420

ttcacacgga ctacatactc cgataacctt aatgatacta agtttacagg agctgggatt 480

ggttctgatg gtaactggat cgttgttgtt ctgaccacaa gcacacctga gggaagtttt 540

gtgacttcca agacagacgg ttccgattcc aatgcagcca atttgaccgc caagaaaact 600

ggtctaatct atcacttact gttttgctga taggttctct cttcatgttg t 651

<210> 172
<211> 826
<212> DNA
<213> Populus tremula x Populus tremuloides

<400> 172
cccctcggag gtcgacccca cgcgtgccgc taatgcagat aatatacccg gtgaagattc 60

atcggataag aaaaagaaga agaagaagaa aaagaaaaag acccaggata gtggagcaac 120

aactaatgat caagctgttt cagctgtggg aggtgaagcc aagtcttcat tggagtctga 180

ggataagcaa tctacagcca agtcctcaca agttagaacc ttttcaaatg gattggttat 240

tgaagaatta gcaatgggca aaccagatgg taaacgagct tctcctggaa gtcaggtcag 300

tgtgcactat attggtaagc tgaagaacgg aaaagtattt gactctaatg tcggaagggc 360

tccctttaaa ttccgcctag gtgttggaca agtcataaaa ggatgggatg ttggagttaa 420

tggtatgcga gttggggaca aaagaagact cacagttcca ccatcgatgg gttacggaga 480

tcggggtgct ggcgggaaga tacctccaaa ctcatggctt gtgtttgatg ttgagttggt 540

gaacgctcgt tgattatctg ttttctgtgc tccttttctg cttttgaact caatggctgc 600

ttttacagac agtgggtata gatggatctc gaaaattggt gttttaactg taggcttgtt 660

ttacaatact gcaagttcac ccccatcgca taacttaagt gtcaagatgg atggtgtgaa 720

taaaattttg gttaattcaa ttgtagcaag actagtgtgc tctgcaaaaa ttttggttaa 780

aactagctgt tctgtttcag cagaatgtaa aaaaaaaaaa aaaaaa 826

<210> 173
<211> 731
<212> DNA
<213> Populus tremula x Populus tremuloides

<400> 173
gggaatacgc tccatctcca tacggctacg ccgatccgta cggttactac cctgcctatt 60

atcctccaca accaccccga cctctctaca atcgagcatc caattacagc ttgccaggtg 120

ggccttctgc tccagttgat ctatctgccc aatcatcacc atcgccctac gatcacaagc 180

cgccgccgcc gccgcgggat tgttccaaaa gacgtccaat tatggtgtgc caagtggacc 240

gtcggctccc gtcgattatt cacatgggaa aggtagtggg tcccttatca gtggggcgat 300

gggagggtta agtttggaag aagggagtaa ctacgagaaa gagaaagttg ctactgataa 360

ggagagtcac agctaccatg attatcgtcg cgaatattga aggaatagag agactgtgtg 420

cgtgtgtgtg tgaaggtata tttgagcttt ttatactcgc attgtggctt tgactgatgg 480

atatactttt gtatgtacat gcaaaaaatt atcataactg ctagatgctg ccctcagtct 540

tccgttgtgc ttcaattatt tcttatctag tgtgtctgtt tctcttactt ctgtaatgga 600

tgtttggttc tatacttgct tctttgtttc aagtcttaat tcgagtttgc tagcttatac 660

acttgggtac tccattgatt gttaattgta gtataaatag tgaatgatta gagaatgatg 720

ggggaactta a 731

<210> 174
<211> 822
<212> DNA
<213> Populus tremula x Populus tremuloides

<400> 174
aaaaaaaaaa aaaaaaaact cgagactaga tcgcttgtaa caaagactga gagttgggaa 60

gaggaaagaa acaaagtatt cctgtatgat ggggtacccc tgctggcaat gctggaagag 120

tacaacatat cgaggcagga gatggaagag gaaaagcaaa gccaatgggg tagttcatcc 180

aacactttac aggcaaagga aaagaaggtc ccaagccatg tagtagttga gcaagaaaat 240

ttgatcgggt caaggccaag caccagcagt cgacgtcttt caaacaagag cttgaatgga 300

ggatttagca atgcaacgcc tttaaacaga aggctttctc taggtcccag acagttggga 360

accaacagca tcaattctgc aaatcaagga atatcttaca taaaggaagg aagaaaaatt 420

caggggcaaa agatgtttct tcgacctgac ctctcttctc atcttcgaga tgaagcagct 480

tcagttgtct catcattttc tgggccatta tctccctgaa ttccaattcg gacatttgaa 540

tcgaaaatca catgtcacgg gagattagac aaaccttatc aaggaccata atagtaactg 600

ttatttctgc aaaacaataa tttacctatt tgcatcaatt tgataagaaa taacttgtgt 660

atctatgcct tgtaccaaca cccgtattta gaacagaacc ccaaagaact gtcattgcaa 720

tgaaaaataa ttattcaggt ttaaaactat gtagagctat gttttgtaat caagaagctt 780

tgattttcct attctattga aattatgaaa catgataatt tc 822

<210> 175
<211> 829
<212> DNA
<213> Populus tremula x Populus tremuloides

<400> 175
ctgtgttgac agtgatggga agcttaaaat taccatttgt cggcagggag gtgaagttga 60

tctacttctt ggaacagaca ttagggatgg taatggtaca acacgagcaa taaattggtg 120

cggctgtctt gttcaggatc ctcatccagc agttcgtgct cttggatttc ttcctggtga 180

aggtcatggt gtctatgcag caatgtgctg tcgaggaagt cccgcagata gatatgctct 240

aggtgctctt agatggattg ttcaagtcaa tggaaaaccc actcctgact tggatgcttt 300

tgtaaatgtg acgaagggat tacgatatga tgagttcgtt cgtgtaaaga ccatcaatct 360

ggatgggaag ccacgagtgc taacgttgaa gcaggacttg cactactggc ctacatggga 420

gttgaggttt gatccaaaca ccgctaggtg gcggagagag acaataaagg ctttagattg 480

caataatgaa taaacagatt gttctgatct cacttcgtgt tctttcctgg aatggttatt 540

gcacgttcaa cactgcttca agagcgtaag tggtccacga aacagttggt gatttgcact 600

ccaactaggt tatgcccttt aattggttgg atttcttttg acaggcaaac ttgcctgttt 660

tagtgtgctc tataataacg gtcttgaaaa ataagtgtac ttttaagaca acaatgcata 720

tggtatttca tcaattaagt gaagtttctg tatttgaatg gaaagctgat gagaggccag 780

ctataataag aacaaaagcg aatgctttat tttgtgaatg taaaaaaaa 829

<210> 176
<211> 487
<212> DNA
<213> Artificial

<220>
<223> Construct sequence

<400> 176
gagttcttga gctaaacgag aggaggagga gaattgtttt tcgtttttca gatatggagg 60

agaggcatgt tatttttggg aagtatgaaa tggggaggct tttagggaag ggaacttttg 120

ctaaagtttt ctatgggaaa cacttggtga caggggagag tgtggcaatc aaagtcataa 180

gcaaagatca agtcaagaaa gaagggatga tggagcaaat ccagagagaa atctcagtca 240

tgcgtcttgt tcgtcatccc aacattgtag agctcaagga agtcatggct accaagacta 300

agatcttctt catcatggag tatgttcgag gaggagagtt gtttgccaaa gtagccaaag 360

gaaggctgaa agaagaagct gctcgaaaat atttccagca actaatcagc gcaattgatt 420

attgccatag cagaggtgtt taccatagag atttgaagcc agagaatttg ttgcttgatg 480

aagatga 487

<210> 177
<211> 452
<212> DNA
<213> Artificial

<220>
<223> Construct sequence

<400> 177
taactcacca aattaagatc aaagcctttc tacaacattt gaatctcagg atcttctaca 60

tgactattgt tgttatcttg attacaaaca ttgtctcctt gccatgtcca aacgatgtct 120

tttagtgcag gcctaacatc ttcctcgcag aacctagtat actccaaggt atctccggaa 180

gacttggaga actccaccac cgcaacctcc ggtgccacct cgaacacctc cgccgtcacc 240

gccagcttcc cctttctccc ctcacatgga ccttgtaacg tcactttgaa atctttgact 300

tttgccacct tataactcag cccccttgca accccttcaa ttttttccat gatcgcactc 360

gctgaaaatt ttgaagtgaa catagagctt gatttcctct tagtctcaaa aagaccagac 420

agatcaaagc cggatgacat cgaggaaata aa 452

<210> 178
<211> 1560
<212> DNA
<213> Artificial

<220>
<223> Construct sequence

<400> 178
cggtcttatt tgtattcgcc tgcaatatcg tacggcctgg gatctctctt ctcttcttac 60

tgagcaagtt gcatctcata agcactggcg atcatggagc tgaatggtca aacaagagtg 120

agaagaaagg accattttgc tcatacaaac ggtgatttag cattagcaag tgttggtgat 180

gtagatccct ggactgcatg ggcatataag cctcgaacta tttcgctgtt acttattggt 240

gcttgctttc taatatgggc aagtggagcc ctagatccag agagctgtac atctggtgat 300

gttgttacat ctgtgaaaag gggtatatgg gcaatgactg cagtttttct tggttattgc 360

ttgctacaag ccccttcaac ggttctaata aggccacatc cagcaatttg gcgcttagtt 420

catggattgg ccattgttta tcttgttgcc ctcacatttc tgctttttca gaagcgtgat 480

gatgcgcggc aatttatgaa gtttctccat cctgaccttg gaattgaact acctgaaaga 540

tcatatggtg ctgattgtcg catttatgtg cctgaaaatc ctacaagcaa gtttaagaac 600

gttttggaca ctctttttga tgaatttgtt ctagcacata tctttggatg gtggggcaag 660

gctatattaa tccgtaatca gccacttcta tgggtattgt caattggttt tgagctgatg 720

gagtttacct tccgccacat gctaccaaac ttcaatgaat gctggtggga cagtatcatt 780

cttgatattt tgatatgcaa ttggtttggc atttgggctg gaatgcatac agtcgggtat 840

tttgatggaa aaacatacga gtgggttggt ataagtcgcc aacctaatat tatgagcaag 900

gtcaaacgaa cattggaaca atttacacct gcacagtggg acaaagatga atggcatccc 960

ttgcttggtc catggcgatt tatccaagtt cttagtctct gtattgtctt cctgactgta 1020

gagctcaaca cattcttttt gaagttttgt ctatgggttc ctcctcggaa ccctgtaata 1080

gtgtacaggc tgatcttgtg gtggctaatt gccataccta caacacgtga atacaattca 1140

tatctccaag accgaaagcc tgtgaaaaag gttggtgctt tttgttggct ttcccttgct 1200

atttgcattg tggaacttct catttgcatc aagtttggac atggtcttta tcccaaacca 1260

atgcctgcat ggttggtcat cttctggaca tctgttggag ttagccttgt aatattcctg 1320

attatgtggt catggaaaag tttgggaaga aagagacgat gattttgcta gcatggtaca 1380

ctattctttt attccttcat gttcttatgg gtatacatct tgtaaatgcc ctatagcttc 1440

atggaattgt aaatgcttat tctcttaggt tagtggatca cagttttaga gtattcaatt 1500

cttttatgat aggtagttgg aaaatgcttc taatggtagg ttcatgtgtt gctaatttgc 1560

<210> 179
<211> 561
<212> DNA
<213> Artificial

<220>
<223> Construct sequence

<400> 179
atgcaaccag gcttggatga gatcacaatg acggggtgga agaatctgaa gcaacggctg 60

tcattcaagg gcctgggtag ttgctgcggg agcacaagct ggatttccag aagtaccacc 120

caaaccatgc cctttatcga tatagaggaa gaagaagagg aagagaccac catgcaaaac 180

caagctcaaa gaggaggagg aggaggagca gcagcagcgc caggtgctgg gatgaatctg 240

gcaatggcat tagctgctga gcgcgattta ggggattcaa atgtcaagac attgatgagt 300

ttgatcgaag aaacggacgg tgttgattgg aggaagaaga ataacagtaa taacaaaagc 360

aggagggaca aggaacagga acagaagcag gaagaagaga aggattgggt atgctgcgtg 420

tgcatggaga gaaataaagg agcagctttt attccatgtg gacacacctt ttgtagggtt 480

tgttcaagag aaatgtgggt taatcgaggg tgctgtcctg tctgcaaccg ttccattctc 540

gacatccttg atatcttcta g 561

<210> 180
<211> 369
<212> DNA
<213> Artificial

<220>
<223> Construct sequence

<400> 180
atggtggaga agatcatcaa tatgaacagt caagatcatc tgaggagtac taattataaa 60

gatgatgatg atgaagaaga agaagaagtt caacttccag ggtttcgatt tcacccaaca 120

gatgaagaac ttgttgggtt ctatcttcgt agaatggttg acaagaagcc tctcagaatt 180

gaactcatca aacaagtcga gatctacaaa tatgatccat gggatctacc aattaagtca 240

agctgtgtgg gagataagga agggtacttc ttttgcaaac gaggaagaaa gtataggaat 300

agcataagac ctaacagggt gacaggttct ggattttgga aagcaaccgg cattgataag 360

cctgtgtat 369

<210>  181
<211>  410
<212>  DNA
<213>  Artificial

<220>
<223>  Construct sequence

<400>  181
attacacaaa tattcacact agaaagggat caaaagcaca atctacaact gatgtaagct      60

catcccagtc cccatgcgta agaatctgat ttccaaatgg gctggaaatg ttcaaagatg     120

aagccataga tggtggttga gctacgtagc tcaactcgtc cacatgcagt ggtttcctct     180

ccttgacatg atgaactgga ggcttattat cgtaatgttg aatgagtgga gcaccaaaat     240

tgacgtagct ttcttgattg taattagatt ccacttgaca gcatgtcttg gagctcgcgt     300

cgattggtgc ttgttgaggt gtagttgata attgcttcaa atctggtgtg tattgtctgc     360

atgacacatt tcttttgaaa attctacaca gtgtccatac ctcagcttct                410


<210>  182
<211>  787
<212>  DNA
<213>  Artificial

<220>
<223>  Construct sequence

<400>  182
atggagtttg atgggctccc aggttttcga ttccacccaa cagaagaaga actcttagat      60

ttctacctta caaagactgt cttaggtcaa gacacagctg atatcattgg tttcctcaac     120

atatacaatc acgaaccttg ggacttgcca ggattatcga agatagggga gagggagtgg     180

tatttccttg tgcacaggga gagcgttctt gggagaccca ggaggacaac cgagaaaggt     240

tactggaagg caactggttc ggacaggccc atccgatgct tgatggatcc gaaaaggttg     300

ttgggtcata gaaaaactat ggttttctac agaggaagag ctccacgagg gagcaaaacg     360

gattgggtca tgaatgagta cagattgcct agcaactgct atttatcgaa ggagatcgtg     420

ctttgcaaag tatatagaaa ggcaacatca ttgaaggtct tggagcaaag ggcagccata     480

gaagaaattg ttagagcacc taacgtcaca tctttatctc ctccaatgcg agggaatttc     540

tctttttatg accaccaaaa gagcttcaac aagttcttgt tagaacaaaa taatgttgtt     600

cacaaagtag agggagtgac agtaatagaa gaagaagaag acaacaaaat cggttaccca     660

tcccaaataa tgggagttgg acccatcaaa gagcactact ccgagctcct gtcaccaaag     720

ttagtcctgg attggaccat ggattcactt tggcctaact aaggagccct aagctagata     780

tcccctg                                                               787


<210>  183

<211>  276
<212>  DNA
<213>  Artificial

<220>
<223>  Construct sequence

<400>  183
atgttgatgg ctttaaaatc ccgggttaat tctgctgaaa agacaccttc tgtaagggag      60

ttgtacagca aggaacatgt agatgcaacc cagtccttat atgacttgtc accttttttc     120

aagcttggtt tcatggctgc caatcttgcc atcattgagg ccacaagaga gcaaagccga     180

gagatgaata gctgcagcaa tggcttccat gttgttgatt ttgatatcgg tcaaggaggg     240

caatacatga accttctaca cgcgctttct gggctt                               276


<210>  184
<211>  364
<212>  DNA
<213>  Artificial

<220>
<223>  Construct sequence

<400>  184
gaagttaacg ccaagcagat gcgacagtga gagtttttcc cttccaacca aaacataccc      60

caccgttctc ttctttaaca gtaaatcctg ggttaactcg gtttgttaaa ctcaatctcg     120

ctttcattga ctcagcaacg atttgactca tcggcttcaa ctcgaatcct gccattccca     180

tccgggcccg ccatttccca aagacctcgc atctttcaat ccggtccctg ccttcgcaag     240

caaccgagtt tactatcgtc cgacccagtc ccccctcgac ccttgctcgc tttgagctat     300

cccctttcca ttgttgagtc gattgagtcc gaatagcgcc tccgtaatat gaacatgatt     360

cgtt                                                                  364


<210>  185
<211>  1681
<212>  DNA
<213>  Artificial

<220>
<223>  Construct sequence

<400>  185
atggacaaca atacctctcg ctctgaggtt tccgactgcg gcgatccaac gaggccggag      60

tctggcggcg gcggtgctag gtacaagttg atgtcgccgg ccaagcttcc gatctcgagg     120

tcggcttgca tcacgatccc tcctggactc agtccgacct cgtttcttga gtctccagtt     180

cttctctcta atgttaaggc agagccttcc cccactactg gtacgtttac taagcctcga     240

acagttcttg gctctcttag ctccactcca tattctgcta caactgtttc atccacagcg     300

tgtggagaaa gaaaatccga cagctttgag tttagaccat atgctaggtc aaacatggtt     360

tctgcagata ttaaccatca gaggagcaca caatgtgctc aagttcaaag ccagtgccat     420

tcacaatcat ttgcctcacc acctttggta aaaggtgaga tggaagtatc cacaaatgag 480

ttgagtctgt cagcacccct tcacatggtt acttcagttg ctagtgcacc tgctgaagtt 540

gattcagatg aactaaacca aacagggctc tctggcagtg ggcttcaggc atcacagtct 600

gatcatagag taggaagtgc gccttcaatg tcatctgatg atggatataa ctggagaaaa 660

tatggacaga aacatgttaa aggaagtgag ttccctcgca gctattacaa atgtacgcat 720

cctaactgtg aagtgaaaaa gttatttgag cgctcccatg atggacaaat aacagagatt 780

atctacaagg gtacacatga tcatcctaaa ccacaaccca gccgccgata tgcctctggt 840

tctgttttgt ccatgcagga agatagattt gacaagtctt catctctgcc taaccaagat 900

gacaagtcac ctggtgctta tggacaggtg cctcatgcta ttgagccaaa tggtgccctt 960

gaactgtcta ctggagcaaa tgatgatact ggagaaggtg ctgaagacga tgatgatccc 1020

ttctcgaaaa gaaggaggtt ggatgctgga ggttttgatg ttactccagt agtcaaacct 1080

atccgggaac cacgtgttgt cgtgcaaact ctgagcgagg ttgatatact ggatgatggg 1140

tacaggtggc gcaaatatgg ccagaaagtg gtgagaggaa atcctaatcc aaggagttac 1200

tacaaatgca caaatgctgg atgcccagtt agaaagcacg ttgagagggc atcacatgat 1260

ccaaaagcag ttataaccac atatgagggg aaacacaatc atgatgtacc tacagctagg 1320

acaaacagtc atgacatggc gggaccatca gctgtgaatg gaccctcaag gatcagacca 1380

gatgaaaatg agaccataag ccttgatctt ggggtcggga tcagttctac aactgaaaac 1440

cagtccggcg atcagcaaca agctttccgt gcggaactta tcaaacatga aaaccaagca 1500

agtggttcta gtttcagagt agttcatgca accccaatta cggcttacta cggtgtttta 1560

aatggtggca tgaatcagta tggatctagg caaattccag gtgaaagccg tagcattgaa 1620

attccacctt atccatatcc gcagaacatg ggaagattat taacgggtcc ataagtttgt 1680

c 1681

<210> 186
<211> 312
<212> DNA
<213> Artificial

<220>
<223> Construct sequence

<400> 186
atggaaacaa agtgtccagt agatgcaagg tgcgcagctg aaatatctgc catgctcacc 60

cctccttctc ctcttcaact ccaggagtat tttgaggaga ttatatctga aaggaaatgc 120

catggcattg aagtcaaacg agatggaaac ttgagcaaag gggtttatgc aactatggaa 180

cttaaagaag gagaacttat actgaaggac aagatacttg tgggtttaca gcatgttcca 240

aataagcttg actgtctggt gtgcagctat tgtttccaat ttattgagtc agttgaatat 300

cagattggga gg                                                          312

<210>  187
<211>  235
<212>  DNA
<213>  Artificial

<220>
<223>  Construct sequence

<400>  187
ctactttttg ttggggtctt gctctaagca agcgttgcac ctgcatttga atccataatc      60

tgctagcaat gcctgtctgt cttcaaatgg aaggtcctcg tctatgtatg aaacggtaac     120

ctcttctccc tttagaattg gtttcagggt gatgattgct gcctggccat ctctgtcctc     180

atctcttttg aaggcgtgtg catttgggca gcaggagtga ttcatgcagc tctgt          235

<210>  188
<211>  1356
<212>  DNA
<213>  Populus trichocarpa

<400>  188
atggaggaga ggcatgttat ttttgggaag tatgaaatgg gtaggctttt agggaaggga      60

acttttgcta aagtttacta tgggaaacac ttggtgacag gagagagtgt ggcaatcaaa     120

gtcataagca aagatcaagt caagaaagaa gggatgatgg agcaaatcca gagagaaatc     180

tcagtcatgc gtcttgttcg tcatcccaac attgtagagc tcaaggaagt catggctacc     240

aagacaaaaa tcttcttcat catggagtat gttcgaggag gagagttgtt tgccaaagta     300

gccaaaggaa ggctgaaaga agaagctgct cgaaaatatt tccagcaact aatcagcgca     360

attgattatt gtcatagcag aggtgtttat catagagatt tgaagccaga gaatatgttg     420

cttgatgaag atgaaaactt gaaaatctct gattttggct tgtcagcctt gcctgaacaa     480

tttcggcaag atgggctttt gcatactcag tgtggaactc ctgcttatgt tgccccagaa     540

gtcttgagaa agaaaggcta tgatggatca aaagctgata tatggtcatg tggggtgatt     600

ctttatgtgc ttcttgcagg attcttgcca tttcaagatg agaatgttat gaagatgtac     660

aggaaagtct tcaaggctga atatcagttt ccgccttggt tttcaacaga ttctaagagg     720

ttgatttcaa gacttcttgt tgctgatcct gaaaagagaa tcacaattcc tgccataatg     780

agaaatcatt ggtttcttaa agggttttca agaccagtgg ccttttctat ccaagaatca     840

agcatggatc aaacaggaca agaacaagat cttgattctt gttctgttgt caacaccaag     900

gtatcatcac cagaattctt caatgcattc gagtttattt cctcaatgtc atccggcttt     960

gatctgtcta gtctttttga gactaagagg aaatcaggct ctatgttcac ttcaaaattt    1020

tcagcgagtg ctatcatgga aaaaattgaa ggggttgcaa aagggctgag ttataaggtg    1080

gcaaaagtca aagatttcaa agtgacgtta caaggtccat gcgagggtag aaaggggaag    1140

ctggcagtga cggcggaggt gttcgaggtg gcaccggagg ttgcggtggt ggagttctcc 1200

aagtcttccg gagatacctt ggagtatact aagttctgtg aggaagatgt taggcctgca 1260

ctaaaagaca tcgtttggac atggcaagga gacaatgttt gtaataaaga taacaacaat 1320

agtcatgtag aagatcgtga aattcaaatg ttgtag 1356

<210> 189
<211> 1272
<212> DNA
<213> Populus trichocarpa

<400> 189
atggagctga atggtcaaac aagagtgaga agaaaggacc attttgctca tacaaacggt 60

gatttagcat taccaagtgt tggtgatgta gatccctgga ctgcatgggc atataagcct 120

cgaactattt cgttgttact tattggtgct tgctttctaa tatgggcaag tggagcccta 180

gatccagaga gctgcacatc tggtgatgtt gttacatctg tgaaaagggg tatatgggca 240

atgactgcag tttttcttgg ttattgcttg ctacaggccc cttcaacggt tctaataagg 300

ccacatccag caatttggcg cttagttcat ggattggcca ttgtttatct tgttgccctc 360

acatttctgc tttttcagaa gcgtgatgat gcgcggcaat ttatgaagtt tctccatcct 420

gaccttggaa ttgcagaact acctgaaaga tcatatggtg ctgattgtcg catttatgtg 480

cctgaaaatc ctacaagcaa gtttaagaac gttttggaca ctctttttga tgaatttgtt 540

ctagcacata tctttggatg gtggggcaag gctatattaa tccgtaatca gccacttcta 600

tgggtattgt caattggttt tgagctgatg gagtttacct tccgccacat gctaccaaac 660

ttcaatgaat gctggtggga cagtatcatt cttgatattt tgatatgcaa ttggtttggc 720

atttgggctg gaatgcatac agtcgggtat tttgatggaa aaacatacga gtgggttggt 780

ataagtcgcc aacctaatat tatgagcaag gtcaaacgaa cattggaaca atttacacct 840

gcacagtggg acaaagatga atggcatccc ttgcttggtc catggcgatt tatccaagtt 900

cttagtctct gtattgtctt cctgactgta gagctcaaca cattcttttt gaagttttgt 960

ctatgggttc ctcctcggaa ccctgtaata gtgtacaggt tgatcttgtg gtggctaatt 1020

gccataccta caacacgtga atacaattca tatctccaag accgaaagcc tgtgaaaaag 1080

gtaggcgctt tttgttggct ttcccttgct atttgcatcg tggaacttct catttgcatc 1140

aagtttggac atggtcttta tcccaaacca atgcctgcat ggttggtcat cttctggaca 1200

tctgttggag ttagccttgt aatattcctg attatgtggt catggaaaag tttgggaaga 1260

aagagacgat ga 1272

<210> 190
<211> 564
<212> DNA
<213> Populus trichocarpa

<400> 190
atgcaaccag gcttggatga gatcacaatg acggcgtgga agaatctaaa gcaacggctc 60

tcattcaagg gcctgggtag ttgctgcggg agcacaagct ggagttccag aagtgccacc 120

ccaaccatgc cctttatcga tatagagcaa gaagaagagg aagagcccat catgcaaaac 180

caagctcaaa gaggaggagg agctgcagca gcagcagcag cgccaggtgc tgggatgaat 240

ctggcaatgg cattagctgc tgagcgcaat ttaggggatt caaatgtcaa gacattgatg 300

agtttgatcg aagaaacgga cggtgttgat tggaggaaga agaataacag taatgataaa 360

agtaggaggg acaaggaaca ggaacagaag caggaagaag agaaggattg ggtatgctgc 420

gtttgcatgg agagaaataa aggcgcagct tttattccat gtggacacac cttttgtagg 480

gtttgttcaa gagaaatgtg ggttaatcga gggtgctgtc ctatctgcaa ccgttccatt 540

ctcgacatcc ttgatatctt ctag 564

<210> 191
<211> 927
<212> DNA
<213> Populus trichocarpa

<400> 191
atggtggaga agatcatcaa tatgaacagt caagatcatc tgaggagtac taattataaa 60

gatgatgatg atgaagaaga agttcaactt ccagggtttc gatttcaccc aacagatgaa 120

gaacttgttg ggttctatct tcgtagaatg gttgacaaga agcctctcag aattgaactc 180

atcaaacaag tcgagatcta caaatatgac ccttgggatc taccaaagtc aagctgtgtg 240

ggagataagg aagggtactt cttttgcaaa cgaggaagaa agtataggaa tagcataaga 300

cctaacaggg tgacagggtc tggattttgg aaagcaactg gcattgataa gccagtgttt 360

tcacttggag gagaaggccg tgacagcatt ggactcaaga aaacacttgt ttactaccgc 420

ggaagtgcag gaaaaggtac aaaaaccgat tggatgatgc acgagtttcg ccttcccacg 480

aaagataaca gcacctccac ggccactgtc aaagctaaaa tctctgacca agaagctgag 540

gtatggacac tgtgtagaat tttcaaaaga aatgtgtcat gcagaaaata cacaccagat 600

ttgaagcaat tatcaactac acctcaacaa ccaccaatcg acacaagctc caagttatgc 660

tgtcaagtgg aatctaatta cactcaagaa agctacgtca attttggtgc tccactcatt 720

caacattacg ataataagcc tccagttcat catgtcaagg aaaggaaacc actgcatgtg 780

gatcagttga gctacgtagc tcaaccacca tctatggctt catctttaaa catttccagc 840

ccatatggaa atcagattct tacgcatggg gactgggatg agcttacatc agttgtagat 900

tgtgcttttg atccctttct tgtgtga 927

<210> 192
<211> 762
<212> DNA
<213> Populus trichocarpa

<400>  192
atggagtttg atgggctccc aggttttcga ttccacccaa cagaagaaga actcttagat      60

ttctacctta caaagactgt cttaggtcaa gacacagctg atatcattgg tttcctcaac     120

atatacaatc acgaaccttg ggacttgcca ggattatcga agatagggga gagggagtgg     180

tatttccttg tgcacaggga gagcgttctt gggagaccca ggaggacaac cgagaaaggt     240

tactggaagg caactggttc ggacaggccc atccgatgct tgatggatcc gaaaaggttg     300

ttgggtcata gaaaaactat ggttttctac agaggaagag ctccacgagg gagcaaaacg     360

gattgggtca tgaatgagta cagattgcct agcaactgct atttatcgaa ggagattgtg     420

ctttgcaaag tatatagaaa ggcaacatca ttgaaggtct tggagcaaag ggcagccata     480

gaagaaattg ttagagcacc taacgtcaca tctttatctc ctccaatgca agggaatttc     540

tctttttatg acccccaaaa gagcttcaac aagttcttgt tagaacaaaa taatgttgtt     600

cacaaagtag agggagtgac agtaatagaa gaagaagaag acaacaaaat tggttaccca     660

tcccaaataa tgggagttgg acccatcaaa gagcactact ccgagctcct gtcaccaaag     720

ttagtcctgg attggaccat ggattcactt tggcctaact aa                        762


<210>  193
<211>  1005
<212>  DNA
<213>  Populus trichocarpa

<400>  193
atgttgatgg ctttaaaatc ccggcttaat tcttctgaaa acacaacttc tgtaatggag      60

ctgtatagca aggaacatgt ggatgcaacc cagttgttat acgacctgtc tccttgtttc     120

aaacttggtt tcatggctgc caatcttgcc atcattgacg ctacaagaga gcaagaacaa     180

gaggcgaaca cttccagcaa tggcttccat gttgttgatt ttgatattgg tcatggaggg     240

caatacaaga accttctaca cgcgctttct gggcttcaga attcgaagcc agccattgtt     300

aagatcacag ccgttgctgc tgatagcaat ggcgtagagg aggagaggct gaggctagtt     360

ggtgaaacgc ttacccaact cgctcgtcga gttggtctga atttgtgttt caacgttgtg     420

agctgcaaac tcagtgagtt gactcgggag tccctcgggt gcgagccgga cgaggcattg     480

gctgtcaatt ttgcgttcaa gctgtataga atgccggacg agagcgtgtc ctccaccgag     540

aatcccaggg atgagctttt gaggcgcgtg aaggggctgg cgccgcgcgt ggtgactgta     600

gtggagcaag aaatgaatac caacacggcc ccattcatgg cgcgcgtgaa cgaatcctgt     660

tcatattacg gagcgctatt cgactcgatt gagtcaacgg ttaaagggga taactcggag     720

cgagccaagg tcgaggaggg actggggcgg aggatggtaa actcggttgc ttgtgaaggg     780

agggaccgtg ttgaaagatg cgaggtgttt ggaaaatggc gggcccggat gggcatggca     840

gggttcgagt tgaagcctct gagtcacaac atagccgagt cgatgaaaac gagattgagt     900

ttagcaaaca gagttaaccc aggatttagt gttaaagaag agaatggtgg ggtatgtttc 960

ggttggatgg gaaaaactct cacagtcgca tctgcttggc gttaa 1005

<210> 194
<211> 1674
<212> DNA
<213> Populus trichocarpa

<400> 194
atggacaaca atacctctcg ctctgaggtt tccgactgcg gcgatccaac gaggccggag 60

tccggcggcg gcggtgctag gtacaagctg atgtcgccgg ccaagcttcc gatctcgagg 120

tcggcttgca tcacgatccc tcctggactc agtccgacct cgtttcttga gtctccagtt 180

cttctctcta atgttaaggc agagccttcc cccactactg gtacgtttac taagcctcga 240

acagctcttg gctctcttag ctccactcca tattctgcta caactgtttc atccacagcg 300

tgcggagaaa gaaaatccga ctactttgag tttagaccat atgctaggtc aaacatggtt 360

tctgcagata taaaccatca gaggagcaca caatgtgctc aagtccaaag ccagtgccat 420

tcacaatcat ttgcctcacc acctttggta aaaggtgaga tggaagtatc cacaaatgag 480

ttgagtctgt cagcatccct tcacatggtt acttcagttg ctagtgcacc tgctgaagtt 540

gattcagatg aactaaacca aacggggctc tctagcagtg ggcttcaggc atcacagtct 600

gatcatagag caggaactgc gccttcaatg tcatctgatg atggatataa ctggagaaaa 660

tatggacaga aacatgttaa gggaagtgag ttccctcgaa gctattacaa atgtacacat 720

cctaactgtg aagtgaaaaa gttatttgag cgctcccatg atggacaaat aacagagatt 780

atctacaagg gtacacatga tcatcctaaa ccacaaccca gccgccgata tgcctctggt 840

tctgttttgt ccatgcagga agatagattt gacaagtctt catctctgcc taaccaaggt 900

gacaagtcac ctggtgctta tggacaggtg cctcatgcta tcgagccaaa tggtgccctt 960

gaactgtcta ctggagcaaa tgatgatact ggagaaggtg ccgaagacga tgatgatccc 1020

ttctcaaaaa gaaggaggtt ggatgctgga ggttttgatg ttactccagt ggtcaaacct 1080

atccgggaac cacgtgttgt cgtgcaaact ctgagtgagg ttgatatact ggatgatggg 1140

tacaggtggc gcaaatatgg ccagaaagtg gtgagaggaa atcctaatcc aaggagttac 1200

tacaaatgca caaatgctgg atgcccagtt agaaagcacg ttgagagggc atcgcatgat 1260

ccaaaagcag ttataaccac atatgagggg aaacacaatc atgatgtacc tacagctagg 1320

acaaacagtc atgacatggc gggaccatca gctgtgaatg gaccctcaag gattagacca 1380

gatgaaaacg agacaattag ccttgatctt ggggtcggga tcagttctac aactgaaaac 1440

cagtccagcg atcagcaaca agctttgcat gctgaactta tcaaacacga gaaccaagca 1500

agtggttcta gtttcagagt agttcatgca accccaatta cggcttacta tggtgtttta 1560

aatggtggca tgaatcagta tggatctagg caaattccag gtgaaagccg tagcattgaa 1620

attccacctt atccatatcc gcagaacatg ggaagattat taacgggtcc ataa 1674

<210> 195
<211> 1371
<212> DNA
<213> Populus trichocarpa

<400> 195
atggaaacaa agtgtccagt agatgcaaag tgcgcagctg aaatatctgc catgctcacc 60

cctccttctc ctcttcaact ccaggagtat ttcgaggaga ttatatctga aaggaaatgc 120

catggcattg aagtcaaaca agatggaaac ttgagcaaag gggtttatgc aactatggaa 180

cttaaagaag gagaacttat actgaaggac aagatacttg tgggtttaca gcatgttcca 240

aataagcttg actgtctggt gtgtggctat tgtttccaat ttattgagtc agttgaatat 300

cagattggga ggaaacttta tttgcaaagt ctaggcgtgc ccagctgcaa tggatgtgat 360

gagggggaat gtagttccag cagctcatac aataaagctt gtttgcctga aggagttata 420

gaagcattaa tgaatggtga attggtattg ccttactctg acaaatttcc cttgccttca 480

actgttcctt gtcctggggg gtgtcaagaa gcttattatt gcagcaaatc ttgtgcgcag 540

actgattggg aatcttcaca ttctttactt tgcaccgggg agaggtcaga atcattatct 600

atagaggcac tttcaaaatt tatacagcat gctactgaaa caaatgatat tttcctccta 660

gcagccaaga caatttcttt caccatttta aggtatagga agctgaaagc agctaatgca 720

gatcgttctg aactttcctt acttttggag gcatggaagc caatatcaat gggatacaag 780

agaaggtggt gggagtgcat ttcatttcca gatgatgttg atcgttctga tgacactgca 840

tttaggatgc aaatacagca gcttgcattc aagtcactgc agcttctcaa ggctgccata 900

tttgatgagg aatgcgagcc attattctcc cttgaaatct atgggaatat tattggcatg 960

tttgagctga ataatcttga tctggtcgta gcatctccag ttgaggatta ttttctgtac 1020

attgatgatc ttccagatcc tgaaaaggaa aaggccgaaa aaattgcacg gcaacttcta 1080

gatgctcttg gagatgacta ttcaatttgt tgccaaggta ctgcgttcta tcctttacag 1140

agctgcatga atcactcctg ctgcccaaat gcacacgcct tcaaaagaga tgaggacaga 1200

gatggccagg cagcaatcat caccctgaaa ccaattcgaa agggagaaga ggttaccgtt 1260

tcatacatag acgaggacct tccatttgaa gacagacagg cattgctagc agattatgga 1320

ttcaaatgca ggtgcaacgc ttgcttagag caagacccca acaaaaagta g 1371